# EUROPÄISCHE PATENTSCHRIFT

(11) **EP 2 035 375 B1**
(45) Veröffentlichungstag und Bekanntmachung des Hinweises auf die Patenterteilung: **19.01.2011**
(21) Anmeldenummer: 07725929.9
(22) Anmeldetag: 08.06.2007
(51) Int. Cl.: C07D 207/40, C07D 401/12, C07D 403/12, C07D 405/12, C07D 405/14, C07D 417/12

(54) **1,3-DISUBSTITUIERTE 4-METHYL-1 H-PYRROL-2-CARBONSÄUREAMIDE UND IHRE VERWENDUNG ZUR HERSTELLUNG VON ARZNEIMITTELN**
1,3-DISUBSTITUTED 4-METHYL-1H-PYRROLE-2-CARBOXAMIDES AND THEIR USE FOR THE MANUFACTURE OF MEDICAMENTS
AMIDE DE L'ACIDE 4-MÉTHYL-1H-PYRROL-2-CARBOXYLIQUE 1,3-DISUBSTITUÉ ET SON UTILISATION POUR LA FABRICATION DE MÉDICAMENTS

(30) Priorität: 09.06.2006 DE 102006027229
(43) Veröffentlichungstag der Anmeldung: 18.03.2009
(73) Patentinhaber: Grünenthal GmbH, 52078 Aachen (DE)
(72) Erfinder: OBERBÖRSCH, Stefan, 52074 Aachen (DE); Dr. SUNDERMANN, Bernd, 61381 Friedrichsdorf (DE); SUNDERMANN, Corinna, 61381 Friedrichsdorf (DE); Dr. BIJSTERVELD, Edward, NL-6546 BB Nijmegen (NL); HENNIES, Hagen-Heinrich, 52152 Simmerath (DE)
(74) Vertreter: Brosch, Oliver
(86) Internationale Anmeldenummer: PCT/EP2007/005098
(87) Internationale Veröffentlichungsnummer: WO 2007/141039

(56) Entgegenhaltungen:
- EP-A2- 0 957 099
- WO-A-90/02733
- WO-A-2004/018455
- WO-A-2004/108685
- WO-A-2005/113497
- ALAZARD, J.P.; BOYE, O.; GILLET, B.; GUENARD, D.; BELOEIL, J.C.; THAL, C.: "Composés interagissant avel la tubuline. Partie I. Synthèse de phenylpyrroles ortho-ortho' substitués en rotation libre ou empêchée" BULL. SOC. CHIM. FR., Bd. 130, Nr. 6, 1993, Seiten 779-787, XP009090907

## Beschreibung

Die vorliegende Erfindung betrifft 1,3-disubstituierte 4-Methyl-1H-pyrrol-2-carbonsäureamide, Verfahren zu ihrer Herstellung, Arzneimittel enthaltend diese Verbindungen sowie deren Verwendung zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nozizeption in letzter zeit erschienen sind.

Klassische Opioide, wie beispielsweise Morphin, sind bei der Therapie starker bis sehr starker Schmerzen wirksam, führen jedoch oftmals zu unerwünschten Begleiterscheinungen wie beispielsweise Atemdepression, Erbrechen, Sedierung, Obstipation oder Toleranzentwicklung. Des Weiteren sind sie bei neuropathischen Schmerzen, unter denen insbesondere Tumorpatienten leiden, häufig nicht ausreichend wirksam.

Die WO2005/113497 und die EP-A-957099 beschreiben substituierte 5-Aminomethyl-1H-pyrrol-2-carbonsäureamide und deren Verwendung als Arzneimittel zur Behandlung von Schmerzen. Die WO2004/018455 offenbart Pyrrol-3-carboxamid-derivate und deren Verwendung in der Behandlung von neurodegenerativen Krankheiten.

Eine Aufgabe der vorliegenden Erfindung bestand daher darin, neue Verbindungen zur Verfügung zu stellen, die sich insbesondere als pharmazeutische Wirkstoffe in Arzneimitteln eignen, bevorzugt in Arzneimitteln zur Prophylaxe und/oder Behandlung von Schmerzen, insbesondere akuten Schmerzen, chronischen Schmerzen und neuropathischen Schmerzen.

Es wurde nun überraschenderweise gefunden, dass sich die disubstituierten 4-Methyl-1H-pyrrol-2-carbonsäureamide der nachstehend angegebenen allgemeinen Formel I zur Noradrenalin-Rezeptor-Regulation, insbesondere zur Hemmung der Noradrenalin-Wiederaufnahme (Noradrenalin-Uptake) und/oder zur 5-HT-Rezeptor-Regulation, insbesondere zur Hemmung der 5-Hydroxy-Tryptophan-Wiederaufnahme (5-HT-Uptake) und/oder zur Opioid-Rezeptor-Regulation und/oder zur Batrachotoxin-(BTX)-Rezeptor-Regulation eignen und daher insbesondere als pharmazeutische Wirkstoffe in Arzneimitteln zur Prophylaxe und/oder Behandlung von mit diesen Rezeptoren bzw. Prozessen in Verbindung stehenden Störungen oder Erkrankungen eingesetzt werden können.

Ein Gegenstand der vorliegenden Erfindung sind daher 1,3-disubstituierte 4-Methyl-1H-pyrrol-2-carbonsäureamide der allgemeinen Formel I, 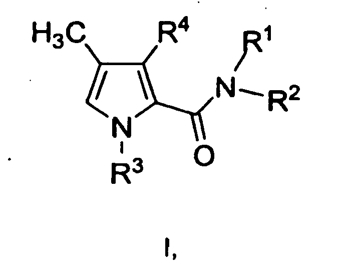 worin
R¹ für unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl; unsubstituiertes oder wenigstens einfach substituiertes -(Alkylen)-Cycloalkyl, -(Alkenylen)-Cycloalkyl, - (Alkinylen)-Cycloalkyl, -(Alkylen)-Cycloalkenyl, -(Alkenylen)-Cycloalkenyl oder - (Alkinylen)-Cycloalkenyl; unsubstituiertes oder wenigstens einfach substituiertes - (Heteroalkylen)-Cycloalkyl, -(Heteroalkenylen)-Cycloalkyl, -(Heteroalkylen)-Cycloalkenyl oder -(Heteroalkenylen)-Cycloalkenyl; unsubstituiertes oder wenigstens einfach substituiertes -(Alkylen)-Heterocycloalkyl, -(Alkenylen)-Heterocycloalkyl, - (Alkinylen)-Heteröcycloalkyl, -(Alkylen)-Heterocycloalkenyl, -(Alkenylen)-Heterocycloalkenyl oder -(Alkinylen)-Heterocycloalkenyl; unsubstituiertes oder wenigstens einfach substituiertes -(Heteroalkylen)-Heterocycloalkyl, - (Heteroalkenylen)-Heterocycloalkyl, -(Heteroalkylen)-Heterocycloalkenyl oder - (Heteroalkenylen)-Heterocycloalkenyl;
Phenyl, das unsubstituiert oder mit 1, 2, 3, 4 oder 5 Substituenten unabhängig voneinander ausgewählt aus der Gruppe bestehend aus F, Cl, Br, I, -CN, -CH₂-CN, - NO₂, -OH, -SH, -NH₂, -C₁₋₅-Alkyl, -(CH₂)-O-C₁₋₅-Alkyl, -C₂₋₅-Alkenyl, -C₂₋₅-Alkinyl, - C≡C-Si(CH₃)₃, -C≡C-Si(C₂H₅)₃, -S-C₁₋₅-Alkyl, -S-Phenyl, -S-CH₂-Phenyl, -O-C₁₋₅-Alkyl, -O-Phenyl, -O-CH₂-Phenyl, -CF₃, -CHF₂, -CH₂F, -O-CF₃, -O-CHF₂, -O-CH₂F, - C(=O)-CF₃, -S-CF₃, -S-CHF₂, -S-CH₂F, -S(=O)₂-Phenyl, -S(=O)₂-C₁₋₅-Alkyl, -S(=O)-C₁₋₅-Alkyl, -NH-C₁₋₅-Alkyl, -N(C₁₋₅Alkyl)₂, -C(=O)-H, -C(=O)-C₁₋₅-Alkyl, -CH₂-O-C(=O)-Phenyl, -O-C(=O)-Phenyl, -NH-S(=O)₂-C₁₋₅-Alkyl, -NH-C(=O)-C₁₋₅-Alkyl, -C(=O)-NH₂, -C(=O)-NH-C₁₋₅-Alkyl, -C(=O)-N(C₁₋₅-Alkyl)₂, -S(=O)₂-NH₂, -S(=O)₂-NH-C₁₋₅-Alkyl, - S(=O)₂-N(C₁₋₅-Alkyl)₂, -S(=O)₂-NH-Phenyl, [1,2,3]-Thiadiazolyl, Cyclopropyl, Cyclobutyl, Cyclopentyl, Cyclohexyl, Piperazinyl, Pyrrolidinyl, Piperidinyl, Pyrazolyl, Phenyl, Furyl (Furanyl), Thiazolyl, Thiadiazolyl, Thiophenyl (Thienyl), Benzyl und Phenethyl substituiert ist, wobei die zyklischen Substituenten bzw. die zyklischen Reste dieser Substituenten selbst jeweils unsubstituiert oder mit ggf. 1, 2, 3, 4 oder 5, Substituenten unabhängig voneinander ausgewählt aus der Gruppe bestehend aus F, Cl, Br, I, -CN, -NO₂, -OH, -SH, -NH₂, -C(=O)-OH, -C₁₋₅-Alkyl, -(CH₂)-O-C₁₋₅-Alkyl, -C₂₋₅-Alkenyl, -C₂₋₅-Alkinyl, -C≡C-Si(CH₃)₃, -C≡C-Si(C₂H₅)₃, -S-C₁₋₅-Alkyl, -S-Phenyl, -S-CH₂-Phenyl, -O-C₁₋₅-Alkyl, -O-Phenyl, -O-CH₂-Phenyl, -CF₃, -CHF₂, - CH₂F, -O-CF₃, -O-CHF₂, -O-CH₂F, -C(=O)-CF₃, -S-CF₃, -S-CHF₂ und -S-CH₂F substituiert sein können; einen unsubstituierten oder wenigstens einfach substituierten Rest ausgewählt aus der Gruppe bestehend aus Naphthyl, Indolizinyl, Benzirmdazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Indazolyl, Chinoxalinyl, Chinazolinyl, Chinolinyl, Naphthridinyl und Isochinolinyl; unsubstituiertes oder wenigstens einfach substituiertes -(Alkylen)-Aryl, -(Alkenylen)-Aryl, -(Alkinylen)-Aryl, -(Heteroalkylen)-Aryl oder -(Heteroalkenylen)-Aryl; unsubstituiertes oder wenigstens einfach substituiertes -(Alkylen)-Heteroaryl,-(Alkenylen)-Heteroaryl, -(Alkinylen)-Heteroaryl, -(Heteroalkylen)-Heteroaryl oder-(Heteroalkenylen)-Heteroaryl oder -NH-C(=O)-R⁵ steht;
R² für H; unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Aryl; oder unsubstituiertes oder wenigstens einfach substituiertes - (Alkylen)-Aryl, -(Alkenylen)-Aryl, -(Alkinylen)-Aryl- steht;
oder R¹ und R² zusammen mit dem sie verbindenden Stickstoffatom für Heterocycloalkyl oder Heterocycloalkenyl stehen, das unsubstituiert oder mit wenigstens einem Rest R⁶ substituiert ist;
R³ für unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes -(Alkylen)-Aryl, -(Alkenylen)-Aryl, -(Alkinylen)-Aryl, -(Heteroalkylen)-Aryl oder -(Heteroalkenylenn)-Aryl; oder unsubstituiertes oder wenigstens einfach substituiertes -(Alkylen)-Heteroaryl, -(Alkenylen)-Heteroaryl, -(Alkinylen)-Heteroaryl, - (Heteroalkylen)-Heteroaryl oder -(Heteroalkenylen)-Heteroaryl steht;
R⁴ für Phenyl, das jeweils unsubstituiert oder mit 1, 2, 3, 4 oder 5 Substituenten unabhängig voneinander ausgewählt aus der Gruppe bestehend aus F, Cl, Br, I, - CN, -CH₂-CN, -OH, -SH, -NH₂, -C(=O)-OH, -C₁₋₅-Alkyl, -(CH₂)-O-C₁₋₅-Alkyl, -C₂₋₅-Alkenyl, -C₂₋₅-Alkinyl, -C≡C-Si(CH₃)₃, -C≡C-Si(C₂H₅)₃, -S-C₁₋₅-Alkyl, -S-Phenyl, -S-CH₂-Phenyl, -O-C₁₋₅-Alkyl, -O-Phenyl, -O-CH₂-Phenyl, -CF₃, -CHF₂, -CH₂F, -O-CF₃, - O-CHF₂, -O-CH₂F, -C(=O)-CF₃, -S-CF₃, -S-CHF₂, -S-CH₂F, -S(=O)₂-Phenyl, -S(=O)₂-C₁₋₅-Alkyl, -S(=O)-C₁₋₅-Alkyl, -NH-C₁₋₅-Alkyl, -N(C₁₋₅Alkyl)₂, -C(=O)-O-C₁₋₅-Alkyl, - C(=O)-H, -C(=O)-C₁₋₅-Alkyl, -CH₂-O-C(=O)-Phenyl, -O-C(=O)-Phenyl, -NH-S(=O)₂-C₁₋₅-Alkyl, -NH-C(=O)C₁₋₅-Alkyl, -C(=O)-NH₂, -C(=O)NH-C₁₋₅-Alkyl, -C(=O)-N(C₁₋₅-Alkyl)₂, -S(=O)₂-NH₂, -S(=O)₂-NH-C₁₋₅-Alkyl, -S(=O)₂-N(C₁₋₅-Alkyl)₂, -S(=O)₂-NH-Phenyl, [1,2,3]-Thiadiazolyl, Cyclopropyl, Cyclobutyl, Cyclopentyl, Cyclohexyl, Pyrazolyl, Phenyl, Furyl (Furanyl), Thiazolyl, Thiadiazolyl, Thiophenyl (Thienyl), Benzyl und Phenethyl substituiert ist, wobei die zyklischen Substituenten bzw. die zyklischen Reste dieser Substituenten selbst jeweils unsubstituiert oder mit ggf. 1, 2, 3, 4 oder 5, Substituenten unabhängig voneinander ausgewählt aus der Gruppe bestehend aus F, Cl, Br, I, -CN, -NO₂, -OH, -SH, -NH₂, -C(=O)-OH, -C₁₋₅-Alkyl, - (CH₂)-O-C₁₋₅-Alkyl, -C₂₋₅-Alkenyl, -C₂₋₅-Alkinyl, -C≡C-Si(CH₃)₃, -C≡C-Si(C₂H₅)₃, -S-C₁₋₅-Alkyl, -S-Phenyl, -S-CH₂-Phenyl, -O-C₁₋₅-Alkyl, -O-Phenyl, -O-CH₂-Phenyl, -CF₃, - CHF₂, -CH₂F, -O-CF₃, -O-CHF₂, -O-CH₂F, -C(=O)-CF₃, -S-CF₃, -S-CHF₂ und -S-CH₂F substituiert sein können;
oder für einen Rest ausgewählt aus der Gruppe bestehend aus Naphthyl, Benzimidazolyl, Triazinyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiadiazolyl, Oxadiazolyl, Pyridazinyl, Chinoxalinyl, Chinazolinyl, Chinolinyl, Naphthridinyl und Isochinolinyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, -CH₂-CN, - NO₂, -OH, -SH, -NH₂, -C(=O)-OH, -C₁₋₅-Alkyl, -(CH₂)-O-C₁₋₅-Alkyl, -C₂₋₅-Alkenyl, -C₂₋₅-Alkinyl, -C≡C-Si(CH₃)₃, -C≡C-Si(C₂H₅)₃, -S-C₁₋₅-Alkyl, -S-Phenyl, -S-CH₂-Phenyl, -O-C₁₋₅-Alkyl, -O-Phenyl, -O-CH₂-Phenyl, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-Phenyl, -S(=O)₂-C₁₋₅-Alkyl; - S(=O)C₁₋₅-Alkyl, -NH-C₁₋₅-Alkyl, -N(C₁₋₅Alkyl)₂, -C(=O)-O-C₁₋₅-Alkyl, -C(=O)-H, - C(=O)-C₁₋₅-Alkyl, -CH₂-O-C(=O)-Phenyl, -O-C(=O)-Phenyl, -NH-S(=O)₂-C₁₋₅-Alkyl, - NH-C(=O)-C₁₋₅-Alkyl, -C(=O)-NH₂, -C(=O)-NH-C₁₋₅-Alkyl, -C(=O)-N(C₁₋₅-Alkyl)₂, - S(=O)₂-NH₂, -S(=O)₂-NH-C₁₋₅-Alkyl, -S(=O)₂-N(C₁₋₅-Alkyl)₂, -S(=O)₂-NH-Phenyl, [1,2,3]-Thiadiazolyl, Cyclopropyl, Cyclobutyl, Cyclopentyl, Cyclohexyl, Pyrazolyl, Phenyl, Furyl (Furanyl), Thiazolyl, Thiadiazolyl, Thiophenyl (Thienyl), Benzyl und Phenethyl substituiert ist, wobei die zyklischen Substituenten bzw. die zyklischen Reste dieser Substituenten selbst jeweils unsubstituiert oder mit ggf. 1, 2, 3, 4 oder 5, Substituenten unabhängig voneinander ausgewählt aus der Gruppe bestehend aus F, Cl, Br, I, -CN, -NO₂, -OH, -SH, -NH₂, -C(=O)-OH, -C₁₋₅-Alkyl, -(CH₂)-O-C₁₋₅-Alkyl, -C₂₋₅-Alkenyl, -C₂₋₅-Alkinyl, -C≡C-Si(CH₃)₃, -C≡C-Si(C₂H₅)₃, -S-C₁₋₅-Alkyl, -S-Phenyl, -S-CH₂-Phenyl, -O-C₁₋₅-Alkyl, -O-Phenyl, -O-CH₂-Phenyl, -CF₃, -CHF₂, - CH₂F, -O-CF₃, -O-CHF₂, -O-CH₂F, -C(=O)-CF₃, -S-CF₃, -S-CHF₂ und -S-CH₂F substituiert sein können;
R⁵ für unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, - (Alkenylen)-Aryl, -(Alkinylen)-Aryl, -(Heteroalkylen)-Aryl oder -(Heteroalkenylen)-Aryl; oder unsubstituiertes oder wenigstens einfach substituiertes -(Alkylen)-Heteroaryl,-(Alkenylen)-Heteroaryl, -(Alkinylen)-Heteroaryl, -(Heteroalkylen)-Heteroaryl oder-(Heteroalkenylen)-Heteroaryl steht;
R⁶ für -OH; F; Cl; Br, I; -SH; -NO₂; -NH₂; -NH-C(=O)-O-R⁷; -C(=O)-O-R⁸; -C(=O)-R⁹; für unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl; unsubstituiertes oder wenigstens einfach substituiertes -(Alkylen)-Cycloalkyl, -(Alkenylen)-Cycloalkyl, - (Alkinylen)-Cycloalkyl, -(Alkylen)-Cycloalkenyl, -(Alkenylen)-Cycloalkenyl oder - (Alkinylen)-Cycloalkenyl; unsubstituiertes oder wenigstens einfach substituiertes - (Heteroalkylen)-Cycloalkyl, -(Heteroalkenylen)-Cycloalkyl, -(Heteroalkylen)-Cycloalkenyl oder -(Heteroalkenylen)-Cycloalkenyl; unsubstituiertes oder wenigstens einfach substituiertes -(Alkylen)-Heterocycloalkyl, -(Alkenylen)-Heterocycloalkyl, - (Alkinylen)-Heterocycloalkyl, -(Alkylen)-Heterocycloalkenyl, -(Alkenylen)-Heterocycloalkenyl oder -(Alkinylen)-Heterocycloalkenyl; unsubstituiertes oder wenigstens einfach substituiertes -(Heteroalkylen)-Heterocycloalkyl, - (Heteroalkenylen)-Heterocycloalkyl, -(Heteroalkylen)-Heterocycloalkenyl oder - (Heteroalkenylen)-Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, - (Alkenylen)-Aryl, -(Alkinylen)-Aryl, -(Heteroalkylenn)-Aryl oder -(Heteroalkenylen)-Aryl; oder unsubstituiertes oder wenigstens einfach substituiertes -(Alkylen)-Heteroaryl, - (Alkenylen)-Heteroaryl, -(Alkinylen)-Heteroaryl, -(Heteroalkylen)-Heteroaryl oder - (Heteroalkenylen)-Heteroaryl steht;
und R⁷, R⁸ und R⁹, unabhängig voneinander, jeweils für unsubstituiertes oder wenigstens einfach substituiertes Alkyl, Alkenyl oder Alkinyl; unsubstituiertes oder wenigstens einfach substituiertes Heteroalkyl, Heteroalkenyl oder Heteroalkinyl; unsubstituiertes oder wenigstens einfach substituiertes Cycloalkyl oder Cycloalkenyl; unsubstituiertes oder wenigstens einfach substituiertes Heterocycloalkyl oder Heterocycloalkenyl; unsubstituiertes oder wenigstens einfach substituiertes Aryl; unsubstituiertes oder wenigstens einfach substituiertes Heteroaryl; unsubstituiertes oder wenigstens einfach substituiertes -(Alkylen)-Aryl, -(Alkenylen)-Aryl, -(Alkinylen)-Aryl, -(Heteroalkylen)-Aryl oder -(Heteroalkenylen)-Aryl; oder unsubstituiertes oder wenigstens einfach substituiertes -(Alkylen)-Heteroaryl, -(Alkenylen)-Heteroaryl, - (Alkinylen)-Heteroaryl, -(Heteroalkylen)-Heteroaryl oder -(Heteroalkenylen)-Heteroaryl stehen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Vorzugsweise kann R⁴ nicht für einen 1,4-Dihydroxy-naphthyl-Rest stehen.

Der Begriff "Alkyl" umfaßt im Sinne der vorliegenden Erfindung azyklische gesättigte Kohlenwasserstoffreste, die verzweigt oder geradkettig sowie unsubstituiert oder wenigstens einfach substituiert sein können mit wie im Fall von C₁₋₁₂-Alkyl 1 bis 12 (d. h. 1, 2, 3, 4, 5, 6, 7, 8, 9, 10, 11 oder 12) C-Atomen bzw. mit wie im Fall von C₁₋₆-Alkyl 1 bis 6 (d. h. 1, 2, 3, 4, 5 oder 6) C-Atomen. Sofern einer oder mehrere der Substituenten für einen Alkyl-Rest stehen oder einen Alkyl-Rest aufweisen, der einfach oder mehrfach substituiert ist, kann dieser bevorzugt mit ggf. 1, 2, 3, 4 oder 5, besonders bevorzugt mit 1, 2 oder 3, Substituenten unabhängig voneinander ausgewählt aus der Gruppe bestehend aus F, Cl, Br, I, -NO₂, -CN, -OH, -SH, -NH₂, - N(C₁₋₅-Alkyl)₂, -N(C₁₋₅-Alkyl)(Phenyl), -N(C₁₋₅-Alkyl)(CH₂-Phenyl), -N(C₁₋₅-Alkyl)(CH₂-CH₂-Phenyl), -NH-C(=O)-O-C₁₋₅-Alkyl, -C(=O)-H, -C(=O)-C₁₋₅-Alkyl, -C(=O)-Phenyl, - C(=S)-C₁₋₅-Alkyl, -C(=S)-Phenyl, -C(=O)-OH, -C(=O)-O-C₁₋₅-Alkyl, -C(=O)-O-Pheny), - C(=O)-O-CH₂-Phenyl, -C(=O)-NH₂, -C(=O)-NH-C₁₋₅-Alkyl, -C(=O)-N(C₁₋₅-Alkyl)₂, - C(=O)-NH-Phenyl, -C(=O)-N(C₁₋₅-Alkyl)-Phenyl, -C(=O)-NH-Naphthyl, -C(=O)-Pyrrolidinyl, -C(=O)-Piperidinyl, -S(=O)-C₁₋₅-Alkyl, -S(=O)-Phenyl, -S(=O)₂-C₁₋₅-Alkyl, -S(=O)₂-Phenyl, -S(=O)₂-NH₂ und -SO₃H substituiert sein, wobei die vorstehend genannten C₁₋₅-Alkyl-Reste jeweils linear oder verzweigt sein können und die vorstehend genannten Phenyl- oder Naphthyl-Reste jeweils unsubstituiert oder mit 1, 2, 3, 4 oder 5 Substituenten, bevorzugt mit 1, 2 oder 3 Substituenten, unabhängig voneinander ausgewählt aus der Gruppe bestehend aus F, Cl, Br, I, -CN, -CF₃, -OH, -NH₂, -O-CF₃, -SH, -O-CH₃, -O-C₂H₅, -O-C₃H₇, Methyl, Ethyl, n-Propyl, Isopropyl, n-Butyl, 2-Butyl, Isobutyl und tert-Butyl substituiert sein können.

Besonders bevorzugte Substituenten für Alkyl können unabhängig voneinander ausgewählt werden aus der Gruppe bestehend aus F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O-C₂H₅, -C(=O)-O-C(CH₃)₃, -NH-C(=O)-O-C(CH₃)₃, -N(CH₃)-Phenyl, -N-(C₂H₅)-(*p-*Toluyl), -C(=O)-O-CH₂-Phenyl, -C(=O)-NH-Naphthyl, -C(=O)-Pyrrolidinyl, -C(=O)-N(CH₃₎-Phenyl und -C(=O)-NH-CH(CH₃)₂.

Als geeignete C₁₋₁₂-Alkyl-Reste, die jeweils unsubstituiert oder einfach oder mehrfach substituiert sein können, seien beispielsweise Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, 2-Hexyl, 3-Hexyl, n-Heptyl, n-Octyl, -C(H)(C₂H₅)₂, -C(H)(n-C₃H₇)₂, (3,3)-Dimethylbutyl, 4-Methyl-2-pentyl und -CH₂-CH₂-C(H)(CH₃)-(CH₂)₃-CH₃ genannt. Als geeignete C₁₋₆-Alkyl-Reste seien beispielsweise Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, 2-Hexyl und 3-Hexyl genannt.

Unter mehrfach substituierten Alkyl-Resten sind solche Alkyl-Reste zu verstehen, die entweder an verschiedenen oder an gleichen C-Atomen mehrfach, bevorzugt zweioder dreifach, substituiert sind, beispielsweise dreifach am gleichen C-Atom wie im Fall von -CF₃ oder an verschiedenen Stellen wie im Fall von -(CHCl)-(CH₂F). Die Mehrfachsubstitution kann mit dem gleichen oder mit verschiedenen Substituenten erfolgen. Als geeignete substituierte Alkyl-Reste seien beispielsweise -CF₃, -CF₂H, - CFH₂, -CH₂Cl, -(CH₂)-OH, -(CH₂)-NH₂, -(CH₂)-CN, -(CH₂)-(CF₃), -(CH₂)-(CHF₂), - (CH₂)-(CH₂F), -(CH₂)-(CH₂Cl), -(CH₂)-(CH₂)-OH, -(CH₂)-(CH₂)-NH₂, -(CH₂)-(CH₂)-CN, -(CF₂)-(CF₃), -(CH₂)-(CH₂)-(CF₃), -(CH₂)-(CH₂)-(CH₂)-OH, -(CH₂)-N(CH₃)₂, -(CH₂)-(CH₂)-(CH₂)-Cl, -(CH₂)-(CH₂)-(CH₂)-(CH₂)-Cl, -(CH₂)-C(=O)-OH, -(CH₂)-(CH₂)-C(=O)-OH, -(CH₂)-C(=O)-O-CH₃ und -(CH₂)-(CH₂)-NH-C(=O)-O-C(CH₃)₃ genannt.

Der Begriff "Alkenyl" umfaßt im Sinne der vorliegenden Erfindung azyklische ungesättigte Kohlenwasserstoffreste, die verzweigt oder geradkettig sowie unsubstituiert oder wenigstens einfach substituiert sein können und wenigstens eine Doppelbindung, bevorzugt 1, 2 oder 3 Doppelbindungen, aufweisen, mit wie im Fall von C₂₋₁₂-Alkenyl 2 bis 12 (d. h. 2, 3, 4, 5, 6, 7, 8, 9, 10, 11 oder 12) C-Atomen bzw. mit wie im Fall von C₂₋₆-Alkeny) 2 bis 6 (d. h. 2, 3, 4, 5 oder 6) C-Atomen. Sofern einer oder mehrere der Substituenten für einen Alkenyl-Rest stehen oder einen Alkenyl-Rest aufweisen, der einfach oder mehrfach substituiert ist, kann dieser bevorzugt mit ggf. 1, 2, 3, 4 oder 5, besonders bevorzugt mit 1, 2 oder 3, Substituenten unabhängig voneinander ausgewählt aus der Gruppe bestehend aus F, Cl, Br, I, -NO₂, -CN, -OH, -SH, -NH₂, -N(C₁₋₅-Alkyl)₂, -N(C₁₋₅-Alkyl)(Phenyl), -N(C₁₋₅-Alkyl)(CH₂-Phenyl), -N(C₁₋₅-Alkyl)(CH₂-CH₂-Phenyl), -NH-C(=O)-O-C₁₋₅-Alkyl, - C(=O)-H, -C(=O)-C₁₋₅-Alkyl, -C(=O)-Phenyl, -C(=S)-C₁₋₅-Alkyl, -C(=S)-Phenyl, - C(=O)-OH, -C(=O)-O-C₁₋₅-Alkyl, -C(=O)-O-Phenyl, -C(=O)-O-CH₂-Phenyl, -C(=O)-NH₂, -C(=O)-NH-C₁₋₅-Alkyl, -C(=O)-N(C₁₋₅-Alkyl)₂, -C(=O)-NH-Phenyl, -C(=O)-N(C₁₋₅-Alkyl)-Phenyl, -C(=O)-NH-Naphthyl, -C(=O)-Pyrrolidinyl, -C(=O)-Piperidinyl, -S(=O)-C₁₋₅-Alkyl, -S(=O)-Phenyl, -S(=O)₂-C₁₋₅-Alkyl, -S(=O)₂-Phenyl, -S(=O)₂-NH₂ und - SO₃H substituiert sein, wobei die vorstehend genannten C₁₋₅-Alkyl-Reste jeweils linear oder verzweigt sein können und die vorstehend genannten Phenyl- oder Naphthyl-Reste jeweils unsubstituiert oder mit 1, 2, 3, 4 oder 5 Substituenten, bevorzugt mit 1, 2 oder 3 Substituenten, unabhängig voneinander ausgewählt aus der Gruppe bestehend aus F, Cl, Br, I, -CN, -CF₃, -OH, -NH₂, -O-CF₃, -SH, -O-CH₃, - O-C₂H₅, -O-C₃H₇, Methyl, Ethyl, n-Propyl, Isopropyl, n-Butyl, 2-Butyl, Isobutyl und tert-Butyl substituiert sein können.

Besonders bevorzugte Substituenten für Alkenyl können unabhängig voneinander ausgewählt werden aus der Gruppe bestehend aus F, Cl, Br, I, -NO₂, -CN, -OH, -SH, -NH₂, -N(CH₃)₂, -N(C₂H₅)₂ und -N(CH₃)(C₂H₅).

Als geeignete C₂₋₁₂-Alkenyl-Reste seien beispielsweise Ethenyl, 1-Propenyl, 2-Propenyl, 1-Butenyl, 2-Butenyl, 3-Butenyl, 1-Pentenyl, 2-Pentenyl, 3-Pentenyl, 4-Pentenyl, Hexenyl, -CH=C(CH₃)₂, -CH=CH-CH=CH-CH₃ und -CH₂-CH₂-CH=CH₂ genannt.

Unter mehrfach substituierten Alkenyl-Resten sind solche Alkenyl-Reste zu verstehen, die entweder an verschiedenen oder an gleichen C-Atomen mehrfach, bevorzugt zweifach, substituiert sind, beispielsweise zweifach am gleichen C-Atom wie im Fall von -CH=CCl₂ oder an verschiedenen Stellen wie im Fall von -CCl=CH-(CH₂)-NH₂. Die Mehrfachsubstitution kann mit dem gleichen oder mit verschiedenen Substituenten erfolgen. Als geeignete substituierte Alkenyl-Reste seien beispielsweise -CH=CH-(CH₂)-OH, -CH=CH-(CH₂)-NH₂ und -CH=CH-CN genannt.

Der Begriff "Alkinyl" umfaßt im Sinne der vorliegenden Erfindung azyklische ungesättigte Kohlenwasserstoffreste, die verzweigt oder geradkettig sowie unsubstituiert oder wenigstens einfach substituiert sein können und wenigstens eine Dreifachbindung, bevorzugt 1 oder 2 Dreifachbindungen, aufweisen, mit wie im Fall von C₂₋₁₂-Alkinyl 2 bis 12 (d. h. 2, 3, 4, 5, 6, 7, 8, 9, 10, 11 oder 12) C-Atomen bzw. mit wie im Fall von C₂₋₆-Alkinyl 2 bis 6 (d. h. 2, 3, 4, 5 oder 6) C-Atomen. Sofern einer oder mehrere der Substituenten für einen Alkinyl-Rest stehen oder einen Alkinyl-Rest aufweisen, der einfach oder mehrfach substituiert ist, kann dieser bevorzugt mit ggf. 1, 2, 3, 4 oder 5, besonders bevorzugt mit ggf. 1 oder 2, Substituenten unabhängig voneinander ausgewählt aus der Gruppe bestehend aus F, Cl, Br, I, -NO₂, -CN, -OH, -SH, -NH₂, -N(C₁₋₅-Alkyl)₂, -N(C₁₋₅-Alkyl)(Phenyl), -N(C₁₋₅-Alkyl)(CH₂-Phenyl), -N(C₁₋₅-Alkyl)(CH₂-CH₂-Phenyl), -NH-C(=O)-O-C₁₋₅-Alkyl, -C(=O)-H. -C(=O)-C₁₋₅-Alkyl, - C(=O)-Phenyl, -C(=S)-C₁₋₅-Alkyl, -C(=S)-Phenyl, -C(=O)-OH, -C(=O)-O-C₁₋₅-Alkyl, C(=O)-O-Phenyl, -C(=O)-O-CH₂-Phenyl, -C(=O)-NH₂, -C(=O)-NH-C₁₋₅-Alkyl, -C(=O)-N(C₁₋₅-Alkyl)₂, -C(=O)-NH-Phenyl, -C(=O)-N(C₁₋₅-Alkyl)-Phenyl, -C(=O)-NH-Naphthyl, -C(=O)-Pyrrolidinyl, -C(=O)-Piperidinyl, -S(=O)-C₁₋₅-Alkyl, -S(=O)-Phenyl, -S(=O)₂-C₁. ₅-Alkyl, -S(=O)₂-Phenyl, -S(=O)₂-NH₂ und -SO₃H substituiert sein, wobei die vorstehend genannten C₁₋₅-Alkyl-Reste jeweils linear oder verzweigt sein können und die vorstehend genannten Phenyl- oder Naphthyl-Reste jeweils unsubstituiert oder mit 1, 2, 3, 4 oder 5 Substituenten, bevorzugt mit 1, 2 oder 3 Substituenten, unabhängig voneinander ausgewählt aus der Gruppe bestehend aus F, Cl, Br, I,-CN, -CF₃, -OH, -NH₂, -O-CF₃, -SH, -O-CH₃, -O-C₂H₅, -O-C₃H₇, Methyl, Ethyl, n-Propyl, Isopropyl, n-Butyl, 2-Butyl, Isobutyl und tert-Butyl substituiert sein können.

Besonders bevorzugte Substituenten für Alkinyl können unabhängig voneinander ausgewählt werden aus der Gruppe bestehend aus F, Cl, Br, I, -NO₂, -CN, -OH, -SH, -NH₂, -N(CH₃)₂, -N(C₂H₅)₂ und -N(CH₃)(C₂H₅).

Als geeignete C₂₋₁₂-Alkinyl-Reste seien beispielsweise Ethinyl, 1-Propinyl, 2-Propinyl, 1-Butinyl, 2-Butinyl, 3-Butinyl, 1-Pentinyl, 2-Pentinyl, 3-Pentinyl, 4-Pentinyl und Hexinyl genannt.

Unter mehrfach substituierten Alkinyl-Resten sind solche Alkinyl-Reste zu verstehen, die entweder an verschiedenen C-Atomen mehrfach substituiert sind, beispielsweise zweifach an verschiedenen C-Atomen wie im Fall von -CHCl-C≡CCl. Als geeignete substituierte Alkinyl-Reste seien beispielsweise -C≡C-F, -C≡C-Cl und -C≡C-l genannt.

Der Begriff "Heteroalkyl" bezeichnet einen wie vorstehend beschriebenen Alkyl-Rest, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkyl-Reste können bevorzugt 1, 2 oder 3 Heteroatom(e) unabhängig voneinander ausgewählt aus der Gruppe bestehend aus Sauerstoff, Schwefel und Stickstoff (NH) als Kettenglied(er) aufweisen. Heteroalkyl-Reste können bevorzugt 2- bis 12-gliedrig, besonders bevorzugt 2- bis 6-gliedrig, sein.

Als geeignete Heteroalkyl-Reste, die jeweils unsubstituiert oder einfach oder mehrfach substituiert sein können, seien beispielsweise -CH₂-O-CH₃, -CH₂-O-C₂H₅, - CH₂-O-CH(CH₃)₂, -CH₂-O-C(CH₃)₃, -CH₂-S-CH₃, -CH₂-S-C₂H₅, -CH₂-S-CH(CH₃)₂, - CH₂-S-C(CH₃)₃, -CH₂-NH-CH₃, -CH₂-NH-C₂H₅, -CH₂-NH-CH(CH₃)₂, -CH₂-NH-C(CH₃)₃, -CH₂-CH₂-O-CH₃, -CH₂-CH₂-O-C₂H₅, -CH₂-CH₂-O-CH(CH₃)₂, -CH₂-CH₂-O-C(CH₃)₃, -CH₂-CH₂-S-CH₃, -CH₂-CH₂-S-C₂H₅, -CH₂-CH₂-S-CH(CH₃)₂, -CH₂-CH₂-S-C(CH₃)₃, -CH₂-CH₂-NH-CH₃, -CH₂-CH₂-NH-C₂H₅, -CH₂-CH₂-NH-CH(CH₃)₂, -CH₂-CH₂-NH-C(CH₃)₃, -CH₂-S-CH₂-O-CH₃, -CH₂-O-CH₂-O-C₂H₅, -CH₂-O-CH₂-O-CH(CH₃)₂, -CH₂-S-CH₂-O-C(CH₃)₃, -CH₂-O-CH₂-S-CH₃, -CH₂-O-CH₂-S-C₂H₅, -CH₂-O-CH₂-S-CH(CH₃)₂, -CH₂-NH-CH₂-S-C(CH₃)₃, -CH₂-O-CH₂-NH-CH₃, -CH₂-O-CH₂-NH-C₂H₅, -CH₂-O-CH₂-NH-CH(CH₃)₂, -CH₂-S-CH₂-NH-C(CH₃)₃, -CH₂-CH₂-CH₂-O-CH₃, -CH₂-CH₂-CH₂-O-C₂H₅ und -CH₂-CH₂-C(H)(CH₃)-(CH₂)₃-CH₃ genannt.

Als geeignete substituierte Heteroalkyl-Reste seien beispielsweise -(CH₂)-O-(CF₃), - (CH₂)-O-(CHF₂), -(CH₂)-O-(CH₂F), -(CH₂)-S-(CF₃), -(CH₂)-S-(CHF₂), -(CH₂)-S-(CH₂F), -(CH₂)-(CH₂)-O-(CF₃), -(CF₂)-O-(CF₃), -(CH₂)-(CH₂)-S-(CF₃) und -(CH₂)-(CH₂)-(CH₂)-O-(CF₃) genannt.

Der Begriff "Heteroalkenyl" bezeichnet einen wie vorstehend beschriebenen Alkenyl-Rest, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkenyl-Reste können bevorzugt 1, 2 oder 3 Heteroatom(e) unabhängig voneinander ausgewählt aus der Gruppe bestehend aus Sauerstoff, Schwefel und Stickstoff (NH) als Kettenglied(er) aufweisen. Heteroalkenyl-Reste können bevorzugt 2- bis 12-gliedrig, besonders bevorzugt 2-bis 6-gliedrig, sein.

Als geeignete Heteroalkenyl-Reste seien beispielsweise -CH₂-O-CH=CH₂, -CH=CH-O-CH=CH-CH₃, -CH₂-CH₂-O-CH=CH₂, -CH₂-S-CH=CH₂, -CH=CH-S-CH=CH-CH₃, - CH₂-CH₂-S-CH=CH₂, -CH₂-NH-CH=CH₂, -CH=CH-NH-CH=CH-CH₃ und -CH₂-CH₂-NH-CH=CH₂ genannt.

Als geeignete substituierte Heteroalkenyl-Reste seien beispielsweise -CH₂-O-CH=CH-(CH₂)-OH, -CH₂-S-CH=CH-(CH₂)-NH₂ und -CH₂-NH-CH=CH-CN genannt.

Der Begriff "Heteroalkinyl" bezeichnet einen wie vorstehend beschriebenen Alkinyl-Rest, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkinyl-Reste können bevorzugt 1, 2 oder 3 Heteroatom(e) unabhängig voneinander ausgewählt aus der Gruppe bestehend aus Sauerstoff, Schwefel und Stickstoff (NH) als Kettenglied(er) aufweisen. Heteroalkinyl-Reste können bevorzugt 2- bis 12-gliedrig, besonders bevorzugt 2- bis 6-gliedrig, sein.

Als geeignete Heteroalkinyl-Reste seien beispielsweise -CH₂-O-C≡CH, -CH₂-CH₂-O-C≡CH, -CH₂-O-C≡C-CH₃, -CH₂-CH₂-O-C≡C-CH₃. -CH₂-S-C≡CH, -CH₂-CH₂-S-C≡CH, -CH₂-S-C≡C-CH₃, -CH₂-CH₂-S-C≡C-CH₃ genannt.

Als geeignete substituierte Heteroalkinyl-Reste seien beispielsweise -CH₂-O-C≡C-Cl, -CH₂-CH₂-O-C≡C-l, -CHF-O-C≡C-CH₃, -CHF-CH₂-O-C≡C-CH₃, -CH₂-S-C≡C-Cl, - CH₂-CH₂-S-C≡C-Cl, -CHF-S-C≡C-CH₃, -CHF-CH₂-S-C≡C-CH₃ genannt.

Der Begriff "Cycloalkyl" bedeutet im Sinne der vorliegenden Erfindung einen zyklischen gesättigten Kohlenwasserstoff-Rest mit bevorzugt 3, 4, 5, 6, 7, 8 oder 9 C-Atomen, besonders bevorzugt mit 3, 4, 5, 6 oder 7 C-Atomen, ganz besonders bevorzugt mit 5 oder 6 C-Atomen, wobei der Rest unsubstituiert oder einfach substituiert oder mehrfach gleich oder verschieden substituiert sein kann.

Als geeignete C₃₋₉-Cycloalkyl-Reste, die jeweils unsubstituiert oder einfach oder mehrfach substituiert sein können, seien beispielsweise Cyclopropyl, Cyclobutyl, Cyclopentyl, Cyclohexyl, Cycloheptyl, Cyclooctyl und Cyclononyl genannt. Als geeignete C₃₋₇-Cycloalkyl-Reste seien Cyclopropyl, Cyclobutyl, Cyclopentyl, Cyclohexyl und Cycloheptyl genannt.

Der Begriff "Cycloalkenyl" bedeutet im Sinne der vorliegenden Erfindung einen zyklischen ungesättigten Kohlenwasserstoff-Rest mit bevorzugt 3, 4, 5, 6, 7, 8 oder 9 C-Atomen, besonders bevorzugt mit 3, 4, 5, 6 oder 7 C-Atomen, ganz besonders bevorzugt mit 5 oder 6 C-Atomen, der wenigstens eine Doppelbindung, vorzugsweise eine Doppelbindung, aufweist und unsubstituiert oder einfach substituiert oder mehrfach gleich oder verschieden substituiert sein kann.

Als geeignete C₃₋₉-Cycloalkenyl-Reste, die jeweils unsubstituiert oder einfach oder mehrfach substituiert sein können, seien Cyclobutenyl, Cyclopentenyl, Cyclohexenyl, Cycloheptenyl, Cyclononenyl und Cyclooctenyl genannt. Als geeignete C₅₋₆-Cycloalkenyl-Reste seien Cyclopentenyl und Cyclohexenyl genannt.

Der Begriff "Heterocycloalkyl" bedeutet im Sinne der vorliegenden Erfindung einen zyklischen gesättigten Kohlenwasserstoff-Rest mit bevorzugt 3, 4, 5, 6, 7, 8 oder 9 C-Atomen, besonders bevorzugt mit 3, 4, 5, 6 oder 7 C-Atomen, ganz besonders bevorzugt mit 5 oder 6 C-Atomen, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heterocycloalkyl-Reste können bevorzugt 1, 2 oder 3 Heteroatom(e) unabhängig voneinander ausgewählt aus der Gruppe bestehend aus Sauerstoff, Schwefel und Stickstoff (NH) als Ringglied(er) aufweisen. Ein Heterocycloalkyl-Rest kann unsubstituiert oder einfach substituiert oder mehrfach gleich oder verschieden substituiert sein. Heterocycloalkyl-Reste können bevorzugt 3- bis 9-gliedrig, besonders bevorzugt 3-bis 7-gliedrig, ganz besonders bevorzugt 5- bis 7-gliedrig, sein.

Als geeignete 3- bis 9-gliedrige Heterocycloalkyl-Reste, die jeweils unsubstituiert oder einfach oder mehrfach substituiert sein können, seien beispielsweise Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Oxetanyl, Azepanyl, Azocanyl, Diazepanyl, Dithiolanyl, (1,3)-Dioxolan-2-yl, Isoxazolidinyl, Isothioazolidinyl, Pyrazolidinyl, Oxazolidinyl, (1,2,4)-Oxadiazolidinyl, (1,2,4)-Thiadiazolidinyl, (1,2,4)-Triazolidin-3-yl, (1,3,4)-Thiadiazolidin-2-yl, (1,3,4)-Triazolidin-1-yl, (1,3,4)-Triazoldidin-2-yl, Tetrahydropyridazinyl, Tetrahydropyrimidinyl, Tetrahydropyrazinyl, (1,3,5)-Tetrahydrotriazinyl, (1,2,4)-Tetrahydrotriazin-1-yl, (1,3)-Dithian-2-yl und (1,3)-Thiazolidinyl genannt. Als geeignete 5- bis 7-gliedrige Heterocycloalkyl-Reste seien beispielsweise Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Oxetanyl, Azepanyl, Diazepanyl und (1,3)-Dioxolan-2-yl genannt.

Der Begriff "Heterocycloalkenyl" bedeutet im Sinne der vorliegenden Erfindung einen zyklischen ungesättigten Kohlenwasserstoff-Rest mit bevorzugt 4, 5, 6, 7, 8 oder 9 C-Atomen, besonders bevorzugt mit 4, 5, 6 oder 7 C-Atomen, ganz besonders bevorzugt mit 5 oder 6 C-Atomen, der wenigstens eine Doppelbindung, vorzugsweise eine Doppelbindung, aufweist und in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heterocycloalkenyl-Reste können bevorzugt 1, 2 oder 3 Heteroatom(e) unabhängig voneinander ausgewählt aus der Gruppe bestehend aus Sauerstoff, Schwefel und Stickstoff (NH) als Ringglied(er) aufweisen. Ein Heterocycloalkenyl-Rest kann unsubstituiert oder einfach substituiert oder mehrfach gleich oder verschieden substituiert sein. Heterocycloalkenyl-Reste können bevorzugt 4- bis 9-gliedrig, besonders bevorzugt 4- bis 7-gliedrig, ganz besonders bevorzugt 5- bis 7-gliedrig, sein.

Als geeignete Heterocycloalkenyl-Reste bzw. als geeignete 5- bis 7-gliedrige Heterocycloalkenyl-Reste, die jeweils unsubstituiert oder einfach oder mehrfach substituiert sein können, seien beispielsweise (2,3)-Dihydrofuranyl, (2,5)-Dihydrofuranyl, (2,3)-Dihydrothienyl, (2,5)-Dihydrothienyl, (2,3)-Dihydropyrrolyl, (2,5)-Dihydropyrrolyl, (2,3)-Dihydroisoxazolyl, (4,5)-Dihydroisoxazolyl, (2,5)-Dihydroisothiazolyl, (2,3)-Dihydropyrazolyl, (4,5)-Dihydropyrazolyl, (2,5)-Dihydropyrazolyl, (2,3)-Dihydrooxazolyl, (4,5)-Dihydrooxazolyl, (2,5)-Dihydrooxazolyl, (2,3)-Dihydrothiazolyl, (4,5)-Dihydrothiazolyl, (2,5)-Dihydrothiazolyl, (2,3)-Dihydroimidazolyl, (4,5)-Dihydroimidazolyl, (2,5)-Dihydroimidazolyl, (3,4,5,6)-Tetrahydropyridin-2-yl-(1,2,5,6)-Tetrahydropyridin-1-yl, (1,2)-Dihydropyridin-1-yl, (1,4)-Dihydropyridin-1-yl, Dihydropyranyl und (1,2,3,4)-Tetrahydropyridin-1-yl genannt.

Cycloalkyl-Rest, Heterocycloalkyl-Rest, Cycloalkenyl-Rest oder Heterocyclalkenyl-Rest können im Sinne der vorliegenden Erfindung mit einem unsubstituierten oder wenigstens einfach substituierten mono- bzw. bizyklischem Ringsystem kondensiert (anneliert) sein. Unter einem mono- bzw. bizyklischem Ringsystem werden im Sinne der vorliegenden Erfindung mono- bzw. bizyklische Kohlenwasserstoffreste verstanden, die gesättigt, ungesättigt oder aromatisch sein und ggf. eines oder mehrere Heteroatome als Ringglieder aufweisen können. Vorzugsweise sind die Ringe der vorstehend genannten mono- oder bizyklischen Ringsysteme jeweils 4-, 5-oder 6-gliedrig und können jeweils bevorzugt ggf. 0, 1, 2, 3, 4 oder 5 Heteroatom(e), besonders bevorzugt ggf. 0, 1 oder 2 Heteroatom(e) als Ringglied(er) aufweisen, die unabhängig voneinander aus der Gruppe bestehend aus Sauerstoff, Stickstoff und Schwefel ausgewählt sind. Sofern ein bizyklisches Ringsystem vorliegt, können die verschiedenen Ringe, jeweils unabhängig voneinander, einen unterschiedlichen Sättigungsgrad aufweisen, d.h. gesättigt, ungesättigt oder aromatisch sein.

Sofern einer oder mehrere der Substituenten ein monozyklisches oder bizyklisches Ringsystem aufweisen, das einfach oder mehrfach substituiert ist, kann dieses bevorzugt mit ggf. 1, 2, 3, 4 oder 5, besonders bevorzugt mit ggf. 1, 2 oder 3, Substituenten substituiert sein, die unabhängig voneinander ausgewählt werden können aus der Gruppe bestehend aus F, Cl, Br, I, -CN, -NO₂, -OH, -SH, -NH₂, Oxo (=O), Thioxo (=S), -C(=O)-OH, C₁₋₅-Alkyl, -C₂₋₅-Alkenyl, -C₂₋₅-Alkinyl, -C≡C-Si(CH₃)₃, - C≡C-Si(C₂H₅)₃, -(CH₂)-O-C₁₋₅-Alkyl, -S-C₁₋₅-Alkyl, -S-Phenyl, -S-CH₂-Phenyl, -O-C₁₋₅-Alkyl, -O-Phenyl, -O-CH₂-Phenyl, -CF₃, -CHF₂, -CH₂F, -O-CF₃, -O-CHF₂, -O-CH₂F, - C(=O)-CF₃, -S-CF₃, -S-CHF₂, -S-CH₂F, -S(=O)₂-Phenyl, -S(=O)₂-C₁₋₅-Alkyl, -S(=O)-C₁₋₅-Alkyl, -NH-C₁₋₅-Alkyl, -N(C₁₋₅Alkyl)(C₁₋₅-Alkyl), -C(=O)-O-C₁₋₅-Alkyl, -C(=O)-H, - C(=O)-C₁₋₅-Alkyl, -CH₂-O-C(=O)-Phenyl, -O-C(=O)-Phenyl, -NH-S(=O)₂-C₁₋₅-Alkyl, - NH-C(=O)-C₁₋₅-Alkyl, -C(=O)-NH₂, -C(=O)-NH-C₁₋₅-Alkyl, -C(=O)-N(C₁₋₅-Alkyl)₂, Pyrazolyl, Phenyl, Furyl (Furanyl), Thiadiazolyl, Thiophenyl (Thienyl) und Benzyl, wobei die vorstehend genannten C₁₋₅-Alkyl-Reste jeweils linear oder verzweigt sein können und die zyklischen Substituenten bzw. die zyklischen Reste dieser Substituenten selbst jeweils unsubstituiert oder mit ggf. 1, 2, 3, 4 oder 5, bevorzugt mit ggf. 1, 2, 3 oder 4, Substituenten unabhängig voneinander ausgewählt aus der Gruppe bestehend aus F, Cl, Br, I, -CN, -CF₃, -OH, -NH₂, -O-CF₃, -SH, -O-C₁₋₅-Alkyl, -O-Phenyl, -O-CH₂-Phenyl, -(CH₂)-O-C₁₋₅-Alkyl, -S-C₁₋₅-Alkyl, -S-Phenyl, -S-CH₂-Phenyl, -C₁₋₅-Alkyl, -C₂₋₅-Alkenyl, -C₂₋₅-Alkinyl, -C≡CSi(CH₃)₃, -C≡C-Si(C₂H₅)₃, - C(=O)-O-C₁₋₅-Alkyl und -C(=O)-CF₃ substituiert sein können.

Besonders bevorzugt können die Substituenten, jeweils unabhängig voneinander, ausgewählt werden aus der Gruppe bestehend aus F, Cl, Br, I, -CN, -NO₂, -OH, -SH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -C≡C-Si(CH₃)₃, -C≡C-Si(C₂H₅)₃, -C≡C-Si(CH₃)₃, -C≡C-Si(C₂H₅)₃, -CH₂-O-CH₃, -CH₂-O-C₂H₅, -OH, -SH, -NH₂, Oxo (=O), - C(=O)-OH, -S-CH₃, -S-C₂H₅, -S(=O)-CH₃, -S(=O)₂-CH₃, -S(=O)-C₂H₅, -S(=O)₂-C₂H₅, - O-CH₃, -O-C₂H₅, -O-C₃H₇, -O-C(CH₃)₃, -CF₃, -CHF₂, -CH₂F, -O-CF₃. -O-CHF₂, -O-CH₂F, -C(=O)-CF₃, -S-CF₃, -S-CHF₂, -S-CH₂F, -S(=O)₂-Phenyl, Pyrazolyl, Phenyl, - N(CH₃)₂, -N(C₂H₅)₂, -NH-CH₃, -NH-C₂H₅, -CH₂-O-C(=O)-Phenyl, -NH-S(=O)₂-CH₃, - C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -O-C(=O)-Phenyl, -C(=O)-NH₂, -C(=O)-NH-CH₃, -C(=O)-N(CH₃)₂, Phenyl, Furyl (Furanyl), Thiadiazolyl, Thiophenyl (Thienyl) und Benzyl, wobei die zyklischen Substituenten bzw. die zyklischen Reste dieser Substituenten selbst mit ggf. 1, 2, 3, 4 oder 5, bevorzugt mit ggf. 1, 2, 3 oder 4, Substituenten unabhängig voneinander ausgewählt aus der Gruppe bestehend aus F, Cl, Br, I, -CN, -CF₃, -OH, -NH₂, -O-CF₃, -SH, -O-CH₃, -O-C₂H₅, -O-C₃H₇, Methyl, Ethyl, n-Propyl, Isopropyl, n-Butyl, 2-Butyl, Isobutyl, tert-Butyl, Ethenyl, Allyl, Ethinyl, Propinyl, -C≡C-Si(CH₃)₃, -C≡C-Si(C₂H₅)₃, -C(=O)-O-C₁₋₅-Alkyl und -C(=O)-CF₃ substituiert sein können.

Als geeignete Cycloalkyl-Rest, Heterocycloalkyl-Rest, Cycloalkenyl-Rest oder Heterocyclalkenyl-Rest, die jeweils unsubstituiert oder einfach oder mehrfach substituiert sein können, und mit einem mono- bzw. bizyklischem Ringsystem kondensiert sind, seien beispielhaft (1,2,3,4)-Tetrahydrochinolinyl, (1,2,3,4)-Tetrahydroisochinolinyl, (2,3)-Dihydro-1H-isoindolyl, Indolinyl, Indanyl, (1,2,3,4)-Tetrahydronaphthyl, (2,3)-Dihydro-benzo[1.4]dioxinyl, Benzo[1.3]dioxolyl, (3,4)-Dihydro-2H-benzo[1.4]oxazinyl, Octahydro-1H-isoindolyl, [1,3,4,9]-Tetrahydro-b-carbolinyl und Octahydro-pyrrolo[3,4-c]pyrrolyl genannt.

Cycloalkyl-Rest, Heterocycloalkyl-Rest, Cycloalkenyl-Rest oder Heterocyclalkenyl-Rest können im Sinne der vorliegenden Erfindung mit einem weiteren Cycloalkyl-Rest, Heterocycloalkyl-Rest, Cycloalkenyl-Rest oder Heterocyclalkenyl-Rest über ein gemeinsames Kohlenstoffatom im Ring einen spirozyklischen Rest bilden.

Als geeigneter spirozyklischer Rest sei beispielsweise ein 8-Azaspiro[4.5]decyl-Rest oder (1,4)-Dioxo-8-aza-spiro[4.5]decyl-Rest genannt.

Sofern einer oder mehrere der Substituenten für einen Cycloalkyl-Rest, Heterocycloalkyl-Rest, Cycloalkenyl-Rest oder Heterocyclalkenyl-Rest stehen oder einen solchen Rest aufweisen, der einfach oder mehrfach substituiert ist, kann dieser bevorzugt mit ggf. 1, 2, 3, 4 oder 5, besonders bevorzugt mit ggf. 1, 2 oder 3, Substituenten unabhängig voneinander ausgewählt aus der Gruppe bestehend aus F, Cl, Br, I, -CN, -CF₃, -OH, -NH₂, -O-CF₃, -SH, -O-C₁₋₅-Alkyl, -O-Phenyl, -O-CH₂-Phenyl, -(CH₂)-O-C₁₋₅-Alkyl, -S-C₁₋₅-Alkyl, -S-Phenyl, -S-CH₂-Phenyl, -C₁₋₅-Alkyl, -C₂₋₅-Alkenyl, -C₂₋₅-Alkinyl, -C≡C-Si(CH₃)₃, -C≡C-Si(C₂H₅)₃, -C(=O)-O-C₁₋₅-Alkyl, -C(=O)-CF₃, -S(=O)₂-C₁₋₅-Alkyl, -S(=O)-C₁₋₅-Alkyl, -S(=O)₂-Phenyl, Oxo (=O), Thioxo (=S), - N(C₁₋₅-Alkyl)₂, -N(H)(C₁₋₅-Alkyl), -NO₂, -S-CF₃, -C(=O)-OH, -NH-S(=O)₂-C₁₋₅-Alkyl, - NH-C(=O)-C₁₋₅-Alkyl, -NH-C(=O)-O-C₁₋₅-Alkyl, -NH-C(=O)-CF₃, -C(=O)-H, -C(=O)-C₁₋₅-Alkyl, -C(=O)-NH₂, -C(=O)-N(C₁₋₅-Alkyl)₂, -C(=O)-N(H)(C₁₋₅-Alkyl); -(CH₂)-Pyrrolidinyl, Benzyl, Phenethyl, Naphthyl, -(CH₂)-Naphthyl und Phenyl substituiert sein, wobei die vorstehend genannten C₁₋₅-Alkyl-Reste jeweils linear oder verzweigt sein können und die zyklischen Substituenten bzw. die zyklischen Reste dieser Substituenten selbst jeweils unsubstituiert oder mit 1, 2, 3, 4 oder 5, bevorzugt mit 1, 2, 3 oder 4, Substituenten unabhängig voneinander ausgewählt aus der Gruppe bestehend aus F, Cl, Br, I, -CN, -CF₃, -OH, -NH₂, -O-CF₃, -SH, -O-C₁₋₅-Alkyl, -0-Phenyl, -O-CH₂-Phenyl, -(CH₂)-O-C₁₋₅-Alkyl, -S-C₁₋₅-Alkyl, -S-Phenyl, -S-CH₂-Phenyl, -C₁₋₅-Alkyl, -C₂₋₅-Alkenyl, -C₂₋₅-Alkinyl, -C≡C-Si(CH₃)₃, -C≡C-Si(C₂H₅)₃, -C(=P)-O-C₁₋₅-Alkyl und -C(=O)-CF₃ substituiert sein können.

Besonders bevorzugt können die Substituenten, jeweils unabhängig voneinander, ausgewählt werden aus der Gruppe bestehend aus F, CI, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, -C≡C-Si(CH₃)₃, -C≡C-Si(C₂H₅)₃, -OH, -O-Phenyl, -O-CH₂-Phenyl, Oxo, Thioxo, -0-CH₃, -O-C₂H₅, -O-C₃H₇, -(CH₂)-O-CH₃, -(CH₂)-O-C₂H₅, -NH₂, -N(CH₃)₂, -N(C₂H₅)₂, - NH-CH₃, -NH-C₂H₅, -NO₂, -CF₃, -O-CF₃, -S-CF₃, -SH, -S-CH₃, -S-C₂H₅, -S(=O)-CH₃, - S(=O)₂-CH₃, -S(=O)-C₂H₅, -S(=O)₂-C₂H₅, -NH-S(=O)₂-CH₃, -C(=O)-OH, -C(=O)-H; - C(=O)-CH₃, -C(=O)-C₂H₅, -C(=O)-N(CH₃)₂, -C(=O)-NH-CH₃, -C(=O)-NH₂, -NH-C(=O)-CF₃, -NH-C(=O)-CH₃, -NH-C(=O)-C₂H₅, -C(=O)-O-CH₃, -C(=O)-O-C₂H₅, -C(=O)-O-C(CH₃)₃, Benzyl, -CH₂-Naphthyl und Phenyl, wobei die zyklischen Substituenten bzw. die zyklischen Reste dieser Substituenten mit 1, 2, 3, 4 oder 5, bevorzugt 1, 2 oder 3, Substituenten unabhängig voneinander ausgewählt aus der Gruppe bestehend aus F, Cl, Br, I, -CN, -CF₃, -OH, -NH₂, -O-CF₃, -SH, -O-CH₃, -O-C₂H₅, -0-C₃H₇, Methyl, Ethyl, n-Propyl, Isopropyl, n-Butyl, 2-Butyl, Isobutyl, tert-Butyl, Ethenyl, Allyl, Ethinyl, Propinyl, -C≡C-Si(CH₃)₃, -C≡C-Si(C₂H₅)₃, -C(=O)-O-C₁₋₅-Alkyl und - C(=O)-CF₃ substituiert sein können.

Der Begriff "Aryl" bedeutet im Sinne der vorliegenden Erfindungen einen mono- oder polyzyklischen, bevorzugt einen mono- oder bizyklischen, aromatischen Kohlenwasserstoff-Rest mit bevorzugt 6, 10 oder 14 C-Atomen. Ein Aryl-Rest kann unsubstituiert oder einfach substituiert oder mehrfach gleich oder verschieden substituiert sein. Als geeignete Aryl-Reste seien beispielsweise Phenyl-, 1-Naphthyl, 2-Naphthyl und Anthracenyl genannt. Besonders bevorzugt ist ein Aryl-Rest ein Phenyl-Rest.

Der Begriff "Heteroaryl" bedeutet im Sinne der vorliegenden Erfindung einen monozyklischen oder polyzyklischen, bevorzugt einen mono-, bi- oder trizyklischen, aromatischen Kohlenwasserstoff-Rest mit bevorzugt 5, 6, 7, 8, 9, 10, 11, 12, 13 oder 14 C-Atomen, besonders bevorzugt mit 5, 6, 9, 10, 13 oder 14 C-Atomen, ganz besonders bevorzugt mit 5 oder 6 C-Atomen, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroaryl-Reste können bevorzugt 1, 2, 3, 4 oder 5, besonders bevorzugt 1, 2 oder 3, Heteroatom(e) unabhängig voneinander ausgewählt aus der Gruppe bestehend aus Sauerstoff, Schwefel und Stickstoff (NH) als Ringglied(er) aufweisen. Ein Heteroanyl-Rest kann unsubstituiert oder einfach substituiert oder mehrfach gleich oder verschieden substituiert sein.

Als geeignete Heteroaryl-Reste seien beispielsweise Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Pyrimidinyl, Indazolyl, Chinoxalinyl, Chinazolinyl, Chinolinyl, Naphthridinyl, Thieno[2,3-d]pyrimidinyl und Isochinolinyl genannt.

Aryl- oder Heteroaryl-Reste können im Sinne der vorliegenden Erfindung mit einem mono- bzw. bizyklischem Ringsystem kondensiert (anneliert) sein.

Beispielhaft für Aryl-Reste, die mit einem mono- bzw. bizyklischen Ringsystem kondensiert sind, seien (2,3)-Dihydrobenzo[b]thiophenyl, (2,3)-Dihydro-1 H-indenyl, Indolinyl, (2,3)-Dihydrobenzofuranyl, (2,3)-Dihydrobenzo[d]oxazolyl, Benzo[d][1,3]dioxolyl, Benzo[d][1,3]oxathiolyl, Isoindolinyl, (1,3)-Diyhydroisobenzofuranyl, (1,3)-Dihydrobenzo[c]thiophenyl, (1,2,3,4)-Tetrahydronaphthyl, (1,2,3,4)-Tetrahydrochinolinyl, Chromanyl, Thiochromanyl, (1,2,3,4)-Tetrahydroisochinolinyl, (1,2,3,4)-Tetrahydrochinoxalinyl, (3,4)-Dihydro-2H-benzo[b][1,4]oxazinyl, (3,4)-Dihydro-2H-benzo[b][1,4]thiazinyl, (2,3)-Dihydrobenzo[b][1,4]dioxinyl, (2,3)-Dihydrobenzo[b][1,4]oxathiinyl, (6,7,8,9)-Tetrahydro-5H-benzo[7]annulenyl, (2,3,4,5)-Tetrahydro-1 H-benzo[b]azepinyl und (2,3,4,5)-Tetrahydro-1 H-benzo[c]azepinyl genannt.

Falls nicht anders angegeben, können, sofern einer oder mehrere der Substituenten für einen Aryl- oder Heteroaryl-Rest stehen oder einen Aryl- oder Heteroaryl-Rest aufweisen, der einfach oder mehrfach substituiert ist, diese Aryl- oder Heteroaryl-Reste bevorzugt mit ggf. 1, 2, 3, 4 oder 5, besonders bevorzugt mit ggf. 1, 2 oder 3, Substituenten unabhängig voneinander ausgewählt aus der Gruppe bestehend aus F, CI, Br, I, -CN, -CH₂-CN, -NO₂, -OH, -SH, -NH₂, -C(=O)-OH, -C₁₋₅-Alkyl, -(CH₂)-O-C₁₋₅-Alkyl, -C₂₋₅-Alkenyl, -C₂₋₅-Alkinyl, -C≡C-Si(CH₃)₃, -C≡C-Si(C₂H₅)₃, -S-C₁₋₅-Alkyl,-S-Phenyl, -S-CH₂Phenyl, -O-C₁₋₅-Alkyl, -O-Phenyl, -O-CH₂-Phenyl, -CF₃, -CHF₂, - CH₂F, -O-CF₃, -O-CHF₂, -O-CH₂F, -C(=O)-CF₃, -S-CF₃, -S-CHF₂, -S-CH₂F, -S(=O)₂-Phenyl, -S(=O)₂-C₁₋₅-Alkyl, -S(=O)-C₁₋₅-Alkyl, -NH-C₁₋₅-Alkyl, -N(C₁₋₅Alkyl)₂, -C(=O)-O-C₁₋₅-Alkyl, -C(=O)-O-Phenyl, -C(=O)-H; -C(=O)-C₁₋₅-Alkyl, -CH₂-O-C(=O)-Phenyl, - O-C(=O)-C₁₋₅-Alkyl, -O-C(=O)-Phenyl, -NH-S(=O)₂-C₁₋₅-Alkyl, -NH-C(=O)-C₁₋₅-Alkyl, - C(=O)-NH₂, -C(=O)-NH-C₁₋₅-Alkyl, -C(=P)-N(C₁₋₅-Alkyl)₂, -C(=O)-N(C₁₋₅-Alkyl)(Phenyl), -C(=O)-NH-Phenyl, -S(=O)₂-NH₂, -S(=O)₂-NH-C₁₋₅-Alkyl, -S(=O)₂-N(C₁₋₅-Alkyl)₂, -S(=O)₂-NH-Phenyl, [1,2,3]-Thiadiazolyl, Cyclopropyl, Cyclobutyl, Cyclopentyl, Cyclohexyl, Piperazinyl, Pyrrolidinyl, Piperidinyl, Pyrazolyl, Phenyl, Furyl (Furanyl), Thiazolyl, Thiadiazolyl, Thiophenyl (Thienyl), Benzyl und Phenethyl substituiert sein, wobei die vorstehend genannten C₁₋₅-Alkyl-Reste jeweils linear oder verzweigt sein können und die zyklischen Substituenten bzw. die zyklischen Reste dieser Substituenten selbst jeweils unsubstituiert oder mit ggf. 1, 2, 3, 4 oder 5, bevorzugt mit ggf. 1, 2, 3 oder 4, Substituenten unabhängig voneinander ausgewählt aus der Gruppe bestehend aus F, Cl, Br, I, -CN, -NO₂, -OH, -SH, -NH₂, -C(=O)-OH, - C₁₋₅-Alkyl, -(CH₂)-O-C₁₋₅-Alkyl, -C₂₋₅-Alkenyl, -C₂₋₅-Alkinyl, -C≡C-Si(CH₃)₃, -C≡C-Si(C₂H₅)₃, -S-C₁₋₅-Alkyl, -S-Phenyl, -S-CH₂-Phenyl, -O-C₁₋₅-Alkyl, -O-Phenyl, -O-CH₂-Phenyl, -CF₃, -CHF₂, -CH₂F, -O-CF₃, -O-CHF₂, -O-CH₂F, -C(=O)-CF₃, -S-CF₃, -S-CHF₂ und -S-CH₂F substituiert sein können.

Besonders bevorzugt können die Substituenten, jeweils unabhängig voneinander ausgewählt werden aus der Gruppe bestehend aus F, CI, Br, I, -CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -C≡C-Si(CH₃)₃ C≡C-Si(C₂H₅)₃, -CH₂-O-CH₃, -CH₂-O-C₂H₅, -NH₂, -C(=O)-OH, -S-CH₃, -S-C₂H₅, - S(=O)-CH₃, -S(=O)₂-CH₃, -S(=O)-C₂H₅, -S(=O)₂-C₂H₅. -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-Phenyl, Pyrazolyl, Phenyl, -N(CH₃)₂, -N(C₂H₅)₂, -NH-CH₃, - NH-C₂H₅, -CH₂-O-C(=O)-Phenyl, -NH-S(=O)₂-CH₃, -C(=O)-O-CH₃, -C(=O)-O-C₂H₅, - C(=O)-O-C(CH₃)₃, -C(=O)-H, -C(=O)-CH₃, -C(=O)-C₂H₅, -NH-C(=O)-CH₃, -NH-C(=O)-C₂H₅, -O-C(=O)-Phenyl, -C(=O)-NH₂, -C(=O)-NH-CH₃, -C(=O)-N(CH₃)₂, -C(=O)-O-CH(CH₃)₂, -C(=O)-O-(CH₂)₃-CH₃, -C(=O)-O-Phenyl, -O-C(=O)-CH₃, -O-C(=O)-C₂H₅, - C(=O)-NH-C₂H₅, -C(=O)-NH-C(CH₃)₃, -C(=O)-N(C₂H₅)₂. -C(=O)-NH-Phenyl, -C(=O)-N(CH₃)-Phenyl, -C(=O)-N(C₂H₅)-Phenyl, -S(=O)-NH₂, [1.2.3]-Thiadiazolyl, Cyclopropyl, Cyclobutyl, Cyclopentyl, Cyclohexyl, Piperazinyl, Pyrrolidinyl, Piperidinyl, Phenyl, Furyl (Furanyl), Thiadiazolyl, Thiophenyl (Thienyl) und Benzyl, wobei die zyklischen Substituenten bzw. die zyklischen Reste dieser Substituenten selbst jeweils mit ggf. 1, 2, 3, 4, oder 5, bevorzugt mit ggf. 1, 2, 3 oder 4, Substituenten unabhängig voneinander ausgewählt aus der Gruppe bestehend aus F, Cl, Br, I, -CN, -NO₂, -OH, -SH, -NH₂, -C(=O)-OH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -C≡C-Si(CH₃)₃, -C≡C-Si(C₂H₅)₃, -CH₂-O-CH₃, -CH₂-O-C₂H₅, -S-CH₃, -S-C₂H₅, -O-CH₃, -O-C₂H₅, -O-C₃H₇, -O-C(CH₃)₃, -CF₃, -CHF₂, -CH₂F, -O-CF₃, - O-CHF₂, -O-CH₂F, -C(=O)-CF₃, -S-CF₃, -S-CHF₂ und -S-CH₂F substituiert sein können.

Ganz besonders bevorzugt kann ein substituierter Aryl-Rest aus der Gruppe bestehend aus 2-Methyl-phenyl, 3-Methyl-phenyl, 4-Methyl-phenyl, 2-Fluor-phenyl, 3-Fluor-phenyl, 4-Fluor-phenyl, 2-Cyano-phenyl, 3-Cyano-phenyl, 4-Cyano-phenyl, 2-Hydroxy-phenyl, 3-Hydroxy-phenyl, 4-Hydroxy-phenyl, 2-Amino-phenyl, 3-Amino-phenyl, 4-Amino-phenyl, 2-Dimethylamino-phenyl, 3-Dimethylamino-phenyl, 4-Dimethylamino-phenyl, 2-Methylamino-phenyl, 3-Methylamino-phenyl, 4-Methylamino-phenyl, 2-Acetyl-phenyl, 3-Acetyl-phenyl, 4-Acetyl-phenyl, 2-Methylsulfinyl-phenyl, 3-Methylsulfinyl-phenyl, 4-Methylsulfinyl-phenyl, 2-Methylsulfonyl-phenyl, 3-Methylsulfonyl-phenyl, 4-Methylsulfonyl-phenyl, 2-Methoxy-phenyl, 3-Methoxy-phenyl, 4-Methoxy-phenyl, 2-Chlor-phenyl, 3-Chlor-phenyl, 4-Chlor-phenyl, 2-Ethoxy-phenyl, 3-Ethoxy-phenyl, 4-Ethoxyphenyl, 2-Trifluormethyl-phenyl, 3-Trifluormethyl-phenyl, 4-Trifluormethyl-phenyl, 2-Difluormethyl-phenyl, 3-Difluormethyl-phenyl, 4-Difluormethyl-phenyl, 2-Fluormethyl-phenyl, 3-Fluormethyl-phenyl, 4-Fluormethyl-phenyl, 2-Nitro-phenyl, 3-Nitro-phenyl, 4-Nitro-phenyl, 2-Ethyl-phenyl, 3-Ethyl-phenyl, 4-Ethyl-phenyl, 2-Propyl-phenyl, 3-Propyl-phenyl, 4-Propyl-phenyl, 2-Isopropyl-phenyl, 3-Isopropyl-phenyl, 4-Isopropyl-phenyl, 2-tert-Butyl-phenyl, 3-tert-Butyl-phenyl, 4-tert-Butyl-phenyl, 2-Carboxyphenyl, 3-Carboxy-phenyl, 4-Carboxyphenyl, 2-Ethenyl-phenyl, 3-Ethenyl-phenyl, 4-Ethenyl-phenyl, 2-Ethinyl-phenyl, 3-Ethinyl-phenyl, 4-Ethinyl-phenyl, 2-Allyl-phenyl, 3-Allyl-phenyl, 4-Allyl-phenyl, 2-Trimethylsilanylethinyl-phenyl, 3-Trimethylsilanylethinyl-phenyl, 4-Trimethylsilanylethinyl-phenyl, 2-Formyl-phenyl, 3-Formyl-phenyl, 4-Formyl-phenyl, 2-Acetamino-phenyl, 3-Acetamino-phenyl, 4-Acetamino-phenyl, 2-Dimethylaminocarbonyl-phenyl, 3-Dimethylaminocarbonyl-phenyl, 4-Dimethylaminocarbonyl-phenyl, 2-Methoxymethyl-phenyl, 3-Methoxymethyl-phenyl, 4-Methoxymethyl-phenyl, 2-Ethoxymethyl-phenyl, 3-Ethoxymethyl-phenyl, 4-Ethoxymethyl-phenyl, 2-Aminocarbonyl-phenyl, 3-Aminocarbonyl-phenyl, 4-Aminocarbonyl-phenyl, 2-Methylaminocarbonyl-phenyl, 3-Methylaminocarbonyl-phenyl, 4-Methylaminocarbonyl-phenyl, 2-Carboxymethylester-phenyl, 3-Carboxymethylester-phenyl, 4-Carboxymethylester-phenyl, 2-Carboxyethylester phenyl, 3-Carboxyethylester-phenyl, 4-Carboxyethylester-phenyl, 2-Carboxy-tert-butylester-phenyl, 3-Carboxy-tert-butylester-phenyl, 4-Carboxy-tert-butylester-phenyl, 2-Methylmercapto-phenyl, 3-Methylmercapto-phenyl, 4-Methylmercapto-phenyl, 2-Ethylmercapto-phenyl, 3-Ethylmercapto-phenyl, 4-Ethylmercaptophenyl, 2-Biphenyl, 3-Biphenyl, 4-Biphenyl, 2-Bromphenyl, 3-Bromphenyl, 4-Bromphenyl, 2-lod-phenyl, 3-lodphenyl, 4-lodphenyl, 2-Trifluormethoxy-phenyl, 3-Trifluormethoxy-phenyl, 4-Trifluormethoxy-phenyl, 2-Fluor-3-trifluormethylphenyl, 2-Fluor-4-methyl-phenyl, (2,3)-Difluorphenyl, (2,3)-Dimethyl-phenyl, (2,3)-Dichlorphenyl, 3-Fluor-2-trifluormethylphenyl, (2,4)-Dichlor-phenyl, (2,4)-Difluorphenyl, 4-Fluor-2-trifluormethyl-phenyl, (2,4)-Dimethoxyphenyl, 2-Chlor-4-fluor-phenyl, 2-Chlor-4-nitro-phenyl, 2-Chlor-4-methyl-phenyl, 2-Chlor-5-tritluormethyl-phenyl, 2-Chlor-5-methoxy-phenyl, 2-Brom-5-trifluormethyl-phenyl, 2-Brom-5-methoxy-phenyl, (2,4)-Dibrom-phenyl, (2,4)-Dimethyl-phenyl, 2-Fluor-4-trifluormethyl-phenyl, (2,5)-Difluor-phenyl, 2-Fluor-5-trifluormethyl-phenyl, 5-Fluor-2-trifluormethyl-phenyl, 5-Chlor-2-trifluormethyl-phenyl, 5-Brom-2-trifluormethyl-phenyl, (2,5)-Dimethoxy-phenyl, (2,5)-Bis-trifluormethyl-phenyl, (2,5)-Dichlor-phenyl, (2,5)-Dibrom-phenyl, 2-Methoxy-5-nitro-phenyl, 2-Fluor-6-trifluormethyl-phenyl, (2,6)-Dimethoxy-phenyl, (2,6)-Dimethyl-phenyl, (2,6)-Dichlor-phenyl, 2-Chlor-6-fluor-phenyl, 2-Brom-6-chlor-phenyl, 2-Brom-6-fluor-phenyl, (2,6)-Difluor-phenyl, (2,6)-Difluor-3-methyl-phenyl, (2,6)-Dibrom-phenyl, (2,6)-Dichlorphenyl, 3-Chlor-2-fluor-phenyl, 3-Chlor-5-methyl-phenyl, (3,4)-Dichlorphenyl, (3,4)-Dimethyl-phenyl, 3-Methyl-4-methoxy-phenyl, 4-Chlor-3-nitro-phenyl, (3,4)-Dimethoxy-phenyl, 4-Fluor-3-trifluormethylphenyl, 3-Fluor-4-trifluormethyl-phenyl, (3,4)-Difluor-phenyl, 3-Cyano-4-fluor-phenyl, 3-Cyano-4-methyl-phenyl, 3-Cyano-4-methoxy-phenyl, 3-Brom-4-fluor-phenyl, 3-Brom-4-methyl-phenyl, 3-Brom-4-methoxy-phenyl, 4-Chlor-2-fluor-phenyl, 4-Chlor-3-trifluormethyl, 4-Brom-3-methyl-phenyl, 4-Brom-5-methyl-phenyl, 3-Chlor-4-fluor-phenyl, 4-Fluor-3-nitro-phenyl, 4-Brom-3-nitro-phenyl, (3,4)-Dibrom-phenyl, 4-Chlor-3-methyl-phenyl, 4-Brom-3-methyl-phenyl, 4-Fluor-3-methyl-phenyl, 3-Fluor-4-methyl-phenyl, 3-Fluor-5-methyl-phenyl, 2-Fluor-3-methyl-phenyl, 4-Methyl-3-nitro-phenyl, (3,5)-Dimethoxy-phenyl, (3,5)-Dimethyl-phenyl, (3,5)-Bis-trifluormethyl-phenyl, (3,5)-Difluor-phenyl, (3,5)-Dinitro-phenyl, (3,5)-Dichlor-phenyl, 3-Fluor-5-trifluormethyl-phenyl, 5-Fluor-3-trifluormethyl-phenyl, (3,5)-Dibrom-phenyl, 5-Chlor-4-fluor-phenyl, 5-Chlor-4-fluor-phenyl, 5-Brom-4-methyl-phenyl, (2,3,4)-Trifluorphenyl, (2,3,4)-Trichlorphenyl, (2,3,6)-Trifluor-phenyl, 5-Chlor-2-methoxy-phenyl, (2,3)-Difluor-4-methyl, (2,4,5)-Trifluor-phenyl, (2,4,5)-Trichlor-phenyl, (2,4)-Dichlor-5-fluor-phenyl, (2,4,6)-Trichlor-phenyl, (2,4,6)-Trimethylphenyl, (2,4,6)-Trifluor-phenyl, (2,4,6)-Trimethoxy-phenyl, (3,4,5)-Trimethoxy-phenyl, (2,3,4,5)-Tetrafluor-phenyl, 4-Methoxy-(2,3,6)-trimethyl-phenyl, 4-Methoxy-(2,3,6)-trimethyl-phenyl, 4-Chlor-2,5-dimethyl-phenyl, 2-Chlor-6-fluor-3-methyl-phenyl, 6-Chlor-2-fluor-3-methyl, (2,4,6)-Trimethylphenyl und (2,3,4,5,6)-Pentafluor-phenyl ausgewählt werden.

Ganz besonders bevorzugt kann ein substituierter Heteroaryl-Rest aus der Gruppe bestehend aus 3-Methyl-pyrid-2-yl, 4-Methyl-pyrid-2-yl, 5-Methyl-pyrid-2-yl, 6-Methyl-pyrid-2-yl, 2-Methyl-pyrid-3-yl, 4-Methyl-pyrid-3-yl, 5-Methyl-pyrid-3-yl, 6-Methyl-pyrid-3-yl, 2-Methyl-pyrid-4-yl, 3-Methyl-pyrid-4-yl, 3-Fluor-pyrid-2-yl, 4-Fluor-pyrid-2-yl, 5-Fluor-pyrid-2-yl, 6-Fluor-pyrid-2-yl, 3-Chlor-pyrid-2-yl, 4-Chlor-pyrid-2-yl, 5-Chlor-pyrid-2-yl, 6-Chlor-pyrid-2-yl, 3-Trifluormethyl-pyrid-2-yl, 4-Trifluormethyl-pyrid-2-yl, 5-Trifluotmethyl-pyrid-2-yl, 6-Trifluormethyl-pyrid-2-yl, 3-Methoxy-pyrid-2-yl, 4-Methoxy-pyrid-2-yl, 5-Methoxy-pyrid-2-yl, 6-Methoxy-pyrid-2-yl, 4-Methyl-thiazol-2-yl, 5-Methyl-thiazol-2-yl, 4-Trifluormethyl-thiazol-2-yl, 5-Trifluormethyl-thiazol-2-yl, 4-Chlor-thiazol-2-yl, 5-Chlor-thiazol-2-yl, 4-Brom-thiazol-2-yl, 5-Brom-thiazol-2-yl, 4-Fluor-thiazol-2-yl, 5-Fluor-thiazol-2-yl, 4-Cyano-thiazol-2-yl, 5-Cyano-thiazol-2-yl, 4-Methoxy-thiazol-2-yl, 5-Methoxy-thiazol-2-yl, 4-Methyl-oxazol-2-yl, 5-Methyl-oxazol-2-yl, 4-Trifluormethyl-oxazol-2-yl, 5-Trifluormethyl-oxazol-2-yl, 4-Chlor-oxazol-2-yl, 5-Chlor-oxazol-2-yl, 4-Brom-oxazol-2-yl, 5-Brom-oxazol-2-yl, 4-Fluor-oxazol-2-yl, 5-Fluor-oxazol-2-yl, 4-Cyano-oxazol-2-yl, 5-Cyano-oxazol-2-yl, 4-Methoxy-oxazol-2-yl, 5-Methoxy-oxazol-2-yl, 2-Methyl-(1,2,4)-thiadiazol-5-yl, 2-Trifluormethyl-(1,2,4)-thiadiazol-5-yl, 2-Chlor-(1,2,4)-thiadiazol-5-yl, 2-Fluor-(1,2,4)-thiadiazol-5-yl, 2-Methoxy-(1,2,4)-thiadiazol-5-yl, 2-Cyano-(1,2,4)-thiadiazol-5-yl, 2-Methyl-(1,2,4)-oxadiazol-5-yl, 2-Trifluormethyl-(1,2,4)-oxadiazol-5-yl, 2-Chlor-(1,2,4)-oxadiazol-5-yl, 2-Fluor-(1,2,4)-oxadiazol-5-yl, 2-Methoxy-(1,2,4)-oxadiazol-5-yl und 2-Cyano-(1,2,4)-oxadiazol-5-yl ausgewählt werden.

Der Begriff "Alkylen" umfaßt im Sinne der vorliegenden Erfindung acyclische gesättigte Kohlenwasserstoffketten, die einen Aryl-, Heteroaryl-, Cycloalkyl-, Heterocyloalkyl-, Cycloalkenyl- oder Heterocycloalkenyl-Rest mit den Verbindungen der allgemeinen Formel I bzw. mit einem anderen Substituenten verbinden. AlkylenKetten können verzweigt oder geradkettig sowie unsubstituiert oder wenigstens einfach substituiert sein mit wie im Fall von C₁₋₁₂-Alkylen 1 bis 12 (d. h. 1, 2, 3, 4, 5, 6, 7, 8, 9, 10, 11 oder 12) C-Atomen, mit wie im Fall von C₁₋₆-Alkylen 1 bis 6 (d. h. 1, 2, 3, 4, 5 oder 6) C-Atomen bzw. mit wie im Fall von C₁₋₃-Alkylen 1 bis 3 (d. h. 1, 2 oder 3) C-Atomen. Beispielhaft seien C₁₋₆-Alkylen-Gruppen wie -(CH₂)-, -(CH₂)₂-, - C(H)(CH₃)-, -(CH₂)₃-, -(CH₂)₄-, -(CH₂)₅-, -C(CH₃)₂-, -C(H)(CH₃)-, -C(H)(C(H)(CH₃)₂)-und C(C₂H₅)(H)- genannt. Als geeignete C₁₋₃-Alkylen-Gruppe seien beispielhaft - (CH₂)-, -(CH₂)₂- und -(CH₂)₃- genannt.

Der Begriff "Alkenylen" umfaßt im Sinne der vorliegenden Erfindung azyklische ungesättigte Kohlenwasserstoffketten, die einen Aryl-, Heteroaryl-, Cycloalkyl-, Heterocyloalkyl-, Cycloalkenyl- oder Heterocycloalkenyl-Rest mit den Verbindungen der allgemeinen Formel I bzw. mit einem anderen Substituenten verbinden. Alkenylen-Ketten weisen wenigstens eine Doppelbindung, bevorzugt 1, 2 oder 3 Doppelbindungen, auf und können verzweigt oder geradkettig sowie unsubstituiert oder wenigstens einfach substituiert sein mit wie im Fall von C₂₋₁₂-Alkenylen 2 bis 12 (d. h. 2, 3, 4, 5, 6, 7, 8, 9, 10, 11 oder 12) C-Atomen, mit wie im Fall von C₂₋₆-Alkenylen 2 bis 6 (d. h. 2, 3, 4, 5 oder 6) C-Atomen bzw. mit wie im Fall von C₂₋₃-Alkenylen 2 bis 3 (d. h. 2 oder 3) C-Atomen. Beispielhaft seien C₂₋₃-Alkenylen-Gruppen wie -CH=CH- und -CH₂-CH=CH- genannt.

Der Begriff "Alkinylen" umfaßt im Sinne der vorliegenden Erfindung azyklische ungesättigte Kohlenwasserstoffketten, die einen Aryl-, Heteroaryl-, Cycloalkyl-, Heterocyloalkyl-, Cycloalkenyl- oder Heterocycloalkenyl-Rest mit den Verbindungen der allgemeinen Formel I bzw. mit einem anderen Substituenten verbinden. Alkinylen-Ketten weisen wenigstens eine Dreifachbindung, bevorzugt 1 oder 2 Dreifachbindungen, auf und können verzweigt oder geradkettig sowie unsubstituiert oder wenigstens einfach substituiert sein mit wie im Fall von C₂₋₁₂-Alkinylen 2 bis 12 (d. h. 2, 3, 4, 5, 6, 7, 8, 9, 10, 11 oder 12) C-Atomen, mit wie im Fall von C₂₋₆-Alkinylen 2 bis 6 (d. h. 2, 3, 4, 5 oder 6) C-Atomen bzw. mit wie im Fall von C₂₋₃-Alkinylen 2 bis 3 (d. h. 2 oder 3) C-Atomen. Beispielhaft seien C₂₋₃-Alkinylen-Gruppen wie -C≡C- und -CH₂-C≡C- genannt.

Der Begriff "Heteroalkylen" bezeichnet eine wie vorstehend beschriebenen AlkylenKette, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkylen-Gruppen können bevorzugt 1, 2 oder 3 Heteroatom(e), besonders bevorzugt ein Heteroatom, ausgewählt aus der Gruppe bestehend aus Sauerstoff, Schwefel und Stickstoff (NH) als Kettenglied(er) aufweisen. Heteroalkylen-Gruppen können bevorzugt 2- bis 12-gliedrig, besonders bevorzugt 2- bis 6-gliedrig, ganz besonders bevorzugt 2- oder 3-gliedrig, sein.

Beispielhaft seien Heteroalkylen-Gruppen wie -(CH₂)-O-, -(CH₂)₂-O-, -(CH₂)₃-O-, - (CH₂)₄-O-, -O-(CH₂)-, -O-(CH₂)₂-, -O-(CH₂)₃-, -O-(CH₂)₄-, -C(C₂H₅)(H)-O-, -O-C(C₂H₅)(H)-, -CH₂-O-CH₂-, -CH₂-S-CH₂-, -CH₂-NH-CH₂-, -CH₂-NH- und -CH₂-CH₂-NH-CH₂-CH₂- genannt.

Der Begriff "Heteroalkenylen" bezeichnet eine wie vorstehend beschriebene Alkenylen-Kette, in dem ein oder mehrere C-Atome jeweils durch ein Heteroatom unabhängig voneinander ausgewählt aus der Gruppe bestehend aus Sauerstoff, Schwefel und Stickstoff (NH) ersetzt wurden. Heteroalkenylen-Gruppen können bevorzugt 1, 2 oder 3 Heteroatom(e), besonders bevorzugt 1 Heteroatom, ausgewählt aus der Gruppe bestehend aus Sauerstoff, Schwefel und Stickstoff (NH) als Kettenglied(er) aufweisen. Heteroalkenylen-Gruppen können bevorzugt 2- bis 12-gliedrig, besonders bevorzugt 2- bis 6-gliedrig, ganz besonders bevorzugt 2- oder 3-gliedrig, sein. Beispielhaft seien Heteroalkenylen-Gruppen wie -CH=CH-NH-, - CH=CH-O- und -CH=CH-S- genannt.

Sofern einer oder mehrere der Substituenten für eine Alkylen-, Alkenylen-, Alkinylen-, Heteroalkylen- oder Heteroalkenylen-Gruppe stehen oder eine solche Gruppe aufweisen, die einfach oder mehrfach substituiert ist, kann diese bevorzugt mit ggf. 1, 2, 3, 4 oder 5, besonders bevorzugt mit ggf. 1, 2 oder 3, Substituenten unabhängig voneinander ausgewählt aus der Gruppe bestehend aus Phenyl, F, Cl, Br, I, -NO₂, - CN, -OH, -O-Phenyl, -O-CH₂-Phenyl, -SH, -S-Phenyl, -S-CH₂-Phehyl, -NH₂, -N(C₁₋₅-Alkyl)₂, -NH-Phenyl, -N(C₁₋₅-Alkyl)(Phenyl), -N(C₁₋₅-Alkyl)(CH₂-Phenyl), -N(C₁₋₅-Alkyl)(CH₂-CH₂-Phenyl), -C(=O)-H, -C(=O)-C₁₋₅-Alkyl, -C(=O)-Phenyl, -C(=S)-C₁₋₅-Alkyl, -C(=S)-Phenyl, -C(=O)-OH, -C(=O)-O-C₁₋₅-Alkyl, -C(=O)-O-Phenyl, -C(=O)-NH₂, -C(=O)-NH-C₁₋₅-Alkyl, -C(=O)-N(C₁₋₅-Alkyl)₂, -S(=O)-C₁₋₅-Alkyl, -S(=O)-Phenyl, - S(=O)₂-C₁₋₅-Alkyl, -S(=O)₂-Phenyl, -S(=O)₂-NH₂ und -SO₃H substituiert sein, wobei die vorstehend genannten C₁₋₅-Alkyl-Reste jeweils linear oder verzweigt sein können und die vorstehend genannten Phenyl-Reste mit 1, 2, 3, 4 oder 5, bevorzugt mit 1, 2, 3 oder 4, Substituenten unabhängig voneinander ausgewählt aus der Gruppe bestehend aus F, CI, Br, I, -CN, -NO₂, -OH, -SH, -NH₂, -C(=O)-OH, -C₁₋₅-Alkyl,-(CH₂)-O-C₁₋₅-Alkyl, -C₂₋₅-Alkenyl, -C₂₋₅-Alkinyl, -C≡C-Si(CH₃)₃, -C≡C-Si(C₂H₅)₃, -S-C₁₋₅-Alkyl, -S-Phenyl, -S-CH₂-Phenyl, -O-C₁₋₅-Alkyl, -O-Phenyl, -O-CH₂-Phenyl, -CF₃, - CHF₂, -CH₂F, -O-CF₃, -O-CHF₂, -O-CH₂F, -C(=O)-CF₃, -S-CF₃, -S-CHF₂ und -S-CH₂F substituiert sein können.

Besonders bevorzugt können Alkylen-, Alkenylen-, Alkinylen-, Heteroalkylen- oder Heteroalkenylen-Gruppen mit 1, 2 oder 3 Substituenten unabhängig voneinander ausgewählt werden aus der Gruppe bestehend aus Phenyl, F, Cl, Sr, I, -NO₂, -CN, - OH, -O-Phenyl, -SH, -S-Phenyl, -C(=O)-O-CH₃, -C(=O)-O-C₂H₅, -NH₂, -N(CH₃)₂, - N(C₂H₅)₂ und -N(CH₃)(C₂H₅) substituiert sein, wobei der Phenyl-Rest mit 1, 2, 3, 4 oder 5 Substituenten unabhängig voneinander ausgewählt aus der Gruppe bestehend aus F, CI, Br, I, -OH, -SH, -NO₂, -CN, -O-CH₃, -O-CF₃ und -O-C₂H₅ substituiert sein kann.

Bevorzugt sind 1,3-disubstituierte 4-Methyl-1 H-pyrrol-2-carbonsäureamide der vorstehend angegebenen allgemeinen Formel I, worin R¹

für C₁₋₆-Alkyl, C₂₋₆-Alkenyl oder C₂₋₆-Alkinyl, das jeweils unsubstituiert oder mit ggf. 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, -N(C₂H₅)₂, - N(CH₃)(C₂H₅), -C(=O)-OH, -C(=O)-O-CH₃, -C(=O)-O-C₂H₅, -C(=O)-O-C(CH₃)₃, - C(=O)-O-CH₂-Phenyl, -C(=O)-NH-Naphthyl, -N(CH₃)-Phenyl, -N(C₂H₅)-Phenyl, - N(C₂H₅)-(m-Toluyl), -N(C₂H₅)-(p-Toluyl), -N(CH₃)-(p-Toluyl) und -NH-C(=O)-O-C(CH₃)₃ substituiert ist;

2- bis 6-gliedriges Heteroalkyl, das jeweils unsubstituiert oder mit ggf. 1, 2, 3, 4 oder 5 Substituenten unabhängig voneinander ausgewählt aus der Gruppe bestehend aus F, Cl, Br, I, -NO₂, -CN, -OH, -SH und -NH₂ substituiert ist und jeweils 1 oder 2 Heteroatom(e) unabhängig voneinander ausgewählt aus der Gruppe bestehend aus Sauerstoff, Schwefel und Stickstoff (NH) als Kettenglied(er) aufweist; C₃₋₇-Cycloalkyl, C₅₋₆-Cycloalkenyl, 5- bis 7-gliedriges Heterocycloalkyl, 5- bis 7-gliedriges Heterocycloalkenyl, (1,2,3,4)-Tetrahydrochinollnyl, (1,2,3,4)-Tetrahydroisochinolinyl, (2,3)-Dihydro-1H-isoindolyl, Indolinyl, Indanyl, (1,2,3,4)-Tetrahydronaphthyl, (2,3)-Dihydro-benzo[1.4]dioxinyl oder Benzo[1.3]dioxolyl, das jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, -0-CH₃, -O-C₂H₅, -O-C₃H₇, -O-Phenyl, -O-CH₂-Phenyl, -NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, - C(=O)-C₂H₅, -C(=O)-C(CH₃)₃, -C(=O)-NH-CH₃, -C(=O)-NH-C₂H₅, -CH₂-Naphthyl, Benzyl und Phenyl substituiert ist und/oder jeweils über eine unsubstituierte C₁₋₃-Alkylen-, C₂₋₃-Alkenylen- oder C₂₋₃-Alkinylen-Gruppe gebunden sein kann und/oder jeweils 1 oder 2 Heteroatom(e) unabhängig voneinander ausgewählt aus der Gruppe bestehend aus Sauerstoff, Schwefel und Stickstoff (NH) als Ringglied(er) aufweisen kann;

Phenyl, das jeweils über eine C₁₋₃-Alkylen-, C₂₋₃-Alkenylen- oder C₂₋₃-Alkinylen-Gruppe, die jeweils unsubstituiert oder mit 1 oder 2 Substituenten ausgewählt aus der Gruppe bestehend aus F, C, Br, -C(=O)-O-CH₃ und -C(=O)-O-C₂H₅ substituiert ist, oder über eine -CH₂-CH₂-O-, -CH₂-O- oder -CH₂-CH₂-CH₂-O-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I,-CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, - C≡C-Si(CH₃)₃, -C≡C-Si(C₂H₅)₃, -CH₂-O-CH₃, -CH₂-O-C₂H₅, -NH₂, -S-CH₃, -S-C₂H₅, - S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-Phenyl, Pyrazolyl, -N(CH₃)₂, -N(C₂H₅)₂, -NH-CH₃, -NH-C₂H₅, -CH₂-O-C(=O)-Phenyl, -NH-S(=O)₂-CH₃, -C(=O)-H, -C(=O)-CH₃, -C(=O)-C₂H₅, -NH-C(=O)-CH₃, -NH-C(=O)-C₂H₅, -O-C(=O)-Phenyl, -C(=O)-NH₂, -C(=O)-NH-CH₃, - C(=O)-NH-C₂H₅, -C(=O)-NH-C(CH₃)₃, -C(=O)-N(C₂H₅)₂, -S(=O)-NH₂, [1.2.3]-Thiadiazolyl, Cyclopropyl, Cyclobutyl, Cyclopentyl, Cyclohexyl, Piperazinyl, Pyrrolidinyl, Piperidinyl, Phenyl, Furyl (Furanyl), Thiadiazolyl, Thiophenyl (Thienyl) und Benzyl substituiert ist, wobei die zyklischen Substituenten bzw. die zyklischen Reste dieser Substituenten selbst jeweils mit ggf. 1, 2, 3, 4, oder 5 Substituenten unabhängig voneinander ausgewählt aus der Gruppe bestehend aus F, Cl, Br, I, - CN, -NO₂, -OH, -SH, -NH₂, Methyl, Ethyl, n-Propyl, Isopropyl, n-Butyl, Isobutyl, 2-Butyl, tert-Butyl, n-Pentyl, neo-Pentyl, -O-CH₃, -O-C₂H₅, -O-C₃H₇, -O-C(CH₃)₃, -CF₃, - CHF₂, -CH₂F, -O-CF₃, -O-CHF₂ und -O-CH₂F substituiert sein können;
einen Rest ausgewählt aus der Gruppe bestehend aus Naphthyl, Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Indazolyl, Chinoxalinyl, Chinazolinyl, Chinolinyl, Naphthridinyl und Isochinolinyl, der jeweils über eine C₁₋₃-Alkylen-, C₂₋₃-Alkenylen- oder C₂₋₃-Alkinylen-Gruppe gebunden sein kann und/oder jeweils unsubstituiert oder mit ggf. 1, 2, 3, 4 oder 5 Substituenten unabhängig voneinander ausgewählt aus der Gruppe bestehend aus F, Cl, Br, I, - CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, - C≡C-Si(CH₃)₃, -C≡C-Si(C₂H₅)₃, -CH₂-O-CH₃, -CH₂-O-C₂H₅, -NH₂, -C(=O)-OH, -S-CH₃, -S-C₂H₅, -S(=O)-CH₃, -S(=O)₂-CH₃, -S(=O)-C₂H₅, -S(=O)₂-C₂H₅, -O-CH₃, -0-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-Phenyl, Pyrazolyl, -N(CH₃)₂, -N(C₂H₅)₂, - NH-CH₃, -NH-C₂H₅, -CH₂-O-C(=O)-Phenyl, -NH-S(=O)₂-CH₃, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, - NH-C(=O)-C₂H₅, -O-C(=O)-Phenyl, -C(=O)-NH₂, -C(=O)-NH-CH₃, -C(=O)-N(CH₃)₂, - C(=O)-O-CH(CH₃)₂, -C(=O)-O-(CH₂)₃-CH₃, -C(=O)-O-Phenyl, -O-C(=O)-CH₃, -0-C(=O)-C₂H₅, -C(=O)-NH-C₂H₅, -C(=O)-NH-C(CH₃)₃, -C(=O)-N(C₂H₅)₂, -C(=O)-NH-Phenyl, -C(=O)-N(CH₃)-Phenyl, -C(=O)-N(C₂H₅)-Phenyl, -S(=O)-NH₂, [1.2.3]-Thiadiazolyl, Cyclopropyl, Cyclobutyl, Cyclopentyl, Cyclohexyl, Piperazinyl, Pyrrolidinyl, Piperidinyl, Phenyl, Furyl (Furanyl), Thiadiazolyl, Thiophenyl (Thienyl) und Benzyl substituiert ist, wobei die zyklischen Substituenten bzw. die zyklischen Reste dieser Substituenten selbst jeweils mit ggf. 1, 2, 3, 4, oder 5 Substituenten unabhängig voneinander ausgewählt aus der Gruppe bestehend aus F, Cl, Br, I, - CN, -NO₂, -OH, -SH, -NH₂, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ und -S-CH₂F substituiert sein können oder -NH-C(=O)-R⁵ steht;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ferner bevorzugt sind 1,3-disubstituierte 4-Methyl-1 H-pyrrol-2-carbonsäureamide der vorstehend angegebenen allgemeinen Formel I, worin
R² für H;
C₁₋₆-Alkyl, C₂₋₆-Alkenyl oder C₂₋₆-Alkinyl, das jeweils unsubstituiert oder mit ggf. 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, -N(C₂H₅)₂, - N(CH₃)(C₂H₅), -C(=O)-OH, -C(=O)-O-CH₃, -C(=O)-O-C₂H₅, -C(=O)-O-C(CH₃)₃, - C(=O)-O-CH₂-Phenyl, -C(=O)-NH-Naphthyl, -N(CH₃)-Phenyl, -N(C₂H₅)-Phenyl, - N(C₂H₅)-(m-Toluyl), -N(C₂H₅)-(p-Toluyl), -N(CH₃)-(p-Toluyl) und -NH-C(=O)-O-C(CH₃)₃ substituiert ist;
oder für einen Rest ausgewählt aus der Gruppe bestehend aus Phenyl und Naphthyl steht, der jeweils über eine C₁₋₃-Alkylen-, C₂₋₃-Alkenylen- oder C₂₋₃-Alkinylen-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -C≡C-Si(CH₃)₃, -C≡C-Si(C₂H₅)₃, -CH₂-O-CH₃, -CH₂-O-C₂H₅, -NH₂, - S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇. -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-Phenyl, Pyrazolyl, -N(CH₃)₂, -N(C₂H₅)₂, - NH-CH₃, -NH-C₂H₅, -CH₂-O-C(=O)-Phenyl, -NH-S(=O)₂-CH₃, -C(=O)-H, -C(=O)-CH₃, -C(=O)-C₂H₅, -NH-C(=O)-CH₃, -NH-C(=O)-C₂H₅, -O-C(=O)-Phenyl, -C(=O)-NH₂, - C(=O)-NH-CH₃, -C(=O)-NH-C₂H₅, -C(=O)-NH-C(CH₃)₃, -C(=O)-N(C₂H₅)₂, -S(=O)-NH₂, [1.2.3]-Thiadiazolyl, Cyclopropyl, Cyclobutyl, Cyclopentyl, Cyclohexyl, Piperazinyl, Pyrrolidinyl, Piperidinyl, Phenyl, Furyl (Furanyl), Thiadiazolyl, Thiophenyl (Thienyl) und Benzyl substituiert ist, wobei die zyklischen Substituenten bzw. die zyklischen Reste dieser Substituenten selbst jeweils mit ggf. 1, 2, 3, 4, oder 5 Substituenten unabhängig voneinander ausgewählt aus der Gruppe bestehend aus F, Cl, Br, I, -CN, -NO₂, -OH, -SH, -NH₂, Methyl, Ethyl, n-Propyl, Isopropyl, n-Butyl, Isobutyl, 2-Butyl, tert-Butyl, n-Pentyl, neo-Pentyl, -O-CH₃, -O-C₂H₅, -O-C₃H₇, -O-C(CH₃)₃, -CF₃, -CHF₂, -CH₂F, -O-CF₃, -O-CHF₂ und -O-CH₂F substituiert sein können;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind 1,3-disubstituierte 4-Methyl-1H-pyrrol-2-carbonsäureamide der vorstehend angegebenen allgemeinen Formel I, worin
R¹ und R² zusammen mit dem sie verbindenden Stickstoffatom einen Rest ausgewählt aus der Gruppe bestehend aus Imidazolidinyl, [1,3,4,9]-Tetrahydro-b-carbolinyl, Piperidinyl, Morpholinyl, Thiomorpholinyl, Pyrrolidinyl, Piperazinyl, Azepanyl, Diazepanyl und (1,4)-Dioxo-8-aza-spiro[4.5]decyl bilden, der jeweils unsubstituiert oder mit 1 oder 2 Resten R⁶ substituiert ist;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind 1,3-disubstituierte 4-Methyl-1 H-pyrrol-2-carbonsäureamide der vorstehend angegebenen allgemeinen Formel I, worin
R³ für C₁₋₆-Alkyl, C₂₋₆-Alkenyl oder C₂₋₆-Alkinyl, das jeweils unsubstituiert oder mit ggf. 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, -N(C₂H₅)₂, - N(CH₃)(C₂H₅), -C(=O)-OH, -C(=O)-O-CH₃, -C(=O)-O-C₂H₅, -C(=O)-O-C(CH₃)₃, - C(=O)-O-CH₂-Phenyl, -C(=O)-NH-Naphthyl, -N(CH₃)-Phenyl, -N(C₂H₅)-Phenyl, - N(C₂H₅)-(m-Toluyl), -N(C₂H₅)-(p-Toluyl), -N(CH3(-(p-Toluyl) und -NH-C(=O)-O-C(CH₃)₃ substituiert ist;
oder einen Rest ausgewählt aus der Gruppe bestehend aus Phenyl, Naphthyl, Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Indazolyl, Chinoxalinyl, Chinazolinyl, Chinolinyl, Naphthridinyl und Isochinolinyl stoht, der jeweils über eine C₁₋₃-Alkylen-, C₂₋₃-Alkenylen- oder C₂₋₃-Alkinylen-Gruppe gebunden ist und/oder jeweils unsubstituiert oder mit ggf. 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -CH₂-O-CH₃, -CH₂-O-C₂H₅, -NH₂, -C(=O)-OH, -S-CH₃, -S-C₂H₅, - S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-H, -C(=O)-CH₃, - C(=O)-C₂H₅, -C(=O)-NH₂, -C(=O)-NH-CH₃ und -C(=O)-N(CH₃)₂ substituiert ist;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind 1,3-disubstituierte 4-Methyl-1 H-pyrrol-2-carbonsäureamide der vorstehend angegebenen allgemeinen Formel I, worin
R⁴ für Phenyl, das jeweils unsubstituiert oder mit 1, 2, 3, 4 oder 5 Substituenten unabhängig voneinander ausgewählt aus der Gruppe bestehend aus F, Cl, Br, I, - CN, -CH₂-CN, -OH, -SH, -NH₂, -C(=O)-OH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -C≡C-Si(CH₃)₃, -C≡C-Si(C₂H₅)₃, -S-CH₃, -S-C₂H₅, -S-Phenyl, -S-CH₂-Phenyl, -O-CH₃, -O-C₂H₅, -O-C₃H₇, -O-C(CH₃)₃, -O-Phenyl, -O-CH₂-Phenyl, -CF₃, - CHF₂, -CH₂F, -O-CF₃, -O-CHF₂, -O-CH₂F, -C(=O)-CF₃, -S-CF₃, -S-CHF₂, -S-CH₂F, - S(=O)₂-Phenyl, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -S(=O)₂-NH₂, -S(=O)₂-NH-Phenyl, [1,2,3]-Thiadiazolyl, Cyclopropyl, Cyclobutyl, Cyclopentyl, Cyclohexyl, Phenyl, Benzyl und Phenethyl substituiert ist, wobei die zyklischen Substituenten bzw. die zyklischen Reste dieser Substituenten selbst jeweils unsubstituiert oder mit ggf. 1, 2, 3, 4 oder 5, Substituenten unabhängig voneinander ausgewählt aus der Gruppe bestehend aus F, Cl, Br, I, -CN, -NO₂, -OH, -SH, -NH₂, -C(=O)-OH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ und -S-CH₂F substituiert sein können;
oder für einen Rest ausgewählt aus der Gruppe bestehend aus Thienyl, Furyl, Pyrrolyl, Pyrazolyl, Pyrazinyl, Pyranyl, Triazolyl, Pyridinyl, Imidazolyl, Thiadiazolyl, Oxadiazolyl und Pyridazinyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, -NO₂, -OH, -CH₂-CN, -SH, -NH₂, -C(=O)-OH. Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -S-CH₃, -S-C₂H₅, -S-Phenyl, -S-CH₂-Phenyl, -O-CH₃, -O-C₂H₅, -O-C₃H₇, -O-C(CH₃)₃, -O-Phenyl, -O-CH₂-Phenyl, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, - N(C₂H₅)₂, -NH-CH₃, -NH-C₂H₅, -C(=O)-O-CH₃, -C(=O)-O-C₂H₅, -C(=O)-O-C(CH₃)₃, - C(=O)-H -C(=O)-CH₃, -C(=O)-C₂H₅, -NH-C(=O)-CH₃, -NH-C(=O)-C₂H₅, -C(=O)-NH₂, - C(=O)-NH-CH₃, -C(=O)-N(CH₃)₂, -S(=O)₂-NH₂, Cyclopropyl, Cyclobutyl, Cyclopentyl, Cyclohexyl, Phenyl und Benzyl substituiert ist, wobei die zyklischen Substituenten bzw. die zyklischen Reste dieser Substituenten selbst jeweils unsubstituiert oder mit ggf. 1, 2, 3, 4 oder 5, Substituenten unabhängig voneinander ausgewählt aus der Gruppe bestehend aus F, Cl, Br, I, -CN, -NO₂, -OH, -SH, -NH₂, -C(=O)-OH, Methyl, Ethyl, n-Propyl, Isopropyl, n-Butyl, Isobutyl, 2-Butyl, tert-Butyl, n-Pentyl, neo-Pentyl, - O-CH₃, -O=C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ und -S-CH₂F substituiert sein können;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind 1,3-disubstituierte 4-Methyl-1H-pyrrol-2-carbonsäureamide der vorstehend angegebenen allgemeinen Formel I, worin
R⁵ für einen Rest ausgewählt aus der Gruppe bestehend aus Phenyl, Naphthyl, Thienyl, Furyl, Pyrrolyl, Pyrazolyl, Pyrazinyl, Pyranyl, Triazolyl, Pyridinyl, Imidazolyl, Indolyl, Isoindolyl, Thiazolyl, Thiadiazolyl, Oxazolyl, Oxadiazolyl, Isoxazolyl, Pyridazinyl, Indazolyl, Chinoxalinyl, Chinazolinyl, Chinolinyl, Naphthridinyl und Isochinolinyl steht, der jeweils über eine C₁₋₃-Alkylen-, C₂₋₃-Alkenylen- oder C₂₋₃-Alkinylen-Gruppe gebunden sein kann und/oder jeweils unsubstituiert oder mit ggf. 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, - S-CF₃, -S-CHF₂, -S-CH₂F, -N(CH₃)₂, -N(C₂H₅)₂, -NH-CH₃, -NH-C₂H₅, -C(=O)-H, - C(=O)-CH₃, -C(=O)-C₂H₅, -C(=O)-NH₂, -C(=O)-NH-CH₃ und -C(=O)-N(CH₃)₂ substituiert ist;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind 1,3-disubstituierte 4-Methyl-1 H-pyrrol-2-carbonsäureamide der vorstehend angegebenen allgemeinen Formel I, worin
R⁶ für -OH; F; Cl; Br; I; -SH; -NO₂; -NH₂; -NH-C(=O)-O-R⁷; -C(=O)-O-R⁸; -C(=O)-R⁹;
C₁₋₆-Alkyl, C₂₋₆-Alkenyl oder C₂₋₆-Alkinyl, das jeweils unsubstituiert oder mit ggf. 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, -N(C₂H₅)₂, -C(=O)-Pyrrolidinyl, -C(=O)-N(CH₃)-Phenyl und -C(=O)-NH-CH(CH₃)₂, -N(CH₃)(C₂H₅), - C(=O)-OH, -C(=O)-O-CH₃, -C(=O)-O-C₂H₅ und -C(=OFO-C(CH₃)₃ substituiert ist;
C₃₋₇-Cycloalkyl, C₅₋₆-Cycloalkenyl, 5- bis 7-gliedriges Heterocycloalkyl oder 5- bis 7-gliedriges Heterocycloalkenyl, das jeweils unsubstituiert oder mit ggf. 1, 2, 3, 4 oder 5 Substituenten unabhängig voneinander ausgewählt aus der Gruppe bestehend aus F, Cl. Br, I, -CN, Methyl, Ethyl, n-Propyl, Isopropyl, n-Butyl, 2-Butyl, Isobutyl, tert-Butyl, -OH, Oxo, Thioxo, -O-CH₃, -O-C₂H₅, -O-C₃H₇, -O-Phenyl, -O-CH₂-Phenyl, - NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂H₅, -CH₂-Naphthyl, Benzyl und Phenyl substituiert ist und/oder jeweils über eine unsubstituierte C₁₋₃-Alkylen-, C₂₋₃-Alkenylen- oder C₂₋₃-Alkinylen-Gruppe gebunden sein kann und/oder jeweils 1 oder 2 Heteroatom(e) unabhängig voneinander ausgewählt aus der Gruppe bestehend aus Sauerstoff, Schwefel und Stickstoff (NH) als Ringglied(er) aufweisen kann;
oder für einen Rest ausgewählt aus der Gruppe bestehend aus Phenyl, Naphthyl, Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, lsoxazolyl, Pyridazinyl, Indazolyl, Chinoxalinyl, Chinazolinyl, Chinolinyl, Naphthridinyl, Thieno[2,3-dipyrimidinyl und Isochinolinyl steht, der jeweils über eine C₁₋₃-Alkylen-, C₂₋₃-Alkenylen- oder C₂₋₃-Alkinylen-Gruppe gebunden sein kann und/oder jeweils unsubstituiert oder mit ggf. 1, 2, 3, 4 oder 5 Substituenten unabhängig voneinander ausgewählt aus der Gruppe bestehend aus F, Cl, Br, I, -CN, -CH₂-CN, -NO₂, -OH, - SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, - NH-C₂H₅, -C(=O)-O-CH₃, -C(=O)-O-C₂H₅, -C(=O)-O-C(CH₃)₃, -C(=O)-H, -C(=O)-CH -C(=O)-C₂H₅, -NH-C(=O)-CH₃, -NH-C(=O)-C₂H₅, -C(=O)-NH₂, -C(=O)-NH-CH₃, - C(=O)-N(CH₃)₂, -C(=O)-O-CH(CH₃)₂, -C(=O)-O-(CH₂)₃-CH₃, -C(=O)-NH-C₂H₅, - C(=O)-NH-C(CH₃)₃, -C(=O)-N(C₂H₅)₂, Cyclopropyl, Cyclobutyl, Cyclopentyl, Cyclohexyl, Phenyl, Furyl (Furanyl) und Benzyl substituiert ist, wobei die zyklischen Substituenten bzw. die zyklischen Reste dieser Substituenten selbst jeweils mit ggf. 1, 2, 3, 4, oder 5 Substituenten unabhängig voneinander ausgewählt aus der Gruppe bestehend aus F, Cl, Br, I, -CN, -NO₂, -OH, -SH, -NH₂, Methyl, Ethyl, n-Propyl, Isopropyl, n-Butyl, Isobutyl, 2-Butyl, tert-Butyl, n-Pentyl, neo-Pentyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, - S-CF₃, -S-CHF₂ und -S-CH₂F substituiert sein können;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ferner bevorzugt sind 1,3-disubstituierte 4-Methyl-1 H-pyrrol-2-carbonsäureamide der vorstehend angegebenen allgemeinen Formel I, worin
R⁷, R⁸ und R⁹, unabhängig voneinander, jeweils für
C₁₋₆-Alkyl, C₂₋₆-Alkenyl oder C₂₋₆-Alkinyl, das jeweils unsubstituiert oder mit ggf. 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, -N(C₂H₅)₂, - N(CH₃)(C₂H₅), -C(=O)-OH, -C(=O)-O-CH₃, -C(=O)-O-C₂H₅ und -C(=O)-O-C(CH₃)₃ substituiert ist;
für einen Rest ausgewählt aus der Gruppe bestehend aus Indolinyl, Indanyl, (1,2,3,4)-Tetrahydronaphthyl, (2,3)-Dihydro-benzo[1.4]dioxinyl und Benzo[1.3]dioxolyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, -O-CH₃, -O-C₂H₅, -O-C₃H₇, -O-Phenyl, -O-CH₂-Phenyl,-NH₂, -N(CH₃)₂, -N(C₂H₅)₂. -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂H₅, -CH₂-Naphthyl, Benzyl und Phenyl substituiert ist;
oder einen Rest ausgewählt aus der Gruppe bestehend aus Phenyl, Naphthyl, Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Thiazolyl, Thiadiazolyl, Oxazolyl, Oxadiazolyl, Isoxazolyl und Pyridazinyl stehen, der jeweils über eine C₁₋₃-Alkylen-, C₂₋₃-Alkenylen- oder C₂₋₃-Alkinylen-Gruppe gebunden sein kann und/oder jeweils unsubstituiert oder mit ggf. 1, 2, 3, 4 oder 5 Substituenten unabhängig voneinander ausgewählt aus der Gruppe bestehend aus F, Cl, Br, I, -CN, -CH₂-CN, -NO₂, -OH, - SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, - NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, - C(=O)-N(CH₃)₂, -C(=O)-O-CH(CH₃)₂, -C(=O)-O-(CH₂)₃-CH₃, -C(=O)-NH-C₂H₅, - C(=O)-NH-C(CH₃)₃. -C(=O)-N(C₂H₅)₂, Cyclopropyl, Cyclobutyl, Cyclopentyl, Cyclohexyl, Phenyl, Furyl (Furanyl) und Benzyl substituiert ist;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind 1,3-disubstituierte 4-Methyl-1 H-pyrrol-2-carbonsäureamide der vorstehend angegebenen allgemeinen Formel I, worin
R¹ für C₁₋₆-Alkyl, C₂₋₆-Alkenyl oder C₂₋₆-Alkinyl, das jeweils unsubstituiert oder mit ggf. 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, -N(C₂H₅)₂, - N(CH₃)(C₂H₅), -C(=O)-OH, -C(=O)-O-CH₃, -C(=O)-O-C₂H₅, -C(=O)-O-C(CH₃)₃, - C(=O)-O-CH₂-Phenyl, -C(=O)-NH-Naphthyl, -N(CH₃)-Phenyl, -N(C₂H₅)-Phenyl, - N(C₂H₅)-(m-Toluyl), -N(C₂H₅)-(p-Toluyl), -N(CH₃)-(p-Toluyl) und -NH-C(=O)-O-C(CH₃)₃ substituiert ist;
2- bis 6-gliedriges Heteroalkyl, das jeweils unsubstituiert oder mit ggf. 1, 2, 3, 4 oder 5 Substituenten unabhängig voneinander ausgewählt aus der Gruppe bestehend aus F, Cl, Br, I, -NO₂, -CN, -OH, -SH und -NH₂ substituiert ist und jeweils 1 oder 2 Heteroatom(e) unabhängig voneinander ausgewählt aus der Gruppe bestehend aus Sauerstoff, Schwefel und Stickstoff (NH) als Kettenglied(er) aufweist;
C₃₋₇-Cycloalkyl, C₅₋₆-Cycloalkenyl, 5- bis 7-gliedriges Heterocycloalkyl, 5- bis 7-gliedriges Heterocycloalkenyl, (1,2,3,4)-Tetrahydrochinolinyl, (1,2,3,4)-Tetrahydroisochinolinyl, (2,3)-Dihydro-1H-isoindotyl, Indolinyl, Indanyl, (1,2,3,4)-Tetrahydronaphthyl, (2,3)-Dihydro-benzo[1.4]dioxinyl oder Benzo[1.3]dioxolyl, das jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, -O-CH₃, -O-C₂H₅, -O-C₃H₇, -O-Phenyl, -O-CH₂-Phenyl, -NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, - C(=O)-C₂H₅, -C(=O)-C(CH₃)₃. -C(=O)-NH-CH₃, -C(=O)-NH-C₂H₅, -CH₂-Naphthyl, Benzyl und Phenyl substituiert ist und/oder jeweils über eine unsubstituierte C₁₋₃-Alkylen-, C₂₋₃-Alkenylen- oder C₂₋₃-Alkinylen-Gruppe gebunden sein kann und/oder jeweils 1 oder 2 Heteroatom(e) unabhängig voneinander ausgewählt aus der Gruppe bestehend aus Sauerstoff, Schwefel und Stickstoff (NH) als Ringglied(er) aufweisen kann;
Phenyl, das jeweils über eine C₁₋₃-Alkylen-, C₂₋₃-Alkenylen- oder C₂₋₃-Alkinylen-Gruppe, die jeweils unsubstituiert oder mit 1 oder 2 Substituenten ausgewählt aus der Gruppe bestehend aus F, C, Br, -C(=O)-O-CH₃ und -C(=O)-O-C₂H₅ substituiert ist, oder über eine -CH₂-CH₂-O-, -CH₂-O- oder -CH₂-CH₂-CH₂-O-Gruppe gebunden sein kann und/oder unsubstituiert oder mit 1, 2, 3, 4 oder 5 Substituenten unabhängig voneinander ausgewählt aus der Gruppe bestehend aus F, Cl, Br, I, - CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, - C≡C-Si(CH₃)₃, -C≡C-Si(C₂H₅)₃, -CH₂-O-CH₃, -CH₂-O-C₂H₅, -NH₂, -S-CH₃, -S-C₂H₅, - S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF3, -S-CF₃, -S-CHF₂, -S-CH₂F, -S(=O)₂-Phenyl, Pyrazolyl, -N(CH₃)₂, -N(C₂H₅)₂, -NH-CH₃, -NH-C₂H₅, -CH₂-O-C(=O)-Phenyl, -NH-S(=O)₂-CH₃, -C(=O)-H, -C(=O)-CH₃, -C(=O)-C₂H₅, -NH-C(=O)-CH₃, -NH-C(=O)-C₂H₅, -O-C(=O)-Phenyl, -C(=O)-NH₂, -C(=O)-NH-CH₃, - C(=O)-NH-C₂H₅, -C(=O)-NH-C(CH₃)₃, -C(=O)-N(C₂H₅)2, -S(=O)-NH₂. [1.2.3]-Thiadiazolyl, Cyclopropyl, Cyclobutyl, Cyclopentyl, Cyclohexyl, Piperazinyl, Pyrrolidinyl, Piperidinyl, Phenyl, Furyl (Furanyl), Thiadiazolyl, Thiophenyl (Thienyl) und Benzyl substituiert ist, wobei die zyklischen Substituenten bzw. die zyklischen Reste dieser Substituenten selbst jeweils mit ggf. 1, 2, 3, 4, oder 5 Substituenten unabhängig voneinander ausgewählt aus der Gruppe bestehend aus F, Cl, Br, I, - CN, -NO₂, -OH, -SH, -NH₂, Methyl, Ethyl, n-Propyl, Isopropyl, n-Butyl, Isobutyl, 2-Butyl, tert-Butyl, n-Pentyl, neo-Pentyl, -O-CH₃, -O-C₂H₅, -O-C₃H₇, -O-C(CH₃)₃, -CF₃, - CHF₂, -CH₂F, -O-CF₃, -O-CHF₂ und -O-CH₂F substituiert sein können;
einen Rest ausgewählt aus der Gruppe bestehend aus Naphthyl, Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Indazolyl, Chinoxalinyl, Chinazolinyl, Chinolinyl, Naphthridinyl und Isochinolinyl, der jeweils über eine C₁₋₃-Alkylen-, C₂₋₃-Alkenylen- oder C₂₋₃-Alkinylen-Gruppe gebunden sein kann und/oder jeweils unsubstituiert oder mit ggf. 1, 2, 3, 4 oder 5 Substituenten unabhängig voneinander ausgewählt aus der Gruppe bestehend aus F, Cl, Br, I, - CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, C≡C-Si(CH₃)₃, -C≡C-Si(C₂H₅)₃, -CH₂-O-CH₃, -CH₂-O-C₂H₅, -NH₂, -C(=O)-OH. -S-CH₃, -S-C₂H₅, -S(=O)-CH₃, -S(₌O)₂-CH₃, -S(=O)-C₂H_{5,} -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F. -S(=O)₂-Phenyl, Pyrazolyl, -N(CH₃)₂, -N(C₂H₅)₂, - NH-CH₃, -NH-C₂H₅, -CH₂-O-C(=O)-Phenyl, -NH-S(=O)₂-CH₃, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, - NH-C(=O)-C₂H₅, -O-C(=O)-Phenyl, -C(=O)-NH₂, -C(=O)-NH-CH₃, -C(=O)-N(CH₃)₂, - C(=O)-O-CH(CH₃)₂, -C(=O)-O-(CH₂)₃-CH₃, -C(=O)-O-Phenyl, -O-C(=O)-CH₃, -O-C(=O)-C₂H₅, -C(=O)-NH-C₂H₅, -C(=O)-NH-C(CH₃)₃, -C(=O)-N(C₂H₅)₂, -C(=O)-NH-Phenyl, -C(=O)-N(CH₃)-Phenyl, -C(=O)-N(C₂H₅)-Phenyl, -S(=O)-NH₂, [1.2.3]-Thiadiazolyl, Cyclopropyl, Cyclobutyl, Cyclopentyl, Cyclohexyl, Piperazinyl, Pyrrolidinyl, Piperidinyl, Phenyl, Furyl (Furanyl), Thiadiazolyl, Thiophenyl (Thienyl) und Benzyl substituiert ist, wobei die zyklischen Substituenten bzw. die zyklischen Reste dieser Substituenten selbst jeweils mit ggf. 1, 2, 3, 4, oder 5 Substituenten unabhängig voneinander ausgewählt aus der Gruppe bestehend aus F, Cl, Br, I, - CN, -NO₂, -OH, -SH, -NH₂, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ und -S-CH₂F substituiert sein können
oder -NH-C(=O)-R⁵ steht;
R² für H;
C₁₋₆-Alkyl, das jeweils unsubstituiert oder mit ggf. 1, 2, 3, 4 oder 5 Substituenten unabhängig voneinander ausgewählt aus der Gruppe bestehend aus F, Cl, Br, I, - NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O-C₂H₅, -C(=O)-O-C(CH₃)₃, -C(=O)-O-CH₂-Phenyl, -C(=O)-NH-Naphthyl, -N(CH₃)-Phenyl, -N(C₂H₅)-Phenyl, -N(C₂H₅)-(m-Toluyl), -N(C₂H₅)-(p-Toluyl), -N(CH₃)-(p-Toluyl) und -NH-C(=O)-O-C(CH₃)₃ substituiert ist;
oder für einen Rest ausgewählt aus der Gruppe bestehend aus Phenyl und Naphthyl steht, der jeweils über eine C₁₋₃-Alkylen-, C₂₋₃-Alkenylen- oder C₂₋₃-Alkinylen-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl,_{.} Propinyl, -C≡C-Si(CH₃)₃, -C≡C-Si(C₂H₅)₃, -CH₂-O-CH₃, -CH₂-O-C₂H₅, -NH₂, - S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃ und -NH-C₂H₅ substituiert ist;
oder R¹ und R² zusammen mit dem sie verbindenden Stickstoffatom einen Rest ausgewählt aus der Gruppe bestehend aus 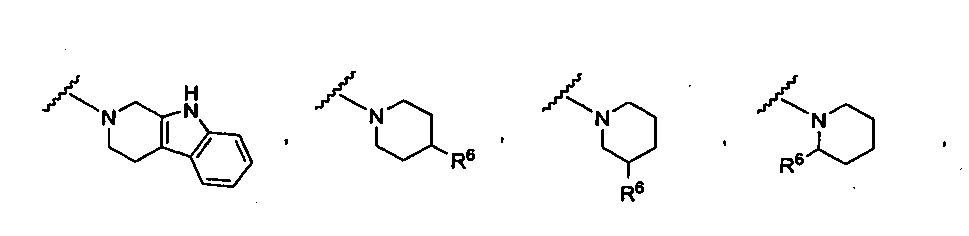 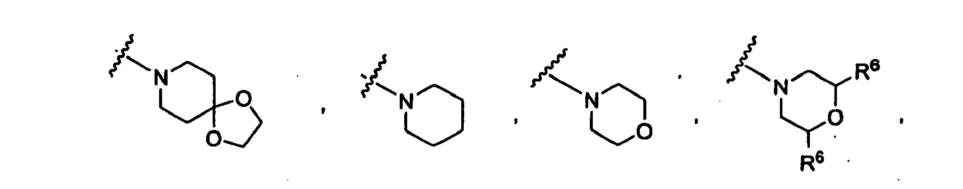 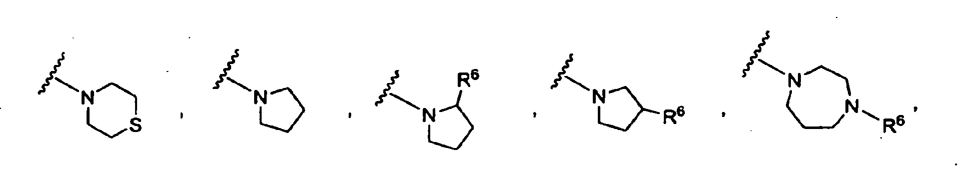 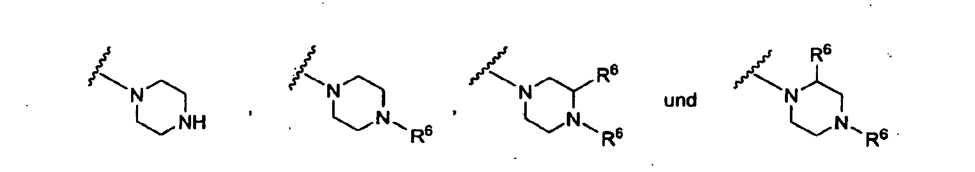 bilden;
R³ für C₁₋₆-Alkyl, das jeweils unsubstituiert oder mit ggf. 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O-C₂H₅ und -C(=O)-O-C(CH₃)₃ substituiert ist;
oder einen Rest ausgewählt aus der Gruppe bestehend aus Phenyl, Isoxazolyl, Thienyl, Furyl, Pyrrolyl, Pyrazolyl, Pyrazinyl, Pyranyl, Triazolyl, Pyridinyl, Imidazolyl, Indolyl, Isoindolyl, Thiazolyl, Thiadiazolyl, Oxazolyl, Oxadiazolyl, Pyridazinyl und Isochinolinyl steht, der jeweils über eine C₁₋₃-Alkylen-, C₂₋₃-Alkenylen- oder C₂₋₃-Alkinylen-Gruppe gebunden ist und/oder jeweils unsubstituiert oder mit ggf. 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -CH₂-O-CH₃, -CH₂-O-C₂H₅, -NH₂, -C(=O)-OH, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂. -N(C₂H₅)₂, -NH-CH₃, -NH-C₂H₅. -C(=O)-H, -C(=O)-CH₃, -C(=O)-C₂H₅, -C(=O)-NH₂, -C(=O)-NH-CH₃ und -C(=O)-N(CH₃)₂ substituiert ist;
R⁴ für Phenyl, das jeweils unsubstituiert oder mit 1, 2, 3, 4 oder 5 Substituenten unabhängig voneinander ausgewählt aus der Gruppe bestehend aus F, Cl, Br, I, - CN, -CH₂-CN, -OH, -SH, -NH₂, -C(=O)-OH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -C≡C-Si(CH₃)₃, -C≡C-Si(C₂H₅)₃, -S-CH₃, -S-C₂H₅, -S-Phenyl, -S-CH₂-Phenyl, -O-CH₃, -O-C₂H₅, -O-C₃H₇, -O-C(CH₃)₃, -O-Phenyl, -O-CH₂-Phenyl, -CF₃, - CHF₂, -CH₂F, -O-CF₃, -O-CHF₂, -O-CH₂F, -C(=O)-CF₃, -S-CF₃, -S-CHF₂, -S-CH₂F, - S(=O)₂-Phenyl, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -S(=O)₂-NH₂, -S(=O)₂-NH-Phenyl, [1,2,3]-Thiadiazolyl, Cyclopropyl, Cyclobutyl, Cyclopentyl, Cyclohexyl, Phenyl, Benzyl und Phenethyl substituiert ist, wobei die zyklischen Substituenten bzw. die zyklischen Reste dieser Substituenten selbst jeweils unsubstituiert oder mit ggf. 1, 2, 3, 4 oder 5, Substituenten unabhängig voneinander ausgewählt aus der Gruppe bestehend aus F, Cl, Br, I, -CN, -NO₂, -OH, -SH, -NH₂, -C(=O)-OH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ und -S-CH₂F substituiert sein können;
oder für einen Rest ausgewählt aus der Gruppe bestehend aus Thienyl, Furyl, Pyrrolyl, Pyrazolyl, Pyrazinyl, Pyranyl, Triazolyl, Pyridinyl, Imidazolyl, Thiadiazolyl, Oxadiazolyl und Pyridazinyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, -NO₂, -OH, -CH₂-CN, -SH, -NH₂, -C(=O)-OH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -S-CH₃, -S-C₂H₅, -S-Phenyl, -S-CH₂-Phenyl, -O-CH₃, -O-C₂H₅, -O-C₃H₇, -O-C(CH₃)₃, -O-Phenyl, -O-CH₂-Phenyl, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂,-N(C₂H₅)₂, -NH-CH₃, -NH-C₂H₅, -C(=O)-O-CH₃, -C(=O)-O-C₂H₅, -C(=O)-O-C(CH₃)₃, - C(=O)-H -C(=O)-CH₃, -C(=O)-C₂H₅, -NH-C(=O)-CH₃, -NH-C(=O)-C₂H₅, -C(=O)-NH₂, - C(=O)-NH-CH₃, -C(=O)-N(CH₃)₂, -S(=O)₂-NH₂, Cyclopropyl, Cyclobutyl, Cyclopentyl, Cyclohexyl, Phenyl und Benzyl substituiert ist, wobei die zyklischen Substituenten bzw. die zyklischen Reste dieser Substituenten selbst jeweils unsubstituiert oder mit ggf. 1, 2, 3, 4 oder 5, Substituenten unabhängig voneinander ausgewählt aus der Gruppe bestehend aus F, Cl, Br, I, -CN, -NO₂, -OH, -SH, -NH₂, -C(=O)-OH. Methyl, Ethyl, n-Propyl, Isopropyl, n-Butyl, Isobutyl, 2-Butyl, tert-Butyl, n-Pentyl, neo-Pentyl, - O-CH₃, -O-C₂H₅. -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ und -S-CH₂F substituiert sein können;
R⁵ für einen Rest ausgewählt aus der Gruppe bestehend aus Phenyl, Naphthyl, Thienyl, Furyl, Pyrrolyl, Pyrazolyl, Pyrazinyl, Pyranyl, Triazolyl, Pyridinyl, Imidazolyl, Indolyl, Isoindolyl, Thiazolyl, Thiadiazolyl, Oxazolyl, Oxadiazolyl, Isoxazolyl und Pyridazinyl steht, der jeweils über eine C₁₋₃-Alkylen-, C₂₋₃-Alkenylen-oder C₂₋₃-Alkinylen-Gruppe gebunden sein kann und/oder jeweils unsubstituiert oder mit ggf. 1, 2, 3, 4 oder 5 Substituenten unabhängig voneinander ausgewählt aus der Gruppe bestehend aus F, Cl, Br, I, -CN, -CH₂-CN, -NO₂, -OH, -SH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -NH₂, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃ und -NH-C₂H₅ substituiert ist;
R⁶ für -OH; F; Cl; Br; I; -SH; -NO₂; -NH₂; -NH-C(=O)-O-R⁷; -C(=O)-O-R⁸; -C(=O)-R⁹;
C₁₋₆-Alkyl, C₂₋₆-Alkenyl oder C₂₋₆-Alkinyl, das jeweils unsubstituiert oder mit ggf. 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, -C(=O)-Pyrrolidinyl, -C(=O)-N(CH₃)-Phenyl und -C(=O)-NH-CH(CH₃)₂, -N(C₂H₅)₂, -N(CH₃)(C₂H₅), -C(=O)-OH, -C(=O)-O-CH₃, -C(=O)-O-C₂H₅ und -C(=O)-O-C(CH₃)₃ substituiert ist;
C₃₋₇-Cycloalkyl, C₅₋₆-Cycloalkenyl, 5- bis 7-gliedriges Heterocycloalkyl oder 5- bis 7-gliedriges Heterocycloalkenyl, das jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, -O-CH₃. -O-C₂H₅, -O-C₃H₇, -O-Phenyl, -O-CH₂-Phenyl,-NH₂, -N(CH₃)₂, -N(C2H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂H₅, -CH₂-Naphthyl, Benzyl und Phenyl substituiert ist und/oder jeweils über eine unsubstituierte C₁₋₃-Alkylen-, C₂₋₃-Alkenylen- oder C₂₋₃-Alkinylen-Gruppe gebunden sein kann und/oder jeweils 1 oder 2 Heteroatom(e) unabhängig voneinander ausgewählt aus der Gruppe bestehend aus Sauerstoff, Schwefel und Stickstoff (NH) als Ringglied(er) aufweisen kann;
oder für einen Rest ausgewählt aus der Gruppe bestehend aus Phenyl, Naphthyl, Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Indazolyl, Chinoxalinyl, Chinazolinyl, Chinolinyl, Naphthridinyl, Thieno[2,3-d]pyrimidinyl und Isochinolinyl steht, der jeweils über eine C₁₋₃-Alkylen-, C₂₋₃-Alkenylen- oder C₂₋₃-Alkinylen-Gruppe gebunden sein kann und/oder jeweils unsubstituiert oder mit ggf. 1, 2, 3, 4 oder 5 Substituenten unabhängig voneinander ausgewählt aus der Gruppe bestehend aus F, Cl, Br, I, -CN, -CH₂-CN, -NO₂, -OH, - SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, - NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, - C(=O)-N(CH₃)₂, -C(=O)-O-CH(CH₃)₂, -C(=O)-O-(CH₂)₃-CH₃, -C(=O)-NH-C₂H₅, - C(=O)-NH-C(CH₃)₃. -C(=O)-N(C₂H₅)₂, Cyclopropyl, Cyclobutyl, Cyclopentyl, Cyclohexyl, Phenyl, Furyl (Furanyl) und Benzyl substituiert ist, wobei die zyklischen Substituenten bzw. die zyklischen Reste dieser Substituenten selbst jeweils mit ggf. 1, 2, 3, 4, oder 5 Substituenten unabhängig voneinander ausgewählt aus der Gruppe bestehend aus F, Cl, Br, I, -CN, -NO₂, -OH, -SH, -NH₂, Methyl, Ethyl, n-Propyl, Isopropyl, n-Butyl, Isobutyl, 2-Butyl, tert-Butyl, n-Pentyl, neo-Pentyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, - S-CF₃, -S-CHF₂ und -S-CH₂F substituiert sein können
und R⁷, R⁸ und R⁹, unabhängig voneinander, jeweils für
C₁₋₆-Alkyl, C₂₋₆-Alkenyl oder C₂₋₆-Alkinyl, das jeweils unsubstituiert oder mit ggf. 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, -N(C₂H₅)₂, - N(CH₃)(C₂H₅), -C(=O)-OH, -C(=O)-O-CH₃, -C(=O)-O-C₂H₅ und -C(=O)-O-C(CH₃)₃ substituiert ist;
für einen Rest ausgewählt aus der Gruppe bestehend aus Indolinyl, Indanyl, (1,2,3,4)-Tetrahydronaphthyl, (2,3)-Dihydro-benzo[1.4]dioxinyl und Benzo[1.3]dioxolyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, -O-CH₃, -O-C₂H₅, -O-C₃H₇, -O-Phenyl, -O-CH₂-Phenyl, - NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂H₅, -CH₂-Naphthyl, Benzyl und Phenyl substituiert ist;
oder einen Rest ausgewählt aus der Gruppe bestehend aus Phenyl, Naphthyl, Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Thiazolyl, Thiadiazolyl, Oxazolyl, Oxadiazolyl, Isoxazolyl und Pyridazinyl stehen, der jeweils über eine C₁₋₃-Alkylen-, C₂₋₃-Alkenylen- oder C₂₋₃-Alkinylen-Gruppe gebunden sein kann und/oder jeweils unsubstituiert oder mit ggf. 1, 2, 3, 4 oder 5 Substituenten unabhängig voneinander ausgewählt aus der Gruppe bestehend aus F, Cl, Br, I, -CN, -CH₂-CN, -NO₂, -OH, - SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, - NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, - C(=O)-N(CH₃)₂, -C(=O)-O-CH(CH₃)₂, -C(=O)-O-CH₂)₃-CH₃, -C(=O)-NH-C₂H₅, - C(=O)-NH-C(CH₃)₃, -C(=O)-N(C₂H₅)₂, Cyclopropyl, Cyclobutyl, Cyclopentyl, Cyclohexyl, Phenyl, Furyl (Furanyl) und Benzyl substituiert ist;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Ebenfalls besonders bevorzugt sind 1,3-disubstituierte 4-Methyl-1 H-pyrrol-2-carbonsäureamide der vorstehend angegebenen allgemeinen Formel I, worin
R¹ für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert oder mit ggf. 1, 2, 3, 4 oder 5 Substituenten unabhängig voneinander ausgewählt aus der Gruppe bestehend aus F, Cl, Br, I, -CN, -OH, -SH, -NH₂, -N(CH₃)₂, -N(C₂H₅)₂, - N(CH₃)(C₂H₅), -C(=O)-O-CH₃, -C(=O)-O-C₂H₅, -C(=O)-O-C(CH₃)₃, -C(=O)-O-CH₂-Phenyl, -C(=O)-NH-Naphthyl, -N(CH₃)-Phenyl, -N(C₂H₅)-Phenyl, -N(C₂H₅)-(m-Toluyl), -N(C₂H₅)-(p-Toluyl), -N(CH₃)-(p-Toluyl) und -NH-C(=O)-O-C(CH₃)₃ substituiert ist;
einen Heteroalkyl-Rest ausgewählt aus der Gruppe bestehend aus -CH₂-O-CH₃, - CH₂-O-C₂H₅, -CH₂-CH₂-O-CH₃, -CH₂-CH₂-O-C₂H₅, -CH₂-CH₂-CH₂-O-CH₃ und -CH₂-CH₂-CH₂-O-C₂H₅, der jeweils unsubstituiert oder mit ggf. 1, 2, 3, 4 oder 5 Substituenten unabhängig voneinander ausgewählt aus der Gruppe bestehend aus F, Cl und Br substituiert ist;
für einen Rest ausgewählt aus der Gruppe bestehend aus Cyclopropyl, Cyclobutyl, Cyclopentyl, Cyclohexyl, Cycloheptyl, Cyclohexenyl, Cyclopentenyl, Pyrrolidinyl, Tetrahydrothiophenyl, Tetrahydrofuranyl, Piperidinyl, Morpholinyl, Piperazinyl, Azepanyl, Diazepanyl, (1,2,3,4)-Tetrahydrochinolinyl, (1,2,3,4)-Tetrahydroisochinolinyl, (2,3)-Dihydro-1H-isoindolyl, Indolinyl, Indanyl, (1,2,3,4)-Tetrahydronaphthyl, (2,3)-Dihydro-benzo[1.4]dioxinyl und Benzo[1.3]dioxolyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, -O-CH₃, -O-C₂H₅, -O-C₃H₇, -O-Phenyl, -O-CH₂-Phenyl, -C(=O)-CH₃, -C(=O)-C₂H₅, -C(=O)-C(CH₃)₃, -C(=O)-NH-CH₃, -C(=O)-NH-C₂H₅, -CH₂-Naphthyl, Benzyl und Phenyl substituiert ist und/oder jeweils über eine -CH₂-, -CH(CH₃)-, -CH₂-CH₂-, -CH(CH₃-CH₂-, -CH₂-CH(CH₃)- oder -CH₂-CH₂-CH₂-Gruppe gebunden sein kann;
Phenyl, das jeweils über eine -CH₂-CH₂-O-, -CH₂-O-, -CH₂-CH₂-CH₂-O-, -CH[C(=O)-O-CH₃]-CH₂-, -CH[C(=O)-O-C₂H₅]-CH₂-, -CH₂-, -CH(CH₃)-, -CH₂-CH₂-, -CH(CH₃)-CH₂-, -CH₂-CH(CH₃)- oder -CH₂-CH₂-CH₂-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, - NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -NH₂, -S-CH₃, - S-C₂H₅ -O-CH₃, -O-C₂H₅, -O-C₃H₇, -O-C(CH₃)₃, -CF₃, -CHF₂, -CH₂F, -O-CF₃, -O-CHF₂, -O-CH₂F, -N(CH₃)₂, -N(C₂H₅)₂, -NH-CH₃, -NH-C₂H₅, -NH-S(=O)₂-CH₃, -C(=O)-H, -C(=O)-CH₃, -C(=O)-C₂H₅, -NH-C(=O)-CH₃, -NH-C(=O)-C₂H₅, -S(=O)-NH₂, [1.2.3]-Thiadiazolyl, Cyclopropyl, Cyclobutyl, Cyclopentyl, Cyclohexyl, Phenyl, Furyl (Furanyl), Thiadiazolyl, Thiophenyl (Thienyl) und Benzyl substituiert ist, wobei die zyklischen Substituenten bzw. die zyklischen Reste dieser Substituenten selbst jeweils mit ggf. 1, 2, 3, 4, oder 5 Substituenten unabhängig voneinander ausgewählt aus der Gruppe bestehend aus F, Cl, Br, I, -CN, -NO₂, Methyl, Ethyl, n-Propyl, Isopropyl, n-Butyl, Isobutyl, 2-Butyl, tert-Butyl, -O-CH₃, -O-C₂H₅, -O-C₃H₇ und -O-C(CH₃)₃ substituiert sein können;
einen Rest ausgewählt aus der Gruppe bestehend aus Naphthyl, Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Indazolyl, Chinoxalinyl, Chinazolinyl, Chinolinyl, Naphthridinyl und Isochinolinyl, der jeweils über -CH₂-, -CH(CH₃)-, -CH₂-CH₂-, -CH(CH₃)-CH₂-, -CH₂-CH(CH₃)- oder -CH₂-CH₂-CH₂-Gruppe gebunden sein kann und/oder jeweils unsubstituiert oder mit ggf. 1, 2, 3, 4 oder 5 Substituenten unabhängig voneinander ausgewählt aus der Gruppe bestehend aus F, Cl, Br, I, -CN, -CH₂-CN, -NO₂, -OH. -SH, -O-Phenyl, Methyl, Ethyl, n-Propyl, Isopropyl, n-Butyl, Isobutyl, 2-Butyl, tert-Butyl, n-Pentyl, neo-Pentyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -C(=O)-NH₂, -C(=O)-NH-CH₃, -C(=O)-N(CH₃)₂, -C(=O)-O-CH(CH₃)₂ und -C(=O)-O-(CH₂)₃-CH₃, substituiert ist
oder -NH-C(=O)-R⁵ steht;
R² für H;
einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert ist;
oder für einen Rest ausgewählt aus der Gruppe bestehend aus Phenyl und Naphthyl steht, der jeweils über eine -CH₂-, -CH(CH₃)-, -CH₂-CH₂-, -CH(CH₃)-CH₂-, -CH₂-CH(CH₃)- oder -CH₂-CH₂-CH₂-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, Isobutyl, 2-Butyl, tert-Butyl, n-Pentyl, neo-Pentyl, -O-CH₃, -O-C₂H₅, -O-C₃H₇, -O-C(CH₃)₃, -CF₃, -CHF₂ und -CH₂F substituiert ist;
oder R¹ und R² zusammen mit dem sie verbindenden Stickstoffatom einen Rest ausgewählt aus der Gruppe bestehend aus 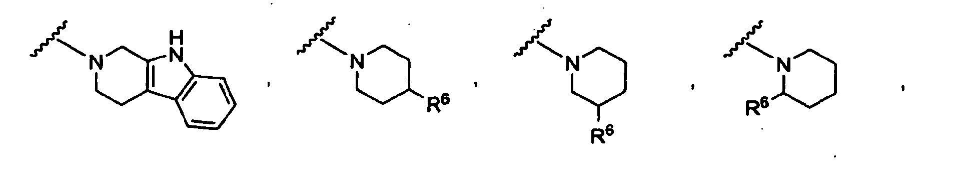 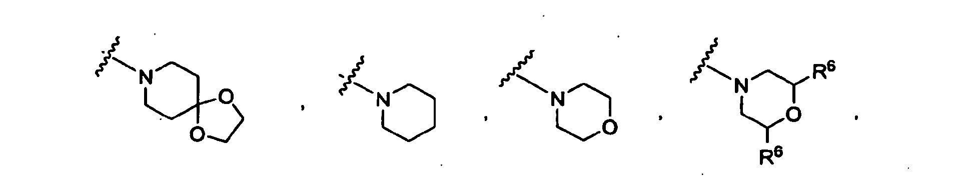 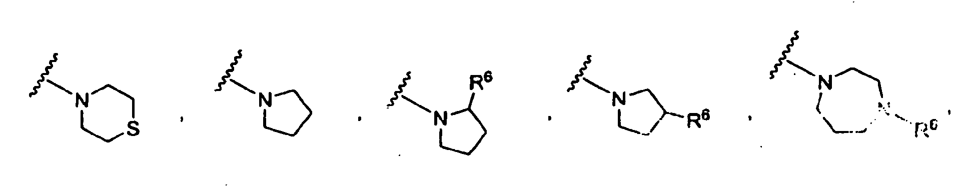 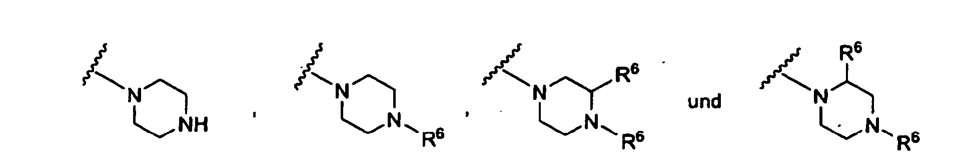 bilden;
R³ für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert ist;
oder für einen Phenyl-Rest steht, der jeweils über eine -CH₂-, -CH(CH₃)-, -CH₂-CH₂-, -CH(CH₃)-CH₂-, -CH₂-CH(CH₃)- oder -CH₂-CH₂-CH₂-Gruppe gebunden ist und/oder jeweils unsubstituiert oder mit ggf. 1, 2, 3, 4 oder 5 Substituenten unabhängig voneinander ausgewählt aus der Gruppe bestehend aus F, Cl, Br, I, -CN, -CH₂-CN, - NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -CH₂-O-CH₃, - CH₂-O-C₂H₅, -NH₂, -C(=O)-OH, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂ und -O-CH₂F substituiert ist;
R⁴ für Phenyl, das jeweils unsubstituiert oder mit 1, 2, 3, 4 oder 5 Substituenten unabhängig voneinander ausgewählt aus der Gruppe bestehend aus F, Cl, Br, I, - CN, -CH₂-CN, -OH, -SH, -NH₂, -C(=O)-OH, Methyl, Ethyl, n-Propyl, Isopropyl, n-Butyl, Isobutyl, 2-Butyl, tert-Butyl, n-Pentyl, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂ und -NH-CH₃ substituiert ist;
oder für einen Rest ausgewählt aus der Gruppe bestehend aus Thienyl, Furyl, Pyrrolyl, Pyrazolyl, Pyrazinyl, Pyranyl, Triazolyl, Pyridinyl, Imidazolyl, Thiadiazolyl Oxadiazolyl und Pyridazinyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, -NO₂, -OH, -CH₂-CN, -SH, -NH₂, -C(=O)-OH, Methyl, Ethyl, n-Propyl, Isopropyl, n-Butyl, Isobutyl, 2-Butyl, tert-Butyl, n-Pentyl, neo-Pentyl, - O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂ und -NH-CH₃ substituiert ist;
R⁵ für einen Rest ausgewählt aus der Gruppe bestehend aus Phenyl und Naphthyl steht, der jeweils unsubstituiert oder mit 1, 2, 3, 4 oder 5 Substituenten unabhängig voneinander ausgewählt aus der Gruppe bestehend aus F, Cl, Br, I, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -NH₂, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃ und -NH-C₂H₅ substituiert ist;
R⁶ für -OH; F; Cl; Br; I; -SH; -NO₂; -NH₂; -NH-C(=O)-O-R⁷; -C(=O)-O-R⁸; -C(=O)-R⁹;
einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert oder mit ggf. 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, -N(C₂H₅)₂, - N(CH₃)(C₂H₅), -C(=O)-Pyrrolidinyl, -C(=O)-N(CH₃)-Phenyl und -C(=O)-NH-CH(CH₃)₂, -C(=O)-OH, -C(=O)-O-CH₃, -C(=O)-O-C₂H₅ und -C(=O)-O-C(CH₃)₃ substituiert ist;
für einen Rest ausgewählt aus der Gruppe bestehend aus Cyclopropyl, Cyclobutyl, Cyclopentyl, Cyclohexyl, Cycloheptyl, Cyclohexenyl, Cyclopentenyl, Pyrrolidinyl, Tetrahydrothiophenyl, Tetrahydrofuranyl, Piperidinyl, Morpholinyl, Piperazinyl, Azepanyl und Diazepanyl, der jeweils unsubstituiert oder mit ggf. 1, 2, 3, 4 oder 5 Substituenten unabhängig voneinander ausgewählt aus der Gruppe bestehend aus Methyl, Ethyl, n-Propyl, Isopropyl, n-Butyl, 2-Butyl, Isobutyl, tert-Butyl, -OH, -O-CH₃, - O-C₂H₅, -O-C₃H₇, -O-CF₃, -C(=O)-CH₃, -C(=O)-C₂H₅. -C(=O)-C(CH₃)₃, -C(=O)-NH-CH₃ und -C(=O)-NH-C₂H₅ substituiert ist und/oder jeweils über eine -CH₂-, -CH(CH₃)-, -CH₂-CH₂-, -CH(CH₃)-CH₂-, -CH₂-CH(CH₃)- oder -CH₂-CH₂-CH₂-Gruppe gebunden sein kann;
oder für einen Rest ausgewählt aus der Gruppe bestehend aus Phenyl, Naphthyl, Indolizinyl, Benzimidazolyl, Tetrazolyl, Triazinyl, Isoxazolyl, Phthalazinyl, Thienyl, Furyl, Pyrrolyl, Pyrazolyl, Pyrazinyl, Pyranyl, Triazolyl, Pyridinyl, Imidazolyl, Indolyl, Isoindolyl, Thiazolyl, Thiadiazolyl, Oxazolyl, Oxadiazolyl, Isoxazolyl, Pyridazinyl, Thieno[2,3-d]pyrimidinyl und Isochinolinyl steht, der jeweils über eine CH₂-, - CH(CH₃)-, -CH₂-CH₂-, -CH(CH₃)-CH₂-, -CH₂-CH(CH₃)- oder -CH₂-CH₂-CH₂-Gruppe gebunden sein kann und/oder jeweils unsubstituiert oder mit ggf. 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, - C(=O)-CF₃, -C(=O)-H, -C(=O)-CH₃ und -C(=O)-C₂H₅ substituiert ist;
und R⁷, R⁸ und R⁹, unabhängig voneinander, jeweils für
einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert oder mit ggf. 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, -N(C₂H₅)₂ und - N(CH₃)(C₂H₅) substituiert ist;
einen Rest ausgewählt aus der Gruppe bestehend aus Indolinyl, Indanyl, (1,2,3,4)-Tetrahydronaphthyl, (2,3)-Dihydro-benzo[1.4]dioxinyl und Benzo[1.3]dioxolyl, der jeweils unsubstituiert ist;
oder einen Rest ausgewählt aus der Gruppe bestehend aus Phenyl, Naphthyl, Isoxazolyl, Thienyl, Furyl, Pyrrolyl, Pyrazolyl, Pyrazinyl, Pyranyl, Triazolyl, Pyridinyl, Imidazolyl, Indolyl, Isoindolyl, Thiazolyl, Thiadiazolyl, Oxazolyl, Oxadiazolyl, Isoxazolyl und Pyridazinyl stehen, der jeweils über eine -CH₂-, -CH(CH₃)-, -CH₂-CH₂-, -CH(CH₃)-CH₂-, -CH₂-CH(CH₃)- oder -CH₂-CH₂-CH₂-Gruppe gebunden sein kann und/oder jeweils unsubstituiert oder mit ggf. 1, 2, 3, 4 oder 5 Substituenten unabhängig voneinander ausgewählt aus der Gruppe bestehend aus F, Cl, Br, I, - CN, -NO₂, -O-Phenyl, Methyl, Ethyl, n-Propyl, Isopropyl, n-Butyl, Isobutyl, 2-Butyl, tert-Butyl, n-Pentyl, neo-Pentyl, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂ und -O-CH₂F substituiert ist;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Ganz besonders bevorzugt sind 1,3-disubstituierte 4-Methyl-1 H-pyrrol-2-carbonsäureamide der vorstehend angegebenen allgemeinen Formel I, worin
R¹ für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert oder mit 1, 2 oder 3 Substituenten unabhängig voneinander ausgewählt aus der Gruppe bestehend aus -CN, -OH, -N(CH₃)₂, -N(C₂H₅)₂ -C(=O)-O-CH₃, -C(=O)-O-C₂H₅, -C(=O)-O-C(CH₃)₃, -C(=O)-O-CH₂-Phenyl, -C(=O)-NH-Naphthyl, -N(CH₃)-Phenyl, -N(C₂H₅)-(m-Toluyl) und -N(C₂H₅)-(p-Toluyl) substituiert ist;
einen Heteroalkyl-Rest ausgewählt aus der Gruppe bestehend aus -CH₂-O-CH₃, - CH₂-O-C₂H₅, -CH₂-CH₂-O-CH₃, -CH₂-CH₂-O-C₂H₅, -CH₂-CH₂-CH₂-O-CH₃ und -CH₂-CH₂-CH₂-O-C₂H₅;
für einen Rest ausgewählt aus der Gruppe bestehend aus Cyclopropyl, Cyclobutyl, Cyclopentyl, Cyclohexyl, Cycloheptyl, Pyrrolidinyl, Tetrahydrofuranyl, Piperidinyl, Morpholinyl, Piperazinyl, Azepanyl, Dihydrofuran-2(3H)-onyl, Indanyl und (1,2,3,4)-Tetrahydronaphthyl, der jeweils unsubstituiert oder mit ggf. 1, 2, 3, 4 oder 5 Substituenten unabhängig voneinander ausgewählt aus der Gruppe bestehend aus, Methyl, Ethyl, n-Propyl, Isopropyl, n-Butyl, 2-Butyl, Isobutyl, tert-Butyl, -CH₂-Naphthyl, -O-Phenyl, -O-CH₂-Phenyl, Benzyl und Phenyl substituiert ist und/oder jeweils über eine -CH₂-, -CH(CH₃)-, -CH₂-CH₂-, -CH(CH₃)-CH₂-, -CH₂-CH(CH₃)- oder -CH₂-CH₂-CH₂-Gruppe gebunden sein kann;
Phenyl, das jeweils über eine -CH₂-CH₂-O-, -CH₂-O-, -CH₂-CH₂-CH₂-O-, -CH[C(=O)-O-CH₃]-CH₂-, -CH[C(=O)-O-C₂H₅]-CH₂-, -CH₂-, -CH(CH₃)-, -CH₂-CH₂-, -CH(CH₃)-CH₂-, -CH₂-CH(CH₃)- oder -CH₂-CH₂-CH₂-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CH₂-CN, Methyl, Ethyl, n-Propyl, Isopropyl, n-Butyl, Isobutyl, 2-Butyl, tert-Butyl, n-Pentyl, neo-Pentyl, -O-Phenyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -N(CH₃)₂, -N(C₂H₅)₂, -S(=O)-NH₂ und [1.2.3]-Thiadiazolyl substituiert ist;
einen Rest ausgewählt aus der Gruppe bestehend aus Thienyl, Furyl, Pyrrolyl, Pyrazolyl, Pyridinyl, Imidazolyl, Indolyl und Isoindolyl, der jeweils über -CH₂-, - CH(CH₃)-, -CH₂-CH₂-, -CH(CH₃)-CH₂-, -CH₂-CH(CH₃)- oder -CH₂-CH₂-CH₂-Gruppe gebunden sein kann und/oder jeweils unsubstituiert oder mit ggf. 1, 2 oder 3 Substituenten unabhängig voneinander ausgewählt aus der Gruppe bestehend aus - NO₂, -OH, Methyl, Ethyl, n-Propyl, Isopropyl, n-Butyl, Isobutyl, 2-Butyl, tert-Butyl, n-Pentyl und neo-Pentyl substituiert ist
oder -NH-C(=O)-R⁵ steht;
R² für H;
einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert ist;
oder für einen Rest ausgewählt aus der Gruppe bestehend aus Phenyl und Naphthyl steht, der jeweils über eine -CH₂-, -CH(CH₃)-, -CH₂-CH₂-, -CH(CH₃)-CH₂-, -CH₂-CH(CH₃)- oder -CH₂-CH₂-CH₂-Gruppe gebunden sein kann und/oder jeweils unsubstituiert oder mit 1, 2 oder 3 Substituenten unabhängig voneinander ausgewählt aus der Gruppe bestehend aus -O-CH₃, -O-C₂H₅, -O-C₃H₇ und -0-C(CH₃)₃ substituiert ist;
oder R¹ und R² zusammen mit dem sie verbindenden Stickstoffatom einen Rest ausgewählt aus der Gruppe bestehend aus 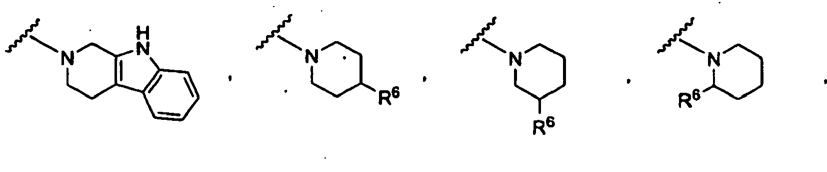 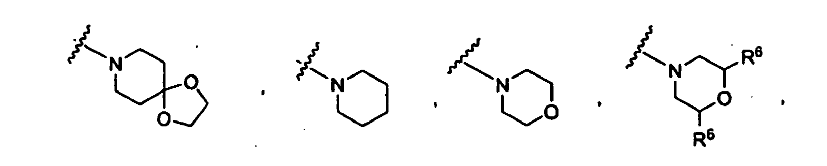 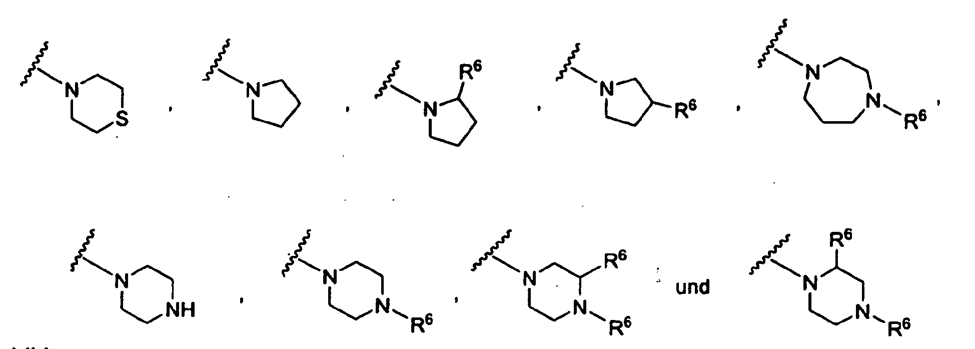 bilden;
R³ für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert ist;
oder für einen Phenyl-Rest steht, der jeweils über eine -CH₂-, -CH(CH₃)-, -CH₂-CH₂-, -CH(CH₃)-CH₂-, -CH₂-CH(CH₃)- oder -CH₂-CH₂-CH₂-Gruppe gebunden ist und/oder jeweils unsubstituiert oder mit ggf. 1, 2, 3, 4 oder 5 Substituenten unabhängig voneinander ausgewählt aus der Gruppe bestehend aus F, Cl, Br, I, -CN, -CH₂-CN, - NO₂, Methyl, Ethyl, n-Propyl, Isopropyl, n-Butyl, Isobutyl, 2-Butyl, tert-Butyl, n-Pentyl, neo-Pentyl, -O-CH₃, -O-C₂H₅, -O-C₃H₇, -O-C(CH₃)₃, -CF₃, -CHF₂, -CH₂F, -O-CF₃, -0-CHF₂ und -O-CH₂F substituiert ist;
R⁴ für Phenyl, das jeweils unsubstituiert oder mit 1, 2, 3, 4 oder 5 Substituenten unabhängig voneinander ausgewählt aus der Gruppe bestehend aus F, Cl, Br, I, Methyl, Ethyl, n-Propyl, Isopropyl, n-Butyl, Isobutyl, 2-Butyl, tert-Butyl, n-Pentyl, -0-CH₃, -O-C₂H₅, -O-C₃H₇, -O-C(CH₃)₃, -O-CF₃, -O-CHF₂ und -O-CH₂F substituiert ist;
oder für einen Rest ausgewählt aus der Gruppe bestehend aus Thienyl, Furyl und Pyrrolyl steht, der jeweils unsubstituiert oder mit ggf. 1, 2, 3, 4 oder 5 Substituenten unabhängig voneinander ausgewählt aus der Gruppe bestehend aus F, Cl, Br, I, Methyl, Ethyl, n-Propyl, Isopropyl, n-Butyl, Isobutyl, 2-Butyl, -O-CH₃, -O-C₂H₅, -O-C₃H₇ und -O-C(CH₃)₃ substituiert ist;
R⁵ für einen Rest ausgewählt aus der Gruppe bestehend aus Phenyl und Naphthyl steht, der jeweils unsubstituiert oder mit 1, 2, 3, 4 oder 5 Substituenten unabhängig voneinander ausgewählt aus der Gruppe bestehend aus F, Cl, Br, I, Methyl, Ethyl, n-Propyl, Isopropyl, n-Butyl, Isobutyl, 2-Butyl, tert-Butyl, -NH₂, -O-CH₃, -O-C₂H₅, -O-C₃H₇, -O-C(CH₃)₃, -N(CH₃)₂, -N(C₂H₅)₂, -NH-CH₃ und -NH-C₂H₅ substituiert ist;
R⁶ für -OH; -NH-C(=O)-O-R⁷; -C(=O)-O-R⁸; -C(=O)-R⁹;
einen Alkyl-Rest ausgewählt aus der Gruppe bestehend aus Methyl, Ethyl, n-Propyl. Isopropyl, n-Butyl, 2-Butyl, Isobutyl, tert-Butyl, n-Pentyl, sec-Pentyl, neo-Pentyl, (3,3,-Dimethyl-butyl, 4-Methyl-2-pentyl und n-Hexyl, der jeweils unsubstituiert oder mit 1, 2 oder 3 Substituenten unabhängig voneinander ausgewählt aus der Gruppe bestehend aus -OH, -N(CH₃)₂, -N(C₂H₅)₂, -C(=O)-Pyrrolidinyl, -C(=O)-N(CH₃)-Phenyl und -C(=O)-NH-CH(CH₃)₂ substituiert ist;
für einen Rest ausgewählt aus der Gruppe bestehend aus Cyclopropyl, Cyclobutyl, Cyclopentyl, Cyclohexyl, Cycloheptyl, Pyrrolidinyl, Piperidinyl und Azepanyl, der jeweils unsubstituiert ist und/oder jeweils über eine -CH₂-, -CH(CH₃)-, -CH₂-CH₂-, - CH(CH₃)-CH₂-, -CH₂-CH(CH₃)- oder -CH₂-CH₂-CH₂-Gruppe gebunden sein kann;
oder für einen Rest ausgewählt aus der Gruppe bestehend aus Phenyl, Naphthyl, Thienyl, Furyl, Pyrazinyl, Pyridinyl, Pyridazinyl und Thieno[2,3-d]pyrimidinyl steht, der jeweils über eine -CH₂-, -CH(CH₃)-, -CH₂-CH₂-, -CH(CH₃)-CH₂-, -CH₂-CH(CH₃)- oder -CH₂-CH₂-CH₂-Gruppe gebunden sein kann und/oder jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, tert-Butyl, -O-CH₃, -O-C₂H₅, -CF₃, - C(=O)-CH₃ und -C(=O)-C₂H₅ substituiert ist;
und R⁷, R⁸ und R⁹, unabhängig voneinander, jeweils für
einen Alkyl-Rest ausgewählt aus der Gruppe bestehend aus Methyl, Ethyl, n-Propyl, Isopropyl, n-Butyl, 2-Butyl, Isobutyl, tert-Butyl und n-Pentyl, der jeweils unsubstituiert ist;
einen Rest ausgewählt aus der Gruppe bestehend aus (2,3)-Dihydro-benzo[1.4]dioxinyl und Benzo[1.3]dioxolyl, der jeweils unsubstituiert ist;
oder einen Rest ausgewählt aus der Gruppe bestehend aus Phenyl, Thienyl, Furyl und Pyrazinyl stehen, der jeweils über eine -CH₂-, -CH(CH₃)-, -CH₂-CH₂-, -CH(CH₃)-CH₂-, -CH₂-CH(CH₃)- oder -CH₂-CH₂-CH₂-Gruppe gebunden sein kann und/oder jeweils unsubstituiert oder mit ggf. 1, 2, 3, 4 oder 5 Substituenten unabhängig voneinander ausgewählt aus der Gruppe bestehend aus F, Cl, Br, I, Methyl, Ethy., n-propyl, Isopropyl, n-Butyl, Isobutyl, 2-Butyl, tert-Butyl, n-Pentyl, neo-Pentyl, -O-CH₃, - O-C₂H₅, -O-C₃H₇ und -O-C(CH₃)₃ substituiert ist;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Ebenfalls ganz besonders bevorzugt sind 1,3-disubstituierte 4-ethyl-1H-pyrrol-2-carbonsäureamide der vorstehend angegebenen allgemeinen Formel I, worin
R¹ für einen Rest ausgewählt aus der Gruppe bestehend aus -CH₂-CH₂-N(C₂H₅)-(m-Toluyl), -CH₂-CH₂-N(C₂H₅)-(p-Toluyl), -CH₂-CH₂-CH₂-N(CH₃)-Phenyl, -CH₂-CH₂-CH₂-N(CH₃)₂, -CH₂-CH₂-CH₂-N(C₂H₅)₂, -CH(CH₃)-CH₂-CH₂-CH(CH₃)₂, -CH₂-CH₂-CN, -CH₂-CH₂-OH, -CH₂-CH₂-N(CH₃)₂, -CH₂-CH₂-N(C₂H₅)₂, -CH₂-CH₂-C(CH₃)₃, n-Pentyl, n-Butyl, Methyl, Ethyl, n-Propyl, -CH(CH₃)-C(=O)-O-CH₂-Phenyl, -CH[CH(CH₃)₂]-C(=O)-O-C(CH₃)₃, -CH₂-C(=O)-O-CH₂-Phenyl, -CH₂-CH₂-C(=O)-O-C(CH₃)₃, -CH₂-CH₂-C(=O)-O-C₂H₅, -CH₂-C(=O)-NH-Naphthyl und -CH₂-CH₂-CH₂-O-CH₃;
für einen Rest ausgewählt aus der Gruppe bestehend aus Cyclopropyl, Cyclobutyl, Cyclopentyl, Cyclohexyl, Cycloheptyl, Pyrrolidinyl, Tetrahydrofuranyl, Piperidinyl, Morpholinyl, Piperazinyl, Azepanyl, Dihydrofuran-2(3H)-onyl, Indanyl und (1,2,3,4)-Tetrahydronaphthyl, der jeweils unsubstituiert oder mit ggf. 1, 2, 3, 4 oder 5 Substituenten unabhängig voneinander ausgewählt aus der Gruppe bestehend aus, Methyl, Ethyl, -CH₂-Naphthyl, -O-Phenyl, -O-CH₂-Phenyl und Benzyl substituiert ist und/oder jeweils über eine -CH₂-, -CH₂-CH₂- oder -CH₂-CH₂-CH₂-Gruppe gebunden sein kann;
Phenyl, das jeweils über eine -CH₂-CH₂-O-, -CH[C(=O)-O-CH₃]-CH₂-, -CH[C(=O)-O-C₂H₅]-CH₂-, -CH₂-, -CH(CH₃)-, -CH₂-CH₂-, -CH(CH₃)-CH₂-, -CH₂-CH(CH₃)- oder - CH₂-CH₂-CH₂-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, **I,** -CH₂-CN, Methyl, Ethyl, n-Propyl, Isopropyl, n-Butyl, Isobutyl, 2-Butyl, tert-Butyl, -O-Phenyl, -O-CH₃, -O-C₂H₅, -CF₃, -O-CF₃, -N(CH₃)₂, - N(C₂H₅)₂, -S(=O)-NH₂ und [1.2.3]-Thiadiazolyl substituiert ist;
einen Rest ausgewählt aus der Gruppe bestehend aus Thienyl, Furyl, Pyridinyl, Imidazolyl, Indolyl und Isoindolyl, der jeweils über -CH₂-, -CH₂-CH₂- oder -CH₂-CH₂-CH₂-Gruppe gebunden sein kann und/oder jeweils unsubstituiert oder mit ggf. 1, 2 oder 3 Substituenten unabhängig voneinander ausgewählt aus der Gruppe bestehend aus -NO₂, -OH, Methyl, Ethyl und n-Propyl substituiert ist
oder -NH-C(=O)-R⁵ steht;
R² für H;
einen Alkyl-Rest ausgewählt aus der Gruppe bestehend aus Methyl, Ethyl und n-Propyl, der jeweils unsubstituiert ist;
oder für einen Rest ausgewählt aus der Gruppe bestehend aus Phenyl und Naphthyl steht, der jeweils über eine -CH₂-Gruppe gebunden sein kann und/oder jeweils unsubstituiert oder mit 1, 2 oder 3 Substituenten unabhängig voneinander ausgewählt aus der Gruppe bestehend aus -O-CH₃, -O-C₂H₅, -O-C₃H₇ und -0-C(CH₃)₃ substituiert ist;
oder R¹ und R² zusammen mit dem sie verbindenden Stickstoffatom einen Rest ausgewählt aus der Gruppe bestehend aus 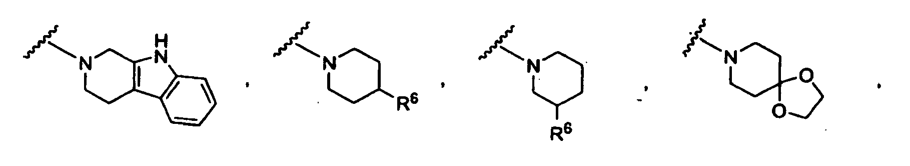 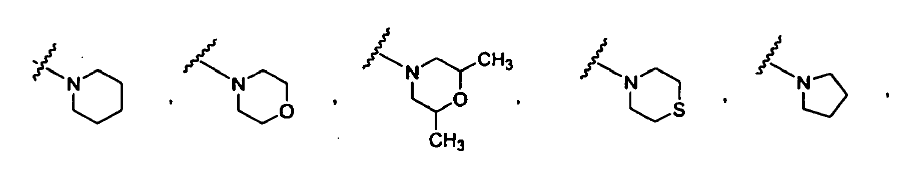 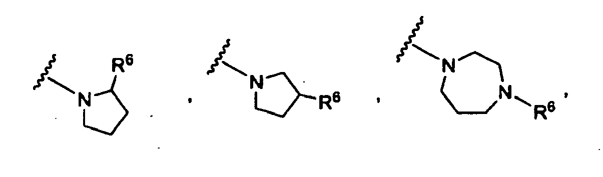 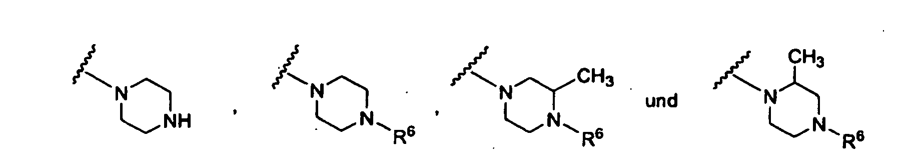 bilden;
R³ für einen Alkyl-Rest ausgewählt aus der Gruppe bestehend aus Methyl, Ethyl, n-Propyl, Isopropyl, n-Butyl, 2-Butyl, Isobutyl und tert-Butyl, der jeweils unsubstituiert ist;
oder für einen Phenyl-Rest steht, der jeweils über eine -CH₂-Gruppe gebunden ist und/oder jeweils unsubstituiert oder mit ggf. 1, 2, 3, 4 oder 5 Substituenten unabhängig voneinander ausgewählt aus der Gruppe bestehend aus F, Cl, Br, Methyl, Ethyl, n-Propyl, -O-CH₃, -O-C₂H₅, und -CF₃ substituiert ist;
R⁴ für Phenyl, das jeweils unsubstituiert oder mit 1, 2, 3, 4 oder 5 Substituenten unabhängig voneinander ausgewählt aus der Gruppe bestehend aus F, Cl, Br, Methyl, Ethyl, n-Propyl, -O-CH₃ und -O-C₂H₅, substituiert ist;
oder für einen Rest ausgewählt aus der Gruppe bestehend aus Thienyl, Furyl und Pyrrolyl steht, der jeweils unsubstituiert ist;
R⁵ für einen Rest ausgewählt aus der Gruppe bestehend aus Phenyl und Naphthyl steht, der jeweils unsubstituiert oder mit 1, 2, 3, 4 oder 5 Substituenten unabhängig voneinander ausgewählt aus der Gruppe bestehend aus F, Cl, Br, Methyl, Ethyl, -O-CH₃, -O-C₂H₅, -N(CH₃)₂, -N(C₂H₅)₂, -NH-CH₃ und -NH-C₂H₅ substituiert ist;
R⁶ für -OH; -NH-C(=O)-O-R⁷; -C(=O)-O-R⁸; -C(=O)-R⁹;
für einen Rest ausgewählt aus der Gruppe bestehend aus Methyl, Ethyl, n-Propyl, Isopropyl, n-Butyl, -CH₃-CH(CH₃)₂, -CH₂-CH₂-OH, -CH₂-CH₂-N(CH₃)₂, -CH₂-CH₂-N(C₂H₅), -CH₂-C(=O)-Pyrrolidinyl, -CH₂-C(=O)-NH-CH(CH₃)₂ und -CH₂-C(=O)-N(CH₃)-Phenyl;
für einen Rest ausgewählt aus der Gruppe bestehend aus Cyclopropyl, Cyclobutyl, Cyclopentyl, Cyclohexyl, Cycloheptyl, Pyrrolidinyl, Piperidinyl und Azepanyl, der jeweils unsubstituiert ist und/oder jeweils über eine -CH₂-Gruppe gebunden sein kann;
oder für einen Rest ausgewählt aus der Gruppe bestehend aus Phenyl, Naphthyl, Thienyl, Pyrazinyl, Pyridinyl und Thieno[2,3-d]pyrimidinyl steht, der jeweils über eine -CH₂-, -CH₂-CH₂- oder -CH₂-CH₂-CH₂-Gruppe gebunden sein kann und/oder jeweils unsubstituiert oder mit ggf. 1, 2, 3, 4 oder 5 Substituenten unabhängig voneinander ausgewählt aus der Gruppe bestehend aus F, Cl, Br, -CN, Methyl, tert-Butyl, -O-CH₃, -O-C₂H₅, -CF₃, -C(=O)-CH₃ und -C(=O)-C₂H₅ substituiert ist;
R⁷ für einen Alkyl-Rest ausgewählt aus der Gruppe bestehend aus Methyl, Ethyl, n-Propyl, Isopropyl, n-Butyl, 2-Butyl, Isobutyl, tert-Butyl und n-Pentyl steht, der jeweils unsubstituiert ist;
R⁸ für einen Alkyl-Rest ausgewählt aus der Gruppe bestehend aus Methyl, Ethyl, n-Propyl, Isopropyl, n-Butyl, 2-Butyl, Isobutyl, tert-Butyl und n-Pentyl, der jeweils unsubstituiert ist;
oder für einen Phenyl- oder Benzyl-Rest steht;
und R⁹ für einen Rest ausgewählt aus der Gruppe bestehend aus (2,3)-Dihydro-benzo[1.4]dioxinyl und Benzo[1.3]dioxolyl, der jeweils unsubstituiert ist;
oder einen Rest ausgewählt aus der Gruppe bestehend aus Phenyl, Thienyl, Furyl und Pyrazinyl steht, der jeweils über eine -CH₂-, -CH₂-CH₂- oder -CH₂-CH₂-CH₂-Gruppe gebunden sein kann und/oder jeweils unsubstituiert oder mit ggf. 1, 2, 3, 4 oder 5 Substituenten unabhängig voneinander ausgewählt aus der Gruppe bestehend aus F, Cl, Br, Methyl, Ethyl, -O-CH₃ und -O-C₂H₅ substituiert ist;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Ganz besonders bevorzugt sind 1,3-disubstituierte 4-Methyl-1H-pyrrol-2-carbonsäureamide der vorstehend angegebenen allgemeinen Formel I ausgewählt aus der Gruppe bestehend aus

| | |
|---|---|
| [1] | 1-Benzyl-4-methyl-3-p-tolyl-1H-pyrrol-2-carbonsäure 4-[1,2,3]thiadiazol-4-yl-benzylamid, |
| [2] | 3-Furan-2-yl-1,4-dimethyl-1H-pyrrol-2-carbonsäure 4-sulfamoyl-benzylamid, |
| [3] | 1-(4-Fluor-benzyl)-3-furan-2-yl-4-methyl-1H-pyrrol-2-carbonsäure 2,4-dimethoxy-benzylamid, |
| [4] | 1-Butyl-4-methyl-3-p-tolyl-1H-pyrrol-2-carbonsäure (2-pyridin-2-yl-ethyl)-amid, |
| [5] | 1-(4-Fluor-benzyl)-3-(4-methoxy-phenyl)-4-methyl-1H-pyrrol-2-carbonsäure [2-(5-hydroxy-1H-indol-3-yl)-ethyl]-amid, |
| [6] | (1,4-Dimethyl-3-p-tolyl-1H-pyrrol-2-yl)-(4-pyrrolidin-1-yl-piperidin-1-yl)-methanon, |
| [7] | 1-Benzyl-3-furan-2-yl-4-methyl-1H-pyrrol-2-carbonsäure 4-brom-2-fluor-benzylamid, |
| [8] | [1-Benzyl-3-(4-chlor-phenyl)-4-methyl-1H-pyrrol-2-yl]-[4-(2-chlor phenyl)-piperazin-1-yl]-methanon, |
| [9] | 1,4-Dimethyl-3-phenyl-1H-pyrrol-2-carbonsäure (3-dimethylamino-propyl)-amid, |
| [10] | 1,4-Dimethyl-3-p-tolyl-1H-pyrrol-2-carbonsäure (2-pyrrolidin-1-yl-ethyl)-amid, |
| [11] | 1-(4-Fluor-benzyl)-3-furan-2-yl-4-methyl-1H-pyrrol-2-carbonsäure 4-dimethylamino-benzylamid, |
| [12] | 1-[4-(1-Benzyl-4-methyl-3-phenyl-1H-pyrrol-2-carbonyl)-piperazin-1-yl]-2-phenyl-ethanon, |
| [13] | 1-Benzyl-3-furan-2-yl-4-methyl-1H-pyrrol-2-carbonsäure 2,5-difluor benzylamid, |
| [14] | 1-(2,6-Dichlor-benzyl)-4-methyl-3-p-tolyl-1H-pyrrol-2-carbonsäure 3,5-difluor-benzylamid, |
| [15] | 1-(2-Brom-benzyl)-3-(4-chlor-phenyl)-4-methyl-1H-pyrrol-2-carbonsäure (2-methyl-cyclohexyl)-amid, |
| [16] | 1,4-Dimethyl-3-p-tolyl-1H-pyrrol-2-carbonsäure (3-morpholin-4-yl-propyl)-amid, |
| [17] | 1-Butyl-4-methyl-3-p-tolyl-1H-pyrrol-2-carbonsäure (1,3-dimethyl-butyl)-amid, |
| [18] | [4-(2,4-Dimethyl-phenyl)-piperazin-1-yl]-[4-methyl-1-(4-methyl-benzyl)-3-p-tolyl-1H-pyrrol-2-yl]-methanon, |
| [19] | 1-(2,6-Dichlor-benzyl)-4-methyl-3-p-tolyl-1H-pyrrol-2-carbonsäure methyl-(2-pyridin-2-yl-ethyl)-amid, |
| [20] | 1,4-Dimethyl-3-phenyl-1H-pyrrol-2-carbonsäure (2-cyano-ethyl)-methyl-amid, |
| [21] | 1-(2,6-Dichlor-benzyl)-4-methyl-3-p-tolyl-1H-pyrrol-2-carbonsäure (3-imidazol-1-yl-propyl)-amid, |
| [22] | 1-(4-Fluor-benzyl)-3-(4-methoxy-phenyl)-4-methyl-1H-pyrrol-2-carbonsäure (1,3-dimethyl-butyl)-amid, |
| [23] | 3-(4-Methoxy-phenyl)-1,4-dimethyl-1H-pyrrol-2-carbonsäure 2-ethoxy-benzylamid, |
| [24] | (4-Cycloheptyl-piperazin-1-yl)-(1,4-dimethyl-3-p-tolyl-1H-pyrrol-2-yl)-methanon, |
| [25] | 1-(4-Fluor-benzyl)-3-(4-methoxy-phenyl)-4-methyl-1H-pyrrol-2-carbonsäure (2-dimethylamino-ethyl)-amid, |
| [26] | 1-Benzyl-4-methyl-3-phenyl-1H-pyrrol-2-carbonsäure (pyridin-2-ylmethyl)-amid, |
| [27] | 3-(4-Chlor-phenyl)-1-ethyl-4-methyl-1H-pyrrol-2-carbonsäure [2-(4-chlor-phenyl)-propyl]-amid, |
| [28] | 3-(4-Chlor-phenyl)-1-(2-fluor-benzyl)-4-methyl-1H-pyrrol-2-carbonsäure 3,5-difluor-benzylamid, |
| [29] | 2-{[1-(4-Fluor-benzyl)-3-(4-methoxy-phenyl)-4-methyl-1H-pyrrol-2-carbonyl]-amino}-propionsäure benzyl ester, |
| [30] | (1-Benzyl-4-methyl-3-p-tolyl-1H-pyrrol-2-yl)-[4-(2-dimethylamino-ethyl)-piperazin-1-yl]-methanon, |
| [31] | 3-Furan-2-yl-1-(4-methoxy-benzyl)-4-methyl-1H-pyrrol-2-carbonsäure (3,3-dimethyl-butyl)-amid, |
| [32] | 3-(4-Chlor-phenyl)-1-isobutyl-4-methyl-1H-pyrrol-2-carbonsäure 4-dimethylamino-benzylamid, |
| [33] | 3-(4-Chlor-phenyl)-1-ethyl-4-methyl-1H-pyrrol-2-carbonsäure (2-pyridin-2-yl-ethyl)-amid, |
| [34] | 3-(4-Chlor-phenyl)-1-ethyl-4-methyl-1H-pyrrol-2-carbonsäure 2,3-dimethoxy-benzylamid, |
| [35] | 1-(4-Fluor-benzyl)-3-(4-methoxy-phenyl)-4-methyl-1H-pyrrol-2-carbonsäure (2-thiophen-2-yl-ethyl)-amid, |
| [36] | 2-{[3-(4-Chlor-phenyl)-1-ethyl-4-methyl-1H-pyrrol-2-carbonyl]-amino}-3-methyl-buttersäure tert-butyl ester, |
| [37] | 3-Furan-2-yl-1-(4-methoxy-benzyl)-4-methyl-1H-pyrrol-2-carbonsäure (1,2,3,4-tetrahydro-naphthalin-1-yl)-amid, |
| [38] | 3-Furan-2-yl-4-methyl-1-(4-trifluormethyl-benzyl)-1H-pyrrol-2-carbonsäure (2-pyrrolidin-1-yl-ethyl)-amid, |
| [39] | {[1-(4-Methoxy-benzyl)-4-methyl-3-p-tolyl-1H-pyrrol-2-carbonyl]-methyl-amino}-essigsäure benzyl ester, |
| [40] | 3-Furan-2-yl-1-(4-methoxy-benzyl)-4-methyl-1H-pyrrol-2-carbonsäure [2-(1-methyl-pyrrolidin-2-yl)-ethyl]-amid, |
| [41] | [4-(5-Brom-2-ethoxy-benzyl)-piperazin-1-yl]-(1,4-dimethyl-3-phenyl-1H-pyrrol-2-yl)-methanon, |
| [42] | (1-Benzyl-3-furan-2-yl-4-methyl-1H-pyrrol-2-yl)-[4-(3-fluor-4-methoxy-benzyl)-piperazin-1-yl]-methanon, |
| [43] | 3-{[3-(4-Chlor-phenyl)-1-ethyl-4-methyl-1H-pyrrol-2-carbonyl]-amino}-propionsäure tert-butyl ester, |
| [44] | 1-Benzyl-4-methyl-3-phenyl-1H-pyrrol-2-carbonsäure 3-methoxy-benzylamid, |
| [45] | (1-Benzyl-3-furan-2-yl-4-methyl-1H-pyrrol-2-yl)-[4-(5-brom-2-ethoxy-benzyl)-piperazin-1-yl]-methanon, |
| [46] | [3-(4-Chlor-phenyl)-1,4-dimethyl-1H-pyrrol-2-yl]-(4-o-tolyl-piperazin-1-yl)-methanon, |
| [47] | 1-(4-Fluor-benzyl)-3-(4-methoxy-phenyl)-4-methyl-1H-pyrrol-2-carbonsäure [2-(4-ethoxy-phenyl)-ethyl]-amid, |
| [48] | [1-(2,6-Dichlor-benzyl)-4-methyl-3-p-tolyl-1H-pyrrol-2-yl]-(4-hydroxy-piperidin-1-yl)-methanon, |
| [49] | (1,4-Dimethyl-3-p-tolyl-1H-pyrrol-2-yl)-[4-(2,4,6-trimethoxy-benzyl)-piperazin-1-yl]-methanon, |
| [50] | 1-(4-Fluor-benzyl)-3-furan-2-yl-4-methyl-1H-pyrrol-2-carbonsäure (2-morpholin-4-yl-ethyl)-amid, |
| [51] | 1-Benzyl-3-(4-chlor-phenyl)-4-methyl-1H-pyrrol-2-carbonsäure [2-(4-fluor-phenyl)-ethyl]-amid, |
| [52] | [4-(2,5-Dimethyl-phenyl)-piperazin-1-yl]-[3-(4-methoxy-phenyl)-1,4-dimethyl-1H-pyrrol-2-yl]-methanon, |
| [53] | 3-(4-Methoxy-phenyl)-1,4-dimethyl-1H-pyrrol-2-carbonsäure (3-imidazol-1-yl-propyl)-amid, |
| [54] | 1-(4-Fluor-benzyl)-3-(4-methoxy-phenyl)-4-methyl-1H-pyrrol-2-carbonsäure [2-(3-trifluormethyl-phenyl)-ethyl]-amid, |
| [55] | [3-(4-Chlor-phenyl)-1-(2-fluor-benzyl)-4-methyl-1H-pyrrol-2-yl]-[4-(5-methyl-pyrazin-2-carbonyl)-piperazin-1-yl]-methanon, |
| [56] | 3-(4-Chlor-phenyl)-1-isobutyl-4-methyl-1H-pyrrol-2-carbonsäure 3-fluor-5-trifluormethyl-benzylamid, |
| [57] | [3-(4-Chlor-phenyl)-1,4-dimethyl-1H-pyrrol-2-yl]-(4-pyridin-4-yl-piperazin-1-yl)-methanon, |
| [58] | 3-(4-Chlor-phenyl)-1-(2-fluor-benzyl)-4-methyl-1H-pyrrol-2-carbonsäure (3-imidazol-1-yl-propyl)-amid, |
| [59] | (1,4-Dimethyl-3-phenyl-1H-pyrrol-2-yl)-[4-(3-fluor-4-methoxy-benzyl)-piperazin-1-yl]-methanon, |
| [60] | 1-Benzyl-3-furan-2-yl-4-methyl-1H-pyrrol-2-carbonsäure [2-(4-brom-phenyl)-ethyl]-amid, |
| [61] | (4-Cycloheptyl-piperazin-1-yl)-(3-furan-2-yl-1,4-dimethyl-1H-pyrrol-2-yl)-methanon, |
| [62] | 1-Benzyl-4-methyl-3-phenyl-1H-pyrrol-2-carbonsäure [2-(4-methoxy-phenoxy)-ethyl]-amid, |
| [63] | 1-(4-Fluor-benzyl)-3-furan-2-yl-4-methyl-1H-pyrrol-2-carbonsäure (3-dimethylamino-propyl)-amid, |
| [64] | [1-(4-Fluor-benzyl)-3-(4-methoxy-phenyl)-4-methyl-1H-pyrrol-2-yl]-(2-pyrrolidin-1-ylmethyl-pyrrolidin-1-yl)-methanon, |
| [65] | 3-(4-Chlor-phenyl)-1-isobutyl-4-methyl-1H-pyrrol-2-carbonsäure (3-diethylamino-propyl)-amid, |
| [66] | [3-(4-Chlor-phenyl)-1,4-dimethyl-1H-pyrrol-2-yl]-[4-(2-fluor-5-methoxy-benzyl)-piperazin-1-yl]-methanon, |
| [67] | 3-(4-Methoxy-phenyl)-1,4-dimethyl-1H-pyrrol-2-carbonsäure pentylamid, |
| [68] | (1-Butyl-4-methyl-3-p-tolyl-1H-pyrrol-2-yl)-[4-(2,5-dimethoxy-benzyl)-piperazin-1-yl]-methanon, |
| [69] | [4-(2-Diethylamino-ethyl)-piperazin-1-yl]-[4-methyl-1-(4-methyl-benzyl)-3-p-tolyl-1H-pyrrol-2-yl]-methanon, |
| [70] | [4-(3-Chlor-phenyl)-piperazin-1-yl]-[3-(4-methoxy-phenyl)-1,4-dimethyl-1H-pyrrol-2-yl]-methanon, |
| [71] | [1-(2-Brom-benzyl)-3-(4-chlor-phenyl)-4-methyl-1H-pyrrol-2-yl]-(4-isopropyl-piperazin-1-yl)-methanon, |
| [72] | [4-(2-Dimethylamino-ethyl)-piperazin-1-yl]-[1-(4-fluor-benzyl)-3-(4-methoxy-phenyl)-4-methyl-1H-pyrrol-2-yl]-methanon, |
| [73] | 3-(4-Chlor-phenyl)-1-(2-fluor-benzyl)-4-methyl-1H-pyrrol-2-carbonsäure 2,4-dimethoxy-benzylamid, |
| [74] | 1-Benzyl-4-methyl-3-p-tolyl-1H-pyrrol-2-carbonsäure (2-pyrrolidin-1-yl-ethyl)-amid, |
| [75] | [1-(4-Fluor-benzyl)-3-furan-2-yl-4-methyl-1H-pyrrol-2-yl]-[4-(3-fluor-4-methoxy-benzyl)-piperazin-1-yl]-methanon, |
| [76] | 3-(4-Methoxy-phenyl)-1,4-dimethyl-1H-pyrrol-2-carbonsäure 2,4-dimethoxy-benzylamid, |
| [77] | 1-Benzyl-4-methyl-3-phenyl-1H-pyrrol-2-carbonsäure (2-pyrrolidin-1-yl-ethyl)-amid, |
| [78] | 3-(4-Chlor-phenyl)-1,4-dimethyl-1H-pyrrol-2-carbonsäure [3-(2-methyl-piperidin-1-yl)-propyl]-amid, |
| [79] | 1-(4-Fluor-benzyl)-3-furan-2-yl-4-methyl-1H-pyrrol-2-carbonsäure cyclohexylamid, |
| [80] | 3-Furan-2-yl-1,4-dimethyl-1H-pyrrol-2-carbonsäure 4-fluor-2-trifluormethyl-benzylamid, |
| [81] | 1-Benzyl-3-furan-2-yl-4-methyl-1H-pyrrol-2-carbonsäure (1-naphthalin-2-yl-ethyl)-amid, |
| [82] | 3-(4-Chlor-phenyl)-1-ethyl-4-methyl-1H-pyrrol-2-carbonsäure (furan-2-ylmethyl)-amid, |
| [83] | 1-Benzyl-4-methyl-3-phenyl-1H-pyrrol-2-carbonsäure (3-dimethylamino-propyl)-methyl-amid, |
| [84] | [3-(4-Methoxy-phenyl)-1,4-dimethyl-1H-pyrrol-2-yl]-(4-p-tolyl-piperazin-1-yl)-methanon, |
| [85] | 1-(4-Methoxy-benzyl)-4-methyl-3-p-tolyl-1H-pyrrol-2-carbonsäure (thiophen-2-ylmethyl)-amid, |
| [86] | 4-Methyl-1-(4-methyl-benzyl)-3-p-tolyl-1H-pyrrol-2-carbonsäure phenethyl-amid, |
| [87] | 1,4-Dimethyl-3-p-tolyl-1H-pyrrol-2-carbonsäure 3,5-difluor-benzylamid, |
| [88] | 3-(4-Methoxy-phenyl)-1 ,4-dimethyl-1H-pyrrol-2-carbonsäure indan-1-ylamid, |
| [89] | 1-Benzyl-4-methyl-3-phenyl-1H-pyrrol-2-carbonsäure 4-dimethylamino-benzylamid, |
| [90] | 1-(4-Fluor-benzyl)-3-furan-2-yl-4-methyl-1H-pyrrol-2-carbonsäure (1-naphthalin-2-ylmethyl-pyrrolidin-3-yl)-amid, |
| [91] | [3-Furan-2-yl-4-methyl-1-(4-trifluormethyl-benzyl)-1H-pyrrol-2-yl]-(4-methyl-[1,4]diazepan-1-yl)-methanon, |
| [92] | [1-(2-Brom-benzyl)-3-(4-chlor-phenyl)-4-methyl-1H-pyrrol-2-yl]-[4-(3-trifluormethyl-phenyl)-piperazin-1-yl]-methanon, |
| [93] | [3-Furan-2-yl-1-(4-methoxy-benzyl)-4-methyl-1H-pyrrol-2-yl]-(4-thiophen-3-ylmethyl-piperazin-1-yl)-methanon, |
| [94] | 1,4-Dimethyl-3-p-tolyl-1H-pyrrol-2-carbonsäure (2-benzyloxy-cyclohexyl)-amid, |
| [95] | 2-{4-[1-Benzyl-3-(4-chlor-phenyl)-4-methyl-1H-pyrrol-2-carbonyl]-piperazin-1-yl}-N-isopropyl-acetamid, |
| [96] | (2,6-Dimethyl-morpholin-4-yl)-[3-(4-methoxy-phenyl)-1,4-dimethyl-1H-pyrrol-2-yl]-methanon, |
| [97] | 3-Furan-2-yl-1-(4-methoxy-benzyl)-4-methyl-1H-pyrrol-2-carbonsäure (thiophen-2-ylmethyl)-amid, |
| [98] | 1,4-Dimethyl-3-p-tolyl-1H-pyrrol-2-carbonsäure (furan-2-ylmethyl)-amid, |
| [99] | 4-Methyl-1-(4-methyl-benzyl)-3-p-tolyl-1H-pyrrol-2-carbonsäure (3-dimethylamino-propyl)-amid, |
| [100] | 1-(4-Fluor-benzyl)-3-(4-methoxy-phenyl)-4-methyl-1H-pyrrol-2-carbonsäure (2-oxo-tetrahydro-furan-3-yl)-amid, |
| [101] | 3-(4-Chlor-phenyl)-1,4-dimethyl-1H-pyrrol-2-carbonsäure (2-phenoxy-ethyl)-amid, |
| [102] | 3-(4-(1-Benzyl-4-methyl-3-p-tolyl-1H-pyrrole-2-carbonyl)piperazin-1-yl)pyrazin-2-carbonitril |
| [103] | 2-{[3-(4-Methoxy-phenyl)-1,4-dimethyl-1H-pyrrol-2-carbonyl]-amino}-3-phenyl-propionsäure methyl ester, |
| [104] | 3-(4-Chlor-phenyl)-1,4-dimethyl-1H-pyrrol-2-carbonsäure (2-morpholin-4-yl-ethyl)-amid, |
| [105] | 3-Furan-2-yl-1,4-dimethyl-1H-pyrrol-2-carbonsäure indan-2-ylamid, |
| [106] | (1-Butyl-4-methyl-3-p-tolyl-1H-pyrrol-2-yl)-[4-(2-fluor-phenyl)-piperazin-1-yl]-methanon, |
| [107] | [4-(3-Chlor-phenyl)-piperazin-1-yl]-(1,4-dimethyl-3-p-tolyl-1H-pyrrol-2-yl)-methanon, |
| [108] | [1-(4-Fluor-benzyl)-3-furan-2-yl-4-methyl-1H-pyrrol-2-yl]-(4-naphthalin-2-ylmethyl-piperazin-1-yl)-methanon, |
| [109] | 3-(4-Chlor-phenyl)-1-isobutyl-4-methyl-1H-pyrrol-2-carbonsäure p-tolylamid, |
| [110] | (1-Benzyl-4-methyl-3-p-tolyl-1H-pyrrol-2-yl)-[4-(2-hydroxy-ethyl)-piperazin-1-yl]-methanon, |
| [111] | 1-Butyl-4-methyl-3-p-tolyl-1H-pyrrol-2-carbonsäure [2-(2,5-dimethoxy-phenyl)-ethyl]-amid, |
| [112] | 1-Butyl-4-methyl-3-p-tolyl-1H-pyrrol-2-carbonsäure (3-imidazol-1-yl-propyl)-amid, |
| [113] | 1-Benzyl-3-(4-chlor-phenyl)-4-methyl-1H-pyrrol-2-carbonsäure [2-(3,4-dimethoxy-phenyl)-ethyl]-methyl-amid, |
| [114] | 4-Methyl-1-(4-methyl-benzyl)-3-p-tolyl-1H-pyrrol-2-carbonsäure (3-morpholin-4-yl-propyl)-amid, |
| [115] | 4-Methyl-1-(4-methyl-benzyl)-3-p-tolyl-1H-pyrrol-2-carbonsäure 4-fluor-2-trifluormethyl-benzylamid, |
| [116] | 2-{[1-Benzyl-3-(4-chlor-phenyl)-4-methyl-1H-pyrrol-2-carbonyl]-amino}-3-(4-chlor-phenyl)-propionsäure ethyl ester, |
| [117] | 1-Benzyl-4-methyl-3-phenyl-1H-pyrrol-2-carbonsäure 3-fluor-4-trifluormethyl-benzylamid, |
| [118] | 1-(4-Brom-benzyl)-4-methyl-3-p-tolyl-1H-pyrrol-2-carbonsäure (2-pyridin-2-yl-ethyl)-amid, |
| [119] | 1-(4-Fluor-benzyl)-3-furan-2-yl-4-methyl-1H-pyrrol-2-carbonsäure benzyl-(2-hydroxy-ethyl)-amid, |
| [120] | 2-[4-(1-Butyl-4-methyl-3-p-tolyl-1H-pyrrol-2-carbonyl)-piperazin-1-yl]-N-methyl-N-phenyl-acetamid, |
| [121] | [4-Methyl-1-(4-methyl-benzyl)-3-p-tolyl-1H-pyrrol-2-yl]-(4-phenyl-piperazin-1-yl)-methanon, |
| [122] | 1-Benzyl-4-methyl-3-p-tolyl-1H-pyrrol-2-carbonsäure (1-benzyl-pyrrolidin-3-yl)-amid, |
| [123] | [1-(4-Fluor-benzyl)-3-furan-2-yl-4-methyl-1H-pyrrol-2-yl]-[4-(3-trifluormethyl-phenyl)-piperazin-1-yl]-methanon, |
| [124] | 1-(4-Methoxy-benzyl)-4-methyl-3-p-tolyl-1H-pyrrol-2-carbonsäure butylamid, |
| [125] | 1-Benzyl-4-methyl-3-p-tolyl-1H-pyrrol-2-carbonsäure [2-(2-fluor-phenyl)-ethyl]-amid, |
| [126] | 4-Methyl-1-(4-methyl-benzyl)-3-p-tolyl-1H-pyrrol-2-carbonsäure 3,4-dimethoxy-benzylamid, |
| [127] | 3-(4-Chlor-phenyl)-1-isobutyl-4-methyl-1H-pyrrol-2-carbonsäure (2-diethylamino-ethyl)-amid, |
| [128] | 1-{4-[3-(4-Chlor-phenyl)-1-ethyl-4-methyl-1H-pyrrol-2-carbonyl]-piperazin-1-yl}-2-phenyl-ethanon, |
| [129] | 1-(4-Fluor-benzyl)-4-methyl-3-p-tolyl-1H-pyrrol-2-carbonsäure 3-trifluormethoxy-benzylamid, |
| [130] | [1-Benzyl-3-(4-chlor-phenyl)-4-methyl-1H-pyrrol-2-yl]-(3-methyl-piperidin-1-yl)-methanon, |
| [131] | 1-(4-Fluor-benzyl)-4-methyl-3-p-tolyl-1H-pyrrol-2-carbonsäure (2-p-tolyl-ethyl)-amid, |
| [132] | 1-(4-Methoxy-benzyl)-4-methyl-3-p-tolyl-1H-pyrrol-2-carbonsäure [2-(2-methyl-5-nitro-imidazol-1-yl)-ethyl]-amid, |
| [133] | 1,4-Dimethyl-3-p-tolyl-1H-pyrrol-2-carbonsäure 2,3-dimethoxy-benzylamid, |
| [134] | 1-(2-Brom-benzyl)-3-(4-chlor-phenyl)-4-methyl-1H-pyrrol-2-carbonsäure [2-(ethyl-m-tolyl-amino)-ethyl]-amid, |
| [135] | 1-(4-Fluor-benzyl)-3-furan-2-yl-4-methyl-1H-pyrrol-2-carbonsäure (4-isopropyl-phenyl)-amid, |
| [136] | 5-Chlor-2-methoxy-benzoesäure N'-[1-(4-fluor-benzyl)-3-(4-methoxy-phenyl)-4-methyl-1H-pyrrol-2-carbonyl]-hydrazid, |
| [137] | (1-Benzyl-4-methyl-3-p-tolyl-1H-pyrrol-2-yl)-[1,4']bipiperidinyl-1'-yl-methanon, |
| [138] | 3-Furan-2-yl-1,4-dimethyl-1H-pyrrol-2-carbonsäure (4-butyl-phenyl)-amid, |
| [139] | 1-Benzyl-4-methyl-3-phenyl-1H-pyrrol-2-carbonsäure [2-(1H-indol-3-yl)-ethyl]-amid, |
| [140] | [4-(4-tert-Butyl-benzyl)-piperazin-1-yl]-[1-(4-fluor-benzyl)-4-methyl-3-p-tolyl-1H-pyrrol-2-yl]-methanon, |
| [141] | [1-(4-Fluor-benzyl)-4-methyl-3-p-tolyl-1H-pyrrol-2-yl]-morpholin-4-yl-methanon, |
| [142] | 3-[(1-Benzyl-3-furan-2-yl-4-methyl-1H-pyrrol-2-carbonyl)-amino]-propionsäure ethyl ester, |
| [143] | 1,4-Dimethyl-3-phenyl-1H-pyrrol-2-carbonsäure (naphthalin-2-ylcarbamoylmethyl)-amid, |
| [144] | [3-(4-Chlor-phenyl)-1,4-dimethyl-1H-pyrrol-2-yl]-[4-(2-ethoxy-phenyl)-piperazin-1-yl]-methanon, |
| [145] | 1-(4-Fluor-benzyl)-3-furan-2-yl-4-methyl-1H-pyrrol-2-carbonsäure (4-cyanomethyl-phenyl)-amid, |
| [146] | 4-[1-Benzyl-3-(4-chlor-phenyl)-4-methyl-1H-pyrrol-2-carbonyl]-piperazin-1-carbonsäure tert-butyl ester, |
| [147] | (1,4-Dioxa-8-aza-spiro[4.5]dec-8-yl)-[1-(4-fluor-benzyl)-3-furan-2-yl-4-methyl-1H-pyrrol-2-yl]-methanon, |
| [148] | 3-Furan-2-yl-4-methyl-1-(4-trifluormethyl-benzyl)-1H-pyrrol-2-carbonsäure pentylamid, |
| [149] | 3-Furan-2-yl-1-(4-methoxy-benzyl)-4-methyl-1H-pyrrol-2-carbonsäure [3-(methyl-phenyl-amino)-propyl]-amid, |
| [150] | 1-(2,6-Dichlor-benzyl)-4-methyl-3-p-tolyl-1H-pyrrol-2-carbonsäure (2-azepan-1-yl-ethyl)-amid, |
| [151] | 1-(4-Brom-benzyl)-4-methyl-3-p-tolyl-1H-pyrrol-2-carbonsäure cyclopentylamid, |
| [152] | [1-(2,6-Dichlor-benzyl)-4-methyl-3-p-tolyl-1H-pyrrol-2-yl]-[4-(2,4-dimethoxy-phenyl)-piperazin-1-yl]-methanon, |
| [153] | 3-(4-Chlor-phenyl)-1-(2-fluor-benzyl)-4-methyl-1H-pyrrol-2-carbonsäure [2-(2-chlor-phenoxy)-ethyl]-amid, |
| [154] | 1-(2,6-Dichlor-benzyl)-4-methyl-3-p-tolyl-1H-pyrrol-2-carbonsäure (3,3-dimethyl-butyl)-amid, |
| [155] | 1,4-Dimethyl-3-p-tolyl-1H-pyrrol-2-carbonsäure (2-benzyloxy-cyclohexyl)-amid, |
| [156] | [4-Methyl-1-(4-methyl-benzyl)-3-p-tolyl-1H-pyrrol-2-yl]-thiomrpholin-4-yl-methanon, |
| [157] | 3-(4-Chlor-phenyl)-1-isobutyl-4-methyl-1H-pyrrol-2-carbonsäure (3-dimethylamino-propyl)-methyl-amid, |
| [158] | [4-(2,5-Dimethyl-phenyl)-piperazin-1-yl]-[3-furan-2-yl-1-(4-methoxy-benzyl)-4-methyl-1H-pyrrol-2-yl]-methanon, |
| [159] | 5-Chlor-2-methoxy-benzoesäure N'-[3-(4-methoxy-phenyl)-1,4-dimethyl-1H-pyrrol-2-carbonyl]-hydrazide, |
| [160] | 2-(3,4-Difluor-phenyl)-1-{4-[1-(4-fluor-benzyl)-3-furan-2-yl-4-methyl-1H-pyrrol-2-carbonyl]-piperazin-1-yl}-ethanon, |
| [161] | 2-{4-[1-(4-Fluor-benzyl)-3-furan-2-yl-4-methyl-1H-pyrrol-2-carbonyl]-piperazin-1-yl}-N-isopropyl-acetamid, |
| [162] | 3-Furan-2-yl-4-methyl-1-(4-trifluormethyl-benzyl)-1H-pyrrol-2-carbonsäure [1-(3-methoxy-phenyl)-ethyl]-amid, |
| [163] | 2-[(1-Benzyl-3-furan-2-yl-4-methyl-1H-pyrrol-2-carbonyl)-amino]-3-(4-chlor-phenyl)-propionsäure methyl ester, |
| [164] | [3-Furan-2-yl-1-(4-methoxy-benzyl)-4-methyl-1H-pyrrol-2-yl]-(4-methyl-piperazin-1-yl)-methanon, |
| [165] | 1,4-Dimethyl-3-phenyl-1H-pyrrol-2-carbonsäure (1-benzyl-pyrrolidin-3-yl)-amid, |
| [166] | (1,4-Dimethyl-3-p-tolyl-1H-pyrrol-2-yl)-(4-thieno[2,3-d]pyrimidin-4-yl-piperazin-1-yl)-methanon, |
| [167] | 4-Methyl-1-(4-methyl-benzyl)-3-p-tolyl-1H-pyrrol-2-carbonsäure (3-methoxy-propyl)-amid, |
| [168] | [3-(4-Methoxy-phenyl)-1,4-dimethyl-1H-pyrrol-2-yl]-[4-(5-thfluormethyl-pyridin-2-yl)-piperazin-1-yl]-methanon, |
| [169] | 1-(4-Brom-benzyl)-4-methyl-3-p-tolyl-1H-pyrrol-2-carbonsäure (3-morpholin-4-yl-propyl)-amid, |
| [170] | 1,4-Dimethyl-3-p-tolyl-1H-pyrrol-2-carbonsäure [2-(2,3-dimethoxy-phenyl)-ethyl]-amid, |
| [171] | [1-(4-Methoxy-benzyl)-4-methyl-3-p-tolyl-1H-pyrrol-2-yl]-(3-methyl-4-p-tolyl-piperazin-1-yl)-methanon, |
| [172] | [3-(4-Chlor-phenyl)-1-ethyl-4-methyl-1H-pyrrol-2-yl]-[4-(2,4-dimethoxy-phenyl)-piperazin-1-yl]-methanon, |
| [173] | 2-{4-[1-(2-Brom-benzyl)-3-(4-chlor-phenyl)-4-methyl-1H-pyrrol-2-carbonyl]-piperazin-1-yl}-benzonitril, |
| [174] | 1-(4-Brom-benzyl)-4-methyl-3-p-tolyl-1H-pyrrol-2-carbonsäure (1-ethyl-pyrrolidin-2-ylmethyl)-amid, |
| [175] | 3-(4-Chlor-phenyl)-1-isobutyl-4-methyl-1H-pyrrol-2-carbonsäure [2-(2-chlor-phenoxy)-ethyl]-amid, |
| [176] | 3-(4-Chlor-phenyl)-1,4-dimethyl-1H-pyrrol-2-carbonsäure [2-(1H-indol-3-yl)-ethyl]-amid, |
| [177] | 1-(4-Fluor-benzyl)-4-methyl-3-p-tolyl-1H-pyrrol-2-carbonsäure (naphthalin-2-ylcarbamoylmethyl)-amid, |
| [178] | 4-(1-Benzyl-3-furan-2-yl-4-methyl-1H-pyrrol-2-carbonyl)-piperazin-1-carbonsäure benzyl ester, |
| [179] | 1,4-Dimethyl-3-phenyl-1H-pyrrol-2-carbonsäure (1-benzyl-pyrrolidin-3-yl)-amid, |
| [180] | [3-(4-Chlor-phenyl)-1-(2-fluor-benzyl)-4-methyl-1H-pyrrol-2-yl]-[4-(4-trifluormethyl-phenyl)-piperazin-1-yl]-methanon, |
| [181] | [3-(4-Chlor-phenyl)-1-isobutyl-4-methyl-1H-pyrrol-2-yl]-(4-phenyl-piperazin-1-yl)-methanon, |
| [182] | 2-{4-[1-Butyl-3-(4-chlor-phenyl)-4-methyl-1H-pyrrol-2-carbonyl]-piperazin-1-yl}-1-pyrrolidin-1-yl-ethanon, |
| [183] | [1-Benzyl-3-(4-chlor-phenyl)-4-methyl-1H-pyrrol-2-yl]-[4-(2-chlor-4-fluor-benzoyl)-piperazin-1-yl]-methanon, |
| [184] | (4-Benzoyl-piperidin-1-yl)-[1-(4-methoxy-benzyl)-4-methyl-3-p-tolyl-1H-pyrrol-2-yl]-methanon, |
| [185] | 3-Furan-2-yl-1,4-dimethyl-1H-pyrrol-2-carbonsäure ethyl-pyridin-4-ylmethyl-amid, |
| [186] | 1-(4-Brom-benzyl)-4-methyl-3-p-tolyl-1H-pyrrol-2-carbonsäure (2-pyrrolidin-1-yl-ethyl)-amid, |
| [187] | [3-(4-Chlor-phenyl)-1-isobutyl-4-methyl-1H-pyrrol-2-yl]-[4-(4-methyl-benzoyl)-piperazin-1-yl]-methanon, |
| [188] | [1-Benzyl-3-(4-chlor-phenyl)-4-methyl-1H-pyrrol-2-yl]-(4-thieno[2,3-d]pyrimidin-4-yl-piperazin-1-yl)-methanon, |
| [189] | 1-(1,4-Dimethyl-3-phenyl-1H-pyrrol-2-carbonyl)-piperidin-3-carbonsäure ethyl ester, |
| [190] | 1,4-Dimethyl-3-phenyl-1H-pyrrol-2-carbonsäure (1-phenyl-ethyl)-amid, |
| [191] | [3-Furan-2-yl-1-(4-methoxy-benzyl)-4-methyl-1H-pyrrol-2-yl]-(1,3,4,9-tetrahydro-b-carbolin-2-yl)-methanon, |
| [192] | 1-{4-[1-Benzyl-3-(4-chlor-phenyl)-4-methyl-1H-pyrrol-2-carbonyl]-piperazin-1-yl}-2-(3,4-difluor-phenyl)-ethanon, |
| [193] | [1-(4-Brom-benzyl)-4-methyl-3-p-tolyl-1H-pyrrol-2-yl]-[4-(3-phenyl-propyl)-piperazin-1-yl]-methanon, |
| [194] | 1-(2-Brom-benzyl)-3-(4-chlorphenyl)-4-methyl-1H-pyrrol-2-carbonsäure [2-(1H-indol-3-yl)-ethyl]-methyl-amid, |
| [195] | 1-(4-{4-[1-(4-Brom-benzyl)-4-methyl-3-p-tolyl-1H-pyrrol-2-carbonyl]-piperazin-1-yl}-phenyl)-ethanon, |
| [196] | 1,4-Dimethyl-3-phenyl-1H-pyrrol-2-carbonsäure 4-fluor-2-trifluormethyl-benzylamid, |
| [197] | 4-Methyl-1-(4-methyl-benzyl)-3-p-tolyl-1H-pyrrol-2-carbonsäure (4-phenoxy-phenyl)-amid, |
| [198] | (4-Hydroxy-piperidin-1-yl)-[1-(4-methoxy-benzyl)-4-methyl-3-p-tolyl-1H-pyrrol-2-yl]-methanon, |
| [199] | 4-Diethylamino-benzoesäure N'-(1-benzyl-4-methyl-3-p-tolyl-1H-pyrrol-2-carbonyl)-hydrazide, |
| [200] | [3-(4-Chlor-phenyl)-1-isobutyl-4-methyl-1H-pyrrol-2-yl]-[4-(3-chlor-phenyl)-piperazin-1-yl]-methanon, |
| [201] | 1-(4-Brom-benzyl)-4-methyl-3-p-tolyl-1H-pyrrol-2-carbonsäure [2-(3,4-dichlor-phenyl)-ethyl]-amid, |
| [202] | 3-(4-Chlor-phenyl)-1-ethyl-4-methyl-1H-pyrrol-2-carbonsäure [2-(1H-indol-3-yl)-ethyl]-amid, |
| [203] | [1-(4-Fluor-benzyl)-4-methyl-3-p-tolyl-1H-pyrrol-2-yl]-[4-(5-trifluormethyl-pyridin-2-yl)-piperazin-1-yl]-methanon, |
| [204] | 1,4-Dimethyl-3-phenyl-1H-pyrrol-2-carbonsäure 3-trifluormethoxy-benzylamid, |
| [205] | [1-(4-Fluor-benzyl)-4-methyl-3-p-tolyl-1H-pyrrol-2-yl]-[4-(4-methyl-benzoyl)-piperazin-1-yl]-methanon, |
| [206] | [4-(3,4-Dichlor-phenyl)-piperazin-1-yl]-[1-(4-fluor-benzyl)-4-methyl-3-p-tolyl-1H-pyrrol-2-yl]-methanon, |
| [207] | 3-(4-Chlor-phenyl)-1-ethyl-4-methyl-1H-pyrrol-2-carbonsäure 2,6-dimethoxy-benzylamid, |
| [208] | 4-Methyl-1-(4-methyl-benzyl)-3-p-tolyl-1H-pyrrol-2-carbonsäure [2-(3,4-dimethoxy-phenyl)-ethyl]-amid, |
| [209] | 1-{4-[3-(4-Chlor-phenyl)-1-isobutyl-4-methyl-1H-pyrrol-2-carbonyl]-piperazin-1-yl}-2-(3,4-difluor-phenyl)-ethanon, |
| [210] | [1-(4-Fluor-benzyl)-4-methyl-3-p-tolyl-1H-pyrrol-2-yl]-[4-(2-methoxy-phenyl)-piperidin-1-yl]-methanon, |
| [211] | [3-Furan-2-yl-4-methyl-1-(4-trifluormethyl-benzyl)-1H-pyrrol-2-yl]-thiomorpholin-4-yl-methanon, |
| [212] | 1,4-Dimethyl-3-phenyl-1H-pyrrol-2-carbonsäure (2-thiophen-2-yl-ethyl)-amid, |
| [213] | 4-Diethylamino-benzoesäure N'-(1-bulyl-4'-methyl-3-p-tolyl-1H-pyrrol-2-carbonyl)-hydrazide, |
| [214] | {1-[1-(4-Fluor-benzyl)-3-(4-methoxy-phenyl)-4-methyl-1H-pyrrol-2-carbonyl]-pyrrolidin-3-yl}-carbaminsäure tert-butyl ester, |
| [215] | 2-{4-[3-(4-Chlor-phenyl)-1-isobutyl-4-methyl-1H-pyrrol-2-carbonyl]-piperazin-1-yl}-1-pyrrolidin-1-yl-ethanon, |
| [216] | [4-(2,3-Dihydro-benzo[1,4]dioxine-2-carbonyl)-piperazin-1-yl]-(1,4-dimethyl-3-phenyl-1H-pyrrol-2-yl)-methanon, |
| [217] | 3-Furan-2-yl-1,4-dimethyl-1H-pyrrol-2-carbonsäure 3,4-dimethoxy-benzylamid, |
| [218] | [3-Furan-2-yl-4-methyl-1-(4-trifluormethyl-benzyl)-1H-pyrrol-2-yl]-(4-pyridin-2-yl-piperazin-1-yl)-methanon, |
| [219] | [4-(Furan-2-carbonyl)-piperazin-1-yl]-[3-furan-2-yl-4-methyl-1-(4-trifluormethyl-benzyl)-1H-pyrrol-2-yl]-methanon, |
| [220] | 3-Furan-2-yl-4-methyl-1-(4-trifluormethyl-benzyl)-1H-pyrrol-2-carbonsäure (4-tert-butyl-phenyl)-amid, |
| [221] | 2-{4-[3-(4-Chlor-phenyl)-1-isobutyl-4-methyl-1H-pyrrol-2-carbonyl]-piperazin-1-yl}-benzonitril, |
| [222] | (3-Furan-2-yl-1,4-dimethyl-1H-pyrrol-2-yl}-[4-(4-trifluormethyl-pyridin-2-yl)-piperazin-1-yl]-methanon, |
| [223] | 1,4-Dimethyl-3-phenyl-1H-pyrrol-2-carbonsäure 3,5-difluor-benzylamid, |
| [224] | 1,4-Dimethyl-3-phenyl-1H-pyrrol-2-carbonsäure (pyridin-4-ylmethyl)-amid, |
| [225] | 2-[4-(3-Furan-2-yl-1,4-dimethyl-1H-pyrrol-2-carbonyl)-piperazin-1-yl]-benzonitril, |
| [226] | 3-Furan-2-yl-1,4-dimethyl-1H-pyrrol-2-carbonsäure methyl-(2-pyridin-2-yl-ethyl)-amid, |
| [227] | 3-Furan-2-yl-1,4-dimethyl-1H-pyrrol-2-carbonsäure (3-methoxy-benzyl)-(tetrahydro-furan-2-ylmethyl)-amid, |
| [228] | 3-Furan-2-yl-1,4-dimethyl-1H-pyrrol-2-carbonsäure (4-phenoxy-phenyl)-amid, |
| [229] | 3-Furan-2-yl-1,4-dimethyl-1H-pyrrol-2-carbonsäure pentylamid, |
| [230] | 2-(3,4-Difluor-phenyl)-1-[4-(3-furan-2-yl-1,4-dimethyl-1H-pyrrol-2-carbonyl)-piperazin-1-yl]-ethanon, |
| [231] | 3-Furan-2-yl-1,4-dimethyl-1H-pyrrol-2-carbonsäure phenylamid, |
| [232] | [4-(2,4-Dimethoxy-phenyl)-piperazin-1-yl]-(3-furan-2-yl-1,4-dimethyl-1H-pyrrol-2-yl)-methanon, |
| [233] | 3-Furan-2-yl-1,4-dimethyl-1H-pyrrol-2-carbonsäure [2-(3,4-dichlorphenyl)-ethyl]-amid, |
| [234] | 3-Furan-2-yl-1,4-dimethyl-1H-pyrrol-2-carbonsäure [2-(2-chlor-phenoxy)-ethyl]-amid, |
| [235] | 3-Furan-2-yl-1,4-dimethyl-1H-pyrrol-2-carbonsäure 3-methoxy-benzylamid, |
| [236] | 3-Furan-2-yl-4-methyl-1-(4-trifluormethyl-benzyl)-1H-pyrrol-2-carbonsäure phenethyl-amid, |
| [237] | 3-Furan-2-yl-1,4-dimethyl-1H-pyrrol-2-carbonsäure cyclopentylamid, |
| [238] | 3-Furan-2-yl-1,4-dimethyl-1H-pyrrol-2-carbonsäure (pyridin-2-ylmethyl) amid, |
| [239] | 3-Furan-2-yl-4-methyl-1-(4-trifluormethyl-benzyl)-1H-pyrrol-2-carbonsäure [2-(3,4-dimethoxy-phenyl)-ethyl]-amid, |
| [240] | [1-(4-Brom-benzyl)-4-methyl-3-p-tolyl-1H-pyrrol-2-yl]-(2,6-dimethyl-morpholin-4-yl)-methanon, |
| [241] | 1,4-Dimethyl-3-phenyl-1H-pyrrol-2-carbonsäure (2-p-tolyl-ethyl)-amid, |
| [242] | (1,4-Dimethyl-3-phenyl-1H-pyrrol-2-yl)-[4-(4-fluor-phenyl)-piperazin-1-yl]-methanon und |
| [243] | 4-Methyl-1-(4-methyl-benzyl)-3-p-tolyl-1H-pyrrol-2-carbonsäure [2-(1H-indol-3-yl)-ethyl]-methyl-amid, |

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

Ein weiterer Gegenstand der vorliegenden Erfindung ist ein Verfahren zur Herstellung von 1,3-disubstituierten 4-Methyl-1H-pyrrol-2-carbonsäureamiden der vorstehend angegebenen allgemeinen Formel I, worin wenigstens eine Verbindung der allgemeinen Formel II, 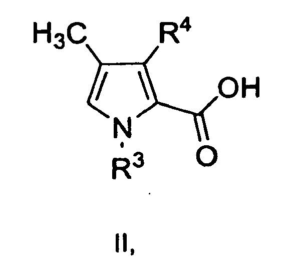 worin R³ und R⁴ die vorstehend genannte Bedeutung haben, ggf. in wenigstens einem Reaktionsmedium, vorzugsweise in wenigstens einem Reaktionsmedium ausgewählt aus der Gruppe bestehend aus Diethylether, Tetrahydrofuran, Acetonitril, Methanol, Ethanol, (1,2)-Dichlorethan, Dimethylformamid, Dichlormethan und entsprechenden Mischungen, ggf. in Gegenwart wenigstens eines geeigneten Kopplungsmittels, vorzugsweise in Gegenwart wenigstens eines Kopplungsmittels ausgewählt aus der Gruppe bestehend aus 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodiimid (EDCI), Diisoproylcarbodiimid, 1,1'-Carbonyl-diimidazol (CDI), N-[(Dimethyamino)-1H-1, 2, 3-triazolo[4, 5-b]pyridino-1-ylmethylen]-N-methylmethanaminium hexafluorophosphat N-oxid (HATU), 0-(Benzotriazol-1-yl)-N,N,N',N'-tetramethyluroniom hexafluorophosphat (HBTU), 0-(Benzotriazol-1-yl)-N,N,N',N'-tetramethyluronium-tetrafluoroborat (TBTU) und 1-Hydroxy-7-azabenzotriazol (HOAt), ggf. in Gegenwart wenigstens einer Base, vorzugsweise in Gegenwart wenigstens einer Base ausgewählt aus der Gruppe bestehend aus Triethylamin, Pyridin, Dimethylaminopyridin, N-Methylmorpholin und Diisopropylethylamin, vorzugsweise bei einer Temperatur von - 70 °C bis 100 °C durch Umsetzung mit wenigstens einer Verbindung der allgemeinen Formel HNR¹R², worin R¹ und R² die vorstehend genannte Bedeutung haben, in eine entsprechende Verbindung der allgemeinen Formel I, ggf. in Form eines entsprechenden Salzes überführt wird, 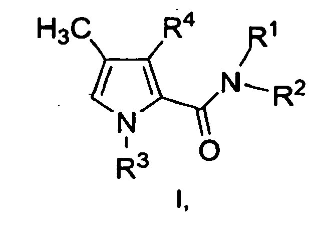 worin R¹, R², R³ und R⁴ die vorstehend genannte Bedeutung haben, und diese ggf. gereinigt und/oder isoliert wird.

Verbindungen der allgemeinen Formel II lassen sich wie im folgenden Schema 1. dargestellt erhalten.

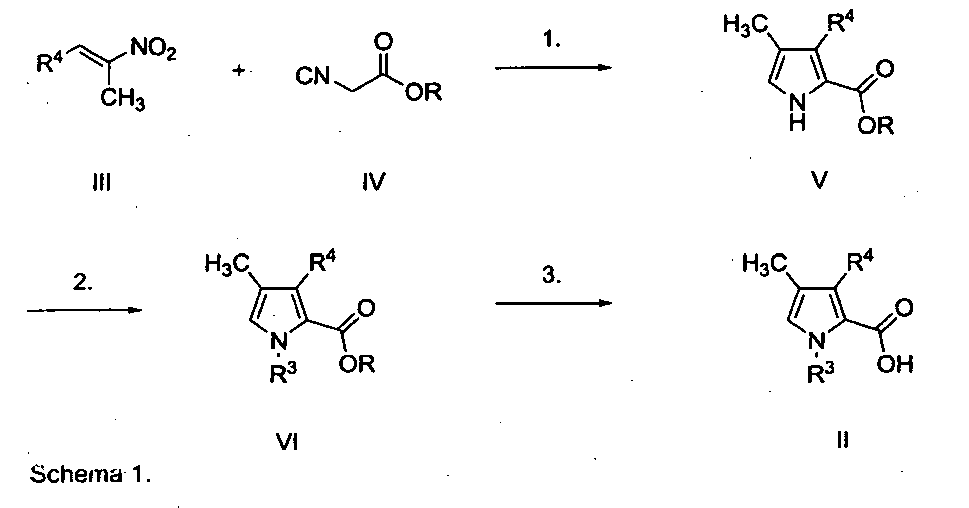

In Stufe 1. wird wenigstens eine Verbindung der allgemeinen Formel II, worin R⁴ die vorstehend genannte Bedeutung hat, in wenigstens einem organischen Lösungsmittel, vorzugsweise in wenigstens einem organischen Lösungsmittel ausgewählt aus der Gruppe bestehend aus Ethanol, Isopropanol, Tetrahydrofuran und entsprechenden Mischungen, in Gegenwart wenigstens einer Base, vorzugsweise in Gegenwart wenigstens einer Guanidinbase, besonders bevorzugt in Gegenwart von 1,5,7-Triazabicyclo[4.4.0]dec-5-en, das ggf. polymergebunden sein kann, mit wenigstens einer Verbindung der allgemeinen Formel IV, worin R für einen linearen oder verzweigten C₁₋₁₀-Alkyl-Rest oder einen Benzyl-Rest stehen kann, zu wenigstens einer Verbindung der allgemeinen Formel V, worin R und R⁴ die vorstehend genannte Bedeutung haben, umgesetzt, und diese ggf. gereinigt und/oder isoliert.

In Stufe 2. wird wenigstens eine Verbindung der allgemeinen Formel V in wenigstens einem organischen Lösungsmittel, vorzugsweise in wenigstens einem organischen Lösungsmittel ausgewählt aus der Gruppe bestehend aus Diethylether, Di-isopropylether, Tetrahydrofuran und entsprechenden Mischungen, in Gegenwart wenigstens einer Base, vorzugsweise in Gegenwart wenigstens einer Base ausgewählt aus der Gruppe bestehend aus Diisopropylethylamin, Triethylamin, Pyridin, Diethylamin, Diisopropylamin, Butyllithium, Lithiumdiisopropylamid, Natriumhydrid, Kaliumhydrid, Lithiumaluminiumhydrid und Natriumborhydrid, mit wenigstens einer Verbindung der allgemeinen Formel R³-LG, worin R³ die vorstehend genannte Bedeutung hat und LG für eine Abgangsgruppe, vorzugsweise für ein Halogentatom, besonders bevorzugt für Chlor oder Brom, steht, zu wenigstens einer Verbindung der allgemeinen Formel VI, worin R³, R⁴ und R die vorstehend genannte Bedeutung haben, umgesetzt, und diese ggf. gereinigt und/oder isoliert.

In Stufe 3. wird wenigstens eine Verbindung der allgemeinen Formel VI in wenigstens einem Lösungsmittel, vorzugsweise in wenigstens einem Lösungsmittel ausgewählt aus der Gruppe bestehend aus Ethanol, Dichlormethan, Methanol, Acetonitril, Tetrahydrofuran, Wasser und entsprechenden Mischungen, in Gegenwart wenigstens einer Säure, vorzugsweise in Gegenwart wenigstens einer Säure ausgewählt aus der Gruppe bestehend aus Schwefelsäure, Salzsäure und LewisSäuren oder in Gegenwart wenigstens einer Base, vorzugsweise in Gegenwart wenigstens einer Base ausgewählt aus der Gruppe bestehend aus Kaliumhydroxid, Lithiumhydroxid, Natriumhydroxid, Natriumcarbonat und Kaliumcarbonat, zu wenigstens einer Verbindung der allgemeinen Formel II, worin R³ und R⁴ die vorstehend genannte Bedeutung haben, umgesetzt, und diese ggf. gereinigt und/oder isoliert.

Die erfindungsgemäßen 1,3-disubstituierten 4-Methyl-1H-pyrrol-2-carbonsäureamide der vorstehend angegebenen allgemeinen Formel I und ggf. entsprechende. Stereoisomere sowie jeweils die entsprechenden Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine erfindungsgemäße 1,3-disubstituierte 4-Methyl-1H-pyrrol-2-carbonsäureamid-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Das erfindungsgemäße Arzneimittel eignet sich zur Noradrenalin-Rezeptor-Regulation, insbesondere zur Hemmung der Noradrenalin-Wiederaufnahme (Noradrenalin-Uptake) und/oder zur 5-HT-Rezeptor-Regulation, insbesondere zur Hemmung der 5-Hydroxy-Tryptophan-Wiederaufnahme (5-HT-Uptake) und/oder zur Opioid-Rezeptor-Regulation und/oder zur Batrachotoxin-(BTX)-Rezeptor-Regulation.

Bevorzugt eignet sich das erfindungsgemäße Arzneimittel zur Prophylaxe und/oder Behandlung von Störungen und/oder Krankheiten, die zumindest teilweise durch Noradrenalin-Rezeptoren und/oder 5-HT-Rezeptoren und/oder Opioid-Rezeptoren und/oder Batrachotoxin-(BTX)-Rezeptoren vermittelt werden.

Bevorzugt eignet sich das erfindungsgemäße Arzneimittel zur Prophylaxe und/oder Behandlung von Schmerz, vorzugsweise von Schmerz ausgewählt aus der Gruppe bestehend aus akutem Schmerz, chronischem Schmerz und neuropathischem Schmerz; zur Prophylaxe und/oder Behandlung von Migräne; Depressionen; Harninkontinenz; Husten; neurodegenerativen Erkrankungen, vorzugsweise ausgewählt aus der Gruppe bestehend aus Morbus Parkinson, Morbus Huntington, Morbus Alzheimer und Multipler Sklerose; Störungen der Nahrungsaufnahme, vorzugsweise ausgewählt aus der Gruppe bestehend aus Bulimie, Anorexie, Fettsucht und Kachexie; kognitiven Dysfunktionen, vorzugsweise Gedächtnisstörungen; Epilepsie; Diarrhoe; Pruritus; Alkohol- und/oder Drogen- und/oder Medikamentenmißbrauch; Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit; zur Prophylaxe und/oder Verminderung einer Toleranzentwicklung gegenüber Medikamenten, insbesondere Medikamenten auf Basis von Opioiden; zur Regulation der Nahrungsaufnahme; zur Modulation der Bewegungsaktivität; zur Regulation des kardiovaskulären Systems; zur Lokalanästhesie; zur Anxiolyse; zur Vigilanzsteigerung; zur Libidosteigerung; zur Diurese und/oder zur Antinatriurese.

Ganz besonders bevorzugt eignet sich das erfindungsgemäße Arzneimittel zur Prophylaxe und/oder Behandlung von Schmerzen, vorzugsweise von akuten Schmerzen, chronischen Schmerzen oder neuropathischen Schmerzen.

Ein weiterer Gegenstand der vorliegenden Erfindung ist die Verwendung wenigstens einer erfindungsgemäßen 1,3-disubstituierten 4-Methyl-1H-pyrrol-2-carbonsäureamid-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Noradrenalin-Rezeptor-Regulation, insbesondere zur Hemmung der Noradrenalin-Wiederaufnahme (Noradrenalin-Uptake) und/oder zur 5-HT-Rezeptor-Regulation, insbesondere zur Hemmung der 5-Hydroxy-Tryptophan-Wiederaufnahme (5-HT-Uptake) und/oder zur Opioid-Rezeptor-Regulation und/oder zur Batrachotoxin-(BTX)-Rezeptor-Regulation.

Bevorzugt ist die Verwendung wenigstens einer erfindungsgemäßen 1,3-disubstituierten 4-Methyl-1H-pyrrol-2-carbonsäureamid-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Störungen und/oder Krankheiten, die zumindest teilweise durch Noradrenalin-Rezeptoren, 5-HT-Rezeptoren, Opioid-Rezeptoren und/oder Batrachotoxin-(BTX)-Rezeptoren vermittelt werden.

Besonders bevorzugt ist die Verwendung wenigstens einer erfindungsgemäßen 1,3-disubstituierten 4-Methyl-1H-pyrrol-2-carbonsäureamid-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz und neuropathischem Schmerz; zur Prophylaxe und/oder Behandlung von Migräne; Depressionen; Harninkontinenz; Husten; neurodegenerativen Erkrankungen, vorzugsweise ausgewählt aus der Gruppe bestehend aus Morbus Parkinson, Morbus Huntington, Morbus Alzheimer und Multipler Sklerose; Störungen der Nahrungsaufnahme, vorzugsweise ausgewählt aus der Gruppe bestehend aus Bulimie, Anorexie, Fettsucht und Kachexie; kognitiven Dysfunktionen, vorzugsweise Gedächtnisstörungen; Epilepsie; Diarrhoe; Pruritus; Alkohol- und/oder Drogen- und/oder Medikamentenmißbrauch; Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit; zur Prophylaxe und/oder Verminderung einer Toleranzentwicklung gegenüber Medikamenten, insbesondere Medikamenten auf Basis von Opioiden; zur Regulation der Nahrungsaufnahme; zur Modulation der Bewegungsaktivität; zur Regulation des kardiovaskulären Systems; zur Lokalanästhesie; zur Anxiolyse; zur Vigilanzsteigerung; zur Libidosteigerung; zur Diurese und/oder zur Antinatriurese.

Ganz besonders ist die Verwendung wenigstens einer erfindungsgemäßen 1,3-disubstituierten 4-Methyl-1H-pyrrol-2-carbonsäureamid-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Schmerzen, vorzugsweise von akuten Schmerzen, chronischen Schmerzen oder neuropathischen Schmerzen.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einer erfindungsgemäßen substituierten 4-Methyl-1H-pyrrol-2-carbonsäureamid-Verbindung der vorstehend angegebenen allgemeinen Formel I, ggf. in Form ihres reinen Stereoisomeren, insbesondere Enantiomeren oder Diastereomeren, ihres Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden 1,3-disubstituierten 4-Methyl-1H-pyrrol-2-carbonsäureamid-Verbindungen in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweiligen 1,3-disubstituierten 4-Methyl-1H-pyrrol-2-carbonsäureamid-Verbindungen auch verzögert freisetzen.

Die Herstellung der erfindungsgemäßen Arzneimittel erfolgt mittels üblichen, aus dem Stande der Technik wohl bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge der jeweiligen 1,3-disubstituierten 4-Methyl-1H-pyrrol-2-carbonsäureamid-Verbindung kann variieren und ist beispielsweise abhängig vom Gewicht oder Alter des Patienten sowie von der Applikationsart, der Indikation und dem Schweregrad der Erkrankung. Üblicherweise werden 0,005 bis 5000 mg/kg, vorzugsweise 0,05 bis 500 mg/kg, besonders bevorzugt 0,05 bis 50 mg/kg, Körpergewicht des Patienten wenigstens einer solchen Verbindung appliziert.

### Pharmakologische Methoden

### 1. Methode zur Bestimmung der Noradrenalin- und der 5HT-Uptake-Inhibierung:

Für in vitro Studien wurden Synaptosomen aus Rattenhimarealen frisch isoliert, wie in der Veröffentlichung "The isolation of nerve endings from brain" von E.G. Gray und V.P. Whittaker, J. Anatomy 96, Seiten 79-88, 1962, beschrieben. Die entsprechende Literaturbeschreibung wird hiermit als Referenz eingeführt und gilt als Teil der Offenbarung.

Das Gewebe (Hypothalamus für die Bestimmung der Noradrenalin-Uptake-Inhibierung und Mark und Pons für die Bestimmung der 5HT-Uptake-Inhibierung) wurde in eisgekühlter 0,32 M Sucrose (100 mg Gewebe / 1mL) in einem Glas-Homogenisierer mit Teflonstößel homogenisiert, indem fünf volle Auf- und Abschläge bei 840 Umdrehungen /Minute benutzt wurden. Das Homogenat wurde bei 4°C für 10 Minuten bei 1000 g zentrifugiert. Nach anschließender Zentrifugierung bei 17000 g für 55 Minuten erhält man die Synaptosomen (P₂-Fraktion), die in 0,32 M Glucose (0,5 mU 100 mg des ursprünglichen Gewichts) noch einmal suspendiert wurden. Der jeweilige Uptake wurde in einer 96-well Mikrotiterplatte gemessen. Das Volumen betrug 250 µl und die Inkubation erfolgte bei Raumtemperatur (ca. 20-25 °C) unter O₂ Atmosphäre.

Die Inkubationszeit betrug 7,5 Minuten für [³H]-NA und 5 Minuten für [³H]-5-HT. Anschließend wurden die 96 Proben durch eine Unifilter GF/B^{®} Mikrotiterplatte (Packard) filtriert und mit 200 mL inkubierten Puffer mit Hilfe eines "Brabdel Cell-Harvester MPXRI-96T" gewaschen. Die Unifilter GF/B Platte wurde bei 55°C 1 h getrocknet. Im Anschluß wurde die Platte mit einem Back seal^{®} (Packard) verschlossen und 35 µl Szintilationsflüssigkeit pro Well (Ultima Gold^{®}, Packard) versetzt. Nach dem Verschließen mit einem top seal^{®} (Packard) wurde, nach der Einstellung des Gleichgewichts (etwa 5 h), die Radioaktivität in einem "Trilux 1450 Microbeta" (Wallac) bestimmt.

Folgende Kenndaten wurden für den NA-Transporter ermittelt:
NA-Uptake : Km = 0,32 ± 0,11 µM

Die Menge des bei der vorstehenden Bestimmung eingesetzten Proteins entsprach den aus der Literatur bekannten Werten, wie z.B. in "Protein measurement with the folin phenol reagent", Lowry et al., J. Biol. Chem., 193, 265-275, 1951 beschrieben. Eine detaillierte Methodenbeschreibung kann auch der Literatur, beispielsweise aus M.Ch. Frink, H.-H. Hennies, W. Engelberger, M. Haurand und B. Wilffert (1996) Arzneim.-Forsch./Drug Res. 46 (III), 11, 1029-1036, entnommen werden. Die entsprechenden Literaturbeschreibungen werden hiermit als Referenz eingeführt und gelten als Teil der Offenbarung.

### II. Methode zur Bestimmung der Affinität zur Batrachotoxin-(BTX)-Bindungsstelle des Natriumkanals:

Die Bindungsstelle 2 des Natriumkanals ist die sogenannte Batrachotoxin-(BTX) Bindungsstelle. Als Ligand wird [³H]-Batrachotoxinin A20 α-Benzoat (10 nM im Ansatz) eingesetzt. Die lonenkanal-Partikel (Synaptosomen) werden aus dem Ratten Cerebrocortex angereichert, wie in der Veröffentlichung von Gray und Whittaker, 1962, J. Anat. 76, 79-88 beschrieben. Die entsprechende Beschreibung wird hiermit als Referenz eingeführt und gilt als Teil der vorliegenden Offenbarung. Als unspezifische Bindung ist die Radioaktivität definiert, die in Gegenwart von Veratridin (3 x 10⁻⁴ M im Ansatz) gemessen wird.

Die Assaybedingungen werden entsprechend der Veröffentlichung von Pauwels, Leysen und Laduron durchgeführt, wie in Eur. J. Pharmacol. 124, 291-298 beschrieben. Die entsprechende Beschreibung wird hiermit als Referenz eingeführt und gilt als Teil der vorliegenden Offenbarung.

Abweichend von dieser Vorschrift wird der Gesamtansatz auf 250 µl verkleinert, so daß der Assay auf 96-well Mikrotiterplatten durchgeführt werden kann. Die Inkubationszeit in diesen Mikrotiterplatten beträgt zwei Stunden bei Raumtemperatur (ca. 20-25°C).

**Folgende Kenndaten wurden für den K_{D}-Wert der Bindungsstelle ermittelt:**
K_{D}: 24,63 ± 1,56 nM.

### Beispiele

Die Ausbeuten der hergestellten Verbindungen sind nicht optimiert.

Alle Temperaturen sind unkorrigiert.

### Abkürzungen

| | |
|---|---|
| aq. | wäßrig |
| DCM | Dichlormethan |
| EDCI | N'-(3-Dimethylaminopropyl)-N-ethylcarbodiimid |
| EtOAc | Ethylacetat |
| ges. | gesättigt |
| h | Stunden |
| HOAt | 1-Hydroxy-7-azabenzotriazol |
| min | Minuten |
| MeOH | Methanol |
| NMR | Kernresonanzspektroskopie |
| RT | Raumtemperatur |
| THF | Tetrahydrofuran |

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach den für den Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt. Die Mischungsverhältnisse von Lösungsmitteln, Laufmitteln oder für chromatographische Untersuchungen sind stets in Volumen/Volumen angegeben.

Die Analytik erfolgte durch Massenspektroskopie und NMR.

### Allgemeines Verfahren zur Synthese der erfindungsgemäßen Beispielverbindungen

### Stufe 1.

Zu einer Reaktionsmischung eines Nitroolefins der allgemeinen Formel III (14 mmol) und Isocyanoessigsäureethylester(14 mmol) in Isopropanol (10 mL) und THF (10 mL) wird 1,5,7-Triazabicyclo[4.4.0]dec-5-en (auf Polystyrol, 72.6 mmol/g, 28 mmol) gegeben und die Reaktionsmischung wird über Nacht bei RT unter Stickstoff als Schutzgas gerührt. Die Reaktionsmischung wird abfiltriert und der Rückstand mit Isopropanol und THF gewaschen und so das gewünschte Produkt der allgemeinen Formel V erhalten.

### Stufe 2.

Zu einer Lösung der Verbindung der allgemeinen Formel V (12 mmol) in THF (20 mL) wird bei 0°C und unter Stickstoff als Inertgas Natriumhydrid (60%-ig in Mineralöl, 24 mmol) gegeben und die Reaktionsmischung für 30 min gerührt. Anschließend wird zur Reaktionsmischung eine Verbindung der allgemeinen Formel R³-LG (24 mmol) gegeben und die Reaktionsmischung wird für 1 h bis 2 h bei 0°C und über Nacht oder Wochenende bei RT gerührt. Nach der Zugabe von wenigen Tropfen Wasser und aq. ges. NaCl-Lösung (200 ml) wird mit DCM (2 x 100 mL) extrahiert Nach Trocknen der vereinigten organischen Phasen über Natriumsulfat, Filtration und Entfemen des Lösungsmittels erfolgt gegebenenfalls die Reinigung mittels Säulen-chromathographie und das gewünschte Produkt der allgemeinen Formel VI wird erhalten.

### Stufe 3.

Eine Reaktionsmischung bestehend aus einer Verbindung der allgemeinen Formel VI (6 mmol) und Natriumhydroxid (120 mmol) in MeOH (180 mL) und Wasser (60 mL) wird für 1 h bis 3 h unter Rückfluss erhitzt. Nach Entfemen des Lösungsmittels wird aq. 1 N NaOH (400 mL) zugegeben und mit EtOAc (3 x 40 ml) extrahiert. Nach Trocknen der vereinigten organischen Phasen über Natriumsulfat, Filtration und Entfemen des Lösungsmittels wird das Produkt der allgemeinen Formel II erhalten, welche ohne weitere Reinigung oder gereinigt mittels Säulenchromathographie verwendet werden kann.

### Reaktion von Aminen der allgemeinen Formel HNR¹R² mit Carbonsäuren der allgemeinen Formel II

Zu einer Reaktionsmischung bestehend aus einer Verbindung der allgemeinen Formel II (0.1 mmol) und einem Amin der allgemeinen Formel HNR¹R² (0.1 mmol) in DCM (1 mL) werden bei 0°C EDCI (0.1 mmol) und HOAt (0.01 mmol) gegeben und die Reaktionsmischung wird für 30 min bei 0°C und über Nacht bei RT gerührt. Nach Entfemen des Lösungsmittels wird der Rückstand in DCM (1 mL) gelöst, mit aq. ges. NaCl-Lösung (1 mL) gewaschen und die abgetrennte wässrige Phase mit DCM (2 x 1 mL) extrahiert. Das Lösungsmittel der vereinigten organischen Phasen wird entfernt und die gewünschten Produkte der allgemeinen Formel I nach Reinigung mittels präparativer HPLC erhalten.

### Synthese von 4-Methyl-3-phenyl-1 H-pyrrol-2-carbonsäureethylester

Zu einer Reaktionsmischung aus trans-β-Methyl-β-nitrostyrol (997 mg, 6.11 mmol) und Isocyanoessigsäureethylester (691 mg, 6.11 mmol) in Isopropanol (5 mL) und THF (5 mL) wurde 1,5,7-Triazabicyclo[4.4.0]dec-5-en auf Polystyrol (4.7 g, 2.6 mmol/g, 12.2 mmol) gegeben und die Reaktionsmischung wurde über Nacht bei RT unter Stickstoff als Schutzgas gerührt. Die Reaktionsmischung wurde abfiltriert und der Rückstand mit Isopropanol und THF gewaschen. Man erhielt das gewünschte Produkt 4-Methyl-3-phenyl-1 H-pyrrol-2-carbonsäureethylester (1.25 g, 89 % der Theorie).

### Synthese von 1,4-Dimethyl-3-phenyl-1H-pyrrol-2-carbonsäureethylester

Zu einer Lösung von 4-Methyl-3-phenyl-1 H-pyrrol-2-carbonsäureethylester (1.25 g, 5.45 mmol) in THF (10 ml) wurde bei 0 °C und unter Stickstoff als Inertgas Natriumhydrid (436 mg, 60%-ig in Mineralöl, 10.9 mmol) gegeben und die Reaktionsmischung wurde für 15 min gerührt. Anschließend wurde zur Reaktionsmischung Methyliodid (1.547 g, 10.9 mmol) gegeben und die Reaktionsmischung wurde für 1 h bei 0°C und über Nacht bei RT. Nach der Zugabe von wenigen Tropfen Wasser und aq. ges. NaCl-Lösung (250 mL) wurde mit DCM (2 x 250 mL) extrahiert. Nach Trocknen der vereinigten organischen Phasen über Natriumsulfat, Filtration und Entfernen des Lösungsmittels erfolgte die Reinigung mittels Säulenchromathographie. Es wurde das gewünschte Produkt 1,4-Dimethyl-3-phenyl-1H-pyrrol-2-carbonsäureethylester (1.02 g, 77 % der Theorie) erhalten.

### Synthese von 1,4-Dimethyl-3-phenyl-1 H-pyrrol-2-carbonsäure

Eine Reaktionsmischung von 1,4-Dimethyl-3-phenyl-1 H-pyrrol-2-carbonsäureethylester (336 mg, 1.38 mmol) und Natriumhydroxid (1.10 g, 28 mmol) in MeOH (45 ml) und Wasser (15 ml) wurde für 1 h unter Rückfluss erhitzt. Nach Entfernen des Lösungsmittels wurde wässrige 1 N NaOH (100 mL) zugegeben und mit EtOAc (2 x 20 mL) extrahiert. Nach Trocknen der vereinigten organischen Phasen über Natriumsulfat, Filtration und Entfernen des Lösungsmittels wurde 4-Dimethyl-3-phenyl-1H-pyrrol-2-carbonsäure (270 mg, 91 % der Theorie) erhalten.

### Beispielverbindung 241:

### 1,4-Dimethyl-3-phenyl-1 H-pyrrol-2-carbonsäure (2-p-tolyl-ethyl)-amid

Zu einer Reaktionsmischung von 4-Dimethyl-3-phenyl-1 H-pyrrol-2-carbonsäure (270 mg, 1.25 mmol) und 2-(Tolyl)ethylamin (169 mg, 1.25 mmol) in DCM (5 mL) wurde bei 0°C EDCl (265 mg, 1.38 mmol) und HOAt (17 mg, 0.12 mmol) gegeben und die Reaktionsmischung für 30 min bei 0°C und über Nacht bei RT gerührt. Nach Entfernen des Lösungsmittels erfolgte die Reinigung mittels Säulen-. chromathographie und es wurde das gewünschte Produkt 1,4-Dimethyl-3-phenyl-1 H-pyrrol-2-carbonsäure (2-p-tolyl-ethyl)-amid (252 mg, 60 % der Theorie) erhalten.

### Beispielverbindung 242:

### (1,4-Dimethyl-3-phenyl-1 H-pyrrol-2-yl)-[4-(4-fluor-phenyl)-piperazin-1 -yl]-methanon

Zu einer Reaktionsmischung von 4-Dimethyl-3-phenyl-1 H-pyrrol-2-carbonsäure (260 mg, 1.21 mmol) und 1-(4-Fluorphenyl)piperazin (215 mg, 1.21 mmol) in DCM (5 ml) wurde bei 0°C EDCI (255 mg, 1.33 mmol) und HOAt (16 mg, 0.12 mmol) gegeben und die Reaktionsmischung wurde für 30 min bei 0°C und über Nacht bei RT gerührt. Nach Entfernen des Lösungsmittels erfolgte die Reinigung mittels Säulenchromathographie und es wurde (1,4-Dimethyl-3-phenyl-1 H-pyrnol-2-yl)-[4-(4-fluor-phenyl)-piperazin-1-yl]-methanon (362 mg, 79 % der Theorie) erhalten.

### Synthese von 4-Methyl-3-p-tolyl-1 H-pyrrol-2-carbonsäureethylester

Zu einer Reaktionsmischung von 4-Methyl-β-ethyl-β-nitrostyrol (1.23 g, 6.94 mmol) und Isocyanoessigsäureethylester (788 mg, 6.90 mmol) in Isopropanol (5 mL) und THF (5 mL) wurde 1,5,7-Triazabicyclo[4.4.0]dec-5-en auf Polystyrol (5.36 g, 2.6 mmol/g, 13.9 mmol) gegeben und die Reaktionsmischung wurde über Nacht bei RT unter Stickstoff als Schutzgas gerührt. Die Reaktionsmischung wurde abfiltriert und der Rückstand mit Isopropanol und THF gewaschen. Es wurde das gewünschte Produkt 4-Methyl-3-p-tolyl-1 H-pyrrol-2-carbonsäureethylester (1.42 g, 85 % der Theorie) erhalten.

### Synthese von 4-Methyl-1-(4-methyl-benzyl)-3-p-tolyl-1 H-pyrrol-2-carbonsäureethylester

Zu einer Lösung von 4-Methyl-3-p-tolyl-1 H-pyrrol-2-carbonsäureethylester (1.42 g, 5.84 mmol) in THF (10 mL) wurde bei 0°C und unter Stickstoff als Inertgas Natriumhydrid (470 mg, 60%-ig in Mineralöl, 11.8 mmol) gegeben und die Reaktionsmischung wurde für 15 min gerührt. Anschließend wurde zur Reaktionsmischung p-Brombenzybromid (2.16 g, 11.7 mmol) gegeben und die Reaktionsmischung wrude für 30 min bei 0°C und über Nacht bei RT gerührt. Nach Zugabe von wenigen Tropfen Wasser und aq. ges. NaCl-Lösung (100 mL) wurde mit DCM (2 x 50 mL) extrahiert. Nach Trocknen der vereinigten organischen Phasen über Natriumsulfat, Filtration und Entfernen des Lösungsmittels erfolgte die Reinigung über Säulenchromathographie. Es wurde 4-Methyl-1-(4-methyl-benzyl)-3-p-tolyl-1 H-pyrrol-2-carbonsäureethylester (1.03 g, 51 % der Theorie) erhalten.

### Synthese von 4-Methyl-1-(4-methyl-benzyl)-3-p-tolyl-1H-pyrrol-2-carbonsäure

Eine Reaktionsmischung von 4-Methyl-1-(4-methyl-benzyl)-3-p-tolyl-1H-pyrrol-2-carbonsäureethylester (550 mg, 1.58 mmol) und NaOH (1.26 g, 32 mmol) in MeOH (45 mL) und H₂O (15 mL) wurde für 1 h unter Rückfluss erhitzt. Nach Entfernen des Lösungsmittels wurde wässrige 1 N NaOH (100 ml) zugegeben und mit EtOAc (2 x 20 ml) extrahiert. Nach Trocknen der vereinigten organischen Phasen über Na₂SO₄, Filtration und Entfernen des Lösungsmittels erhielt man das gewünschte Produkt 4-Methyl-1-(4-methyl-benzyl)-3-p-tolyl-1 H-pyrrol-2-carbonsäure, welches ohne weitere Reinigung verwendet wurde.

### Beispielverbindung 69:

### Synthese von [4-(2-Diethylamino-ethyl)-piperazin-1-yl]-[4-methyl-1-(4-methylbenzyl)-3-p-tolyl-1 H-pyrrol-2-yl]-methanon

Zu einer Reaktionsmischung von 4-Methyl-1 -(4-methyl-benzyl)-3-p-tolyl-1 H-pyrrol-2-carbonsäure (252 mg, 0.79 mmol) und 1-(2-Diethylaminoethyl)-piperazin (146 mg, 0.79 mmol) in DCM (5 mL) wurde bei 0°C EDCI (166 mg, 0.87 mmol) und HOAt (11 mg, 0.08 mmol) gegeben und die Reaktionsmischung wurde für 30 min bei 0°C und über Nacht bei RT gerührt. Nach Entfernen des Lösungsmittels erfolgte die Reinigung mittels Säulenchromathographie und es wurde das gewünschte Produkt [4-(2-Diethylamino-ethyl)-piperazin-1-yl]-[4-methyl-1-(4-methyl-benzyl)-3-p-tolyl-1 H-pyrrol-2-yl]-methanon (148 mg, 38 % der Theorie) erhalten.

### Beispielverbindung 244:

### Synthese von 4-Methyl-1 -(4-methyl-benzyl)-3-p-tolyl-1 H-pyrrol-2-carbonsäure [2-(1H-indol-3-yl)-ethyl]-methyl-amid

Zu einer Reaktionsmischung von 4-Dimethyl-3-phenyl-1 H-pyrrol-2-carbonsäure (0.79 mmol) und [2-(1 H-Indol-3-yl)-ethyl]-methylamin (138 mg, 0.79 mmol) in DCM (5 ml) wurden bei 0°C EDCI (166 mg, 0.87 mmol) und HOAt (11 mg, 0.08 mmol) gegeben und die Reaktionsmischung wurde für 30 min bei 0°C und über Nacht bei RT gerührt. Nach Entfernen des Lösungsmittels erfolgte die Reinigung über Säulenchromathographie und es wurde das gewünschte Produkt 4-Methyl-1 -(4-methyl-benzyl)-3-p-tolyl-1 H-pyrrol-2-carbonsäure [2-(1H-indol-3-yl)-ethyl]-methyl-amid (170 mg, 45 % der Theorie) erhalten.

Die folgenden Beispielverbindungen wurden wie vorstehend im allgemeinen Verfahren beschrieben erhalten. Dabei sind die jeweiligen Ausgangsstoffe dem Fachmann bekannt.

| | |
|---|---|
| [1] | 1-Benzyl-4-methyl-3-p-tolyl-1H-pyrrol-2-carbonsäure 4-[1,2,3]thiadiazol-4-yl-benzylamid, |
| [2] | 3-Furan-2-yl-1,4-dimethyl-1H-pyrrol-2-carbonsäure 4-sulfamoyl-benzylamid, |
| [3] | 1-(4-Fluor-benzyl)-3-furan-2-yl-4-methyl-1H-pyrrol-2-carbonsäure 2,4-dimethoxy-benzylamid, |
| [4] | 1-Butyl-4-methyl-3-p-tolyl-1H-pyrrol-2-carbonsäure (2-pyridin-2-yl-ethyl)-amid, |
| [5] | 1-(4-Fluor-benzyl)-3-(4-methoxy-phenyl)-4-methyl-1H-pyrrol-2-carbonsäure [2-(5-hydroxy-1H-indol-3-yl)-ethyl]-amid, |
| [6] | (1,4-Dimethyl-3-p-tolyl-1H-pyrrol-2-yl)-(4-pyrrolidin-1-yl-piperidin-1-yl)-methanon, |
| [7] | 1-Benzyl-3-furan-2-yl-4-methyl-1H-pyrrol-2-carbonsäure 4-brom-2-fluor-benzylamid, |
| [8] | [1-Benzyl-3-(4-chlor-phenyl)-4-methyl-1H-pyrrol-2-yl]-[4-(2-chlor-phenyl)-piperazin-1-yl]-methanon, |
| [9] | 1,4-Dimethyl-3-phenyl-1H-pyrrol-2-carbonsäure (3-dimethylamino-propyl)-amid, |
| [10] | 1,4-Dimethyl-3-p-tolyl-1H-pyrrol-2-carbonsäure (2-pyrrolidin-1-yl-ethyl)-amid, |
| [11] | 1-(4-Fluor-benzyl)-3-furan-2-yl-4-methyl-1H-pyrrol-2-carbonsäure 4-dimethylamino-benzylamid, |
| [12] | 1-[4-(1-Benzyl-4-methyl-3-phenyl-1H-pyrrol-2-carbonyl)-piperazin-1-yl]-2-phenyl-ethanon, |
| [13] | 1-Benzyl-3-furan-2-yl-4-methyl-1H-pyrrol-2-carbonsäure 2,5-difluor-benzylamid, |
| [14] | 1-(2,6-Dichlor-benzyl)-4-methyl-3-p-tolyl-1H-pyrrol-2-carbonsäure 3,5-difluor-benzylamid, |
| [15] | 1-(2-Brom-benzyl)-3-(4-chlor-phenyl)-4-methyl-1H-pyrrol-2-carbonsäure (2-methyl-cyclohexyl)-amid, |
| [16] | 1,4-Dimethyl-3-p-tolyl-1H-pyrrol-2-carbonsäure (3-morpholin-4-yl-propyl)-amid, |
| [17] | 1-Butyl-4-methyl-3-p-tolyl-1H-pyrrol-2-carbonsäure (1,3-dimethyl-butyl)-amid, |
| [18] | [4-(2,4-Dimethyl-phenyl)-piperazin-1-yl]-[4-methyl-1-(4-methyl-benzyl)-3-p-tolyl-1H-pyrrol-2-yl]-methanon, |
| [19] | 1-(2,6-Dichlor-benzyl)-4-methyl-3-p-tolyl-1H-pyrrol-2-carbonsäure methyl-(2-pyridin-2-yl-ethyl)-amid, |
| [20] | 1,4-Dimethyl-3-phenyl-1H-pyrrol-2-carbonsäure (2-cyano-ethyl)-methyl-amid, |
| [21] | 1-(2,6-Dichlor-benzyl)-4-methyl-3-p-tolyl-1H-pyrrol-2-carbonsäure (3-imidazol-1-yl-propyl)-amid, |
| [22] | 1-(4-Fluor-benzyl)-3-(4-methoxy-phenyl)-4-methyl-1H-pyrrol-2-carbonsäure (1,3-dimethyl-butyl)-amid, |
| [23] | 3-(4-Methoxy-phenyl)-1,4-dimethyl-1H-pyrrol-2-carbonsäure 2-ethoxy-benzylamid, |
| [24] | (4-Cycloheptyl-piperazin-1-yl)-(1,4-dimethyl-3-p-tolyl-1H-pyrrol-2-yl)-methanon, |
| [25] | 1-(4-Fluor-benzyl)-3-(4-methoxy-phenyl)-4-methyl-1H-pyrrol-2-carbonsäure (2-dimethylamino-ethyl)-amid, |
| [26] | 1-Benzyl-4-methyl-3-phenyl-1H-pyrrol-2-carbonsäure (pyridin-2-ylmethyl)-amid, |
| [27] | 3-(4-Chlor-phenyl)-1-ethyl-4-methyl-1H-pyrrol-2-carbonsäure [2-(4-chlor-phenyl)-propyl]-amid, |
| [28] | 3-(4-Chlor-phenyl)-1-(2-fluor-benzyl)-4-methyl-1H-pyrrol-2-carbonsäure 3,5-difluor-benzylamid, |
| [29] | 2-{[1-(4-Fluor-benzyl)-3-(4-methoxy-phenyl)-4-methyl-1H-pyrrol-2-carbonyl]-amino}-propionsäure benzyl ester, |
| [30] | (1-Benzyl-4-methyl-3-p-tolyl-1H-pyrrol-2-yl)-[4-(2-dimethylamino-ethyl)-piperazin-1-yl]-methanon, |
| [31] | 3-Furan-2-yl-1-(4-methoxy-benzyl)-4-methyl-1H-pyrrol-2-carbonsäure (3,3-dimethyl-butyl)-amid, |
| [32] | 3-(4-Chlor-phenyl)-1-isobutyl-4-methyl-1H-pyrrol-2-carbonsäure 4-dimethylamino-benzylamid, |
| [33] | 3-(4-Chlor-phenyl)-1-ethyl-4-methyl-1H-pyrrol-2-carbonsäure (2-pyridin-2-yl-ethyl)-amid, |
| [34] | 3-(4-Chlor-phenyl)-1-ethyl-4-methyl-1H-pyrrol-2-carbonsäure 2,3-dimethoxy-benzylamid, |
| [35] | 1-(4-Fluor-benzyl)-3-(4-methoxy-phenyl)-4-methyl-1H-pyrrol-2-carbonsäure (2-thiophen-2-yl-ethyl)-amid, |
| [36] | 2-{[3-(4-Chlor-phenyl)-1-ethyl-4-methyl-1H-pyrrol-2-carbonyl]-amino}-3-methyl-buttersäure tert-butyl ester, |
| [37] | 3-Furan-2-yl-1-(4-methoxy-benzyl)-4-methyl-1H-pyrrol-2-carbonsäure (1,2,3,4-tetrahydro-naphthalin-1-yl)-amid, |
| [38] | 3-Furan-2-yl-4-methyl-1-(4-trifluormethyl-benzyl)-1H-pyrrol-2-carbonsäure (2-pyrrolidin-1-yl-ethyl)-amid, |
| [39] | {[1-(4-Methoxy-benzyl)-4-methyl-3-p-tolyl-1H-pyrrol-2-carbonyl]-methyl-amino}-essigsäure benzyl ester, |
| [40] | 3-Furan-2-yl-1-(4-methoxy-benzyl)-4-methyl-1H-pyrrol-2-carbonsäure [2-(1-methyl-pyrrolidin-2-yl)-ethyl]-amid, |
| [41] | [4-(5-Brom-2-ethoxy-benzyl)-piperazin-1-yl]-(1,4-dimethyl-3-phenyl-1H-pyrrol-2-yl) methanon, |
| [42] | (1-Benzyl-3-furan-2-yl-4-methyl-1H-pyrrol-2-yl)-[4-(3-fluor-4-methoxy-benzyl)-piperazin-1-yl]-methanon, |
| [43] | 3-{[3-(4-Chlor-phenyl)-1-ethyl-4-methyl-1H-pyrrol-2-carbonyl]-amino}-propionsäure tert-butyl ester, |
| [44] | 1-Benzyl-4-methyl-3-phenyl-1H-pyrrol-2-carbonsäure 3-methoxy-benzylamid, |
| [45] | (1-Benzyl-3-furan-2-yl-4-methyl-1H-pyrrol-2-yl)-[4-(5-brom-2-ethoxy-benzyl)-piperazin-1-yl]-methanon, |
| [46] | [3-(4-Chlor-phenyl)-1,4-dimethyl-1H-pyrrol-2-yl]-(4-o-tolyl-piperazin-1-yl)-methanon, |
| [47] | 1-(4-Fluor-benzyl)-3-(4-methoxy-phenyl)-4-methyl-1H-pyrrol-2-carbonsäure [2-(4-ethoxy-phenyl)-ethyl]-amid, |
| [48] | [1-(2,6-Dichlor-benzyl)-4-methyl-3-p-tolyl-1H-pyrrol-2-yl]-(4-hydroxy-piperidin-1-yl)-methanon, |
| [49] | (1,4-Dimethyl-3-p-tolyl-1H-pyrrol-2-yl)-[4-(2,4,6-trimethoxy-benzyl)-piperazin-1-yl]-methanon, |
| [50] | 1-(4-Fluor-benzyl)-3-furan-2-yl-4-methyl-1H-pyrrol-2-carbonsäure (2-morpholin-4-yl-ethyl)-amid, |
| [51] | 1-Benzyl-3-(4-chlor-phenyl)-4-methyl-1H-pyrrol-2-carbonsäure [2-(4-fluor-phenyl)-ethyl]-amid, |
| [52] | [4-(2,5-Dimethyl-phenyl)-piperazin-1-yl]-[3-(4-methoxy-phenyl)-1,4-dimethyl-1H-pyrrol-2-yl]-methanon, |
| [53] | 3-(4-Methoxy-phenyl)-1,4-dimethyl-1H-pyrrol-2-carbonsäure (3-imidazol-1-yl-propyl)-amid, |
| [54] | 1-(4-Fluor-benzyl)-3-(4-methoxy-phenyl)-4-methyl-1H-pyrrol-2-carbonsäure [2-(3-trifluormethyl-phenyl)-ethyl]-amid, |
| [55] | [3-(4-Chlor-phenyl)-1-(2-fluor-benzyl)-4-methyl-1H-pyrrol-2-yl]-[4-(5-methyl-pyrazin-2-carbonyl)-piperazin-1-yl]-methanon, |
| [56] | 3-(4-Chlor-phenyl)-1-isobutyl-4-methyl-1H-pyrrol-2-carbonsäure 3-fluor-5-trifluormethyl-benzylamid, |
| [57] | [3-(4-Chlor-phenyl)-1,4-dimethyl-1H-pyrrol-2-yl]-(4-pyridin-4-yl-piperazin-1-yl)-methanon, |
| [58] | 3-(4-Chlor-phenyl)-1-(2-fluor-benzyl)-4-methyl-1H-pyrrol-2-carbonsäure (3-imidazol-1-yl-propyl)-amid, |
| [59] | (1,4-Dimethyl-3-phenyl-1H-pyrrol-2-yl)-[4-(3-fluor-4-methoxy-benzyl)-piperazin-1-yl]-methanon, |
| [60] | 1-Benzyl-3-furan-2-yl-4-methyl-1H-pyrrol-2-carbonsäure [2-(4-brom-phenyl)-ethyl]-amid, |
| [61] | (4-Cycloheptyl-piperazin-1-yl)-(3-furan-2-yl-1,4-dimethyl-1H-pyrrol-2-yl)-methanon, |
| [62] | 1-Benzyl-4-methyl-3-phenyl-1H-pyrrol-2-carbonsäure [2-(4-methoxy-phenoxy)-ethyl]-amid, |
| [63] | 1-(4-Fluor-benzyl)-3-furan-2-yl-4-methyl-1H-pyrrol-2-carbonsäure (3-dimethylamino-propyl)-amid, |
| [64] | [1-(4-Fluor-benzyl)-3-(4-methoxy-phenyl)-4-methyl-1H-pyrrol-2-yl]-(2-pyrrolidin-1-ylmethyl-pyrrolidin-1-yl)-methanon, |
| [65] | 3-(4-Chlor-phenyl)-1-isobutyl-4-methyl-1H-pyrrol-2-carbonsäure (3-diethylamino-propyl)-amid, |
| [66] | [3-(4-Chlor-phenyl)-1,4-dimethyl-1H-pyrrol-2-yl]-[4-(2-fluor-5-methoxy-benzyl)-piperazin-1-yl]-methanon, |
| [67] | 3-(4-Methoxy-phenyl)-1,4-dimethyl-1H-pyrrol-2-carbonsäure pentylamid, |
| [68] | (1-Butyl-4-methyl-3-p-tolyl-1H-pyrrol-2-yl)-[4-(2,5-dimethoxy-benzyl)-piperazin-1-yl]-methanon, |
| [69] | [4-(2-Diethylamino-ethyl)-piperazin-1-yl]-[4-methyl-1-(4-methyl-benzyl)-3-p-tolyl-1H-pyrrol-2-yl]-methanon, |
| [70] | [4-(3-Chlor-phenyl)-piperazin-1-yl]-[3-(4-methoxy-phenyl)-1,4-dimethyl-1H-pyrrol-2-yl]-methanon, |
| [71] | [1-(2-Brom-benzyl)-3-(4-chlor-phenyl)-4-methyl-1H-pyrrol-2-yl]-(4-isopropyl-piperazin-1-yl)-methanon, |
| [72] | [4-(2-Dimethylamino-ethyl)-piperain-1-yl]-[1-(4-fluor-benzyl)-3-(4-methoxy-phenyl)-4-methyl-1H-pyrrol-2-2-yl]-methanon, |
| [73] | 3-(4-Chlor-phenyl)-1-(2-fluor-benzyl)-4-methyl-1H-pyrrol-2-carbonsäure 2,4-dimethoxy-benzylamid, |
| [74] | 1-Benzyl-4-methyl-3-p-tolyl-1H-pyrrol-2-carbonsäure (2-pyrrolidin-1-yl-ethyl)-amid, |
| [75] | [1-(4-Fluor-benzyl)-3-furan-2-yl-4-methyl-1H-pyrrol-2-yl]-[4-(3-fluor-4-methoxy-benzyl)-piperazin-1-yl]-methanon, |
| [76] | 3-(4-Methoxy-phenyl)-1,4-dimethyl-1H-pyrrol-2-carbonsäure 2,4-dimethoxy-benzylamid, |
| [77] | 1-Benzyl-4-methyl-3-phenyl-1H-pyrrol-2-carbonsäure (2-pyrrolidin-1-yl-ethyl)-amid, |
| [78] | 3-(4-Chlor-phenyl)-1,4-dimethyl-1H-pyrrol-2-carbonsäure [3-(2-methyl-piperidin-1-yl)-propyl]-amid, |
| [79] | 1-(4-Fluor-benzyl)-3-furan-2-yl-4-methyl-1H-pyrrol-2-carbonsäure cyclohexylamid, |
| [80] | 3-Furan-2-yl-1,4-dimethyl-1H-pyrrol-2-carbonsäure 4-fluor-2-trifluormethyl-benzylamid, |
| [81] | 1-Benzyl-3-furan-2-yl-4-methyl-1H-pyrrol-2-carbonsäure (1-naphthalin-2-yl-ethyl)-amid, |
| [82] | 3-(4-Chlor-phenyl)-1-ethyl-4-methyl-1H-pyrrol-2-carbonsäure (furan-2-ylmethyl)-amid, |
| [83] | 1-Benzyl-4-methyl-3-phenyl-1H-pyrrol-2-carbonsäure (3-dimethylamino-propyl)-methyl-amid, |
| [84] | [3-(4-Methoxy-phenyl)-1,4-dimethyl-1H-pyrrol-2-yl]-(4-p-tolyl-piperazin-1-yl)-methanon, |
| [85] | 1-(4-Methoxy-benzyl)-4-methyl-3-p-tolyl-1H-pyrrol-2-carbonsäure (thiophen-2-ylmethyl)-amid, |
| [86] | 4-Methyl-1-(4-methyl-benzyl)-3-p-tolyl-1H-pyrrol-2-carbonsäure phenethyl-amid, |
| [87] | 1,4-Dimethyl-3-p-tolyl-1H-pyrrol-2-carbonsäure 3,5-difluor-benzylamid, |
| [88] | 3-(4-Methoxy-phenyl)-1,4-dimethyl-1H-pyrrol-2-carbonsäure indan-1-ylamid, |
| [89] | 1-Benzyl-4-methyl-3-phenyl-1H-pyrrol-2-carbonsäure 4-dimethylamino-benzylamid, |
| [90] | 1-(4-Fluor-benzyl)-3-furan-2-yl-4-methyl-1H-pyrrol-2-carbonsäure (1-naphthalin-2-ylmethyl-pyrrolidin-3-yl)-amid, |
| [91] | [3-Furan-2-yl-4-methyl-1-(4-trifluormethyl-benzyl)-1H-pyrrol-2-yl]-(4-methyl-[1,4]diazepan-1-yl)-methanon, |
| [92] | [1-(2-Brom-benzyl)-3-(4-chlor-phenyl)-4-methyl-1H-pyrrol-2-yl]-[4-(3-trifluormethyl-phenyl)-piperazin-1-yl]-methanon, |
| [93] | [3-Furan-2-yl-1-(4-methoxy-benzyl)-4-melhyl-1H-pyrrol-2-yl]-(4-thiophen-3-ylmethyl-piperazin-1-yl)-methanon, |
| [94] | 1,4-Dimethyl-3-p-tolyl-1H-pyrrol-2-carbonsäure (2-benzyloxy-cyclohexyl)-amid, |
| [95] | 2-{4-[1-Benzyl-3-(4-chlor-phenyl)-4-methyl-1H-pyrrol-2-carbonyl]-piperazin-1-yl}-N-isopropyl-acetamid, |
| [96] | (2,6-Dimethyl-morpholin-4-yl)-[3-(4-methoxy-phenyl)-1,4-dimethyl-1H-pyrrol-2-yl]-methanon, |
| [97] | 3-Furan-2-yl-1-(4-methoxy-benzyl)-4-methyl-1H-pyrrol-2-carbonsäure (thiophen-2-ylmethyl)-amid, |
| [98] | 1,4-Dimethyl-3-p-tolyl-1H-pyrrol-2-carbonsäure (furan-2-ylmethyl)-amid, |
| [99] | 4-Methyl-1-(4-methyl-benzyl)-3-p-tolyl-1H-pyrrol-2-carbonsäure (3-dimethylamino-propyl)-amid, |
| [100] | 1-(4-Fluor-benzyl)-3-(4-methoxy-phenyl)-4-methyl-1H-pyrrol-2-carbonsäure (2-oxo-tetrahydro-furan-3-yl)-amid, |
| [101] | 3-(4-Chlor-phenyl)-1,4-dimethyl-1H-pyrrol-2-carbonsäure (2-phenoxy-ethyl)-amid, |
| [102] | 3-(4-(1-Benzyl-4-methyl-3-p-tolyl-1H-pyrrole-2-carbonyl)piperazin-1-yl)pyrazin-2-carbonitril |
| [103] | 2-{[3-(4-Methoxy-phenyl)-1,4-dimethyl-1H-pyrrol-2-carbonyl]-amino}-3-phenyl-propionsäure methyl ester, |
| [104] | 3-(4-Chlor-phenyl)-1,4-dimethyl-1H-pyrrol-2-carbonsäure(2-morpholin-4-yl-ethyl)-amid, |
| [105] | 3-Furan-2-yl-1,4-dimethyl-1H-pyrrol-2-carbonsäure indan-2-ylamid, |
| [106] | (1-Butyl-4-methyl-3-p-tolyl-1H-pyrrol-2-yl)-[4-(2-fluor-phenyl)-piperazin-1-yl]-methanon, |
| [107] | [4-(3-Chlor-phenyl)-piperazin-1-yl]-(1,4-dimethyl-3-p-tolyl-1H-pyrrol-2-yl)-methanon, |
| [108] | [1-(4-Fluor-benzyl)-3-furan-2-yl-4-methyl-1H-pyrrol-2-yl]-(4-naphthalin-2-ylmethyl-piperazin-1-yl)-methanon, |
| [109] | 3-(4-Chlor-phenyl)-1-isobutyl-4-methyl-1H-pyrrol-2-carbonsäure p-tolylamid, |
| [110] | (1-Benzyl-4-methyl-3-p-tolyl-1H-pyrrol-2-yl)-[4-(2-hydroxy-ethyl)-piperazin-1-yl]-methanon, |
| [111] | 1-Butyl-4-methyl-3-p-tolyl-1H-pyrrol-2-carbonsäure [2-(2,5-dimethoxy-phenyl)-ethyl]-amid, |
| [112] | 1-Butyl-4-methyl-3-p-tolyl-1H-pyrrol-2-carbonsäure (3-imidazol-1-yl-propyl)-amid, |
| [113] | 1-Benzyl-3-(4-chlor-phenyl)-4-methyl-1H-pyrrol-2-carbonsäure [2-(3,4-dimethoxy-phenyl)-ethyl]-methyl-amid, |
| [114] | 4-Methyl-1-(4-methyl-benzyl)-3-p-tolyl-1H-pyrrol-2-carbonsäure (3-morpholin-4-yl-propyl)-amid, |
| [115] | 4-Methyl-1-(4-methyl-benzyl)-3-p-tolyl-1H-pyrrol-2-carbonsäure 4-fluor-2-trifluormethyl-benzylamid, |
| [116] | 2-{[1-Benzyl-3-(4-chlor-phenyl)-4-methyl-1H-pyrrol-2-carbonyl]-amino}-3-(4-chlor-phenyl)-propionsäure ethyl ester, |
| [117] | 1-Benzyl-4-methyl-3-phenyl-1H-pyrrol-2-carbonsäure 3-fluor-4-trifluormethyl-benzylamid, |
| [118] | 1-(4-Brom-benzyl)-4-methyl-3-p-tolyl-1H-pyrrol-2-carbonsäure (2-pyridin-2-yl-ethyl)-amid, |
| [119] | 1-(4-Fluor-benzyl)-3-furan-2-yl-4-methyl-1H-pyrrol-2-carbonsäure benzyl-(2-hydroxy-ethyl)-amid, |
| [120] | 2-[4-(1-Butyl-4-methyl-3-p-tolyl-1H-pyrrol-2-carbonyl)-piperazin-1-yl]-N-methyl-N-phenyl-acetamid, |
| [121] | [4-Methyl-1-(4-methyl-behzyl)-3-p-tolyl-1H-pyrrol-2-yl]-(4-phenyl-piperazin-1-yl)-methanon, |
| [122] | 1-Benzyl-4-methyl-3-p-tolyl-1H-pyrrol-2-carbonsäure (1-benzyl-pyrrolidin-3-yl)-amid, |
| [123] | [1-(4-Fluor-benzyl)-3-furan-2-yl-4-methyl-1H-pyrrol-2-yl]-[4-(3-trifluormethyl-phenyl)-piperazin-1-yl]-methanon, |
| [124] | 1-(4-Methoxy-benzyl)-4-methyl-3-p-tolyl-1H-pyrrol-2-carbonsäure butylamid, |
| [125] | 1-Benzyl-4-methyl-3-p-tolyl-1H-pyrrol-2-carbonsäure [2-(2-fluor-phenyl)-ethyl]-amid, |
| [126] | 4-Methyl-1-(4-methyl-benzyl)-3-p-tolyl-1H-pyrrol-2-carbonsäure 3,4-dimethoxy-benzylamid, |
| [127] | 3-(4-Chlor-phenyl)-1-isobutyl-4-methyl-1H-pyrrol-2-carbonsäure (2-diethylamino-ethyl)-amid, |
| [128] | 1-{4-[3-(4-Chlor-phenyl)-1-ethyl-4-methyl-1H-pyrrol-2-carbonyl]-piperazin-1-yl}-2-phenyl-ethanon, |
| [129] | 1-(4-Fluor-benzyl)-4-methyl-3-p-tolyl-1H-pyrrol-2-carbonsäure 3-trifluormethoxy-benzylamid, |
| [130] | [1-Benzyl-3-(4-chlor-phenyl)-4-methyl-1H-pyrrol-2-yl]-(3-methyl-piperidin-1-yl)-methanon, |
| [131] | 1-(4-Fluor-benzyl)-4-methyl-3-p-tolyl-1H-pyrrol-2-carbonsäure (2-p-tolyl-ethyl)-amid, |
| [132] | 1-(4-Methoxy-benzyl)-4-methyl-3-p-tolyl-1H-pyrrol-2-carbonsäure [2-(2-methyl-5-nitro-imidazol-1-yl)-ethyl]-amid, |
| [133] | 1,4-Dimethyl-3-p-tolyl-1H-pyrrol-2-carbonsäure 2,3-dimethoxy-benzylamid, |
| [134] | 1-(2-Brom-benzyl)-3-(4-chlor-phenyl)-4-methyl-1H-pyrrol-2-carbonsäure [2-(ethyl-m-tolyl-amino)-ethyl]-amid, |
| [135] | 1-(4-Fluor-benzyl)-3-furan-2-yl-4-methyl-1H-pyrrol-2-carbonsäure (4-isopropyl-phenyl)-amid, |
| [136] | 5-Chlor-2-methoxy-benzoesäure N'-[1-(4-fluor-benzyl)-3-(4-methoxy-phenyl)-4-methyl-1H-pyrrol-2-carbonyl]-hydrazid, |
| [137] | (1-Benzyl-4-methyl-3-p-tolyl-1H-pyrrol-2-yl)-[1,4']bipiperidinyl-1'-yl-methanon, |
| [138] | 3-Furan-2-yl-1,4-dimethyl-1H-pyrrol-2-carbonsäure (4-butyl-phenyl)-amid, |
| [139] | 1-Benzyl-4-methyl-3-phenyl-1H-pyrrol-2-carbonsäure [2-(1H-indol-3-yl)-ethyl]-amid, |
| [140] | [4-(4-tert-Butyl-benzyl)-piperazin-1-yl]-[1-(4-fluor-benzyl)-4-methyl-3-p-tolyl-1H-pyrrol-2-yl]-methanon, |
| [141] | [1-(4-Fluor-benzyl)-4-methyl-3-p-tolyl-1H-pyrrol-2-yl]-morpholin-4-yl-methanon, |
| [142] | 3-[(1-Benzyl-3-furan-2-yl-4-methyl-1H-pyrrol-2-carbonyl)-amino]-propionsäure ethyl ester, |
| [143] | 1,4-Dimethyl-3-phenyl-1H-pyrrol-2-carbonsäure (naphthalin-2-ylcarbamoylmethyl)-amid, |
| [144] | [3-(4-Chlor-phenyl)-1,4-dimethyl-1H-pyrrol-2-yl]-[4-(2-ethoxy-phenyl)-piperazin-1-yl]-methanon, |
| [145] | 1-(4-Fluor-benzyl)-3-furan-2-yl-4-methyl-1H-pyrrol-2-carbonsäure (4-cyanomethyl-phenyl)-amid, |
| [146] | 4-[1-Benzyl-3-(4-chlor-phenyl)-4-methyl-1H-pyrrol-2-carbonyl]-piperazin-1-carbonsäure tert-butyl ester, |
| [147] | (1,4-Dioxa-8-aza-spiro[4.5]dec-8-yl)-[1-(4-fluor-benzyl)-3-furan-2-yl-4-methyl-1H-pyrrol-2-yl]-methanon, |
| [148] | 3-Furan-2-yl-4-methyl-1-(4-trifluormethyl-benzyl)-1H-pyrrol-2-carbonsäure pentylamid, |
| [149] | 3-Furan-2-yl-1-(4-methoxy-benzyl)-4-methyl-1H-pyrrol-2-carbonsäure [3-(methyl-phenyl-amino)-propyl]-amid, |
| [150] | 1-(2,6-Dichlor-benzyl)-4-methyl-3-p-tolyl-1H-pyrrol-2-carbonsäure (2-azepan-1-yl-ethyl)-amid, |
| [151] | 1-(4-Brom-benzyl)-4-methyl-3-p-tolyl-1H-pyrrol-2-carbonsäure cyclopentylamid, |
| [152] | [1-(2,6-Dichlor-benzyl)-4-methyl-3-p-tolyl-1H-pyrrol-2-yl]-[4-(2,4-dimethoxy-phenyl)-piperazin-1-yl]-methanon, |
| [153] | 3-(4-Chlor-phenyl)-1-(2-fluor-benzyl)-4-methyl-1H-pyrrol-2-carbonsäure [2-(2-chlor-phenoxy)-ethyl]-amid, |
| [154] | 1-(2,6-Dichlor-benzyl)-4-methyl-3-p-tolyl-1H-pyrrol-2-carbonsäure (3,3-dimethyl-butyl)-amid, |
| [155] | 1,4-Dimethyl-3-p-tolyl-1H-pyrrol-2-carbonsäure (2-benzyloxy-cyclohexyl)-amid, |
| [156] | [4-Methyl-1-(4-methyl-benzyl)-3-p-tolyl-1H-pyrrol-2-yl]-thiomrpholin-4-yl-methanon, |
| [157] | 3-(4-Chlor-phenyl)-1-isobutyl-4-methyl-1H-pyrrol-2-carbonsäure (3-dimethylamino-propyl)-methyl-amid, |
| [158] | [4-(2,5-Dimethyl-phenyl)-piperazin-1-yl]-[3-furan-2-yl-1-(4-methoxy-benzyl)-4-methyl-1H-pyrrol-2-yl]-methanon, |
| [159] | 5-Chlor-2-methoxy-benzoesäure N'-[3-(4-methoxy-phenyl)-1,4-dimethyl-1H-pyrrol-2-carbonyl]-hydrazide, |
| [160] | 2-(3,4-Difluor-phenyl)-1-{4-[1-(4-fluor-benzyl)-3-furan-2-yl-4-methyl-1H-pyrrol-2-carbonyl]-piperazin-1-yl}-ethanon, |
| [161] | 2-{4-[1-(4-Fluor-benzyl)-3-furan-2-yl-4-methyl-1H-pyrrol-2-carbonyl]-piperazin-1-yl}-N-isopropyl-acetamid, . |
| [162] | 3-Furan-2-yl-4-methyl-1-(4-trifluormethyl-benzyl)-1H-pyrrol-2-carbonsäure [1-(3-methoxy-phenyl)-ethyl]-amid, |
| [163] | 2-[(1-Benzyl-3-furan-2-yl-4-methyl-1H-pyrrol-2-carbonyl)-amino]-3-(4-chlor-phenyl)-propionsäure methyl ester, |
| [164] | [3-Furan-2-yl-1-(4-methoxy-benzyl)-4-methyl-1H-pyrrol-2-yl]-(4-methyl-piperazin-1-yl)-methanon, |
| [165] | 1,4-Dimethyl-3-phenyl-1H-pyrrol-2-carbonsäure (1-benzyl-pyrrolidin-3-yl)-amid, |
| [166] | (1,4-Dimethyl-3-p-tolyl-1H-pyrrol-2-yl)-(4-thieno[2,3-d]pyrimidin-4-yl-piperazin-1-yl)-methanon, |
| [167] | 4-Methyl-1-(4-methyl-benzyl)-3-p-tolyl-1H-pyrrol-2-carbonsäure (3-methoxy-propyl)-amid, |
| [168] | [3-(4-Methoxy-phenyl)-1,4-dimethyl-1H-pyrrol-2-yl]-[4-(5-trifluormethyl-pyridin-2-yl)-piperazin-1-yl]-methanon, |
| [169] | 1-(4-Brom-benzyl)-4-methyl-3-p-tolyl-1H-pyrrol-2-carbonsäure (3-morpholin-4-yl-propyl)-amid, |
| [170] | 1,4-Dimethyl-3-p-tolyl-1H-pyrrol-2-carbonsäure [2-(2,3-dimethoxy-phenyl)-ethyl]-amid, |
| [171] | [1-(4-Methoxy-benzyl)-4-methyl-3-p-tolyl-1H-pyrrol-2-yl]-(3-methyl-4-p-tolyl-piperazin-1-yl)-methanon, |
| [172] | [3-(4-Chlor-phenyl)-1-ethyl-4-methyl-1H-pyrrol-2-yl]-[4-(2,4-dimethoxy-phenyl)-piperazin-1-yl]-methanon, |
| [173] | 2-{4-[1-(2-Brom-benzyl)-3-(4-chlor-phenyl)-4-methyl-1H-pyrrol-2-carbonyl]-piperazin-1-yl}-benzonitril, |
| [174] | 1-(4-Brom-benzyl)-4-methyl-3-p-tolyl-1H-pyrrol-2-carbonsäure (1-ethyl-pyrrolidin-2-ylmethyl)-amid, |
| [175] | 3-(4-Chlor-phenyl)-1-isobutyl-4-methyl-1H-pyrrol-2-carbonsäure [2-(2-chlor-phenoxy)-ethyl]-amid, |
| [176] | 3-(4-Chlor-phenyl)-1,4-dimethyl-1H-pyrrol-2-carbonsäure [2-(1H-indol-3-yl)-ethyl]-amid, |
| [177] | 1-(4-Fluor-benzyl)-4-methyl-3-p-tolyl-1H-pyrrol-2-carbonsäure (naphthalin-2-ylcarbamoylmethyl)-amid, |
| [178] | 4-(1-Benzyl-3-furan-2-yl-4-methyl-1H-pyrrol-2-carbonyl)-piperazin-1-carbonsäure benzyl ester, |
| [179] | 1,4-Dimethyl-3-phenyl-1H-pyrrol-2-carbonsäure (1-benzyl-pyrrolidin-3-yl)-amid, |
| [180] | [3-(4-Chlor-phenyl)-1-(2-fluor-benzyl)-4-methyl-1H-pyrrol-2-yl]-[4-(4-trifluormethyl-phenyl)-piperazin-1-yl]-methanon, |
| [181] | [3-(4-Chlor-phenyl)-1-isobutyl-4-methyl-1H-pyrrol-2-yl]-(4-phenyl-piperazin-1-yl)-methanon, |
| [182] | 2-{4-[1-Butyl-3-(4-chlor-phenyl)-4-methyl-1H-pyrrol-2-carbonyl]-piperazin-1-yl}-1-pyrrolidin-1-yl-ethanon, |
| [183] | [1-Benzyl-3-(4-chlor-phenyl)-4-methyl-1H-pyrrol-2-yl]-[4-(2-chlor-4-fluor-benzoyl)-piperazin-1-yl]-methanon, |
| [184] | (4-Benzoyl-piperidin-1-yl)-[1-(4-methoxy-benzyl)-4-methyl-3-p-tolyl-1H-pyrrol-2-yl]-methanon, |
| [185] | 3-Furan-2-yl-1,4-dimethyl-1H-pyrrol-2-carbonsäure ethyl-pyridin-4-ylmethyl-amid, |
| [186] | 1-(4-Brom-benzyl)-4-methyl-3-p-tolyl-1H-pyrrol-2-carbonsäure (2-pyrrolidin-1-yl-ethyl)-amid, |
| [187] | [3-(4-Chlor-phenyl)-1-isobutyl-4-methyl-1H-pyrrol-2-yl]-[4-(4-methyl-benzoyl)-piperazin-1-yl]-methanon, |
| [188] | [1-Benzyl-3-(4-chlor-phenyl)-4-methyl-1H-pyrrol-2-yl]-(4-thieno[2,3-d]pyrimidin-4-yl-piperazin-1-yl)-methanon, |
| [189] | 1-(1,4-Dimethyl-3-phenyl-1H-pyrrol-2-carbonyl)-piperidin-3-carbonsäure ethyl ester, |
| [190] | 1,4-Dimethyl-3-phenyl-1H-pyrrol-2-carbonsäure (1-phenyl-ethyl)-amid, |
| [191] | [3-Furan-2-yl-1-(4-methoxy-benzyl)-4-methyl-1H-pyrrol-2-yl]-(1,3,4,9-tetrahydro-b-carbolin-2-yl)-methanon, |
| [192] | 1-{4-[1-Benzyl-3-(4-chlor-phenyl)-4-methyl-1H-pyrrol-2-carbonyl]-piperazin-1-yl}-2-(3,4-difluor-phenyl)-ethanon, |
| [193] | [1-(4-Brom-benzyl)-4-methyl-3-p-tolyl-1H-pyrrol-2-yl]-[4-(3-phenyl-propyl)-piperazin-1-yl]-methanon, |
| [194] | 1-(2-Brom-benzyl)-3-(4-chlor-phenyl)-4-methyl-1H-pyrrol-2-carbonsäure [2-(1H-indol-3-yl)-ethyl]-methyl-amid, |
| [195] | 1-(4-{4-[1-(4-Brom-benzyl)-4-methyl-3-p-tolyl-1H-pyrrol-2-carbonyl]-piperazin-1-yl}-phenyl)-ethanon, |
| [196] | 1,4-Dimethyl-3-phenyl-1H-pyrrol-2-carbonsäure 4-fluor-2-trifluormethyl-benzylamid, |
| [197] | 4-Methyl-1-(4-methyl-benzyl)-3-p-tolyl-1H-pyrrol-2-carbonsäure (4-phenoxy-phenyl)-amid, |
| [198] | (4-Hydroxy-piperidin-1-yl)-[1-(4-methoxy-benzyl)-4-methyl-3-p-tolyl-1H-pyrrol-2-yl]-methanon, |
| [199] | 4-Diethylamino-benzoesäure N'-(1-benzyl-4-methyl-3-p-tolyl-1H-pyrrol-2-carbonyl)-hydrazide, |
| [200] | [3-(4-Chlor-phenyl)-1-isobutyl-4-methyl-1H-pyrrol-2-yl]-[4-(3-chlor-phenyl)-piperazin-1-yl]-methanon, |
| [201] | 1-(4-Brom-benzyl)-4-methyl-3-p-tolyl-1H-pyrrol-2-carbonsäure [2-(3,4-dichlor-phenyl)-ethyl]-amid, |
| [202] | 3-(4-Chlor-phenyl)-1-ethyl-4-methyl-1H-pyrrol-2-carbonsäure [2-(1H-indol-3-yl)-ethyl]-amid, |
| [203] | [1-(4-Fluor-benzyl)-4-methyl-3-p-tolyl-1H-pyrrol-2-yl]-[4-(5-trifluormethyl-pyridin-2-yl)-piperazin-1-yl]-methanon, |
| [204] | 1,4-Dimethyl-3-phenyl-1H-pyrrol-2-carbonsäure 3-trifluormethoxy-benzylamid, |
| [205] | [1-(4-Fluor-benzyl)-4-methyl-3-p-tolyl-1H-pyrrol-2-yl]-[4-(4-methyl-benzoyl)-piperazin-1-yl]-methanon, |
| [206] | [4-(3,4-Dichlor-phenyl)-piperazin-1-yl]-[1-(4-fluor-benzyl)-4-methyl-3-p-tolyl-1H-pyrrol-2-yl]-methanon, |
| [207] | 3-(4-Chlor-phenyl)-1-ethyl-4-methyl-1H-pyrrol-2-carbonsäure 2,6-dimethoxy-benzylamid, |
| [208] | 4-Methyl-1-(4-methyl-benzyl)-3-p-tolyl-1H-pyrrol-2-carbonsäure [2-(3,4-dimethoxy-phenyl)-ethyl]-amid, |
| [209] | 1-{4-[3-(4-Chlor-phenyl)-1-isobutyl-4-methyl-1H-pyrrol-2-carbonyl]-piperazin-1-yl}-2-(3,4-difluor-phenyl)-ethanon, |
| [210] | [1-(4-Fluor-benzyl)-4-methyl-3-p-tolyl-1H-pyrrol-2-yl]-[4-(2-methoxy-phenyl)-piperidin-1-yl]-methanon, |
| [211] | [3-Furan-2-yl-4-methyl-1-(4-trifluormethyl-benzyl)-1H-pyrrol-2-yl]-thiomorpholin-4-yl-methanon, |
| [212] | 1,4-Dimethyl-3-phenyl-1H-pyrrol-2-carbonsäure (2-thiophen-2-yl-ethyl)-amid, |
| [213] | 4-Diethylamino-benzoesäure N'-(1-butyl-4-methyl-3-p-tolyl-1H-pyrrol-2-carbonyl)-hydrazide, |
| [214] | {1-[1-(4-Fluor-benzyl)-3-(4-methoxy-phenyl)-4-methyl-1H-pyrrol-2-carbonyl]-pyrrolidin-3-yl}-carbaminsäure tert-butyl ester, |
| [215] | 2-{4-[3-(4-Chlor-phenyl)-1-isobutyl-4-methyl-1H-pyrrol-2-carbonyl]-piperazin-1-yl}-1-pyrrolidin-1-yl-ethanon, |
| [216] | [4-(2,3-Dihydro-benzo[1,4]dioxine-2-carbonyl)-piperazin-1-yl]-(1,4-dimethyl-3-phenyl-1H-pyrrol-2-yl)-methanon, |
| [217] | 3-Furan-2-yl-1 ,4-dimethyl-1H-pyrrol-2-carbonsäure 3,4-dimethoxy-benzylamid, |
| [218] | [3-Furan-2-yl-4-methyl-1-(4-trifluormethyl-benzyl)-1H-pyrrol-2-yl]-(4-pyridin-2-yl-piperazin-1-yl)-methanon, |
| [219] | [4-(Furan-2-carbonyl)-piperazin-1-yl]-[3-furan-2-yl-4-methyl-1-(4-trifluormethyl-benzyl)-1H-pyrrol-2-yl]-methanon, |
| [220] | 3-Furan-2-yl-4-methyl-1-(4-trifluormethyl-benzyl)-1H-pyrrol-2-carbonsäure (4-tert-butyl-phenyl)-amid, |
| [221] | 2-{4-[3-(4-Chlor-phenyl)-1-isobutyl-4-methyl-1H-pyrrol-2-carbonyl]-piperazin-1-yl}-benzonitril, |
| [222] | (3-Furan-2-yl-1,4-dimethyl-1H-pyrrol-2-yl)-[4-(4-trifluormethyl-pyridin-2-yl)-piperazin-1-yl]-methanon, |
| [223] | 1,4-Dimethyl-3-phenyl-1H-pyrrol-2-carbonsäure 3,5-difluor-benzylamid, |
| [224] | 1,4-Dimethyl-3-phenyl-1H-pyrrol-2-carbonsäure (pyridin-4-ylmethyl)-amid, |
| [225] | 2-[4-(3-Furan-2-yl-1,4-dimethyl-1H-pyrrol-2-carbonyl)-piperazin-1-yl]-benzonitril, |
| [226] | 3-Furan-2-yl-1,4-dimethyl-1H-pyrrol-2-carbonsäure methyl-(2-pyridin-2-yl-ethyl)-amid, |
| [227] | 3-Furan-2-yl-1,4-dimethyl-1H-pyrrol-2-carbonsäure (3-methoxy-benzyl)-(tetrahydro-furan-2-ylmethyl)amid, |
| [228] | 3-Furan-2-yl-1,4-dimethyl-1H-pyrrol-2-carbonsäure (4-phenoxy-phenyl)-amid, |
| [229] | 3-Furan-2-yl-1,4-dimethyl-1H-pyrrol-2-carbonsäure pentylamid, |
| [230] | 2-(3,4-Difluor-phenyl)-1-[4-(3-furan-2-yl-1,4-dimethyl-1H-pyrrol-2-carbonyl)-piperazin-1-yl]-ethanon, |
| [231] | 3-Furan-2-yl-1,4-dimethyl-1H-pyrrol-2-carbonsäure phenylamid, |
| [232] | [4-(2,4-Dimethoxy-phenyl)-piperazin-1-yl]-(3-furan-2-yl-1,4-dimethyl-1H-pyrrol-2-yl)-methanon, |
| [233] | 3-Furan-2-yl-1,4-dimethyl-1H-pyrrol-2-carbonsäure [2-(3,4-dichlor-phenyl)-ethyl]-amid, |
| [234] | 3-Furan-2-yl-1,4-dimethyl-1H-pyrrol-2-carbonsäure [2-(2-chlor-phenoxy)-ethyl]-amid, |
| [235] | 3-Furan-2-yl-1,4-dimethyl-1H-pyrrol-2-carbonsäure 3-methoxy-benzylamid, |
| [236] | 3-Furan-2-yl-4-methyl-1-(4-trifluormethyl-benzyl)-1H-pyrrol-2-carbonsäure phenethyl-amid, |
| [237] | 3-Furan-2-yl-1,4-dimethyl-1H-pyrrol-2-carbonsäure cyclopentylamid, |
| [238] | 3-Furan-2-yl-1,4-dimethyl-1H-pyrrol-2-carbonsäure (pyridin-2-ylmethyl)-amid, |
| [239] | 3-Furan-2-yl-4-methyl-1-(4-trifluormethyl-benzyl)-1H-pyrrol-2-carbonsäure [2-(3,4-dimethoxy-phenyl)-ethyl]-amid, |
| [240] | [1-(4-Brom-benzyl)-4-methyl-3-p-tolyl-1H-pyrrol-2-yl]-(2,6-dimethyl-morpholin-4-yl)-methanon, |
| [241] | 1,4-Dimethyl-3-phenyl-1H-pyrrol-2-carbonsäure (2-p-tolyl-ethyl)-amid, |
| [242] | (1,4-Dimethyl-3-phenyl-1H-pyrrol-2-yl)-[4-(4-fluor-phenyl)-piperazin-1-yl]-methanon |
| [243] | 4-Methyl-1-(4-methyl-benzyl)-3-p-tolyl-1H-pyrrol-2-carbonsäure [2-(1H-indol-3-yl)-ethyl]-methyl-amid, |

### Pharmakologische Daten:

Die Noradrenalin-Wiederaufnahmehemmung (NA-Uptake Inhibierung) und die Serotonin-Wiederaufnahmehemmung (5-HT-Uptake Inhibierung) der erfindungsgemäßen 1,3-disubstituierten 4-Methyl-1H-pyrrol-2-carbonsäureamide wurde wie vorstehend beschrieben bestimmt.

Die erfindungsgemäßen 1,3-disubstituierten 4-Methyl-1H-pyrrol-2-carbonsäureamide weisen eine ausgezeichnete Affinität zum Noradrenalin-Rezeptor und zum 5-HT-Rezeptor auf.

Darüber hinaus zeigen diese erfindungsgemäßen Verbindungen auch ausgezeichnete Affinitäten für die Batrachotoxin-(BTX)-Bindungsstelle des Natriumkanals.

In der nachfolgenden Tabelle I sind die jeweiligen pharmakologischen Daten für einige beispielgemäße 1,3-disubstituierten 4-Methyl-1H-pyrrol-2-carbonsäureamide wiedergegeben.

**Tabelle 1.**

| **Verbindung gemäß Beispiel** | **5-HT Uptake (% Hemmung bei 10** µ**M)** | **NA Uptake (% Hemmung bei 10** µ**M)** | **BTX (Na⁺-Kanal) (% Hemmung bei 10** µ**M)** |
|---|---|---|---|
| 3 | 58 | | |
| 4 | 39 | | |
| 8 | | 70 | |
| 18 | 50 | | |
| 19 | 39 | | |
| 24 | 54 | 33 | |
| 35 | | 37 | |
| 26 | | 33 | |
| 29 | 52 | | |
| 32 | | 35 | |
| 34 | 37 | | |
| 35 | | | 48 |
| 36 | 74 | | |
| 37 | 68 | | |
| 38 | | | 94 |
| 39 | 54 | | 74 |
| 40 | | | 81 |
| 41 | 37 | | 77 |
| 42 | | | 88 |
| 43 | | | 31 |
| 44 | | | 47 |
| 45 | | | 89 |
| 46 | 34 | 63 | 55 |
| 47 | 67 | | 55 |
| 48 | | | 36 |
| 49 | 30 | | 89 |
| 50 | | | 77 |
| 51 | | | 43 |
| 52 | | | 76 |
| 53 | | | 44 |
| 54 | | | 42 |
| 55 | | | 44 |
| 57 | 66 | | 76 |
| 58 | 50 | | 93 |
| 59 | | | 75 |
| 60 | 67 | | |
| 61 | 52 | 34 | 64 |
| 62 | 73 | | 50 |
| 63 | 50 | 57 | 75 |
| 64 | | | 96 |
| 65 | | 35 | 88 |
| 66 | | | 73 |
| 68 | | | 84 |
| 69 | | | 91 |
| 70 | 54 | 69 | 59 |
| 71 | | | 83 |
| 72 | | | 81 |
| 73 | | | 49 |
| 74 | | | 101 |
| 75 | | | 89 |
| 76 | | | 41 |
| 77 | | | 91 |
| 78 | | 32 | 86 |
| 80 | | 30 | |
| 81 | | 57 | 35 |

## Patentansprüche

1. 1,3-Disubstituierte 4-Methyl-1H-pyrrol-2-carbonsäureamide der allgemeinen Formel I, 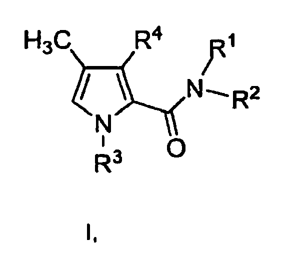 worin
R¹ für C₁₋₆-Alkyl, C₂₋₆-Alkenyl oder C₂₋₆-Alkinyl steht, das jeweils unsubstituiert oder mit 1, 2, 3, 4 oder 5 Substituenten unabhängig voneinander ausgewählt aus der Gruppe bestehend aus F, Cl, Br, I, - NO₂, -CN, -OH, -SH, -NH₂, -N(CH₃)₂, -N(C₂H₅)₂, -N(CH₃)(C₂H₅), -C(=O)- OH, -C(=O)-O-CH₃, -C(=O)-O-C₂H₅, -C(=O)-O-C(CH₃)₃, -C(=O)-O-CH₂- Phenyl, -C(=O)-NH-Naphthyl, -N(CH₃)-Phenyl, -N(C₂H₅)-Phenyl, - N(C₂H₅)-(m-Toluyl), -N(C₂H₅)-(p-Toluyl), -N(CH₃)-(p-Toluyl) und -NH- C(=O)-O-C(CH₃)₃ substituiert sein kann;
für 2- bis 6-gliedriges Heteroalkyl, Heteroalkenyl oder Heteroalkinyl steht, das jeweils unsubstituiert oder mit 1, 2, 3, 4 oder 5 Substituenten unabhängig voneinander ausgewählt aus der Gruppe bestehend aus F, Cl, Br, I, -NO₂, -CN, -OH, -SH und -NH₂ substituiert sein kann und jeweils 1 oder 2 Heteroatom(e) unabhängig voneinander ausgewählt aus der Gruppe bestehend aus Sauerstoff, Schwefel und Stickstoff (NH) als Kettenglied(er) aufweisen kann;
für C₃₋₇-Cycloalkyl, C₅₋₆-Cycloalkenyl, 5- bis 7-gliedriges Heterocycloalkyl, 5- bis 7-gliedriges Heterocycloalkenyl, (1,2,3,4)- Tetrahydrochinolinyl, (1,2,3,4)-Tetrahydroisochinolinyl, (2,3)-Dihydro-1H-isoindolyl, Indolinyl, Indanyl, (1,2,3,4)-Tetrahydronaphthyl, (2,3)-Dihydro-benzo[1.4]dioxinyl oder Benzo[1.3]dioxolyl steht, das jeweils unsubstituiert oder mit 1, 2, 3, 4 oder 5 Substituenten unabhängig voneinander ausgewählt aus der Gruppe bestehend aus F, Cl, Br, I,-CN, Methyl, Ethyl, n-Propyl, Isopropyl, n-Butyl, 2-Butyl, Isobutyl, tert-Butyl, -OH, Oxo, Thioxo, -O-CH₃, -O-C₂H₅, -O-C₃H₇, -O-Phenyl, -O-CH₂-Phenyl, -NH₂, -N(CH₃)₂, -N(C₂H₅)₂, -NH-CH₃, -NH-C₂H₅, -NO₂. - CF₃, -O-CF₃, -S-CF₃, -SH, -S-CH₃, -S-C₂H₅, -C(=O)-CH₃, -C(=O)-C₂H₅, - C(=O)-C(CH₃)₃, -C(=O)-NH-CH₃, -C(=O)-NH-C₂H₅, -CH₂-Naphthyl, Benzyl und Phenyl substituiert sein kann und/oder jeweils über eine unsubstituierte C₁₋₃-Alkylen-, C₂₋₃-Alkenylen- oder C₂₋₃-Alkinylen-Gruppe gebunden sein kann und/oder jeweils 1 oder 2 Heteroatom(e) unabhängig voneinander ausgewählt aus der Gruppe bestehend aus Sauerstoff, Schwefel und Stickstoff (NH) als Ringglied(er) aufweisen kann;
für Phenyl steht, das jeweils über eine C₁₋₃-Alkylen-, C₂₋₃-Alkenylen-oder C₂₋₃-Alkinylen-Gruppe, die jeweils unsubstituiert oder mit 1 oder 2 Substituenten ausgewählt aus der Gruppe bestehend aus F, C, Br, - C(=O)-O-CH₃ und -C(=O)-O-C₂H₅ substituiert sein kann oder über eine -CH₂-CH₂-O-, -CH₂-O- oder -CH₂-CH₂-CH₂-O-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -C≡C-Si(CH₃)₃, - C≡C-Si(C₂H₅)₃, -CH₂-O-CH₃, -CH₂-O-C₂H₅, -NH₂, -S-CH₃, -S-C₂H₅, - S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, - C(=O)-CF₃, -S-CF₃, -S-CHF₂, -S-CH₂F, -S(=O)₂-Phenyl, Pyrazolyl, - N(CH₃)₂, -N(C₂H₅)₂, -NH-CH₃, -NH-C₂H₅, -CH₂-O-C(=O)-Phenyl, -NH-S(=O)₂-CH₃, -C(=O)-H, -C(=O)-CH₃, -C(=O)-C₂H₅, -NH-C(=O)-CH₃,-NH-C(=O)-C₂H₅, -O-C(=O)-Phenyl, -C(=O)-NH₂, -C(=O)-NH-CH₃,-C(=O)-NH-C₂H₅, -C(=O)-NH-C(CH₃)₃, -C(=O)-N(C₂H₅)₂, -S(=O)-NH₂, [1.2.3]-Thiadiazolyl, Cyclopropyl, Cyclobutyl, Cyclopentyl, Cyclohexyl, Piperazinyl, Pyrrolidinyl, Piperidinyl, Phenyl, Furyl (Furanyl), Thiadiazolyl, Thiophenyl (Thienyl) und Benzyl substituiert sein kann, wobei die zyklischen Substituenten bzw. die zyklischen Reste dieser Substituenten selbst jeweils mit 1, 2, 3, 4, oder 5 Substituenten unabhängig voneinander ausgewählt aus der Gruppe bestehend aus F, Cl, Br, I, -CN, -NO₂, -OH, -SH, -NH₂, Methyl, Ethyl, n-Propyl, Isopropyl, n-Butyl, Isobutyl, 2-Butyl, tert-Butyl, n-Pentyl, neo-Pentyl, -O-CH₃, -O-C₂H₅, -O-C₃H₇, -O-C(CH₃)₃, -CF₃, -CHF₂, -CH₂F, -O-CF₃, -O-CHF₂ und - O-CH₂F substituiert sein können;
für einen Rest steht ausgewählt aus der Gruppe bestehend aus Naphthyl, Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Indazolyl, Chinoxalinyl, Chinazolinyl, Chinolinyl, Naphthridinyl und Isochinolinyl, der jeweils über eine C₁₋₃-Alkylen-, C₂₋₃-Alkenylen- oder C₂₋₃-Alkinylen-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -C≡C-Si(CH₃)₃, -C≡C-Si(C₂H₅)₃, -CH₂-O-CH₃, -CH₂-O-C₂H₅, -NH₂, -C(=O)-OH, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, - C(=O)-CF₃, -S-CF₃, -S-CHF₂, -S-CH₂F, -S(=O)₂-Phenyl, Pyrazolyl, - N(CH₃)₂, -N(C₂H₅)₂, -NH-CH₃, -NH-C₂H₅, -CH₂-O-C(=O)-Phenyl, -NH-S(=O)₂-CH₃, -C(=O)-O-CH₃, -C(=O)-O-C₂H₅, -C(=O)-O-C(CH₃)₃, - C(=O)-H, -C(=O)-CH₃, -C(=O)-C₂H₅, -NH-C(=O)-CH₃, -NH-C(=O)-C₂H₅, -O-C(=O)-Phenyl, -C(=O)-NH₂, -C(=O)-NH-CH₃, -C(=O)-N(CH₃)₂, - C(=O)-O-CH(CH₃)₂, -C(=O)-O-(CH₂)₃-CH₃, -C(=O)-O-Phenyl, -O-C(=O)- CH₃, -O-C(=O)-C₂H₅, -C(=O)-NH-C₂H₅, -C(=O)-NH-C(CH₃)₃, -C(=O)- N(C₂H₅)₂, -C(=O)-NH-Phenyl, -C(=O)-N(CH₃)-Phenyl, -C(=O)-N(C₂H₅)- Phenyl, -S(=O)-NH₂, [1.2.3]-Thiadiazolyl, Cyclopropyl, Cyclobutyl, Cyclopentyl, Cyclohexyl, Piperazinyl, Pyrrolidinyl, Piperidinyl, Phenyl, Furyl (Furanyl), Thiadiazolyl, Thiophenyl (Thienyl) und Benzyl substituiert sein kann, wobei die zyklischen Substituenten bzw. die zyklischen Reste dieser Substituenten selbst jeweils mit 1, 2, 3, 4, oder 5 Substituenten unabhängig voneinander ausgewählt aus der Gruppe bestehend aus F, Cl, Br, I, -CN, -NO₂, -OH, -SH, -NH₂, Methyl, Ethyl, n- Propyl, Isopropyl, n-Butyl, Isobutyl, 2-Butyl, tert-Butyl, n-Pentyl, neo- Pentyl, Ethenyl, Allyl, Ethinyl, Propinyl, -O-CH₃, -O-C₂H₅, -O-C₃H₇, -O- C(CH₃)₃, -CF₃, -CHF₂, -CH₂F, -O-CF₃, -O-CHF₂, -O-CH₂F, -C(=O)-CF₃, - S-CF₃, -S-CHF₂ und -S-CH₂F substituiert sein können;
oder für -NH-C(=O)-R⁵ steht
R² für H oder C₁₋₆-Alkyl, C₂₋₆-Alkenyl oder C₂₋₆-Alkinyl steht, das jeweils unsubstituiert oder mit 1, 2, 3, 4 oder 5 Substituenten unabhängig voneinander ausgewählt aus der Gruppe bestehend aus F, Cl, Br, I, - NO₂, -CN, -OH, -SH, -NH₂, -N(CH₃)₂, -N(C₂H₅)₂, -N(CH₃)(C₂H₅), -C(=O)- OH, -C(=O)-O-CH₃, -C(=O)-O-C₂H₅, -C(=O)-O-C(CH₃)₃, -C(=O)-O-CH₂- Phenyl, -C(=O)-NH-Naphthyl, -N(CH₃)-Phenyl, -N(C₂H₅)-Phenyl, - N(C₂H₅)-(m-Toluyl), -N(C₂H₅)-(p-Toluyl), -N(CH₃)-(p-Toluyl) und -NH- C(=O)-O-C(CH₃)₃ substituiert sein kann;
für einen Rest steht ausgewählt aus der Gruppe bestehend aus Phenyl und Naphthyl, der jeweils über eine C₁₋₃-Alkylen-, C₂₋₃-Alkenylen- oder C₂₋₃-Alkinylen-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, - CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -C≡C-Si(CH₃)₃, -C≡C-Si(C₂H₅)₃, -CH₂- O-CH₃, -CH₂-O-C₂H₅, -NH₂, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, - CF₃, -CHF₂, -CH₂F, -O-CF₃, -O-CHF₂, -O-CH₂F, -C(=O)-CF₃, -S-CF₃, - S-CHF₂, -S-CH₂F, -S(=O)₂-Phenyl, Pyrazolyl, -N(CH₃)₂, -N(C₂H₅)₂, -NH- CH₃, -NH-C₂H₅, -CH₂-O-C(=O)-Phenyl, -NH-S(=O)₂-CH₃, -C(=O)-H, - C(=O)-CH₃, -C(=O)-C₂H₅, -NH-C(=O)-CH₃, -NH-C(=O)-C₂H₅, -O-C(=O)- Phenyl, -C(=O)-NH₂, -C(=O)-NH-CH₃, -C(=O)-NH-C₂H₅, -C(=O)-NH- C(CH₃)₃, -C(=O)-N(C₂H₅)₂, -S(=O)-NH₂, [1.2.3]-Thiadiazolyl, Cyclopropyl, Cyclobutyl, Cyclopentyl, Cyclohexyl, Piperazinyl, Pyrrolidinyl, Piperidinyl, Phenyl, Furyl (Furanyl), Thiadiazolyl, Thiophenyl (Thienyl) und Benzyl substituiert sein kann, wobei die zyklischen Substituenten bzw. die zyklischen Reste dieser Substituenten selbst jeweils mit 1, 2, 3, 4, oder 5 Substituenten unabhängig voneinander ausgewählt aus der Gruppe bestehend aus F, Cl, Br, I, -CN, -NO₂, -OH, -SH, -NH₂, Methyl, Ethyl, n-Propyl, Isopropyl, n-Butyl, Isobutyl, 2-Butyl, tert-Butyl, n-Pentyl, neo-Pentyl, -O-CH₃, -O- C₂H₅, -O-C₃H₇, -O-C(CH₃)₃, -CF₃, -CHF₂, -CH₂F, -O-CF₃, -O-CHF₂ und - O-CH₂F substituiert sein können, oder
R¹ und R² zusammen mit dem sie verbindenden Stickstoffatom einen Rest bilden ausgewählt aus der Gruppe bestehend aus Imidazolidinyl, [1,3,4,9]-Tetrahydro-b-carbolinyl, Piperidinyl, Morpholinyl, Thiomorpholinyl, Pyrrolidinyl, Piperazinyl, Azepanyl, Diazepanyl und (1,4)-Dioxo-8-aza-spiro[4.5]decyl, der jeweils unsubstituiert oder mit 1 oder 2 Resten R⁶ substituiert sein kann.
R³ für C₁₋₆-Alkyl, C₂₋₆-Alkenyl oder C₂₋₆-Alkinyl steht, das jeweils unsubstituiert oder mit 1, 2, 3, 4 oder 5 Substituenten unabhängig voneinander ausgewählt aus der Gruppe bestehend aus F, Cl, Br, I, - NO₂, -CN, -OH, -SH, -NH₂, -N(CH₃)₂, -N(C₂H₅)₂, -N(CH₃)(C₂H₅), -C(=O)- OH, -C(=O)-O-CH₃, -C(=O)-O-C₂H₅, -C(=O)-O-C(CH₃)₃, -C(=O)-O-CH₂- Phenyl, -C(=O)-NH-Naphthyl, -N(CH₃)-Phenyl, -N(C₂H₅)-Phenyl, - N(C₂H₅)-(m-Toluyl), -N(C₂H₅)-(p-Toluyl), -N(CH₃)-(p-Toluyl) und -NH- C(=O)-O-C(CH₃)₃ substituiert sein kann; oder
für einen Rest steht ausgewählt aus der Gruppe bestehend aus Phenyl, Naphthyl, Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Indazolyl, Chinoxalinyl, Chinazolinyl, Chinolinyl, Naphthridinyl und Isochinolinyl, der jeweils über eine C₁₋₃-Alkylen-, C₂₋₃- Alkenylen- oder C₂₋₃-Alkinylen-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n- Propyl, Isopropyl, n-Butyl, Isobutyl, 2-Butyl, tert-Butyl, n-Pentyl, neo- Pentyl, Ethenyl, Allyl, Ethinyl, Propinyl, -CH₂-O-CH₃, -CH₂-O-C₂H₅, - NH₂, -C(=O)-OH, -S-CH₃, -S-C₂H₅, -S(=O)-CH₃, -S(=O)₂-CH₃, -S(=O)- C₂H₅, -S(=O)₂-C₂H₅, -O-CH₃, -O-C₂H₅, -O-C₃H₇, -O-C(CH₃)₃, -CF₃, - CHF₂, -CH₂F, -O-CF₃, -O-CHF₂, -O-CH₂F, -C(=O)-CF₃, -S-CF₃, -S- CHF₂, -S-CH₂F, -N(CH₃)₂, -N(C₂H₅)₂, -NH-CH₃, -NH-C₂H₅, -C(=O)-H, - C(=O)-CH₃, -C(=O)-C₂H₅, -C(=O)-NH₂, -C(=O)-NH-CH₃ und -C(=O)- N(CH₃)₂ substituiert sein kann.
R⁴ für Phenyl steht, das jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -OH, -SH, -NH₂, -C(=O)-OH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -C≡C-Si(CH₃)₃, - C≡C-Si(C₂H₅)₃, -S-CH₃, -S-C₂H₅, -S-Phenyl, -S-CH₂-Phenyl, -O-CH₃, - O-C₂H₅, -O-C₃H₇, -O-C(CH₃)₃, -O-Phenyl, -O-CH₂-Phenyl, -CF₃, -CHF₂, -CH₂F, -O-CF₃, -O-CHF₂, -O-CH₂F, -C(=O)-CF₃, -S-CF₃, -S-CHF₂, -S- CH₂F, -S(=O)₂-Phenyl, -N(CH₃)₂, -N(C₂H₅)₂, -NH-CH₃, -NH-C₂H₅, - C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, - C(=O)-NH-CH₃, -C(=O)-N(CH₃)₂, -S(=O)₂-NH₂, -S(=O)₂-NH-Phenyl, [1,2,3]-Thiadiazolyl, Cyclopropyl, Cyclobutyl, Cyclopentyl, Cyclohexyl, Phenyl, Benzyl und Phenethyl substituiert sein kann, wobei die zyklischen Substituenten bzw. die zyklischen Reste dieser Substituenten selbst jeweils unsubstituiert oder mit 1, 2, 3, 4 oder 5, Substituenten unabhängig voneinander ausgewählt aus der Gruppe bestehend aus F, Cl, Br, I, -CN, -NO₂, -OH, -SH, -NH₂, -C(=O)-OH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -O-CH₃, -O-C₂H₅, - O-C₃H₇, -O-C(CH₃)₃, -CF₃, -CHF₂, -CH₂F, -O-CF₃, -O-CHF₂, -O-CH₂F, - C(=O)-CF₃, -S-CF₃, -S-CHF₂ und -S-CH₂F substituiert sein können;
oder
für einen Rest steht ausgewählt aus der Gruppe bestehend aus Thienyl, Furyl, Pyrrolyl, Pyrazolyl, Pyrazinyl, Pyranyl, Triazolyl, Pyridinyl, Imidazolyl, Thiadiazolyl, Oxadiazolyl und Pyridazinyl, der jeweils unsubstituiert oder mit 1, 2, 3, 4 oder 5 Substituenten unabhängig voneinander ausgewählt aus der Gruppe bestehend aus F, Cl, Br, I, - CN, -NO₂, -OH, -CH₂-CN, -SH, -NH₂, -C(=O)-OH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -S-CH₃, -S-C₂H₅, -S-Phenyl, -S-CH₂-Phenyl, -O-CH₃, -O-C₂H₅, -O-C₃H₇, -O-C(CH₃)₃, -O-Phenyl, -O-CH₂-Phenyl, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅. -C(=O)-O-CH₃, -C(=O)-O-C₂H₅, -C(=O)-O-C(CH₃)₃, -C(=O)-H - C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -S(=O)₂-NH₂, Cyclopropyl, Cyclobutyl, Cyclopentyl, Cyclohexyl, Phenyl und Benzyl substituiert sein kann, wobei die zyklischen Substituenten bzw. die zyklischen Reste dieser Substituenten selbst jeweils unsubstituiert oder mit 1, 2, 3, 4 oder 5, Substituenten unabhängig voneinander ausgewählt aus der Gruppe bestehend aus F, Cl, Br, I, -CN, -NO₂, -OH, -SH, -NH₂, -C(=O)-OH, Methyl, Ethyl, n-Propyl, Isopropyl, n-Butyl, Isobutyl, 2-Butyl, tert-Butyl, n-Pentyl, neo-Pentyl, -O-CH₃, -O-C₂H₅, -O-C₃H₇, -O-C(CH₃)₃, -CF₃, - CHF₂, -CH₂F, -O-CF₃, -O-CHF₂, -O-CH₂F, -C(=O)-CF₃, -S-CF₃, -S-CHF₂ und -S-CH₂F substituiert sein können;
R⁵ für einen Rest steht ausgewählt aus der Gruppe bestehend aus Phenyl, Naphthyl, Thienyl, Furyl, Pyrrolyl, Pyrazolyl, Pyrazinyl, Pyranyl, Triazolyl, Pyridinyl, Imidazolyl, Indolyl, Isoindolyl, Thiazolyl, Thiadiazolyl, Oxazolyl, Oxadiazolyl, Isoxazolyl, Pyridazinyl, Indazolyl, Chinoxalinyl, Chinazolinyl, Chinolinyl, Naphthridinyl und Isochinolinyl, der jeweils über eine C₁₋₃-Alkylen-, C₂₋₃-Alkenylen- oder C₂₋₃-Alkinylen-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O- Phenyl, Methyl, Ethyl, n-Propyl, Isopropyl, n-Butyl, Isobutyl, 2-Butyl, tert- Butyl, n-Pentyl, neo-Pentyl, Ethenyl, Allyl, Ethinyl, Propinyl, -NH₂. - C(=O)-OH, -S-CH₃, -S-C₂H₅, -O-CH₃, -O-C₂H₅, -O-C₃H₇, -O-C(CH₃)₃, - CF₃, -CHF₂, -CH₂F, -O-CF₃, -O-CHF₂, -O-CH₂F, -C(=O)-CF₃, -S-CF₃, - S-CHF₂, -S-CH₂F, -N(CH₃)₂, -N(C₂H₅)₂, -NH-CH₃, -NH-C₂H₅, -C(=O)-H, -C(=O)-CH₃, -C(=O)-C₂H₅, -C(=O)-NH₂, -C(=O)-NH-CH₃ und -C(=O)- N(CH₃)₂ substituiert sein kann;
R⁶ für -OH; F; Cl; Br; I; -SH; -NO₂; -NH₂; -NH-C(=O)-O-R⁷; -C(=O)-O-R⁸_{;} -C(=O)-R⁹ steht;
für C₁₋₆-Alkyl, C₂₋₆-Alkenyl oder C₂₋₆-Alkinyl steht, das jeweils unsubstituiert oder mit 1, 2, 3, 4 oder 5 Substituenten unabhängig voneinander ausgewählt aus der Gruppe bestehend aus F, Cl, Br, I, - NO₂, -CN, -OH, -SH, -NH₂, -N(CH₃)₂, -N(C₂H₅)₂, -C(=O)-Pyrrolidinyl, - C(=O)-N(CH₃)-Phenyl und -C(=O)-NH-CH(CH₃)₂, -N(CH₃)(C₂H₅), - C(=O)-OH, -C(=O)-O-CH₃, -C(=O)-O-C₂H₅ und -C(=O)-O-C(CH₃)₃ substituiert sein kann; für C₃₋₇-Cycloalkyl, C₅₋₆-Cycloalkenyl, 5- bis 7-gliedriges Heterocycloalkyl oder 5- bis 7-gliedriges Heterocycloalkenyl steht, das jeweils unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, -O-CH₃, -O-C₂H₅, -O-C₃H₇, -O-Phenyl, -O-CH₂-Phenyl, -NH₃, -N(CH₃)₂. -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂H₅, -CH₂-Naphthyl, Benzyl und Phenyl substituiert sein kann und/oder jeweils über eine unsubstituierte C₁₋₃-Alkylen-, C₂₋₃-Alkenylen- oder C₂₋₃-Alkinylen-Gruppe gebunden sein kann und/oder jeweils 1 oder 2 Heteroatom(e) unabhängig voneinander ausgewählt aus der Gruppe bestehend aus Sauerstoff, Schwefel und Stickstoff (NH) als Ringglied(er) aufweisen kann; oder
für einen Rest steht ausgewählt aus der Gruppe bestehend aus Phenyl, Naphthyl, Indolizinyl, Benzimidazolyl, Tetrazolyl, Triazinyl, Isoxazolyl, Phthalazinyl, Carbazolyl, Carbolinyl, Diaza-naphthyl, Thienyl. Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Indazolyl, Chinoxalinyl, Chinazolinyl, Chinolinyl, Naphthridinyl, Thieno[2,3-d]pyrimidinyl und Isochinolinyl, der jeweils über eine C₁₋₃-Alkylen-, C₂₋₃-Alkenylen- oder C₂₋₃-Alkinylen-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -NH₂, - C(=O)-OH, -S-CH3, -S-C₂H₅, -O-CH₃, -O-C₂H₅, -O-C₃H₇, -O-C(CH₃)₃, - CF₃, -CHF₂, -CH₂F, -O-CF₃, -O-CHF₂, -O-CH₂F, -C(=O)-CF₃, -S-CF₃, - S-CHF₂, -S-CH₂F, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(-O)-CH₃, - C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -C(=O)-O-CH(CH₃)₂, -C(=O)-O-(CH₂)₃-CH₃, - C(=O)-NH-C₂H₅, -C(=O)-NH-C(CH₃)₃, -C(=O)-N(C₂H₅)₂, Cyclopropyl, Cyclobutyl, Cyclopentyl, Cyclohexyl, Phenyl, Furyl (Furanyl) und Benzyl substituiert sein kann, wobei die zyklischen Substituenten bzw. die zyklischen Reste dieser Substituenten selbst jeweils mit 1, 2, 3, 4, oder 5 Substituenten unabhängig voneinander ausgewählt aus der Gruppe bestehend aus F, Cl, Br, I, -CN, -NO₂, -OH, -SH, -NH₂, Methyl, Ethyl, n-Propyl, Isopropyl, n-Butyl, Isobutyl, 2-Butyl, tert-Butyl, n-Pentyl, neo-Pentyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ und -S-CH₂F substituiert sein können; und
R⁷, R⁸ und R⁹, unabhängig voneinander, jeweils steht
für C₁₋₆-Alkyl, C₂₋₆-Alkenyl oder C₂₋₆-Alkinyl, das jeweils unsubstituiert oder mit 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, -N(C₂H₅)₂, -N(CH₃)(C₂H₅). -C(=O)-OH, -C(=O)-O-CH₃, -C(=O)-O-C₂H₅ und -C(=O)-O-C(CH₃)₃ substituiert sein kann;
für einen Rest steht ausgewählt aus der Gruppe bestehend aus Indolinyl, Indanyl, (1,2,3,4)-Tetrahydronaphthyl, (2,3)-Dihydro-benzo[1.4]dioxinyl und Benzo[1.3]dioxolyl, der jeweils unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, -O-CH₃. -O-C₂H₅, -O-C₃H₇, -O-Phenyl, -O-CH₂-Phenyl, - NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃. -C(=O)-NH-CH₃, -C(=O)-NH-C₂H₅, -CH₂-Naphthyl, Benzyl und Phenyl substituiert sein kann; oder
für einen Rest steht ausgewählt aus der Gruppe bestehend aus Phenyl, Naphthyl, Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl. Pyridinyl, Imidazolyl, Indolyl, Isoindotyl, Thiazolyl, Thiadiazolyl, Oxazolyl, Oxadiazolyl, Isoxazolyl und Pyridazinyl, der jeweils über eine C₁₋₃-Alkylen-, C₂₋₃-Alkenylen- oder C₂₋₃-Alkinylen-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, - N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -C(=O)-O-CH(CH₃)₂, -C(=O)-O-(CH₂)₃-CH₃, -C(=O)-NH-C₂H₅, - C(=O)-NH-C(CH₃)₃, -C(=O)-N(C₂H₅)₂, Cyclopropyl, Cyclobutyl, Cyclopentyl, Cyclohexyl, Phenyl, Furyl (Furanyl) und Benzyl substituiert sein kann,
jeweils in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

2. Verbindungen gemäß Anspruch 1, **dadurch gekennzeichnet, dass**
R¹ für C₁₋₆-Alkyl, C₂₋₆-Alkenyl oder C₂₋₆-Alkinyl steht, das jeweils unsubstituiert oder mit 1, 2, 3, 4 oder 5 Substituenten unabhängig voneinander ausgewählt aus der Gruppe bestehend aus F, Cl, Br, I, - NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O-C₂H₅, -C(=O)-O-C(CH₃)₃, -C(=O)-O-CH₂-Phenyl, -C(=O)-NH-Naphthyl, -N(CH₃)-Phenyl, -N(C₂H₅)-Phenyl, - N(C₂H₅)-(m-Toluyl), -N(C₂H₅)-(p-Toluyl), -N(CH₃)-(p-Toluyl) und -NH-C(=O)-O-C(CH₃)₃ substituiert sein kann;
für 2- bis 6-gliedriges Heteroalkyl steht, das jeweils unsubstituiert oder mit 1, 2, 3, 4 oder 5 Substituenten unabhängig voneinander ausgewählt aus der Gruppe bestehend aus F, Cl, Br, I, -NO₂, -CN, -OH, -SH und - NH₂ substituiert sein kann und jeweils 1 oder 2 Heteroatom(e) unabhängig voneinander ausgewählt aus der Gruppe bestehend aus Sauerstoff, Schwefel und Stickstoff (NH) als Kettenglied(er) aufweisen kann;
für C₃₋₇-Cycloalkyl, C₅₋₆-Cycloalkenyl, 5- bis 7-gliedriges Heterocycloalkyl, 5- bis 7-gliedriges Heterocycloalkenyl, (1,2,3,4)-Tetrahydrochinolinyl, (1,2,3,4)-Tetrahydroisochinolinyl, (2,3)-Dihydro-1H-isoindolyl, Indolinyl, Indanyl, (1,2,3,4)-Tetrahydronaphthyl, (2,3)-Dihydro-benzo[1.4]dioxinyl oder Benzo[1.3]dioxolyl steht, das jeweils unsubstituiert oder mit 1, 2, 3, 4 oder 5 Substituenten unabhängig voneinander ausgewählt aus der Gruppe bestehend aus F, Cl, Br, I, - CN, Methyl, Ethyl, n-Propyl, Isopropyl, n-Butyl. 2-Butyl, Isobutyl, tert-Butyl, -OH, Oxo, Thioxo, -O-CH₃, -O-C₂H₅, -O-C₃H₇, -O-Phenyl, -O-CH₂-Phenyl, -NH₂, -N(CH₃)₂, -N(C₂H₅)₂, -NH-CH₃, -NH-C₂H₅, -NO₂, - CF₃, -O-CF₃, -S-CF₃, -SH, -S-CH₃, -S-C₂H₅, -C(=O)-CH₃, -C(=O)-C₂H₅, - C(=O)-C(CH₃)_{3,} -C(=O)-NH-CH₃, -C(=O)-NH-C₂H₅, -CH₂-Naphthyl, Benzyl und Phenyl substituiert sein kann und/oder jeweils über eine unsubstituierte C₁₋₃-Alkylen-, C₂₋₃-Alkenylen- oder C₂₋₃-Alkinylen-Gruppe gebunden sein kann und/oder jeweils 1 oder 2 Heteroatom(e) unabhängig voneinander ausgewählt aus der Gruppe bestehend aus Sauerstoff, Schwefel und Stickstoff (NH) als Ringglied(er) aufweisen kann;
für Phenyl steht, das jeweils über eine C₁₋₃-Alkylen-, C₂₋₃-Alkenylen-oder C₂₋₃-Alkdnylen-Gruppe, die jeweils unsubstituiert oder mit 1 oder 2 Substituenten ausgewählt aus der Gruppe bestehend aus F, Cl, Br, I - C(=O)-O-CH₃ und -C(=O)-O-C₂H₅ substituiert sein kann, oder über eine -CH₂-CH₂-O-. -CH₂-O- oder -CH₂-CH₂-CH₂-O-Gruppe gebunden sein kann und/oder unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -C≡C-Si(CH₃)₃, -C≡C-Si(C₂H₅)₃, -CH₂-O-CH₃, -CH₂-O-C₂H₅, -NH₂, -S-CH₃, -S-C₂H₅, -S(=O)-CH₃, - S(=O)₂-CH₃, -S(=O)-C₂H₅, -S(=O)₂-C₂H₅, -O-CH₃, -O-C₂H₅, -O-C₃H_{7,} - O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-Phenyl, Pyrazolyl, -N(CH₃)₂, - N(C₂H₅)₂, -NH-CH₃, -NH-C₂H₅, -CH₂-O-C(=O)-Phenyl, -NH-S(=O)₂-CH₃, -C(=O)-H, -C(=O)-CH₃, -C(=O)-C₂H₅, -NH-C(=O)-CH₃, -NH-C(=O)-C₂H₅, -O-C(=O)-Phenyl, -C(=O)-NH_{2,} -C(=O)-NH-CH₃, -C(=O)-NH-C₂H_{5,} - C(=O)-NH-C(CH₃)₃, -C(=O)-N(C₂H₅)₂, -S(=O)-NH₂, [1.2.3]-Thiadiazolyl, Cyclopropyl, Cyclobutyl, Cydopentyl, Cyclohexyl. Piperazinyl, Pyrrolidinyl, Piperidinyl, Phenyl, Furyl (Furanyl), Thiadiazolyl, Thiophenyl (Thienyl) und Benzyl substituiert sein kann, wobei die zyklischen Substituenten bzw. die zyklischen Reste dieser Substituenten selbst jeweils mit 1, 2, 3, 4, oder 5 Substituenten unabhängig voneinander ausgewählt aus der Gruppe bestehend aus F, Cl, Br, I, -CN, -NO₂, -OH, -SH, -NH₂, Methyl, Ethyl, n-Propyl, Isopropyl, n-Butyl, Isobutyl, 2-Butyl, tert-Butyl, n-Pentyl, neo-Pentyl, -O-CH₃, -O-C₂H₅, -O-C₃H₇, -O-C(CH₃)₃, -CF₃, -CHF₂, -CH₂F, -O-CF₃, -O-CHF₂ und - O-CH₂F substituiert sein können;
für einen Rest steht ausgewählt aus der Gruppe bestehend aus Naphthyl, Indolizinyl, Benzimidazolyl, Tetrazolyl, Triazinyl, lsoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl. Indolyl, Isoindolyl, Benzo[b]furanyl, Benzo[b]thiophenyl, Benzo[d]thlazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Indazolyl, Chinoxalinyl, Chinazolinyl, Chinolinyl, Naphthridinyl und Isochinolinyl, der jeweils über eine C₁₋₃-Alkylen-, C₂₋₃-Alkenylen- oder C₂₋₃-Alkinylen-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n-Propyl, Isopropyl, n-Butyl, lsobutyl, 2-Butyl, tert-Butyl, n-Pentyl, neo-Pentyl, Ethenyl, Allyl, Ethinyl, Propinyl, -C=C-Si(CH₃)₃, -C=C-Si(C₂H₅)₃, -CH₂-O-CH₃, -CH₂-O-C₂H₅, -NH₂, -C(=O)-OH, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, - C(=O)-CF₃, -S-CF₃, -S-CHF₂, -S-CH₂F, -S(=O)₂-Phenyl, Pyrazolyl, - N(CH₃)₂, -N(C₂H₅)₂, -NH-CH₃, -NH-C₂H₅, -CH₂-O-C(=O)-Phenyl, -NH-S(=O)₂-CH₃, -C(=O)-O-CH₃, -C(=O)-O-C₂H₅, -C(=O)-O-C(CH₃)₃, - C(=O)-H, -C(=O)-CH₃, -C(=O)-C₂H₅, -NH-C(=O)-CH₃, -NH-C(=O)-C₂H₅, -O-C(=O)-Phenyl, -C(=O)-NH₂, -C(=O)-NH-CH₃, -C(=O)-N(CH₃)₂, - C(=O)-O-CH(CH₃)₂, -C(=O)-O-(CH₂)₃-CH₃, -C(=O)-O-Phenyl, -O-C(=O)-CH₃, -O-C(=O)-C₂H₅, -C(=O)-NH-C₂H₅, -C(=O)-NH-C(CH₃)₃, -C(=O)-N(C₂H₅)₂, -C(=O)-NH-Phenyl, -C(=O)-N(CH₃)-Phenyl, -C(=O)-N(C₂H₅)-Phenyl, -S(=O)-NH₂, [1.2.3]-Thiadiazolyl, Cyclopropyl, Cyclobutyl, Cyclopentyl, Cyclohexyl, Piperazinyl, Pyrrolidinyl, Piperidinyl, Phenyl, Furyl (Furanyl), Thiadiazolyl, Thiophenyl (Thienyl) und Benzyl substituiert sein kann, wobei die zyklischen Substituenten bzw. die zyklischen Reste dieser Substituenten selbst jeweils mit 1, 2, 3, 4, oder 5 Substituenten unabhängig voneinander ausgewählt aus der Gruppe bestehend aus F, Cl, Br, I, -CN, -NO₂, -OH, -SH, -NH₂, Methyl, Ethyl, n-Propyl,Isopropyl, n-Butyl, Isobutyl, 2-Butyl, tert-Butyl, n-Pentyl, neo-Pentyl, Ethenyl, Allyl, Ethinyl, Propinyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, - S-CF₃, -S-CHF₂ und -S-CH₂F substituiert sein können;
oder für -NH-C(=O)-R⁵ steht;
R² für H oder C₁₋₆-Alkyl steht, das jeweils unsubstituiert oder mit 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)- O-CH₃, -C(=O)-O-C₂H₅, -C(=O)-O-C(CH₃)₃, -C(=O)-O-CH₂- Phenyl, -C(=O)-NH-Naphthyl, -N(CH₃)-Phenyl, -N(C₂H₅)-Phenyl, -N(C₂H₅)-(m-Toluyl), -N(C₂H₅)-(p-Toluyl), -N(CH₃)-(p-Toluyl) und -NN-C(=O)-O-C(CH₃)₃ substituiert sein kann; oder
für einen Rest steht ausgewählt aus der Gruppe bestehend aus Phenyl und Naphthyl, der jeweils über eine-C₁₋₃-Alkylen-, C₂₋₃-Alkenylen- oder C₂₋₃-Alkinylen-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, - CN, -CH₂-CN, -NO₂. -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -C≡C-Si(CH₃)₃, -C≡C-Si(C₂H₅)₃, -CH₂-O-CH₃, -CH₂-O-C₂H₅, -NH₂, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)_{3,} - CF₃, -CHF₂, -CH₂F, -O-CF₃, -O-CHF₂, -O-CH₂F, -C(=O)-CF₃, -S-CF₃, - S-CHF₂, -S-CH₂F, -N(CH₃)₂, -N(C₂H₅)₂, -NH-CH₃ und -NH-C₂H₅ substituiert sein kann;
oder R¹ und R² zusammen mit dem sie verbindenden Stickstoffatom einen Rest ausgewählt aus der Gruppe bestehend aus 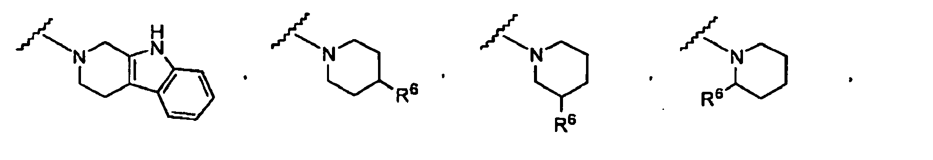 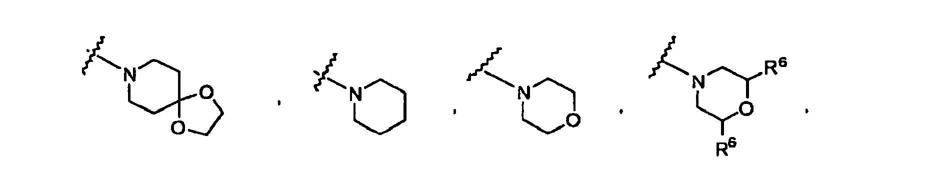 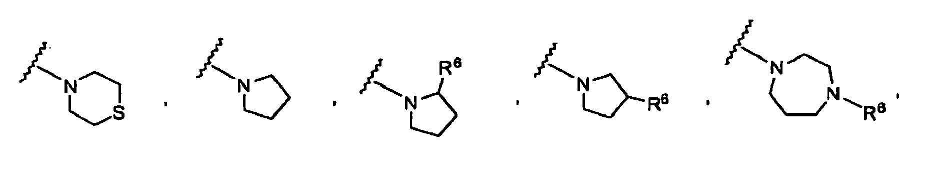 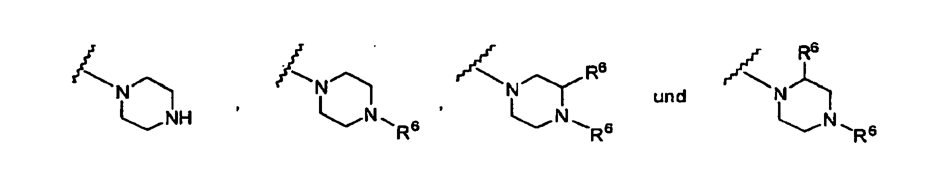 bilden;
R³ für C₁₋₆-Alkyl steht, das jeweils unsubstituiert oder mit 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, - N(C₂H₅)₂, -N(CH₃)(C₂H₅), -C(=O)-OH, -C(=O)-O-CH₃, -C(=O)-O-C₂H₅ und -C(=O)-O-C(CH₃)₃ substituiert sein kann; oder
für einen Rest steht ausgewählt aus der Gruppe bestehend aus Phenyl, Isoxazolyl, Thienyl, Furyl, Pyrrolyl, Pyrazolyl, Pyrazinyl, Pyranyl, Triazolyl, Pyridinyl, Imidazolyl, Indolyl, Isoindolyl, Thiazolyl, Thiadiazolyl, Oxazolyl, Oxadiazolyl, Pyridazinyl und Isochinolinyl, der jeweils über eine C₁₋₃-Alkylen-, C₂₋₃-Alkenylen- oder C₂₋₃-Alkinylen-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O- Phenyl, Methyl, Ethyl, n-Propyl, Isopropyl, n-Butyl, Isobutyl,2-Butyl, tert- Butyl, n-Pentyl, neo-Pentyl, Ethenyl, Allyl, Ethinyl, Propinyl, -CH₂-O- CH₃, -CH₂-O-C₂H₅, -NH₂, -C(=O)-OH, -S-CH₃, -S-C₂H₅, -S(=O)-CH₃, - S(=O)₂-CH₃, -S(=O)-C₂H₅, -S(=O)₂-C₂H₅, -O-CH₃, -O-C₂H₅, -O-C₃H₇, - O-C(CH₃)₃, -CF₃, -CHF₂, -CH₂F, -O-CF₃, -O-CHF₂, -O-CH₂F, -C(=O)- CF₃, -S-CF₃, -S-CHF₂, -S-CH₂F, -N(CH₃)₂, -N(C₂H₅)₂, -NH-CH₃, -NH- C₂H₅, -C(=O)-H, -C(=O)-CH₃, -C(=O)-C₂H₅, -C(=O)-NH₂, -C(=O)-NH- CH₃ und -C(=O)-N(CH₃)₂ substituiert sein kann;
R⁴ für Phenyl steht, das jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -OH, -SH, -NH₂, -C(=O)-OH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -C≡C-Si(CH₃)₃, - C=C-Si(C₂H₅)₃, -S-CH₃, -S-C₂H₅, -S-Phenyl, -S-CH₂-Phenyl, -O-CH₃, - O-C₂H₅, -O-C₃H₇, -O-C(CH₃)₃, -O-Phenyl, -O-CH₂-Phenyl, -CF₃, -CHF₂, -CH₂F, -O-CF₃, -O-CHF₂, -O-CH₂F, -C(=O)-CF₃, -S-CF₃, -S-CHF₂, -S- CH₂F, -S(=O)₂-Phenyl, -N(CH₃)₂, -N(C₂H₅)₂, -NH-CH₃, -NH-C₂H₅, - C(=O)-O-CH₃, -C(=O)-O-C₂H₃, -C(=O)-C(CH₃)₃, -C(=O)-H, -C(=O)- CH₃, -C(=O)-C₂H₅, -NH-C(=O)-CH₃, -NH-C(=O)-C₂H₅, -C(=O)-NH₂, - C(=O)-NH-CH₃, -C(=O)-N(CH₃)₂, -S(=O)₂-NH₂, -S(=O)₂-NH-Phenyl, [1,2,3]-Thiadiazolyl, Cyclopropyl, Cyclobutyl, Cyclopentyl, Cyclohexyl, Phenyl, Benzyl und Phenethyl substituiert sein kann, wobei die zyklischen Substituenten bzw. die zyklischen Reste dieser Substituenten selbst jeweils unsubstituiert oder mit 1, 2, 3, 4 oder 5, Substituenten unabhängig voneinander ausgewählt aus der Gruppe bestehend aus F, Cl, Br, I, -CN, -NO₂, -OH, -SH, -NH₂, -C(=O)-OH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -O-CH₃, -O-C₂H₅, - O-C₃H₇, -O-C(CH₃)₃, -CF₃, -CHFz, -CH₂F, -O-CF₃, -O-CHF₂, -O-CH₂F, - C(=O)-CF₃, -S-CF₃, -S-CHF₂ und -S-CH₂F substituiert sein können; oder
für einen Rest steht ausgewählt aus der Gruppe bestehend aus Thienyl, Furyl, Pyrrolyl, Pyrazolyl, Pyrazinyl, Pyranyl, Triazolyl, Pyridinyl, Imidazolyl, Thiadiazolyl, Oxadiazolyl und Pyridazinyl steht, der jeweils unsubstituiert oder mit 1, 2, 3, 4 oder 5 Substituenten unabhängig voneinander ausgewählt aus der Gruppe bestehend aus F, Cl, Br, I, - CN, -NO₂, -OH, -CH₂-CN, -SH, -NH₂, -C(=O)-OH, Methyl, Ethyl, n- Propyl, Isopropyl, n-Butyl, Isobutyl, 2-Butyl, tert-Butyl, n-Pentyl, neo- Pentyl, Ethenyl, Allyl, Ethinyl, Propinyl, -S-CH₃, -S-C₂H₅, -S-Phenyl, -S- CH₂-Phenyl, -O-CH₃, -O-C₂H₅, -O-C₃H₇, -O-C(CH₃)₃, -O-Phenyl, -O- CH₂-Phenyl, -CF₃, -CHF₂, -CH₂F, -O-CF₃, -O-CHF₂, -O-CH₂F, -C(=O)- CF₃, -S-CF₃, -S-CHF₂, -S-CH₂F, -N(CH₃)₂, -N(C₂H₅)₂, -NH-CH₃, -NH- C₂H₅, -C(=O)-O-CH₃, -C(=O)-O-C₂H₅, -C(=O)-O-C(CH₃)₃, -C(=O)-H - C(=O)-CH₃, -C(=O)-C₂H₅, -NH-C(=O)-CH₃, -NH-C(=O)-C₂H₅, -C(=O)- NH₂, -C(=O)-NH-CH₃, -G(=O)-N(CH₃)₂, -S(=O)₂-NH₂, Cyclopropyl, Cyclobutyl, Cyclopentyl, Cyclohexyl, Phenyl und Benzyl substituiert sein kann, wobei die zyklischen Substituenten bzw. die zyklischen Reste dieser Substituenten selbst jeweils unsubstituiert oder mit 1, 2, 3, 4 oder 5, Substituenten unabhängig voneinander ausgewählt aus der Gruppe bestehend aus F, Cl, Br, I, -CN, -NO₂, -OH, -SH, -NH₂, -C(=O)- OH, Methyl, Ethyl, n-Propyl, Isopropyl, n-Butyl, Isobutyl, 2-Butyl, tert- Butyl, n-Pentyl, neo-Pentyl, -O-CH₃, -O-C₂H₅, -O-C₃H₇, -O-C(CH₃)₃, - CF₃, -CHF₂, -CH₂F, -O-CF₃, -O-CHF₂, -O-CH₂F, -C(O)-CF₃, -S-CF₃, - S-CHF₂ und -S-CH₂F substituiert sein können;
R⁵ für einen Rest ausgewählt aus der Gruppe bestehend aus Phenyl, Naphthyl, Thienyl, Furyl, Pyrrolyl, Pyrazolyl, Pyrazinyl, Pyranyl, Triazolyl, Pyridinyl, Imidazolyl, Indolyl, Isoindolyl, Thiazolyl, Thiadiazolyl, Oxazolyl, Oxadiazolyl, Isoxazolyl und Pyridazinyl steht, der jeweils über eine C₁₋₃₋Alkylen-, C₂₋₃-Alkenylen- oder C₂₋₃-Alkinylen-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -NH₂, -S-CH₃, -S- C₂H₅, -O-CH₃, -O-C₂H₅, -O-C₃H₇, -O-C(CH₃)₃, -CF₃, -CHF₂, -CH₂F, -O- CF₃, -O-CHF₂, -O-CH₂F, -C(=O)-CF₃, -S-CF₃, -S-CHF₂, -S-CH₂F, - N(CH₃)₂, -N(C₂H₅)₂, -NH-CH₃ und -NH-C₂H₅ substituiert sein kann;
R⁶ für -OH; F; Cl; Br, I; -SH; -NO₂; -NH₂; -NH-C(=O)-O-R⁷; -C(=O)-O-R⁸; -C(=O)-R⁹ steht,
für C₁₋₆-Alkyl, C₂₋₆-Alkenyl oder C₂₋₆-Alkinyl steht, das jeweils unsubstituiert oder mit 1, 2, 3, 4 oder 5 Substituenten unabhängig voneinander ausgewählt aus der Gruppe bestehend aus F, Cl, Br, I, - NO₂, -CN, -OH, -SH, -NH₂, -N(CH₃)₂, -C(=O)-Pyrrolidinyl, -C(=O)- N(CH₃)-Phenyl und -C(=O)-NH-CH(CH₃)₂, -N(C₂H₅)₂, -N(CH₃)(C₂H₅), - C(=O)-OH, -C(=O)-O-CH₃, -C(=O)-O-C₂H₅ und -C(=O)-O-C(CH₃)₃ substituiert sein kann;
für C₃₋₇-Cycloalkyl, C₅₋₆-Cycloalkenyl, 5- bis 7-gliedriges Heterocycloalkyl oder 5- bis 7-gliedriges Heterocycloalkenyl steht, das jeweils unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, -O-CH₃, -O-C₂H₅, -O-C₃H₇, -O- Phenyl, -O-CH₂-Phenyl, -NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)- C₂H₅, -C(=O)-C(CH₃)₃, -C(=O)-NH-CH₃, -C(=O)-NH-C₂H₅, -CH₂- Naphthyl, Benzyl und Phenyl substituiert sein kann und/oder jeweils über eine unsubstituierte C₁₋₃-Alkylen-, C₂₋₃-Alkenylen- oder C₂₋₃- Alkinylen-Gruppe gebunden sein kann und/oder jeweils 1 oder 2 Heteroatom(e) unabhängig voneinander ausgewählt aus der Gruppe bestehend aus Sauerstoff, Schwefel und Stickstoff (NH) als Ringglied(er) aufweisen kann; oder
für einen Rest ausgewählt aus der Gruppe bestehend aus Phenyl, Naphthyl, Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Indazolyl, Chinoxalinyl, Chinazolinyl, Chinolinyl, Naphthridinyl, Thieno[2,3-d]pyrimidinyl und Isochinolinyl steht, der jeweils über eine C₁₋₃-Alkylen-, C₂₋₃-Alkenylen- oder C₂₋₃-Alkinylen-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, - O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -NH₂, - C(=O)-OH, -S-CH₃, -S-C₂H₅, -O-CH₃, -O-C₂H₅, -O-C₃H₇, -O-C(CH₃)₃, - CF₃, -CHF₂, -CH₂F, -O-CF₃, -O-CHF₂, -O-CH₂F, -C(=O)-CF₃, -S-CF₃, - S-CHF₂, -S-CH₂F, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(=O)-CH₃, - C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -C(=O)-O-CH(CH₃)₂, -C(=O)-O-(CH₂)₃-CH₃, - C(=O)-NH-C₂H₅, -C(=O)-NH-C(CH₃)₃, -C(=O)-N(C₂H₅)₂, Cyclopropyl, Cyclobutyl, Cyclopentyl, Cyclohexyl, Phenyl, Furyl (Furanyl) und Benzyl substituiert sein kann, wobei die zyklischen Substituenten bzw. die zyklischen Reste dieser Substituenten selbst jeweils mit 1, 2, 3, 4, oder 5 Substituenten unabhängig voneinander ausgewählt aus der Gruppe bestehend aus F, Cl, Br, I, -CN, -NO₂, -OH, -SH, -NH₂, Methyl, Ethyl, n-Propyl, Isopropyl, n-Butyl, Isobutyl, 2-Butyl, tert-Butyl, n-Pentyl, neo-Pentyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ und -S-CH₂F substituiert sein können
und R⁷, R⁸ und R⁹, unabhängig voneinander, jeweils steht
für C₁₋₆-Alkyl, C₂₋₆-Alkenyl oder C₂₋₆-Alkinyl, das jeweils unsubstituiert oder mit 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O-C₂H₅ und -C(=O)-O-C(CH₃)₃ substituiert sein kann;
für einen Rest ausgewählt aus der Gruppe bestehend aus Indolinyl, Indanyl, (1,2,3,4)-Tetrahydronaphthyl, (2,3)-Dihydro-benzo[1.4]dioxinyl und Benzo[1.3]dioxolyl, der jeweils unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, -O-CH₃, -O-C₂H₅, -O-C₃H₇, -O-Phenyl, -O-CH₂-Phenyl, -NH₂, -N(CH₃)₂, - N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂H₅, -CH₂-Naphthyl, Benzyl und Phenyl substituiert sein kann; oder
für einen Rest ausgewählt aus der Gruppe bestehend aus Phenyl, Naphthyl, Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Thiazolyl, Thiadiazolyl, Oxazolyl, Oxadiazolyl, Isoxazolyl und Pyridazinyl stehen, der jeweils über eine C₁₋₃-Alkylen-, C₂₋₃-Alkenylen- oder C₂₋₃-Alkinylen-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, - N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -C(=O)-O-CH(CH₃)₂, -C(=O)-O-(CH₂)₃-CH₃, -C(=O)-NH-C₂H₅, - C(=O)-NH-C(CH₃)₃, -C(=O)-N(C₂H₅)₂, Cyclopropyl, Cyclobutyl, Cyclopentyl, Cyclohexyl, Phenyl, Furyl (Furanyl) und Benzyl substituiert sein kann.

3. Verbindungen gemäß Anspruch 1 oder 2, **dadurch gekennzeichnet, dass**
R¹ für einen Alkyl-Rest steht ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert oder mit 1, 2, 3, 4 oder 5 Substituenten unabhängig voneinander ausgewählt aus der Gruppe bestehend aus F, Cl, Br, I, -CN, -OH, -SH, -NH₂, -N(CH₃)₂, -N(C₂H₅)₂, - N(CH₃)(C₂H₅), -C(=O)-O-CH₃, -C(=O)-O-C₂H₅, -C(=O)-O-C(CH₃)₃, - C(=O)-O-CH₂-Phenyl, -C(=O)-NH-Naphthyl, -N(CH₃)-Phenyl, -N(C₂H₅)- Phenyl, -N(C₂H₅)-(m-Toluyl), -N(C₂H₅)-(p-Toluyl), -N(CH₃)-(p-Toluyl) und -NH-C(=O)-O-C(CH₃)₃ substituiert sein kann;
für einen Heteroalkyl-Rest steht ausgewählt aus der Gruppe bestehend aus -CH₂-O-CH₃, -CH₂-O-C₂H₅, -CH₂-CH₂-O-CH₃, -CH₂-CH₂-O-C₂H₅, - CH₂-CH₂-CH₂-O-CH₃ und -CH₂-CH₂-CH₂-O-C₂H₅, der jeweils unsubstituiert oder mit 1, 2, 3, 4 oder 5 Substituenten unabhängig voneinander ausgewählt aus der Gruppe bestehend aus F, Cl und Br substituiert sein kann;
für einen Rest steht ausgewählt aus der Gruppe bestehend aus Cyclopropyl, Cyclobutyl, Cyclopentyl, Cyclohexyl, Cycloheptyl, Cyclohexenyl, Cyclopentenyl, Pyrrolidinyl, Tetrahydrothiophenyl, Tetrahydrofuranyl, Piperidinyl, Morpholinyl, Piperazinyl, Azepanyl, Diazepanyl, (1,2,3,4)-Tetrahydrochinolinyl, (1,2,3,4)- Tetrahydroisochinolinyl, (2,3)-Dihydro-1H-isoindolyl, Indolinyl, Indanyl, (1,2,3,4)-Tetrahydronaphthyl, (2,3)-Dihydro-benzo[1.4]dioxinyl und Benzo[1.3]dioxolyl, der jeweils unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, -O-CH₃, -O-C₂H₅, -O-C₃H₇, -O-Phenyl, -O-CH₂-Phenyl, -C(=O)-CH₃, -C(=O)-C₂H₅, -C(=O)-C(CH₃)₃, -C(=O)-NH-CH₃, -C(=O)-NH-C₂H₅, -CH₂-Naphthyl, Benzyl und Phenyl substituiert sein kann und/oder jeweils über eine -CH₂-, -CH(CH₃)-, - CH₂-CH₂-, -CH(CH₃)-CH₂-, -CH₂-CH(CH₃)- oder -CH₂-CH₂-CH₂-Gruppe gebunden sein kann;
für Phenyl steht, das jeweils über eine -CH₂-CH₂-O-, -CH₂-O-, -CH₂-CH₂-CH₂-O-, -CH[C(=O)-O-CH₃]-CH₂-, -CH[C(=O)-O-C₂H₅]-CH₂-, -CH₂-, -CH(CH₃)-, -CH₂-CH₂-, -CH(CH₃)-CH₂-, -CH₂-CH(CH₃)- oder -CH₂-CH₂-CH₂-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, - SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, - NH₂, -S-CH₃, -S-C₂H₅ -O-CH₃, -O-C₂H₅, -O-C₃H₇, -O-C(CH₃)₃, -CF₃, - CHF₂, -CH₂F, -O-CF₃, -O-CHF₂, -O-CH₂F, -N(CH₃)₂, -N(C₂H₅)₂, -NH-CH₃, -NH-C₂H₅, -NH-S(=O)₂-CH₃, -C(=O)-H, -C(=O)-CH₃, -C(=O)-C₂H₅, -NH-C(=O)-CH₃, -NH-C(=O)-C₂H₅, -S(=O)-NH₂, [1.2.3]-Thiadiazolyl, Cyclopropyl, Cyclobutyl, Cyclopentyl, Cyclohexyl, Phenyl, Furyl (Furanyl), Thiadiazolyl, Thiophenyl (Thienyl) und Benzyl substituiert sein kann, wobei die zyklischen Substituenten bzw. die zyklischen Reste dieser Substituenten selbst jeweils mit 1, 2, 3, 4, oder 5 Substituenten unabhängig voneinander ausgewählt aus der Gruppe bestehend aus F, Cl, Br, I, -CN, -NO₂, Methyl, Ethyl, n-Propyl, Isopropyl, n-Butyl, Isobutyl, 2-Butyl, tert-Butyl, -O-CH₃, -O-C₂H₅, -O-C₃H₇ und -O-C(CH₃)₃ substituiert sein können;
für einen Rest steht ausgewählt aus der Gruppe bestehend aus Naphthyl, Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Indazolyl, Chinoxalinyl, Chinazolinyl, Chinolinyl, Naphthridinyl und Isochinolinyl, der jeweils über -CH₂-, -CH(CH₃)-, - CH₂-CH₂-, -CH(CH₃)-CH₂-, -CH₂-CH(CH₃)- oder -CH₂-CH₂-CH₂-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O- Phenyl, Methyl, Ethyl, n-Propyl, Isopropyl, n-Butyl, Isobutyl, 2-Butyl, tert- Butyl, n-Pentyl, neo-Pentyl, -O-CH₃, -O-C₂H₅, -O-C₃H₇, -O-C(CH₃)₃, - CF₃, -CHF₂, -CH₂F, -O-CF₃, -O-CHF₂, -O-CH₂F, -C(=O)-CF₃, -S-CF₃, - S-CHF₂, -S-CH₂F, -C(=O)-NH₂, -C(=O)-NH-CH₃, -C(=O)-N(CH₃)₂, - C(=O)-O-CH(CH₃)₂ und -C(=O)-O-(CH₂)₃-CH₃, substituiert sein kann
oder für -NH-C(=O)-R⁵ steht;
R² für H oder einen Alkyl-Rest steht ausgewählt aus der Gruppe bestehend aus Methyl, Ethyl, n-Propyl, Isopropyl, n-Butyl, 2-Butyl, Isobutyl, tert- Butyl, n-Pentyl, sec-Pentyl, neo-Pentyl, (3,3)-Dimethyl-butyl, 4-Methyl-2- pentyl und n-Hexyl, der jeweils unsubstituiert sein kann; oder
für einen Rest steht ausgewählt aus der Gruppe bestehend aus Phenyl und Naphthyl, der jeweils über eine -CH₂-, -CH(CH₃)-, -CH₂-CH₂-, - CH(CH₃)-CH₂-, -CH₂-CH(CH₃)- oder -CH₂-CH₂-CH₂-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n- Butyl, Isobutyl, 2-Butyl, tert-Butyl, n-Pentyl, neo-Pentyl, -O-CH₃, -O- C₂H₅, -O-C₃H₇, -O-C(CH₃)₃, -CF₃, -CHF₂ und -CH₂F substituiert sein kann; oder R¹ und R² zusammen mit dem sie verbindenden Stickstoffatom einen Rest ausgewählt aus der Gruppe bestehend aus 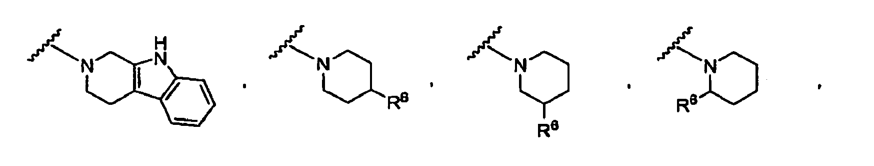 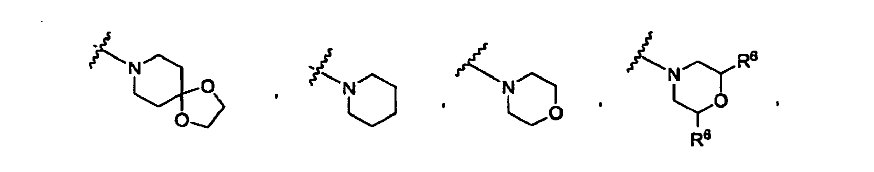 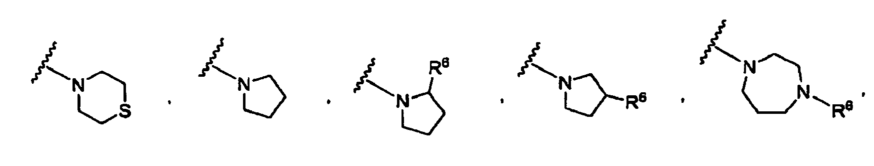 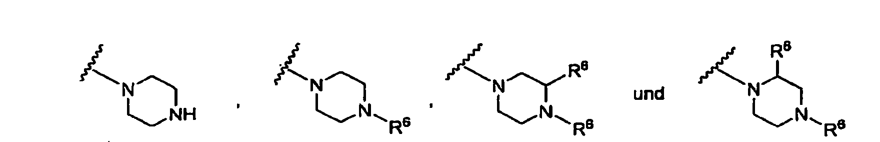 bilden;
R³ für einen Alkyl-Rest steht ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert ist; oder
für einen Phenyl-Rest steht, der jeweils über eine -CH₂-, -CH(CH₃)-, - CH₂-CH₂-, -CH(CH₃)-CH₂-, -CH₂-CH(CH₃)- oder -CH₂-CH₂-CH₂-Gruppe gebunden ist und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -CH₂-O-CH₃, - CH₂-O-C₂H₅, -NH₂, -C(=O)-OH, -S-CH₃, -S-C₂H₅, -S(=O)-CH₃, -S(=O)₂- CH₃, -S(=O)-C₂H₅, -S(=O)₂-C₂H₅, -O-CH₃, -O-C₂H₅, -O-C₃H₇, -O- C(CH₃)₃, -CF₃, -CHF₂, -CH₂F, -O-CF₃, -O-CHF₂ und -O-CH₂F substituiert sein kann;
R⁴ für Phenyl steht, das jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -OH, -SH, -NH₂, -C(=O)-OH, Methyl, Ethyl, n-Propyl, Isopropyl, n-Butyl, Isobutyl, 2-Butyl, tert-Butyl, n-Pentyl, -S-CH₃, -S-C₂H₅, -O-CH₃, -O-C₂H₅, -O-C₃H₇, -O-C(CH₃)₃, - CF₃, -CHF₂, -CH₂F, -O-CF₃, -O-CHF₂, -O-CH₂F, -C(=O)-CF₃, -S-CF₃, - S-CHF₂, -S-CH₂F, -N(CH₃)₂ und -NH-CH₃ substituiert sein kann; oder
für einen Rest steht ausgewählt aus der Gruppe bestehend aus Thienyl, Furyl, Pyrrolyl, Pyrazolyl, Pyrazinyl, Pyranyl, Triazolyl, Pyridinyl, Imidazolyl, Thiadiazolyl, Oxadiazolyl und Pyridazinyl, der jeweils unsubstituiert oder mit 1, 2, 3, 4 oder 5 Substituenten unabhängig voneinander ausgewählt aus der Gruppe bestehend aus F, Cl, Br, I, - CN, -NO₂, -OH, -CH₂-CN, -SH, -NH₂, -C(=O)-OH, Methyl, Ethyl, n- Propyl, Isopropyl, n-Butyl, Isobutyl, 2-Butyl, tert-Butyl, n-Pentyl, neo- Pentyl, -O-CH₃, -O-C₂H₅, -O-C₃H₇, -O-C(CH₃)₃, -CF₃. -CHF₂, -CH₂F, -O- CF₃, -O-CHF₂, -O-CH₂F, -C(=O)-CF₃, -S-CF₃, -S-CHF₂, -S-CH₂F, - N(CH₃)₂ und -NH-CH₃ substituiert sein kann;
R⁵ für einen Rest ausgewählt aus der Gruppe bestehend aus Phenyl und Naphthyl steht, der jeweils unsubstituiert oder mit 1, 2, 3, 4 oder 5 Substituenten unabhängig voneinander ausgewählt aus der Gruppe bestehend aus F, Cl, Br, I, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -NH₂, -O-CH₃, -O-C₂H₅, -O-C₃H₇, -O-C(CH₃)₃, -CF₃, -CHF₂, - CH₂F, -O-CF₃, -O-CHF₂, -O-CH₂F, -C(=O)-CF₃, -S-CF₃, -S-CHF₂, -S- CH₂F, -N(CH₃)₂, -N(C₂H₅)₂, -NH-CH₃ und -NH-C₂H₅ substituiert sein kann;
R⁶ für -OH; F; Cl; Br; I; -SH; -NO₂; -NH₂; -NH-C(=O)-O-R⁷; -C(=O)-O-R⁸; -C(=O)-R⁹ steht;
für einen Alkyl-Rest steht ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert oder mit 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, - N(C₂H₅)₂, -N(CH₃)(C₂H₅), -C(=O)-Pyrrolidinyl, -C(=O)-N(CH₃)-Phenyl und -C(=O)-NH-CH(CH₃)₂, -C(=O)-OH, -C(=O)-O-CH₃, -C(=O)-O-C₂H₅ und -C(=O)-O-C(CH₃)₃ substituiert sein kann;
für einen Rest ausgewählt aus der Gruppe bestehend aus Cyclopropyl, Cyclobutyl, Cyclopentyl, Cyclohexyl, Cycloheptyl, Cyclohexenyl, Cyclopentenyl, Pyrrolidinyl, Tetrahydrothiophenyl, Tetrahydrofuranyl, Piperidinyl, Morpholinyl, Piperazinyl, Azepanyl und Diazepany steht, der jeweils unsubstituiert oder mit 1, 2, 3, 4 oder 5 Substituenten unabhängig voneinander ausgewählt aus der Gruppe bestehend aus Methyl, Ethyl, n-Propyl, Isopropyl, n-Butyl, 2-Butyl, Isobutyl, tert-Butyl, - OH, -O-CH₃, -O-C₂H₅, -O-C₃H₇, -O-CF₃, -C(=O)-CH₃, -C(=O)-C₂H₅, - C(=O)-C(CH₃)₃, -C(=O)-NH-CH₃ und -C(=O)-NH-C₂H₅ substituiert sein kann und/oder jeweils über eine -CH₂-, -CH(CH₃)-, -CH₂-CH₂-, - CH(CH₃)-CH₂-, -CH₂-CH(CH₃)- oder -CH₂-CH₂-CH₂-Gruppe gebunden sein kann; oder
für einen Rest ausgewählt aus der Gruppe bestehend aus Phenyl, Naphthyl, Indolizinyl, Benzimidazolyl, Tetrazolyl, Triazinyl, Isoxazolyl, Phthalazinyl, Thienyl, Furyl, Pyrrolyl, Pyrazolyl, Pyrazinyl, Pyranyl, Triazolyl, Pyridinyl, Imidazolyl, Indolyl, Isoindolyl, Thiazoiyl, Thiadiazolyl, Oxazolyl, Oxadiazolyl, Isoxazolyl, Pyridazinyl, Thieno[2,3-d]pyrimidinyl und Isochinolinyl steht, der jeweils über eine CH₂-, -CH(CH₃)-, -CH₂- CH₂-, -CH(CH₃)-CH₂-, -CH₂-CH(CH₃)- oder -CH₂-CH₂-CH₂-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, -OH, -SH, -O-Phenyl, Methyl, Ethyl, n-Propyl, Isopropyl, n-Butyl, Isobutyl, 2-Butyl, tert-Butyl, n-Pentyl, neo-Pentyl, Ethenyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, - C(=O)-H, -C(=O)-CH₃ und -C(=O)-C₂H₅ substituiert sein kann;
und R⁷, R⁸ und R⁹, unabhängig voneinander, jeweils steht
für einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert oder mit 1, 2, 3, 4 oder 5 Substituenten unabhängig voneinander ausgewählt aus der Gruppe bestehend aus F, Cl, Br, I, -NO₂, -CN, -OH, -SH, -NH₂, -N(CH₃)₂, -N(C₂H₅)₂ und - N(CH₃)(C₂H₅) substituiert sein kann;
für einen Rest ausgewählt aus der Gruppe bestehend aus Indolinyl, Indanyl, (1,2,3,4)-Tetrahydronaphthyl, (2,3)-Dihydro-benzo[1.4]dioxinyl und Benzo[1.3]dioxolyl, der jeweils unsubstituiert ist; oder
für einen Rest steht ausgewählt aus der Gruppe bestehend aus Phenyl, Naphthyl, Isoxazolyl, Thienyl, Furyl, Pyrrolyl, Pyrazolyl, Pyrazinyl, Pyranyl, Triazolyl, Pyridinyl, Imidazolyl, Indolyl, Isoindolyl, Thiazolyl, Thiadiazolyl, Oxazolyl, Oxadiazolyl, Isoxazolyl und Pyridazinyl, der jeweils über eine -CH₂-, -CH(CH₃)-, -CH₂-CH₂-, -CH(CH₃)-CH₂-, -CH₂-CH(CH₃)- oder -CH₂-CH₂-CH₂-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, -NO₂, -O-Phenyl, Methyl, Ethyl, n-Propyl, Isopropyl, n-Butyl, Isobutyl, 2-Butyl, tert-Butyl, n-Pentyl, neo-Pentyl, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂ und -O-CH₂F substituiert sein kann.

4. Verbindungen gemäß einem oder mehreren der Ansprüche 1 bis 3, **dadurch gekennzeichnet, dass**
R¹ für einen Alkyl-Rest steht ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert oder mit 1, 2 oder 3 Substituenten unabhängig voneinander ausgewählt aus der Gruppe bestehend aus -CN, -OH, -N(CH₃)₂, -N(C₂H₅)₂ -C(=O)-O-CH₃, -C(=O)- O-C₂H₅, -C(=O)-O-C(CH₃)₃, -C(=O)-O-CH₂-Phenyl, -C(=O)-NH- Naphthyl, -N(CH₃)-Phenyl, -N(C₂H₅)-(m-Toluyl) und -N(C₂H₅)-(p-Toluyl) substituiert sein kann;
für einen Heteroalkyl-Rest steht ausgewählt aus der Gruppe bestehend aus -CH₂-O-CH₃, -CH₂-O-C₂H₅, -CH₂-CH₂-O-CH₃, -CH₂-CH₂-O-C₂H₅, - CH₂-CH₂-CH₂-O-CH₃ und -CH₂-CH₂-CH₂-O-C₂H₅;
für einen Rest steht ausgewählt aus der Gruppe bestehend aus Cyclopropyl, Cyclobutyl, Cyclopentyl, Cyclohexyl, Cycloheptyl, Pyrrolidinyl, Tetrahydrofuranyl, Piperidinyl, Morpholinyl, Piperazinyl, Azepanyl, Dihydrofuran-2(3H)-onyl, Indanyl und (1,2,3,4)- Tetrahydronaphthyl, der jeweils unsubstituiert oder mit 1, 2, 3, 4 oder 5 Substituenten unabhängig voneinander ausgewählt aus der Gruppe bestehend aus, Methyl, Ethyl, n-Propyl, Isopropyl, n-Butyl, 2-Butyl, Isobutyl, tert-Butyl, -CH₂-Naphthyl, -O-Phenyl, -O-CH₂-Phenyl, Benzyl und Phenyl substituiert sein kann und/oder jeweils über eine -CH₂-, - CH(CH₃)-, -CH₂-CH₂-, -CH(CH₃)-CH₂-, -CH₂-CH(CH₃)- oder -CH₂-CH₂- CH₂-Gruppe gebunden sein kann;
für Phenyl steht, das jeweils über eine -CH₂-CH₂-O-, -CH₂-O-, -CH₂- CH₂-CH₂-O-, -CH[C(=O)-O-CH₃]-CH₂-, -CH[C(=O)-O-C₂H₅]-CH₂-, -CH₂-, -CH(CH₃)-, -CH₂-CH₂-, -CH(CH₃)-CH₂-, -CH₂-CH(CH₃)- oder -CH₂-CH₂- CH₂-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CH₂-CN, Methyl, Ethyl, n- Propyl, Isopropyl, n-Butyl, Isobutyl, 2-Butyl, tert-Butyl, n-Pentyl, neo- Pentyl, -O-Phenyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -N(CH₃)₂, -N(C₂H₅)₂, -S(=O)-NH₂ und [1.2.3]-Thiadiazolyl substituiert sein kann;
einen Rest steht ausgewählt aus der Gruppe bestehend aus Thienyl, Furyl, Pyrrolyl, Pyrazolyl, Pyridinyl, Imidazolyl, Indolyl und Isoindolyl, der jeweils über -CH₂-, -CH(CH₃)-, -CH₂-CH₂-, -CH(CH₃)-CH₂-, -CH₂- CH(CH₃)- oder -CH₂-CH₂-CH₂-Gruppe gebunden sein kann und/oder jeweils unsubstituiert oder mit 1, 2 oder 3 Substituenten unabhängig voneinander ausgewählt aus der Gruppe bestehend aus -NO₂, -OH, Methyl, Ethyl, n-Propyl, Isopropyl, n-Butyl, Isobutyl, 2-Butyl, tert-Butyl, n-Pentyl und neo-Pentyl substituiert sein kann oder für -NH-C(=O)-R⁵ steht;
R² für H oder einen Alkyl-Rest ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert sein kann; oder
für einen Rest ausgewählt aus der Gruppe bestehend aus Phenyl und Naphthyl steht, der jeweils über eine -CH₂-, -CH(CH₃)-, -CH₂-CH₂-, - CH(CH₃)-CH₂-, -CH₂-CH(CH₃)- oder -CH₂-CH₂-CH₂-Gruppe gebunden sein kann und/oder jeweils unsubstituiert oder mit 1, 2 oder 3 Substituenten unabhängig voneinander ausgewählt aus der Gruppe bestehend aus -O-CH₃, -O-C₂H₅, -O-C₃H₇ und -O-C(CH₃)₃ substituiert sein kann; oder
R¹ und R² zusammen mit dem sie verbindenden Stickstoffatom einen Rest ausgewählt aus der Gruppe bestehend aus 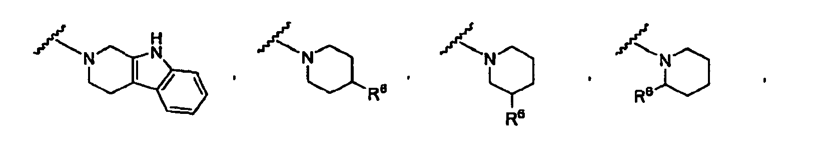 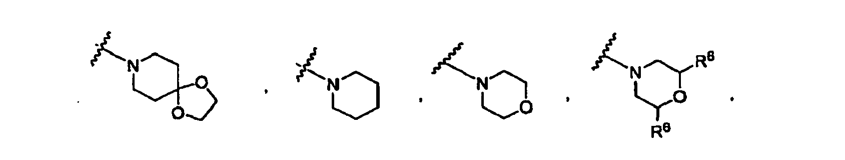 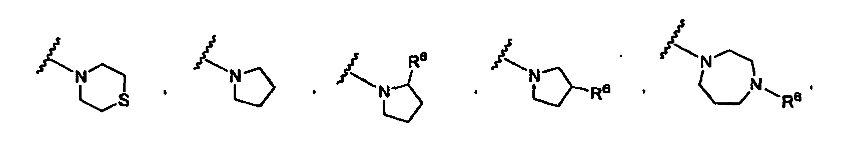 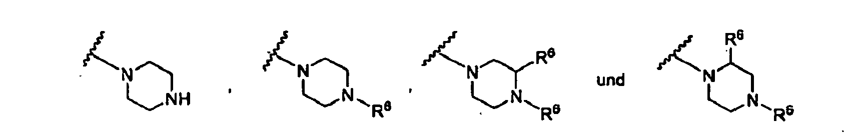 bilden;
R³ für einen Alkyl-Rest steht, ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert sein kann; oder
für einen Phenyl-Rest steht, der jeweils über eine -CH₂-, -CH(CH₃)-, - CH₂-CH₂-, -CH(CH₃)-CH₂-, -CH₂-CH(CH₃)- oder -CH₂-CH₂-CH₂-Gruppe gebunden ist und/oder jeweils unsubstituiert oder mit 1, 2, 3, 4 oder 5 Substituenten unabhängig voneinander ausgewählt aus der Gruppe bestehend aus F, Cl, Br, I, -CN, -CH₂-CN, -NO₂, Methyl, Ethyl, n-Propyl, Isopropyl, n-Butyl, Isobutyl, 2-Butyl, tert-Butyl, n-Pentyl, neo-Pentyl, -O- CH₃, -O-C₂H₅, -O-C₃H₇, -O-C(CH₃)₃, -CF₃, -CHF₂, -CH₂F, -O-CF₃, -O- CHF₂ und -O-CH₂F substituiert sein kann;
R⁴ für Phenyl steht, das jeweils unsubstituiert oder mit 1, 2, 3, 4 oder 5 Substituenten unabhängig voneinander ausgewählt aus der Gruppe bestehend aus F, Cl, Br, I, Methyl, Ethyl, n-Propyl, Isopropyl, n-Butyl, Isobutyl, 2-Butyl, tert-Butyl, n-Pentyl, -O-CH₃, -O-C₂H₅, -O-C₃H₇, -O- C(CH₃)₃, -O-CF₃, -O-CHF₂ und -O-CH₂F substituiert sein kann; oder
für einen Rest ausgewählt aus der Gruppe bestehend aus Thienyl, Furyl und Pyrrolyl steht, der jeweils unsubstituiert oder mit 1, 2, 3, 4 oder 5 Substituenten unabhängig voneinander ausgewählt aus der Gruppe bestehend aus F, Cl, Br, I, Methyl, Ethyl, n-Propyl, Isopropyl, n-Butyl, Isobutyl, 2-Butyl, -O-CH₃, -O-C₂H₅, -O-C₃H₇ und -O-C(CH₃)₃ substituiert sein kann;
R⁵ für einen Rest ausgewählt aus der Gruppe bestehend aus Phenyl und Naphthyl steht, der jeweils unsubstituiert oder mit 1, 2, 3, 4 oder 5 Substituenten unabhängig voneinander ausgewählt aus der Gruppe bestehend aus F, Cl, Br, I, Methyl, Ethyl, n-Propyl, Isopropyl, n-Butyl, Isobutyl, 2-Butyl, tert-Butyl, -NH₂, -O-CH₃, -O-C₂H₅, -O-C₃H₇, -O- C(CH₃)₃, -N(CH₃)₂, -N(C₂H₅)₂, -NH-CH₃ und -NH-C₂H₅ substituiert sein kann;
R⁶ für -OH; -NH-C(=O)-O-R⁷; -C(=O)-O-R⁸; -C(=O)-R⁹ steht,
für einen Alkyl-Rest steht ausgewählt aus der Gruppe bestehend aus Methyl, Ethyl, n-Propyl, Isopropyl, n-Butyl, 2-Butyl, Isobutyl, tert-Butyl, n-Pentyl, sec-Pentyl, neo-Pentyl, (3,3)-Dimethyl-butyl, 4-Methyl-2-pentyl und n-Hexyl, der jeweils unsubstituiert oder mit 1, 2 oder 3 Substituenten unabhängig voneinander ausgewählt aus der Gruppe bestehend aus -OH, -N(CH₃)₂, -N(C₂H₅)₂, -C(=O)-Pyrrolidinyl, -C(=O)- N(CH₃)-Phenyl und -C(=O)-NH-CH(CH₃)₂ substituiert sein kann;
für einen Rest steht ausgewählt aus der Gruppe bestehend aus Cyclopropyl, Cyclobutyl, Cyclopentyl, Cyclohexyl, Cycloheptyl, Pyrrolidinyl, Piperidinyl und Azepanyl, der jeweils unsubstituiert ist und/oder jeweils über eine -CH₂-, -CH(CH₃)-, -CH₂-CH₂-, -CH(CH₃)-CH₂-, -CH₂-CH(CH₃)- oder -CH₂-CH₂-CH₂-Gruppe gebunden sein kann; oder
für einen Rest ausgewählt aus der Gruppe bestehend aus Phenyl, Naphthyl, Thienyl, Furyl, Pyrazinyl, Pyridinyl, Pyridazinyl und Thieno[2,3-d]pyrimidinyl steht, der jeweils über eine -CH₂-, -CH(CH₃)-, - CH₂-CH₂-, -CH(CH₃)-CH₂-, -CH₂-CH(CH₃)- oder -CH₂-CH₂-CH₂-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CN, Methyl, tert-Butyl, -O-CH₃, -O-C₂H₅, -CF₃, -C(=O)-CH₃ und -C(=O)-C₂H₅ substituiert sein kann;
und R⁷, R⁸ und R⁹, unabhängig voneinander, jeweils steht
für einen Alkyl-Rest ausgewählt aus der Gruppe bestehend aus Methyl, Ethyl, n-Propyl, Isopropyl, n-Butyl, 2-Butyl, Isobutyl, tert-Butyl und n-Pentyl, der jeweils unsubstituiert sein kann;
für einen Rest ausgewählt aus der Gruppe bestehend aus (2,3)-Dihydro-benzo[1.4]dioxinyl und Benzo[1.3]dioxolyl, der jeweils unsubstituiert sein kann; oder
für einen Rest ausgewählt aus der Gruppe bestehend aus Phenyl, Thienyl, Furyl und Pyrazinyl, der jeweils über eine -CH₂-, -CH(CH₃)-, - CH₂-CH₂-, -CH(CH₃)-CH₂-, -CH₂-CH(CH₃)- oder -CH₂-CH₂-CH₂-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, Methyl, Ethyl, n-Propyl, Isopropyl, n-Butyl, Isobutyl, 2-Butyl, tert-Butyl, n-Pentyl, neo-Pentyl, -O-CH₃, -0-C₂H₅, -O-C₃H₇ und -O-C(CH₃)₃ substituiert sein kann.

5. Verbindungen gemäß einem oder mehreren der Ansprüche 1 bis 4, **dadurch gekennzeichnet, dass**
R¹ für einen Rest steht ausgewählt aus der Gruppe bestehend aus -CH₂- CH₂-N(C₂H₅)-(m-Toluyl), -CH₂-CH₂-N(C₂H₅)-(p-Toluyl), -CH₂-CH₂-CH₂- N(CH₃)-Phenyl, -CH₂-CH₂-CH₂-N(CH₃)₂, -CH₂-CH₂-CH₂-N(C₂H₅)₂, - CH(CH₃)-CH₂-CH₂-CH(CH₃)₂, -CH₂-CH₂-CN, -CH₂-CH₂-OH, -CH₂-CH₂- N(CH₃)₂, -CH₂-CH₂-N(C₂H₅)₂, -CH₂-CH₂-C(CH₃)₃, n-Pentyl, n-Butyl, Methyl, Ethyl, n-Propyl, -CH(CH₃)-C(=O)-O-CH₂-Phenyl, - CH[CH(CH₃)₂]-C(=O)-O-C(CH₃)₃, -CH₂-C(=O)-O-CH₂-Phenyl, -CH₂- CH₂-C(=O)-O-C(CH₃)₃, -CH₂-CH₂-C(=O)-O-C₂H₅, -CH₂-C(=O)-NH- Naphthyl und -CH₂-CH₂-CH₂-O-CH₃;
für einen Rest steht ausgewählt aus der Gruppe bestehend aus Cyclopropyl, Cyclobutyl, Cyclopentyl, Cyclohexyl, Cycloheptyl, Pyrrolidinyl, Tetrahydrofuranyl, Piperidinyl, Morpholinyl, Piperazinyl, Azepanyl, Dihydrofuran-2(3H)-onyl, Indanyl und (1,2,3,4)- Tetrahydronaphthyl, der jeweils unsubstituiert oder mit 1, 2, 3, 4 oder 5 Substituenten unabhängig voneinander ausgewählt aus der Gruppe bestehend aus, Methyl, Ethyl, -CH₂-Naphthyl, -O-Phenyl, -O-CH₂- Phenyl und Benzyl substituiert sein kann und/oder jeweils über eine - Chor, -CH₂-CH₂- oder -CH₂-CH₂-CH₂-Gruppe gebunden sein kann;
für Phenyl steht, das jeweils über eine -CH₂-CH₂-O-, -CH[C(=O)-O- CH₃]-CH₂-, -CH[C(=O)-O-C₂H₅]-CH₂-, -CH₂-, -CH(CH₃)-, -CH₂-CH₂-, - CH(CH₃)-CH₂-, -CH₂-CH(CH₃)- oder -CH₂-CH₂-CH₂-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, I, -CH₂-CN, Methyl, Ethyl, n-Propyl, Isopropyl, n-Butyl, Isobutyl, 2-Butyl, tert-Butyl, -O-Phenyl, -O-CH₃, -O-C₂H₅, -CF₃, - O-CF₃, -N(CH₃)₂, -N(C₂H₅)₂, -S(=O)-NH₂ und [1.2.3]-Thiadiazolyl substituiert sein kann;
für einen Rest steht ausgewählt aus der Gruppe bestehend aus Thienyl, Furyl, Pyridinyl, Imidazolyl, Indolyl und Isoindolyl, der jeweils über -CH₂- , -CH₂-CH₂- oder -CH₂-CH₂-CH₂-Gruppe gebunden sein kann und/oder jeweils unsubstituiert oder mit 1, 2 oder 3 Substituenten unabhängig voneinander ausgewählt aus der Gruppe bestehend aus -NO₂, -OH, Methyl, Ethyl und n-Propyl substituiert sein kann
oder für -NH-C(=O)-R⁵ steht;
R² für H oder einen Alkyl-Rest steht ausgewählt aus der Gruppe bestehend aus Methyl, Ethyl und n-Propyl, der jeweils unsubstituiert ist; oder
für einen Rest ausgewählt aus der Gruppe bestehend aus Phenyl und Naphthyl steht, der jeweils über eine -CH₂-Gruppe gebunden sein kann und/oder jeweils unsubstituiert oder mit 1, 2 oder 3 Substituenten unabhängig voneinander ausgewählt aus der Gruppe bestehend aus - O-CH₃, -O-C₂H₅, -O-C₃H₇ und -O-C(CH₃)₃ substituiert sein kann; oder
R¹ und R² zusammen mit dem sie verbindenden Stickstoffatom einen Rest ausgewählt aus der Gruppe bestehend aus 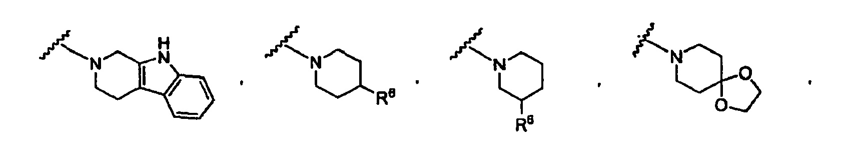 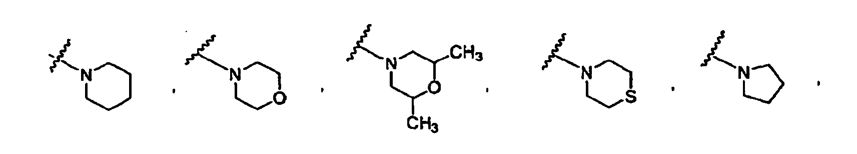 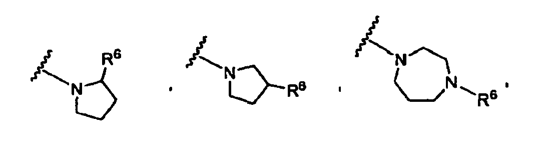 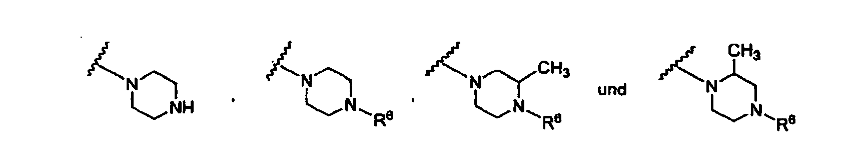 bilden;
R³ für einen Alkyl-Rest steht ausgewählt aus der Gruppe bestehend aus Methyl, Ethyl, n-Propyl, Isopropyl, n-Butyl, 2-Butyl, Isobutyl und tert- Butyl, der jeweils unsubstituiert sein kann; oder
für einen Phenyl-Rest steht, der jeweils über eine -CH₂-Gruppe gebunden ist und/oder jeweils unsubstituiert oder mit 1, 2, 3, 4 oder 5 Substituenten unabhängig voneinander ausgewählt aus der Gruppe bestehend aus F, Cl, Br, Methyl, Ethyl, n-Propyl, -O-CH₃, -O-C₂H₅, und - CF₃ substituiert sein kann;
R⁴ für Phenyl steht, das jeweils unsubstituiert oder mit 1, 2, 3, 4 oder 5 Substituenten unabhängig voneinander ausgewählt aus der Gruppe bestehend aus F, Cl, Br, Methyl, Ethyl, n-Propyl, -O-CH₃ und -O-C₂H₅, substituiert sein kann;
oder für einen Rest ausgewählt aus der Gruppe bestehend aus Thienyl, Furyl und Pyrrolyl steht, der jeweils unsubstituiert sein kann;
R⁵ für einen Rest ausgewählt aus der Gruppe bestehend aus Phenyl und Naphthyl steht, der jeweils unsubstituiert oder mit 1, 2, 3, 4 oder 5 Substituenten unabhängig voneinander ausgewählt aus der Gruppe bestehend aus F, Cl, Br, Methyl, Ethyl, -O-CH₃, -O-C₂H₅, -N(CH₃)₂, - N(C₂H₅)₂, -NH-CH₃ und -NH-C₂H₅ substituiert sein kann;
R⁶ für -OH; -NH-C(=O)-O-R⁷; -C(=O)-O-R⁸; -C(=O)-R⁹ steht,
für einen Rest steht ausgewählt aus der Gruppe bestehend aus Methyl, Ethyl, n-Propyl, Isopropyl, n-Butyl, -CH₃-CH(CH₃)₂, -CH₂-CH₂-OH, -CH₂- CH₂-N(CH₃)₂, -CH₂-CH₂-N(C₂H₅), -CH₂-C(=O)-Pyrrolidinyl, -CH₂-C(=O)- NH-CH(CH₃)₂ und -CH₂-C(=O)-N(CH₃)-Phenyl;
für einen Rest steht ausgewählt aus der Gruppe bestehend aus Cyclopropyl, Cyclobutyl, Cyclopentyl, Cyclohexyl, Cycloheptyl, Pyrrolidinyl, Piperidinyl und Azepanyl, der jeweils unsubstituiert ist und/oder jeweils über eine -CH₂-Gruppe gebunden sein kann; oder
für einen Rest steht ausgewählt aus der Gruppe bestehend aus Phenyl, Naphthyl, Thienyl, Pyrazinyl, Pyridinyl und Thieno[2,3-d]pyrimidinyl steht, der jeweils über eine -CH₂-, -CH₂-CH₂- oder -CH₂-CH₂-CH₂- Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, -CN, Methyl, tert-Butyl, -O-CH₃, - O-C₂H₅, -CF₃, -C(=O)-CH₃ und -C(=O)-C₂H₅ substituiert sein kann;
R⁷ für einen Alkyl-Rest ausgewählt aus der Gruppe bestehend aus Methyl, Ethyl, n-Propyl, Isopropyl, n-Butyl, 2-Butyl, Isobutyl, tert-Butyl und n- Pentyl steht, der jeweils unsubstituiert ist;
R⁸ für einen Alkyl-Rest steht ausgewählt aus der Gruppe bestehend aus Methyl, Ethyl, n-Propyl, Isopropyl, n-Butyl, 2-Butyl, Isobutyl, tert-Butyl und n-Pentyl, der jeweils unsubstituiert ist; oder
für einen Phenyl- oder Benzyl-Rest steht; und
R⁹ für einen Rest steht ausgewählt aus der Gruppe bestehend aus (2,3)-Dihydro-benzo[1.4]dioxinyl und Benzo[1.3]dioxolyl, der jeweils unsubstituiert ist; oder
für einen Rest steht ausgewählt aus der Gruppe bestehend aus Phenyl, Thienyl, Furyl und Pyrazinyl steht, der jeweils über eine -CH₂-, -CH₂- CH₂- oder -CH₂-CH₂-CH₂-Gruppe gebunden sein kann und/oder jeweils unsubstituiert oder mit 1, 2, 3, 4 oder 5 Substituenten unabhängig voneinander ausgewählt aus der Gruppe bestehend aus F, Cl, Br, Methyl, Ethyl, -O-CH₃ und -O-C₂H₅ substituiert sein kann.

6. Verbindungen gemäß einem der Ansprüche 1 bis 5 ausgewählt aus der Gruppe bestehend aus
| | |
|---|---|
| [1] | 1-Benzyl-4-methyl-3-p-tolyl-1H-pyrrol-2-carbonsäure 4-[1,2,3]thiadiazol-4-yl-benzylamid, |
| [2] | 3-Furan-2-yl-1,4-dimethyl-1H-pyrrol-2-carbonsäure 4-sulfamoyl-benzylamid, |
| [3] | 1-(4-Fluor-benzyl)-3-furan-2-yl-4-methyl-1H-pyrrol-2-carbonsäure 2,4-dimethoxy-benzylamid, |
| [4] | 1-Butyl-4-methyl-3-p-tolyl-1H-pyrrol-2-carbonsäure (2-pyridin-2-yl-ethyl)-amid, |
| [5] | 1-(4-Fluor-benzyl)-3-(4-methoxy-phenyl)-4-methyl-1H-pyrrol-2-carbonsäure [2-(5-hydroxy-1H-indol-3-yl)-ethyl]-amid, |
| [6] | (1,4-Dimethyl-3-p-tolyl-1H-pyrrol-2-yl)-(4-pyrrolidin-1-yl-piperidin-1-yl)-methanon, |
| [7] | 1-Benzyl-3-furan-2-yl-4-methyl-1H-pyrrol-2-carbonsäure 4-brom-2-fluor-benzylamid, |
| [8] | [1-Benzyl-3-(4-chlor-phenyl)-4-methyl-1H-pyrrol-2-yl]-[4-(2-chlor-phenyl)-piperazin-1-yl]-methanon, |
| [9] | 1,4-Dimethyl-3-phenyl-1H-pyrrol-2-carbonsäure (3-dimethylamino-propyl)-amid, |
| [10] | 1,4-Dimethyl-3-p-tolyl-1H-pyrrol-2-carbonsäure (2-pyrrolidin-1-yl-ethyl)-amid, |
| [11] | 1-(4-Fluor-benzyl)-3-furan-2-yl-4-methyl-1H-pyrrol-2-carbonsäure 4-dimethylamino-benzylamid, |
| [12] | 1-[4-(1-Benzyl-4-methyl-3-phenyl-1H-pyrrol-2-carbonyl)-piperazin-1-yl]-2-phenyl-ethanon, |
| [13] | 1-Benzyl-3-furan-2-yl-4-methyl-1H-pyrrol-2-carbonsäure 2,5-difluor-benzylamid, |
| [14] | 1-(2,6-Dichlor-benzyl)-4-methyl-3-p-tolyl-1H-pyrrol-2-carbonsäure 3,5-difluor-benzylamid, |
| [15] | 1-(2-Brom-benzyl)-3-(4-chlor-phenyl)-4-methyl-1H-pyrrol-2-carbonsäure (2-methyl-cyclohexyl)-amid, |
| [16] | 1,4-Dimethyl-3-p-tolyl-1H-pyrrol-2-carbonsäure (3-morpholin-4-yl-propyl)-amid, |
| [17] | 1-Butyl-4-methyl-3-p-tolyl-1H-pyrrol-2-carbonsäure (1,3-dimethyl-butyl)-amid, |
| [18] | [4-(2,4-Dimethyl-phenyl)-piperazin-1-yl]-[4-methyl-1-(4-methyl-benzyl)-3-p-tolyl-1H-pyrrol-2-yl]-methanon, |
| [19] | 1-(2,6-Dichlor-benzyl)-4-methyl-3-p-tolyl-1H-pyrrol-2-carbonsäure methyl-(2-pyridin-2-yl-ethyl)-amid, |
| [20] | 1,4-Dimethyl-3-phenyl-1H-pyrrol-2-carbonsäure (2-cyano-ethyl)-methyl-amid, |
| [21] | 1-(2,6-Dichlor-benzyl)-4-methyl-3-p-tolyl-1H-pyrrol-2-carbonsäure (3-imidazol-1-yl-propyl)-amid, |
| [22] | 1-(4-Fluor-benzyl)-3-(4-methoxy-phenyl)-4-methyl-1H-pyrrol-2-carbonsäure (1,3-dimethyl-butyl)-amid, |
| [23] | 3-(4-Methoxy-phenyl)-1,4-dimethyl-1H-pyrrol-2-carbonsäure 2-ethoxy-benzylamid, |
| [24] | (4-Cycloheptyl-piperazin-1-yl)-(1,4-dimethyl-3-p-tolyl-1H-pyrrol-2-yl)-methanon, |
| [25] | 1-(4-Fluor-benzyl)-3-(4-methoxy-phenyl)-4-methyl-1H-pyrrol-2-carbonsäure (2-dimethylamino-ethyl)-amid, |
| [26] | 1-Benzyl-4-methyl-3-phenyl-1H-pyrrol-2-carbonsäure (pyridin-2-ylmethyl)-amid, |
| [27] | 3-(4-Chlor-phenyl)-1-ethyl-4-methyl-1H-pyrrol-2-carbonsäure [2-(4-chlor-phenyl)-propyl]-amid, |
| [28] | 3-(4-Chlor-phenyl)-1-(2-fluor-benzyl)-4-methyl-1H-pyrrol-2-carbonsäure 3,5-difluor-benzylamid, |
| [29] | 2-{[1-(4-Fluor-benzyl)-3-(4-methoxy-phenyl)-4-methyl-1H-pyrrol-2-carbonyl]-amino}-propionsäure benzyl ester, |
| [30] | (1-Benzyl-4-methyl-3-p-tolyl-1H-pyrrol-2-yl)-[4-(2-dimethylamino-ethyl)-piperazin-1-yl]-methanon, |
| [31] | 3-Furan-2-yl-1-(4-methoxy-benzyl)-4-methyl-1H-pyrrol-2-carbonsäure (3,3-dimethyl-butyl)-amid, |
| [32] | 3-(4-Chlor-phenyl)-1-isobutyl-4-methyl-1H-pyrrol-2-carbonsäure 4-dimethylamino-benzylamid, |
| [33] | 3-(4-Chlor-phenyl)-1-ethyl-4-methyl-1H-pyrrol-2-carbonsäure (2-pyridin-2-yl-ethyl)-amid, |
| [34] | 3-(4-Chlor-phenyl)-1-ethyl-4-methyl-1H-pyrrol-2-carbonsäure 2,3-dimethoxy-benzylamid, |
| [35] | 1-(4-Fluor-benzyl)-3-(4-methoxy-phenyl)-4-methyl-1H-pyrrol-2-carbonsäure (2-thiophen-2-yl-ethyl)-amid, |
| [36] | 2-{[3-(4-Chlor-phenyl)-1-ethyl-4-methyl-1H-pyrrol-2-carbonyl]-amino}-3-methyl-buttersäure tert-butyl ester, |
| [37] | 3-Furan-2-yl-1-(4-methoxy-benzyl)-4-methyl-1H-pyrrol-2-carbonsäure (1,2,3,4-tetrahydro-naphthalin-1-yl)-amid, |
| [38] | 3-Furan-2-yl-4-methyl-1-(4-trifluormethyl-benzyl)-1H-pyrrol-2-carbonsäure (2-pyrrolidin-1-yl-ethyl)-amid, |
| [39] | {[1-(4-Methoxy-benzyl)-4-methyl-3-p-tolyl-1H-pyrrol-2-carbonyl]-methyl-amino}-essigsäure benzyl ester, |
| [40] | 3-Furan-2-yl-1-(4-methoxy-benzyl)-4-methyl-1H-pyrrol-2-carbonsäure [2-(1-methyl-pyrrolidin-2-yl)-ethyl]-amid, |
| [41] | [4-(5-Brom-2-ethoxy-benzyl)-piperazin-1-yl]-(1,4-dimethyl-3-phenyl-1H-pyrrol-2-yl)-methanon, |
| [42] | (1-Benzyl-3-furan-2-yl-4-methyl-1H-pyrrol-2-yl)-[4-(3-fluor-4-methoxy-benzyl)-piperazin-1-yl]-methanon, |
| [43] | 3-{[3-(4-Chlor-phenyl)-1-ethyl-4-methyl-1H-pyrrol-2-carbonyl]-amino}-propionsäure tert-butyl ester, |
| [44] | 1-Benzyl-4-methyl-3-phenyl-1H-pyrrol-2-carbonsäure 3-methoxy-benzylamid, |
| [45] | (1-Benzyl-3-furan-2-yl-4-methyl-1H-pyrrol-2-yl)-[4-(5-brom-2-ethoxy-benzyl)-piperazin-1-yl]-methanon, |
| [46] | [3-(4-Chlor-phenyl)-1,4-dimethyl-1H-pyrrol-2-yl]-(4-o-tolyl-piperazin-1-yl)-methanon, |
| [47] | 1-(4-Fluor-benzyl)-3-(4-methoxy-phenyl)-4-methyl-1H-pyrrol-2-carbonsäure [2-(4-ethoxy-phenyl)-ethyl]-amid, |
| [48] | [1-(2,6-Dichlor-benzyl)-4-methyl-3-p-tolyl-1H-pyrrol-2-yl]-(4-hydroxy-piperidin-1-yl)-methanon, |
| [49] | (1,4-Dimethyl-3-p-tolyl-1H-pyrrol-2-yl)-[4-(2,4,6-trimethoxy-benzyl)-piperazin-1-yl]-methanon, |
| [50] | 1-(4-Fluor-benzyl)-3-furan-2-yl-4-methyl-1H-pyrrol-2-carbonsäure (2-morpholin-4-yl-ethyl)-amid, |
| [51] | 1-Benzyl-3-(4-chlor-phenyl)-4-methyl-1H-pyrrol-2-carbonsäure [2-(4-fluor-phenyl)-ethyl]-amid, |
| [52] | [4-(2,5-Dimethyl-phenyl)-piperazin-1-yl]-[3-(4-methoxy-phenyl)-1,4-dimethyl-1H-pyrrol-2-yl]-methanon, |
| [53] | 3-(4-Methoxy-phenyl)-1,4-dimethyl-1H-pyrrol-2-carbonsäure (3-imidazol-1-yl-propyl)-amid, |
| [54] | 1-(4-Fluor-benzyl)-3-(4-methoxy-phenyl)-4-methyl-1H-pyrrol-2-carbonsäure [2-(3-trifluormethyl-phenyl)-ethyl]-amid, |
| [55] | [3-(4-Chlor-phenyl)-1-(2-fluor-benzyl)-4-methyl-1H-pyrrol-2-yl]-[4-(5-methyl-pyrazin-2-carbonyl)-piperazin-1-yl]-methanon, |
| [56] | 3-(4-Chlor-phenyl)-1-isobutyl-4-methyl-1H-pyrrol-2-carbonsäure 3-fluor-5-trifluormethyl-benzylamid, |
| [57] | [3-(4-Chlor-phenyl)-1,4-dimethyl-1H-pyrrol-2-yl]-(4-pyridin-4-yl-piperazin-1-yl)-methanon, |
| [58] | 3-(4-Chlor-phenyl)-1-(2-fluor-benzyl)-4-methyl-1H-pyrrol-2-carbonsäure (3-imidazol-1-yl-propyl)-amid, |
| [59] | (1,4-Dimethyl-3-phenyl-1H-pyrrol-2-yl)-[4-(3-fluor-4-methoxy-benzyl)-piperazin-1-yl]-methanon, |
| [60] | 1-Benzyl-3-furan-2-yl-4-methyl-1H-pyrrol-2-carbonsäure [2-(4-brom-phenyl)-ethyl]-amid, |
| [61] | (4-Cycloheptyl-piperazin-1-yl)-(3-furan-2-yl-1,4-dimethyl-1H-pyrrol-2-yl)-methanon, |
| [62] | 1-Benzyl-4-methyl-3-phenyl-1H-pyrrol-2-carbonsäure [2-(4-methoxy-phenoxy)-ethyl]-amid, |
| [63] | 1-(4-Fluor-benzyl)-3-furan-2-yl-4-methyl-1H-pyrrol-2-carbonsäure (3-dimethylamino-propyl)-amid, |
| [64] | [1-(4-Fluor-benzyl)-3-(4-methoxy-phenyl)-4-methyl-1H-pyrrol-2-yl]-(2-pyrrolidin-1-ylmethyl-pyrrolidin-1-yl)-methanon, |
| [65] | 3-(4-Chlor-phenyl)-1-isobutyl-4-methyl-1H-pyrrol-2-carbonsäure (3-diethylamino-propyl)-amid, |
| [66] | [3-(4-Chlor-phenyl)-1,4-dimethyl-1H-pyrrol-2-yl]-[4-(2-fluor-5-methoxy-benzyl)-piperazin-1-yl]-methanon, |
| [67] | 3-(4-Methoxy-phenyl)-1,4-dimethyl-1H-pyrrol-2-carbonsäure pentylamid, |
| [68] | (1-Butyl-4-methyl-3-p-tolyl-1H-pyrrol-2-yl)-[4-(2,5-dimethoxy-benzyl)-piperazin-1-yl]-methanon, |
| [69] | [4-(2-Diethylamino-ethyl)-piperazin-1-yl]-[4-methyl-1-(4-methyl-benzyl)-3-p-tolyl-1H-pyrrol-2-yl]-methanon, |
| [70] | [4-(3-Chlor-phenyl)-piperazin-1-yl]-[3-(4-methoxy-phenyl)-1,4-dimethyl-1H-pyrrol-2-yl]-methanon, |
| [71] | [1-(2-Brom-benzyl)-3-(4-chlor-phenyl)-4-methyl-1H-pyrrol-2-yl]-(4-isopropyl-piperazin-1-yl)-methanon, |
| [72] | [4-(2-Dimethylamino-ethyl)-piperazin-1-yl]-[1-(4-fluor-benzyl)-3-(4-methoxy-phenyl)-4-methyl-1H-pyrrol-2-yl]-methanon, |
| [73] | 3-(4-Chlor-phenyl)-1-(2-fluor-benzyl)-4-methyl-1H-pyrrol-2-carbonsäure 2,4-dimethoxy-benzylamid, |
| [74] | 1-Benzyl-4-methyl-3-p-tolyl-1H-pyrrol-2-carbonsäure (2-pyrrolidin-1-yl-ethyl)-amid, |
| [75] | [1-(4-Fluor-benzyl)-3-furan-2-yl-4-methyl-1H-pyrrol-2-yl]-[4-(3-fluor-4-methoxy-benzyl)-piperazin-1-yl]-methanon, |
| [76] | 3-(4-Methoxy-phenyl)-1,4-dimethyl-1H-pyrrol-2-carbonsäure 2,4-dimethoxy-benzylamid, |
| [77] | 1-Benzyl-4-methyl-3-phenyl-1H-pyrrol-2-carbonsäure (2-pyrrolidin-1-yl-ethyl)-amid, |
| [78] | 3-(4-Chlor-phenyl)-1,4-dimethyl-1H-pyrrol-2-carbonsäure [3-(2-methyl-piperidin-1-yl)-propyl]-amid, |
| [79] | 1-(4-Fluor-benzyl)-3-furan-2-yl-4-methyl-1H-pyrrol-2-carbonsäure cyclohexylamid, |
| [80] | 3-Furan-2-yl-1,4-dimethyl-1H-pyrrol-2-carbonsäure 4-fluor-2-trifluormethyl-benzylamid, |
| [81] | 1-Benzyl-3-furan-2-yl-4-methyl-1H-pyrrol-2-carbonsäure (1-naphthalin-2-yl-ethyl)-amid, |
| [82] | 3-(4-Chlor-phenyl)-1-ethyl-4-methyl-1H-pyrrol-2-carbonsäure (furan-2-ylmethyl)-amid, |
| [83] | 1-Benzyl-4-methyl-3-phenyl-1H-pyrrol-2-carbonsäure (3-dimethylamino-propyl)-methyl-amid, |
| [84] | [3-(4-Methoxy-phenyl)-1,4-dimethyl-1H-pyrrol-2-yl]-(4-p-tolyl-piperazin-1-yl)-methanon, |
| [85] | 1-(4-Methoxy-benzyl)-4-methyl-3-p-tolyl-1H-pyrrol-2-carbonsäure (thiophen-2-ylmethyl)-amid, |
| [86] | 4-Methyl-1-(4-methyl-benzyl)-3-p-tolyl-1H-pyrrol-2-carbonsäure phenethyl-amid, |
| [87] | 1,4-Dimethyl-3-p-tolyl-1H-pyrrol-2-carbonsäure 3,5-difluor-benzylamid, |
| [88] | 3-(4-Methoxy-phenyl)-1,4-dimethyl-1H-pyrrol-2-carbonsäure indan-1-ylamid, |
| [89] | 1-Benzyl-4-methyl-3-phenyl-1H-pyrrol-2-carbonsäure 4-dimethylamino-benzylamid, |
| [90] | 1-(4-Fluor-benzyl)-3-furan-2-yl-4-methyl-1H-pyrrol-2-carbonsäure (1-naphthalin-2-ylmethyl-pyrrolidin-3-yl)-amid, |
| [91] | [3-Furan-2-yl-4-methyl-1-(4-trifluormethyl-benzyl)-1H-pyrrol-2-yl]-(4-methyl-[1,4]diazepan-1-yl)-methanon, |
| [92] | [1-(2-Brom-benzyl)-3-(4-chlor-phenyl)-4-methyl-1H-pyrrol-2-yl]-[4-(3-trifluormethyl-phenyl)-piperazin-1-yl]-methanon, |
| [93] | [3-Furan-2-yl-1-(4-methoxy-benzyl)-4-methyl-1H-pyrrol-2-yl]-(4-thiophen-3-ylmethyl-piperazin-1-yl)-methanon, |
| [94] | 1,4-Dimethyl-3-p-tolyl-1H-pyrrol-2-carbonsäure (2-benzyloxy-cyclohexyl)-amid, |
| [95] | 2-{4-[1-Benzyl-3-(4-chlor-phenyl)-4-methyl-1H-pyrrol-2-carbonyl]-piperazin-1-yl}-N-isopropyl-acetamid, |
| [96] | (2,6-Dimethyl-morpholin-4-yl)-[3-(4-methoxy-phenyl)-1,4-dimethyl-1H-pyrrol-2-yl]-methanon, |
| [97] | 3-Furan-2-yl-1-(4-methoxy-benzyl)-4-methyl-1H-pyrrol-2-carbonsäure (thiophen-2-ylmethyl)-amid, |
| [98] | 1,4-Dimethyl-3-p-tolyl-1H-pyrrol-2-carbonsäure (furan-2-ylmethyl)-amid, |
| [99] | 4-Methyl-1-(4-methyl-benzyl)-3-p-tolyl-1H-pyrrol-2-carbonsäure (3-dimethylamino-propyl)-amid, |
| [100] | 1-(4-Fluor-benzyl)-3-(4-methoxy-phenyl)-4-methyl-1H-pyrrol-2-carbonsäure (2-oxo-tetrahydro-furan-3-yl)-amid, |
| [101] | 3-(4-Chlor-phenyl)-1,4-dimethyl-1H-pyrrol-2-carbonsäure (2-phenoxy-ethyl)-amid, |
| [102] | 3-(4-(1-Benzyl-4-methyl-3-p-tolyl-1H-pyrrole-2-carbonyl)piperazin-1-yl)pyrazin-2-carbonitril |
| [103] | 2-{[3-(4-Methoxy-phenyl)-1,4-dimethyl-1H-pyrrol-2-carbonyl]-amino}-3-phenyl-propionsäure methyl ester, |
| [104] | 3-(4-Chlor-phenyl)-1,4-dimethyl-1H-pyrrol-2-carbonsäure (2-morpholin-4-yl-ethyl)-amid, |
| [105] | 3-Furan-2-yl-1,4-dimethyl-1H-pyrrol-2-carbonsäure indan-2-ylamid, |
| [106] | (1-Butyl-4-methyl-3-p-tolyl-1H-pyrrol-2-yl)-[4-(2-fluor-phenyl)-piperazin-1-yl]-methanon, |
| [107] | [4-(3-Chlor-phenyl)-piperazin-1-yl]-(1,4-dimethyl-3-p-tolyl-1H-pyrrol-2-yl)-methanon, |
| [108] | [1-(4-Fluor-benzyl)-3-furan-2-yl-4-methyl-1H-pyrrol-2-yl]-(4-naphthalin-2-ylmethyl-piperazin-1-yl)-methanon, |
| [109] | 3-(4-Chlor-phenyl)-1-isobutyl-4-methyl-1H-pyrrol-2-carbonsäure p-tolylamid, |
| [110] | (1-Benzyl-4-methyl-3-p-tolyl-1H-pyrrol-2-yl)-[4-(2-hydroxy-ethyl)-piperazin-1-yl]-methanon, |
| [111] | 1-Butyl-4-methyl-3-p-tolyl-1H-pyrrol-2-carbonsäure [2-(2,5-dimethoxy-phenyl)-ethyl]-amid, |
| [112] | 1-Butyl-4-methyl-3-p-tolyl-1H-pyrrol-2-carbonsäure (3-imidazol-1-yl-propyl)-amid, |
| [113] | 1-Benzyl-3-(4-chlor-phenyl)-4-methyl-1H-pyrrol-2-carbonsäure [2-(3,4-dimethoxy-phenyl)-ethyl]-methyl-amid, |
| [114] | 4-Methyl-1-(4-methyl-benzyl)-3-p-tolyl-1H-pyrrol-2-carbonsäure (3-morpholin-4-yl-propyl)-amid, |
| [115] | 4-Methyl-1-(4-methyl-benzyl)-3-p-tolyl-1H-pyrrol-2-carbonsäure 4-fluor-2-trifluormethyl-benzylamid, |
| [116] | 2-{[1-Benzyl-3-(4-chlor-phenyl)-4-methyl-1H-pyrrol-2-carbonyl]-amino}-3-(4-chlor-phenyl)-propionsäure ethyl ester, |
| [117] | 1-Benzyl-4-methyl-3-phenyl-1H-pyrrol-2-carbonsäure 3-fluor-4-trifluormethyl-benzylamid, |
| [118] | 1-(4-Brom-benzyl)-4-methyl-3-p-tolyl-1H-pyrrol-2-carbonsäure (2-pyridin-2-yl-ethyl)-amid, |
| [119] | 1-(4-Fluor-benzyl)-3-furan-2-yl-4-methyl-1H-pyrrol-2-carbonsäure benzyl-(2-hydroxy-ethyl)-amid, |
| [120] | 2-[4-(1-Butyl-4-methyl-3-p-tolyl-1H-pyrrol-2-carbonyl)-piperazin-1-yl]-N-methyl-N-phenyl-acetamid, |
| [121] | [4-Methyl-1-(4-methyl-benzyl)-3-p-tolyl-1H-pyrrol-2-yl]-(4-phenyl-piperazin-1-yl)-methanon, |
| [122] | 1-Benzyl-4-methyl-3-p-tolyl-1H-pyrrol-2-carbonsäure (1-benzyl-pyrrolidin-3-yl)-amid, |
| [123] | [1-(4-Fluor-benzyl)-3-furan-2-yl-4-methyl-1H-pyrrol-2-yl]-[4-(3-trifluormethyl-phenyl)-piperazin-1-yl]-methanon, |
| [124] | 1-(4-Methoxy-benzyl)-4-methyl-3-p-tolyl-1H-pyrrol-2-carbonsäure butylamid, |
| [125] | 1-Benzyl-4-methyl-3-p-tolyl-1H-pyrrol-2-carbonsäure [2-(2-fluor-phenyl)-ethyl]-amid, |
| [126] | 4-Methyl-1-(4-methyl-benzyl)-3-p-tolyl-1H-pyrrol-2-carbonsäure 3,4-dimethoxy-benzylamid, |
| [127] | 3-(4-Chlor-phenyl)-1-isobutyl-4-methyl-1H-pyrrol-2-carbonsäure (2-diethylamino-ethyl)-amid, |
| [128] | 1-{4-[3-(4-Chlor-phenyl)-1-ethyl-4-methyl-1H-pyrrol-2-carbonyl]-piperazin-1-yl}-2-phenyl-ethanon, |
| [129] | 1-(4-Fluor-benzyl)-4-methyl-3-p-tolyl-1H-pyrrol-2-carbonsäure 3-trifluormethoxy-benzylamid, |
| [130] | [1-Benzyl-3-(4-chlor-phenyl)-4-methyl-1H-pyrrol-2-yl]-(3-methyl-piperidin-1-yl)-methanon, |
| [131] | 1-(4-Fluor-benzyl)4-methyl-3-p-tolyl-1H-pyrrol-2-carbonsäure (2-p-tolyl-ethyl)-amid, |
| [132] | 1-(4-Methoxy-benzyl)-4-methyl-3-p-tolyl-1H-pyrrol-2-carbonsäure [2-(2-methyl-5-nitro-imidazol-1-yl)-ethyl]-amid, |
| [133] | 1,4-Dimethyl-3-p-tolyl-1H-pyrrol-2-carbonsäure 2,3-dimethoxy-benzylamid, |
| [134] | 1-(2-Brom-benzyl)-3-(4-chlor-phenyl)-4-methyl-1H-pyrrol-2-carbonsäure [2-(ethyl-m-tolyl-amino)-ethyl]-amid, |
| [135] | 1-(4-Fluor-benzyl)-3-furan-2-yl-4-methyl-1H-pyrrol-2-carbonsäure (4-isopropyl-phenyl)-amid, |
| [136] | 5-Chlor-2-methoxy-benzoesäure N'-[1-(4-fluor-benzyl)-3-(4-methoxy-phenyl)-4-methyl-1H-pyrrol-2-carbonyl]-hydrazid, |
| [137] | (1-Benzyl-4-methyl-3-p-tolyl-1H-pyrrol-2-yl)-[1,4']bipiperidinyl-1'-yl-methanon, |
| [138] | 3-Furan-2-yl-1,4-dimethyl-1H-pyrrol-2-carbonsäure (4-butyl-phenyl)-amid, |
| [139] | 1-Benzyl-4-methyl-3-phenyl-1H-pyrrol-2-carbonsäure [2-(1H-indol-3-yl)-ethyl]-amid, |
| [140] | [4-(4-tert-Butyl-benryl)-piperazin-1-yl]-[1-(4-fluor-benzyl)-4-methyl-3-p-tolyl-1H-pyrrol-2-yl]-methanon, |
| [141] | [1-(4-Fluor-benzyl)-4-methyl-3-p-tolyl-1H-pyrrol-2-yl]-morpholin-4-yl-methanon, |
| [142] | 3-[(1-Benzyl-3-furan-2-yl-4-methyl-1H-pyrrol-2-carbonyl)-amino]-propionsäure ethyl ester, |
| [143] | 1,4-Dimethyl-3-phenyl-1H-pyrrol-2-carbonsäure (naphthalin-2-ylcarbamoylmethyl)-amid, |
| [144] | [3-(4-Chlor-phenyl)-1,4-dimethyl-1H-pyrrol-2-yl]-[4-(2-ethoxy-phenyl)-piperazin-1-yl]-methanon, |
| [145] | 1-(4-Fluor-benzyl)-3-furan-2-yl-4-methyl-1H-pyrrol-2-carbonsäure (4-cyanomethyl-phenyl)-amid, |
| [146] | 4-[1-Benzyl-3-(4-chlor-phenyl)-4-methyl-1H-pyrrol-2-carbonyl]-piperazin-1-carbonsäure tert-butyl ester, |
| [147] | (1,4-Dioxa-8-aza-spiro[4.5]dec-8-yl)-[1-(4-fluor-benzyl)-3-furan-2-yl-4-methyl-1H-pyrrol-2-yl]-methanon, |
| [148] | 3-Furan-2-yl-4-methyl-1-(4-trifluormethyl-benzyl)-1H-pyrrol-2-carbonsäure pentylamid, |
| [149] | 3-Furan-2-yl-1-(4-methoxy-benzyl)-4-methyl-1H-pyrrol-2-carbonsäure [3-(methyl-phenyl-amino)-propyl]-amid, |
| [150] | 1-(2,6-Dichlor-benzyl)-4-methyl-3-p-tolyl-1H-pyrrol-2-carbonsäure (2-azepan-1-yl-ethyl)-amid, |
| [151] | 1-(4-Brom-benzyl)-4-methyl-3-p-tolyl-1H-pyrrol-2-carbonsäure cyclopentylamid, |
| [152] | [1-(2,6-Dichlor-benzyl)-4-methyl-3-p-tolyl-1H-pyrrol-2-yl]-[4-(2,4-dimethoxy-phenyl)-piperazin-1-yl]-methanon, |
| [153] | 3-(4-Chlor-phenyl)-1-(2-fluor-benzyl)-4-methyl-1H-pyrrol-2-carbonsäure [2-(2-chlor-phenoxy)-ethyl]-amid, |
| [154] | 1-(2,6-Dichlor-benzyl)-4-methyl-3-p-tolyl-1H-pyrrol-2-carbonsäure (3,3-dimethyl-butyl)-amid, |
| [155] | 1,4-Dimethyl-3-p-tolyl-1H-pyrrol-2-carbonsäure (2-benzyloxy-cyclohexyl)-amid, |
| [156] | [4-Methyl-1-(4-methyl-benzyl)-3-p-tolyl-1H-pyrrol-2-yl]-thiomrpholin-4-yl-methanon, |
| [157] | 3-(4-Chlor-phenyl)-1-isobutyl-4-methyl-1H-pyrrol-2-carbonsäure (3-dimethylamino-propyl)-methyl-amid, |
| [158] | [4-(2,5-Dimethyl-phenyl)-piperazin-1-yl]-[3-furan-2-yl-1-(4-methoxy-benzyl)-4-methyl-1H-pyrrol-2-yl]-methanon, |
| [159] | 5-Chlor-2-methoxy-benzoesäure N'-[3-(4-methoxy-phenyl)-1,4-dimethyl-1H-pyrrol-2-carbonyl]-hydrazide, |
| [160] | 2-(3,4-Difluor-phenyl)-1-{4-[1-(4-fluor-benzyl)-3-furan-2-yl-4-methyl-1H-pyrrol-2-carbonyl]-piperazin-1-yl}-ethanon, |
| [161] | 2-{4-[1-(4-Fluor-benzyl)-3-furan-2-yl-4-methyl-1H-pyrrol-2-carbonyl]-piperazin-1-yl}-N-isopropyl-acetamid, |
| [162] | 3-Furan-2-yl-4-methyl-1-(4-trifluormethyl-benzyl)-1H-pyrrol-2-carbonsäure [1-(3-methoxy-phenyl)-ethyl]-amid, |
| [163] | 2-[(1-Benzyl-3-furan-2-yl-4-methyl-1H-pyrrol-2-carbonyl)-amino]-3-(4-chlor-phenyl)-propionsäure methyl ester, |
| [164] | [3-Furan-2-yl-1-(4-methoxy-benzyl)-4-methyl-1H-pyrrol-2-yl]-(4-methyl-piperazin-1-yl)-methanon, |
| [165] | 1,4-Dimethyl-3-phenyl-1H-pyrrol-2-carbonsäure (1-benzyl-pyrrolidin-3-yl)-amid, |
| [166] | (1,4-Dimethyl-3-p-tolyl-1H-pyrrol-2-yl)-(4-thieno[2,3-d]pyrimidin-4-yl-piperazin-1-yl)-methanon, |
| [167] | 4-Methyl-1-(4-methyl-benzyl)-3-p-tolyl-1H-pyrrol-2-carbonsäure (3-methoxy-propyl)-amid, |
| [168] | [3-(4-Methoxy-phenyl)-1,4-dimethyl-1H-pyrrol-2-yl]-[4-(5-trifluormethyl-pyridin-2-yl)-piperazin-1-yl]-methanon, |
| [169] | 1-(4-Brom-benzyl)-4-methyl-3-p-tolyl-1H-pyrrol-2-carbonsäure (3-morpholin-4-yl-propyl)-amid, |
| [170] | 1,4-Dimethyl-3-p-tolyl-1H-pyrrol-2-carbonsäure [2-(2,3-dimethoxy-phenyl)-ethyl]-amid, |
| [171] | [1-(4-Methoxy-benzyl)-4-methyl-3-p-tolyl-1H-pyrrol-2-yl]-(3-methyl-4-p-tolyl-piperazin-1-yl)-methanon, |
| [172] | [3-(4-Chlor-phenyl)-1-ethyl-4-methyl-1H-pyrrol-2-yl]-[4-(2,4-dimethoxy-phenyl)-piperazin-1-yl]-methanon, |
| [173] | 2-{4-[1-(2-Brom-benzyl)-3-(4-chlor-phenyl)-4-methyl-1H-pyrrol-2-carbonyl]-piperazin-1-yl}-benzonitril, |
| [174] | 1-(4-Brom-benzyl)-4-methyl-3-p-tolyl-1H-pyrrol-2-carbonsäure (1-ethyl-pyrrolidin-2-ylmethyl)-amid, |
| [175] | 3-(4-Chlor-phenyl)-1-isobutyl-4-methyl-1H-pyrrol-2-carbonsäure [2-(2-chlor-phenoxy)-ethyl]-amid, |
| [176] | 3-(4-Chlor-phenyl)-1,4-dimethyl-1H-pyrrol-2-carbonsäure [2-(1H-indol-3-yl)-ethyl]-amid, |
| [177] | 1-(4-Fluor-benzyl)-4-methyl-3-p-tolyl-1H-pyrrol-2-carbonsäure (naphthalin-2-ylcarbamoylmethyl)-amid, |
| [178] | 4-(1-Benzyl-3-furan-2-yl-4-methyl-1H-pyrrol-2-carbonyl)-piperazin-1-carbonsäure benzyl ester, |
| [179] | 1,4-Dimethyl-3-phenyl-1H-pyrrol-2-carbonsäure (1-benzyl-pyrrolidin-3-yl)-amid, |
| [180] | [3-(4-Chlor-phenyl)-1-(2-fluor-benzyl)-4-methyl-1H-pyrrol-2-yl]-[4-(4-trifluormethyl-phenyl)-piperazin-1-yl]-methanon, |
| [181] | [3-(4-Chlor-phenyl)-1-isobutyl-4-methyl-1H-pyrrol-2-yl]-(4-phenyl-piperazin-1-yl)-methanon, |
| [182] | 2-{4-[1-Butyl-3-(4-chlor-phenyl)-4-methyl-1H-pyrrol-2-carbonyl]-piperazin-1-yl}-1-pyrrolidin-1-yl-ethanon, |
| [183] | [1-Benzyl-3-(4-chlor-phenyl)-4-methyl-1H-pyrrol-2-yl]-[4-(2-chlor-4-fluor-benzoyl)-piperazin-1-yl]-methanon, |
| [184] | (4-Benzoyl-piperidin-1-yl)-[1-(4-methoxy-benzyl)-4-methyl-3-p-tolyl-1H-pyrrol-2-yl]-methanon, |
| [185] | 3-Furan-2-yl-1,4-dimethyl-1H-pyrrol-2-carbonsäure ethyl-pyridin-4-ylmethyl-amid, |
| [186] | 1-(4-Brom-benzyl)-4-methyl-3-p-tolyl-1H-pyrrol-2-carbonsäure (2-pyrrolidin-1-yl-ethyl)-amid, |
| [187] | [3-(4-Chlor-phenyl)-1-isobutyl-4-methyl-1H-pyrrol-2-yl]-[4-(4-methyl-benzoyl)-piperazin-1-yl]-methanon, |
| [188] | [1-Benzyl-3-(4-chlor-phenyl)-4-methyl-1H-pyrrol-2-yl]-(4-thieno[2,3-d]pyrimidin-4-yl-piperazin-1-yl)-methanon, |
| [189] | 1-(1,4-Dimethyl-3-phenyl-1H-pyrroi-2-carbonyl)-piperidin-3-carbonsäure ethyl ester, |
| [190] | 1,4-Dimethyl-3-phenyl-1H-pyrrol-2-carbonsäure (1-phenyl-ethyl)-amid, |
| [191] | [3-Furan-2-yl-1-(4-methoxy-benzyl)-4-methyl-1H-pyrrol-2-yl]-(1,3,4,9-tetrahydro-b-carbolin-2-yl)-methanon, |
| [192] | 1-{4-[1-Benzyl-3-(4-chlor-phenyl)-4-methyl-1H-pyrrol-2-carbonyl]-piperazin-1-yl}-2-(3,4-difluor-phenyl)-ethanon, |
| [193] | [1-(4-Brom-benzyl)-4-methyl-3-p-tolyl-1H-pyrrol-2-yl]-[4-(3-phenyl-propyl)-piperazin-1-yl]-methanon, |
| [194] | 1-(2-Brom-benzyl)-3-(4-chlor-phenyl)-4-methyl-1H-pyrrol-2-carbonsäure [2-(1H-indol-3-yl)-ethyl]-methyl-amid, |
| [195] | 1-(4-{4-[1-(4-Brom-benzyl)-4-methyl-3-p-tolyl-1H-pyrrol-2-carbonyl]-piperazin-1-yl}-phenyl)-ethanon, |
| [196] | 1,4-Dimethyl-3-phenyl-1H-pyrrol-2-carbonsäure 4-fluor-2-trifluormethyl-benzylamid, |
| [197] | 4-Methyl-1-(4-methyl-benzyl)-3-p-tolyl-1H-pyrrol-2-carbonsäure (4-phenoxy-phenyl)-amid, |
| [198] | (4-Hydroxy-piperidin-1-yl)-[1-(4-methoxy-benzyl)-4-methyl-3-p-tolyl-1H-pyrrol-2-yl]-methanon, |
| [199] | 4-Diethylamino-benzoesäure N'-(1-benzyl-4-methyl-3-p-tolyl-1H-pyrrol-2-carbonyl)-hydrazide, |
| [200] | [3-(4-Chlor-phenyl)-1-isobutyl-4-methyl-1H-pyrrol-2-yl]-[4-(3-chlor phenyl)-piperazin-1-yl]-methanon, |
| [201] | 1-(4-Brom-benzyl)-4-methyl-3-p-tolyl-1H-pyrrol-2-carbonsäure [2-(3,4-dichlor-phenyl)-ethyl]-amid, |
| [202] | 3-(4-Chlor-phenyl)-1-ethyl-4-methyl-1H-pyrrol-2-carbonsäure [2-(1H-indol-3-yl)-ethyl]-amid, |
| [203] | [1-(4-Fluor-benzyl)-4-methyl-3-p-tolyl-1H-pyrrol-2-yl]-[4-(5-trifluormethyl-pyridin-2-yl)-piperazin-1-yl]-methanon, |
| [204] | 1,4-Dimethyl-3-phenyl-1H-pyrrol-2-carbonsäure 3-trifluormethoxy-benzylamid, |
| [205] | [1-(4-Fluor-benzyl)-4-methyl-3-p-tolyl-1H-pyrrol-2-yl]-[4-(4-methyl-benzoyl)-piperazin-1-yl]-methanon, |
| [206] | [4-(3,4-Dichlor-phenyl)-piperazin-1-yl]-[1-(4-fluor-benzyl)-4-methyl-3-p-tolyl-1H-pyrrol-2-yl]-methanon, |
| [207] | 3-(4-Chlor-phenyl)-1-ethyl-4-methyl-1H-pyrrol-2-carbonsäure 2,6-dimethoxy-benzylamid, |
| [208] | 4-Methyl-1-(4-methyl-benzyl)-3-p-tolyl-1H-pyrrol-2-carbonsäure [2-(3,4-dimethoxy-phenyl)-ethyl]-amid, |
| [209] | 1-{4-[3-(4-Chlor-phenyl)-1-isobutyl-4-methyl-1H-pyrrol-2-carbonyl]-piperazin-1-yl}-2-(3,4-difluor-phenyl)-ethanon, |
| [210] | [1-(4-Fluor-benzyl)-4-methyl-3-p-tolyl-1H-pyrrol-2-yl]-[4-(2-methoxy-phenyl)-piperidin-1-yl]-methanon, |
| [211] | [3-Furan-2-yl-4-methyl-1-(4-trifluormethyl-benzyl)-1H-pyrrol-2-yl]-thiomorpholin-4-yl-methanon, |
| [212] | 1,4-Dimethyl-3-phenyl-1H-pyrrol-2-carbonsäure (2-thiophen-2-yl-ethyl)-amid, |
| [213] | 4-Diethylamino-benzoesäure N'-(1-butyl-4-methyl-3-p-tolyl-1H-pyrrol-2-carbonyl)-hydrazide, |
| [214] | {1-[1-(4-Fluor-benzyl)-3-(4-methoxy-phenyl)-4-methyl-1H-pyrrol-2-carbonyl]-pyrrolidin-3-yl}-carbaminsäure tert-butyl ester, |
| [215] | 2-{4-[3-(4-Chlor-phenyl)-1-isobutyl-4-methyl-1H-pyrrol-2-carbonyl]-piperazin-1-yl}-1-pyrrolidin-1-yl-ethanon, |
| [216] | [4-(2,3-Dihydro-benzo[1,4]dioxine-2-carbonyl)-piperazin-1-yl]-(1,4-dimethyl-3-phenyl-1H-pyrrol-2-yl)-methanon, |
| [217] | 3-Furan-2-yl-1,4-dimethyl-1H-pyrrol-2-carbonsäure 3,4-dimethoxy-benzylamid, |
| [218] | [3-Furan-2-yl-4-methyl-1-(4-trifluormethyl-benzyl)-1H-pyrrol-2-yl]-(4-pyridin-2-yl-piperazin-1-yl)-methanon, |
| [219] | [4-(Furan-2-carbonyl)-piperazin-1-yl]-[3-furan-2-yl-4-methyl-1-(4-trifluormethyl-benzyl)-1H-pyrrol-2-yl]-methanon, |
| [220] | 3-Furan-2-yl-4-methyl-1-(4-trifluormethyl-benzyl)-1H-pyrrol-2-carbonsäure (4-tert-butyl-phenyl)-amid, |
| [221] | 2-{4-[3-(4-Chlor-phenyl)-1-isobutyl-4-methyl-1H-pyrrol-2-carbonyl]-piperazin-1-yl}-benzonitril, |
| [222] | (3-Furan-2-yl-1,4-dimethyl-1H-pyrrol-2-yl)-[4-(4-trifluormethyl-pyridin-2-yl)-piperazin-1-yl]-methanon, |
| [223] | 1,4-Dimethyl-3-phenyl-1H-pyrrol-2-carbonsäure 3,5-difluor-benzylamid, |
| [224] | 1,4-Dimethyl-3-phenyl-1H-pyrrol-2-carbonsäure (pyridin-4-ylmethyl)-amid, |
| [225] | 2-[4-(3-Furan-2-yl-1,4-dimethyl-1H-pyrrol-2-carbonyl)-piperazin-1-yl]-benzonitril, |
| [226] | 3-Furan-2-yl-1,4-dimethyl-1H-pyrrol-2-carbonsäure methyl-(2-pyridin-2-yl-ethyl)-amid, |
| [227] | 3-Furan-2-yl-1,4-dimethyl-1H-pyrrol-2-carbonsäure (3-methoxy-benzyl)-(tetrahydro-furan-2-ylmethyl)-amid, |
| [228] | 3-Furan-2-yl-1,4-dimethyl-1H-pyrrol-2-carbonsäure (4-phenoxy-phenyl)-amid, |
| [229] | 3-Furan-2-yl-1,4-dimethyl-1H-pyrrol-2-carbonsäure pentylamid, |
| [230] | 2-(3,4-Difluor-phenyl)-1-[4-(3-furan-2-yl-1,4-dimethyl-1H-pyrrol-2-carbonyl)-piperazin-1-yl]-ethanon, |
| [231] | 3-Furan-2-yl-1,4-dimethyl-1H-pyrrol-2-carbonsäure phenylamid, |
| [232] | [4-(2,4-Dimethoxy-phenyl)-piperazin-1-yl]-(3-furan-2-yl-1,4-dimethyl-1H-pyrrol-2-yl)-methanon, |
| [233] | 3-Furan-2-yl-1,4-dimethyl-1H-pyrrol-2-carbonsäure [2-(3,4-dichlor-phenyl)-ethyl]-amid, |
| [234] | 3-Furan-2-yl-1,4-dimethyl-1H-pyrrol-2-carbonsäure [2-(2-chlor-phenoxy)-ethyl]-amid, |
| [235] | 3-Furan-2-yl-1,4-dimethyl-1H-pyrrol-2-carbonsäure 3-methoxy-benzylamid, |
| [236] | 3-Furan-2-yl-4-methyl-1-(4-trifluormethyl-benzyl)-1H-pyrrol-2-carbonsäure phenethyl-amid, |
| [237] | 3-Furan-2-yl-1,4-dimethyl-1H-pyrrol-2-carbonsäure cyclopentylamid, |
| [238] | 3-Furan-2-yl-1,4-dimethyl-1H-pyrrol-2-carbonsäure (pyridin-2-ylmethyl)-amid, |
| [239] | 3-Furan-2-yl-4-methyl-1-(4-trifluormethyl-benzyl)-1H-pyrrol-2-carbonsäure [2-(3,4-dimethoxy-phenyl)-ethyl]-amid, |
| [240] | [1-(4-Brom-benzyl)-4-methyl-3-p-tolyl-1H-pyrrol-2-yl]-(2,6-dimethyl-morpholin-4-yl)-methanon, |
| [241] | 1,4-Dimethyl-3-phenyl-1H-pyrrol-2-carbonsäure (2-p-tolyl-ethyl)-amid, |
| [242] | (1,4-Dimethyl-3-phenyl-1H-pyrrol-2-yl)-[4-(4-fluor-phenyl)-piperazin-1-yl]-methanon und |
| [243] | 4-Methyl-1-(4-methyl-benzyl)-3-p-tolyl-1H-pyrrol-2-carbonsäure [2-(1H-indol-3-yl)-ethyl]-methyl-amid, |
jeweils in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze oder jeweils in Form entsprechender Solvate.

7. Verfahren zur Herstellung von 1,3-disubstituierten 4-Methyl-1H-pyrrol-2-carbonsäureamiden der allgemeinen Formel I gemäß einem der Ansprüche 1 bis 6, **dadurch gekennzeichnet, dass** wenigstens eine Verbindung der allgemeinen Formel II, 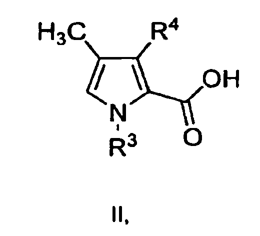 worin R³ und R⁴ die Bedeutung gemäß der Ansprüche 1 bis 6 haben, in wenigstens einem Reaktionsmedium, in Gegenwart wenigstens eines geeigneten Kopplungsmittels, in Gegenwart wenigstens einer Base, vorzugsweise bei einer Temperatur von - 70 °C bis 100 °C, durch Umsetzung mit wenigstens einer Verbindung der allgemeinen Formel HNR¹R², worin R¹ und R² die Bedeutung gemäß einem der Ansprüche 1 bis 6 haben, in eine entsprechende Verbindung der allgemeinen Formel I, in Form eines entsprechenden Salzes überführt wird, 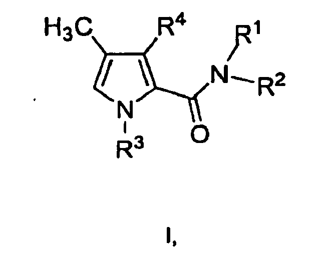 worin R¹, R², R³ und R⁴ die vorstehend genannte Bedeutung haben, und diese gereinigt und/oder isoliert wird.

8. Arzneimittel enthaltend wenigstens eine Verbindung gemäß einem der Ansprüche 1 bis 6 und einen oder mehrere pharmazeutische Hilfsstoffe.

9. Arzneimittel gemäß Anspruch 8 zur Prophylaxe und/oder Behandlung von Schmerz, vorzugsweise von Schmerz ausgewählt aus der Gruppe bestehend aus akutem Schmerz, chronischem Schmerz und neuropathischem Schmerz.

10. Arzneimittel gemäß Anspruch 8 oder 9 zur Prophylaxe und/oder Behandlung von Migräne; Depressionen; Harninkontinenz; Husten; neurodegenerativen Erkrankungen, vorzugsweise ausgewählt aus der Gruppe bestehend aus Morbus Parkinson, Morbus Huntington, Morbus Alzheimer und Multipler Sklerose; Störungen der Nahrungsaufnahme, vorzugsweise ausgewählt aus der Gruppe bestehend aus Bulimie, Anorexie, Fettsucht und Kachexie; kognitiven Dysfunktionen, vorzugsweise Gedächtnisstörungen; Epilepsie; Diarrhoe; Pruritus; Alkohol- und/oder Drogen- und/oder Medikamentenmißbrauch; Alkohol- und/oder Drogen- und/oder Medikamentenabhängigkeit, vorzugsweise zur Prophylaxe und/oder Verminderung von Entzugserscheinungen bei Alkohol- und/oder Drogen-und/oder Medikamentenabhängigkeit; zur Prophylaxe und/oder Verminderung einer Toleranzentwicklung gegenüber Medikamenten, insbesondere Medikamenten auf Basis von Opioiden; zur Regulation der Nahrungsaufnahme; zur Modulation der Bewegungsaktivität; zur Regulation des kardiovaskulären Systems; zur Lokalanästhesie; zur Anxiolyse; zur Vigilanzsteigerung; zur Libidosteigerung; zur Diurese und/oder zur Antinatriurese.

11. Verwendung wenigstens einer Verbindung gemäß einem der Ansprüche 1 bis 6 zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz und neuropathischem Schmerz.

12. Verwendung wenigstens einer Verbindung gemäß einem der Ansprüche 1 bis 6 zur Herstellung eines Arzneimittels zur Prophylaxe und/oder Behandlung von Migräne; Depressionen; Harninkontinenz; Husten; neurodegenerativen Erkrankungen, vorzugsweise ausgewählt aus der Gruppe bestehend aus Morbus Parkinson, Morbus Huntington, Morbus Alzheimer und Multipler Sklerose; Störungen der Nahrungsaufnahme, vorzugsweise ausgewählt aus der Gruppe bestehend aus Bulimie, Anorexie, Fettsucht und Kachexie; kognitiven Dysfunktionen, vorzugsweise Gedächtnisstörungen; Epilepsie; Diarrhoe; Pruritus; Alkohol- und/oder Drogen- und/oder Medikamentenmißbrauch; Alkohol- und/oder Drogen- und/oder Medikamentenabhängigkeit, vorzugsweise zur Prophylaxe und/oder Verminderung von Entzugserscheinungen bei Alkohol- und/oder Drogen-und/oder Medikamentenabhängigkeit; zur Prophylaxe und/oder Verminderung einer Toleranzentwicklung gegenüber Medikamenten, insbesondere Medikamenten auf Basis von Opioiden; zur Regulation der Nahrungsaufnahme; zur Modulation der Bewegungsaktivität; zur Regulation des kardiovaskulären Systems; zur Lokalanästhesie; zur Anxiolyse; zur Vigilanzsteigerung; zur Libidosteigerung; zur Diurese und/oder zur Antinatriurese.

## Claims

1. 1,3-Disubstituted 4-methyl-1H-pyrrole-2-carboxamides of the general formula I, 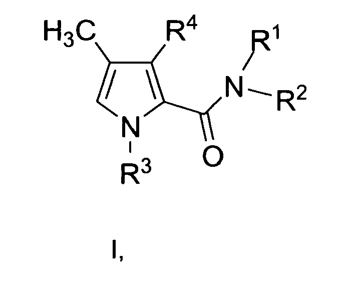 in which
R¹ denotes C₁₋₆ alkyl, C₂₋₆ alkenyl or -C₂₋₆ alkynyl, which may in each case be unsubstituted or substituted with 1, 2, 3, 4 or 5 substituents mutually independently selected from the group consisting of F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O-C₂H₅, -C(=O)-O-C(CH₃)₃, -C(=O)-O-CH₂- phenyl, -C(=O)-NH-naphthyl, -N(CH₃)-phenyl, -N(C₂H₅)-phenyl, -N(C₂H₅)-(m-toluyl), -N(C₂H₅)-(p-toluyl), -N(CH₃)-(p-toluyl) and -NH-C(=O)-O-C(CH₃)₃;
2- to 6-membered heteroalkyl, heteroalkenyl or heteroalkynyl, which may in each case be unsubstituted or substituted with 1, 2, 3, 4 or 5 substituents mutually independently selected from the group consisting of F, Cl, Br, I, -NO₂, -CN, -OH, -SH and -NH₂ and may in each case comprise 1 or 2 heteroatom(s) mutually independently selected from the group consisting of oxygen, sulfur and nitrogen (NH) as chain link(s);
C₃₋₇ cycloalkyl, C₅₋₆ cycloalkenyl, 5- to 7-membered heterocycloalkyl, 5- to 7-membered heterocycloalkenyl, (1,2,3,4)-tetrahydroquinolinyl, (1,2,3,4)-tetrahydroisoquinolinyl, (2,3)-dihydro-1H-isoindolyl, indolinyl, indanyl, (1,2,3,4)-tetrahydronaphthyl, (2,3)-dihydrobenzo[1.4]dioxinyl or benzo[1.3]dioxolyl, which may in each case be unsubstituted or substituted with 1, 2, 3, 4 or 5 substituents mutually independently selected from the group consisting of F, Cl, Br, I, -CN, methyl, ethyl, n-propyl, isopropyl, n-butyl, 2-butyl, isobutyl, tert.-butyl, -OH, oxo, thioxo, -O-CH₃, -O-C₂H₅, -O-C₃H₇, -O-phenyl, -O-CH₂-phenyl, -NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂H₅, -CH₂-naphthyl, benzyl and phenyl and/or may in each case be attached via an unsubstituted C₁₋₃ alkylene, C₂₋₃ alkenylene or C₂₋₃ alkynylene group and/or may in each case comprise 1 or 2 heteroatom(s) mutually independently selected from the group consisting of oxygen, sulfur and nitrogen (NH) as ring member(s);
phenyl, which may in each case be attached via a C₁₋₃ alkylene, C₂₋₃ alkenylene or C₂₋₃ alkynylene group, which may in each case be unsubstituted or substituted with 1 or 2 substituents selected from the group consisting of F, -C(=O)-O-CH₃ and -C(=O)-O-C₂H₅, or be attached via a -CH₂-CH₂-O-, -CH₂-O- or -CH₂-CH₂-CH₂-O- group and/or may in each case be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, allyl, ethynyl, propynyl, -C≡C-Si(CH₃)₃, -C≡C-Si(C₂H₅)₃, -CH₂-O-CH₃, -CH₂-O-C₂H₅, -NH₂, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-phenyl, pyrazolyl, -N(CH₃)₂, -N(C₂H₅)₂, -NH-CH₃, -NH-C₂H₅, -CH₂-O-C(=O)-phenyl, -NH-S(=O)₂-CH₃, -C(=O)-H, -C(=O)-CH₃, -C(=O)-C₂H₅, -NH-C(=O)-CH₃, -NH-C(=O)-C₂H₅, -O-C(=O)-phenyl, -C(=O)-NH₂, -C(=O)-NH-CH₃, -C(=O)-NH-C₂H₅, -C(=O)-NH-C(CH₃)₃, -C(=O)-N(C₂H₅)₂, -S(=O)-NH₂, [1,2,3]-thiadiazolyl, cyclopropyl, cyclobutyl, cyclopentyl, cyclohexyl, piperazinyl, pyrrolidinyl, piperidinyl, phenyl, furyl (furanyl), thiadiazolyl, thiophenyl (thienyl) and benzyl, wherein the cyclic substituents or the cyclic residues of these substituents themselves may in each case be substituted with 1, 2, 3, 4, or 5 substituents mutually independently selected from the group consisting of F, Cl, Br, I, -CN, -NO₂, -OH, -SH, -NH₂, methyl, ethyl, n-propyl, isopropyl, n-butyl, isobutyl, 2-butyl, tert.-butyl, n-pentyl, neopentyl, -O-CH₃, -O-C₂H₅, -O-C₃H₇, -O-C(CH₃)₃, -CF₃, -CHF₂, -CH₂F, -O-CF₃, -O-CHF₂ and -O-CH₂F;
a residue selected from the group consisting of naphthyl, indolizinyl, benzimidazolyl, tetrazolyl, triazinyl, isoxazolyl, phthalazinyl, carbazolyl, carbolinyl, diazanaphthyl, thienyl, furyl, pyrrolyl, pyrazolyl, pyrazinyl, pyranyl, triazolyl, pyridinyl, imidazolyl, indolyl, isoindolyl, benzo[b]furanyl, benzo[b]thiophenyl, benzo[d]thiazolyl, benzodiazolyl, benzotriazolyl, benzoxazolyl, benzisoxazolyl, thiazolyl, thiadiazolyl, oxazolyl, oxadiazolyl, isoxazolyl, pyridazinyl, indazolyl, quinoxalinyl, quinazolinyl, quinolinyl, naphthridinyl and isoquinolinyl, which may in each case be attached via a C₁₋₃ alkylene, C₂₋₃ alkenylene or C₂₋₃ alkynylene group and/or may in each case be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, allyl, ethynyl, propynyl, -C≡C-Si(CH₃)₃, -C≡C-Si(C₂H₅)₃, -CH₂-O-CH₃, -CH₂-O-C₂H₅, -NH₂, -C(=O)-OH, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-phenyl, pyrazolyl, -N(CH₃)₂, -N(C₂H₅)₂, -NH-CH₃, -NH-C₂H₅, -CH₂-O-C(=O)-phenyl, -NH-S(=O)₂-CH₃, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -O-C(=O)-phenyl, -C(=O)-NH₂, -C(=O)-NH-CH₃, -C(=O)-N(CH₃)₂, -C(=O)-O-CH(CH₃)₂, -C(=O)-O-(CH₂)₃-CH₃, -C(=O)-O-phenyl, -O-C(=O)-CH₃, -O-C(=O)-C₂H₅, -C(=O)-NH-C₂H₅, -C(=O)-NH-C(CH₃)₃, -C(=O)-N(C₂H₅)₂, -C(=O)-NH-phenyl, -C(=O)-N(CH₃)-phenyl, -C(=O)-N(C₂H₅)-phenyl, -S(=O)-NH₂, [1,2,3]-thiadiazolyl, cyclopropyl, cyclobutyl, cyclopentyl, cyclohexyl, piperazinyl, pyrrolidinyl, piperidinyl, phenyl, furyl (furanyl), thiadiazolyl, thiophenyl (thienyl) and benzyl, wherein the cyclic substituents or the cyclic residues of these substituents themselves may in each case be substituted with 1, 2, 3, 4, or 5 substituents mutually independently selected from the group consisting of F, Cl, Br, I, -CN, -NO₂, -OH, -SH, -NH₂, methyl, ethyl, n-propyl, isopropyl, n-butyl, isobutyl, 2-butyl, tert.-butyl, n-pentyl, neopentyl, ethenyl, allyl, ethynyl, propynyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ and -S-CH₂F;
or -NH-C(=O)-R⁵.
R² denotes H or C₁₋₆ alkyl, C₂₋₆ alkenyl or C₂₋₆ alkynyl, which may in each case be unsubstituted or substituted with 1, 2, 3, 4 or 5 substituents mutually independently selected from the group consisting of F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O-C₂H₅, -C(=O)-O-C(CH₃)₃, -C(=O)-O-CH₂-phenyl, -C(=O)-NH-naphthyl, -N(CH₃)-phenyl, -N(C₂H₅)- phenyl, -N(C₂H₅)-(m-toluyl), -N(C₂H₅)-(p-toluyl), -N(CH₃)-(p-toluyl) and -NH-C(=O)-O-C(CH₃)₃;
a residue selected from the group consisting of phenyl and naphthyl, which may in each case be attached via a C₁₋₃ alkylene, C₂₋₃ alkenylene or C₂₋₃ alkynylene group and/or may in each case be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, allyl, ethynyl, propynyl, -C≡C-Si(CH₃)_{3,} -C≡C-Si(C₂H₅)₃, -CH₂-O-CH₃, -CH₂-O-C₂H₅, -NH₂, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂- phenyl, pyrazolyl, -N(CH₃)₂, -N(C₂H₅)₂, -NH-CH₃, -NH-C₂H₅, -CH₂-O-C(=O)-phenyl, -NH-S(=O)₂-CH₃, -C(=O)-H, -C(=O)-CH₃, -C(=O)-C₂H₅, -NH-C(=O)-CH₃, -NH-C(=O)-C₂H₅, -O-C(=O)-phenyl, -C(=O)-NH₂, -C(=O)-NH-CH₃, -C(=O)-NH-C₂H₅, -C(=O)-NH-C(CH₃)₃, -C(=O)-N(C₂H₅)₂, -S(=O)-NH₂, [1,2,3]-thiadiazolyl, cyclopropyl, cyclobutyl, cyclopentyl, cyclohexyl, piperazinyl, pyrrolidinyl, piperidinyl, phenyl, furyl (furanyl), thiadiazolyl, thiophenyl (thienyl) and benzyl, wherein the cyclic substituents or the cyclic residues of these substituents themselves may in each case be substituted with 1, 2, 3, 4, or 5 substituents mutually independently selected from the group consisting of F, Cl, Br, I, -CN, -NO₂, -OH, -SH, -NH₂, methyl, ethyl, n-propyl, isopropyl, n-butyl, isobutyl, 2-butyl, tert.-butyl, n-pentyl, neopentyl, -O-CH₃, -O-C₂H₅, -O-C₃H₇, -O-C(CH₃)₃, -CF₃, -CHF₂, -CH₂F, -O-CF₃, -O-CHF₂ and -O-CH₂F, or
R¹ and R², together with the nitrogen atom joining them together, form a residue selected from the group consisting of imidazolidinyl, [1,3,4,9]- tetrahydro-[b]-carbolinyl, piperidinyl, morpholinyl, thiomorpholinyl, pyrrolidinyl, piperazinyl, azepanyl, diazepanyl and (1,4)-dioxo-8-aza- spiro[4.5]decyl, which may in each case be unsubstituted or substituted with 1 or 2 R⁶ residues.
R³ denotes C₁₋₆ alkyl, C₂₋₆ alkenyl or C₂₋₆ alkynyl, which may in each case be unsubstituted or substituted with 1, 2, 3, 4 or 5 substituents mutually independently selected from the group consisting of F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O-C₂H₅, -C(=O)-O-C(CH₃)₃, -C(=O)-O-CH₂- phenyl, -C(=O)-NH-naphthyl, -N(CH₃)-phenyl, -N(C₂H₅)-phenyl, -N(C₂H₅)-(m-toluyl), -N(C₂H₅)-(p-toluyl), -N(CH₃)-(p-toluyl) and -NH-C(=O)-O-C(CH₃)₃; or
a residue selected from the group consisting of phenyl, naphthyl, indolizinyl, benzimidazolyl, tetrazolyl, triazinyl, isoxazolyl, phthalazinyl, carbazolyl, carbolinyl, diazanaphthyl, thienyl, furyl, pyrrolyl, pyrazolyl, pyrazinyl, pyranyl, triazolyl, pyridinyl, imidazolyl, indolyl, isoindolyl, benzo[b]furanyl, benzo[b]thiophenyl, benzo[d]thiazolyl, benzodiazolyl, benzotriazolyl, benzoxazolyl, benzisoxazolyl, thiazolyl, thiadiazolyl, oxazolyl, oxadiazolyl, isoxazolyl, pyridazinyl, indazolyl, quinoxalinyl, quinazolinyl, quinolinyl, naphthridinyl and isoquinolinyl, which may in each case be attached via a C₁₋₃ alkylene, C₂₋₃ alkenylene or C₂₋₃ alkynylene group and/or may in each case be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, allyl, ethynyl, propynyl, -CH₂-O-CH₃, -CH₂-O-C₂H₅, -NH₂, -C(=O)-OH, -S-CH₃, -S-C₂H₅, -S(=O)-CH₃, -S(=O)₂-CH₃, -S(=O)-C₂H₅, -S(=O)₂-C₂H_{5,} -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-H, -C(=O)-CH₃, -C(=O)-C₂H₅, -C(=O)-NH₂, -C(=O)-NH-CH₃ and -C(=O)-N(CH₃)₂.
R⁴ denotes phenyl, which may in each case be unsubstituted or substituted with 1, 2, 3, 4 or 5 substituents mutually independently selected from the group consisting of F, Cl, Br, I, -CN, -CH₂-CN, -OH, -SH, -NH₂, -C(=O)-OH, methyl, ethyl, n-propyl, isopropyl, n-butyl, isobutyl, 2-butyl, tert.-butyl, n-pentyl, neopentyl, ethenyl, allyl, ethynyl, propynyl, -C≡C-Si(CH₃)₃, -C≡C-Si(C₂H₅)₃, -S-CH₃, -S-C₂H₅, -S-phenyl, -S-CH₂- phenyl, -O-CH₃, -O-C₂H₅, -O-C₃H₇, -O-C(CH₃)₃, -O-phenyl, -O-CH₂- phenyl, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-phenyl, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -S(=O)₂-NH₂, -S(=O)₂-NH-phenyl, [1 ,2,3]-thiadiazolyl, cyclopropyl, cyclobutyl, cyclopentyl, cyclohexyl, phenyl, benzyl and phenethyl, wherein the cyclic substituents or the cyclic residues of these substituents may themselves in each case be unsubstituted or substituted with 1, 2, 3, 4 or 5 substituents mutually independently selected from the group consisting of F, Cl, Br, I, -CN, -NO₂, -OH, -SH, -NH₂, -C(=O)-OH, methyl, ethyl, n-propyl, isopropyl, n-butyl, isobutyl, 2-butyl, tert.-butyl, n-pentyl, neopentyl, ethenyl, allyl, ethynyl, propynyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ and -S-CH₂F; or
a residue selected from the group consisting of thienyl, furyl, pyrrolyl, pyrazolyl, pyrazinyl, pyranyl, triazolyl, pyridinyl, imidazolyl, thiadiazolyl, oxadiazolyl and pyridazinyl, which may in each case be unsubstituted or substituted with 1, 2, 3, 4 or 5 substituents mutually independently selected from the group consisting of F, Cl, Br, I, -CN, -NO₂, -OH, -CH₂-CN, -SH, -NH₂, -C(=O)-OH, methyl, ethyl, n-propyl, isopropyl, n-butyl, isobutyl, 2-butyl, tert.-butyl, n-pentyl, neopentyl, ethenyl, allyl, ethynyl, propynyl, -S-CH₃, -S-C₂H₅, -S-phenyl, -S-CH₂-phenyl, -O-CH₃, -O-C₂H₅, -O-C₃H₇, -O-C(CH₃)₃, -O-phenyl, -O-CH₂-phenyl, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -S(=O)₂-NH₂, cyclopropyl, cyclobutyl, cyclopentyl, cyclohexyl, phenyl and benzyl, wherein the cyclic substituents or the cyclic residues of these substituents themselves may in each case be unsubstituted or substituted with 1, 2, 3, 4, or 5 substituents mutually independently selected from the group consisting of F, Cl, Br, I, -CN, -NO₂, -OH, -SH, -NH₂, -C(=O)-OH, methyl, ethyl, n-propyl, isopropyl, n-butyl, isobutyl, 2-butyl, tert.-butyl, n-pentyl, neopentyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ and -S-CH₂F;
R⁵ denotes a residue selected from the group consisting of phenyl, naphthyl, thienyl, furyl, pyrrolyl, pyrazolyl, pyrazinyl, pyranyl, triazolyl, pyridinyl, imidazolyl, indolyl, isoindolyl, thiazolyl, thiadiazolyl, oxazolyl, oxadiazolyl, isoxazolyl, pyridazinyl, indazolyl, quinoxalinyl, quinazolinyl, quinolinyl, naphthridinyl and isoquinolinyl, which may in each case be attached via a C₁₋₃ alkylene, C₂₋₃ alkenylene or C₂₋₃ alkynylene group and/or may in each case be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, allyl, ethynyl, propynyl, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-H, -C(=O)-CH₃, -C(=O)-C₂H₅, -C(=O)-NH₂, -C(=O)-NH-CH₃ and -C(=O)-N(CH₃)₂;
R⁶ denotes -OH; F; Cl; Br; I; -SH; -NO₂; -NH₂; -NH-C(=O)-O-R⁷; -C(=O)-O-R⁸; -C(=O)-R⁹_{;}
C₁₋₆ alkyl, C₂₋₆ alkenyl or C₂₋₆ alkynyl, which may in each case be unsubstituted or substituted with 1, 2, 3, 4 or 5 substituents mutually independently selected from the group consisting of F, Cl, Br, I, -NO₂, -CN, -OH, -SH, -NH₂, -N(CH₃)₂, -N(C₂H₅)₂, -C(=O)-pyrrolidinyl, -C(=O)-N(CH₃)-phenyl and -C(=O)-NH-CH(CH₃)₂, -N(CH₃)(C₂H₅), -C(=O)-OH, -C(=O)-O-CH₃, -C(=O)-O-C₂H₅ and -C(=O)-O-C(CH₃)₃;
C₃₋₇ cycloalkyl, C₅₋₆ cycloalkenyl, 5- to 7-membered heterocycloalkyl or 5- to 7-membered heterocycloalkenyl, which may in each case be unsubstituted or substituted with 1, 2, 3, 4 or 5 substituents mutually independently selected from the group consisting of F, Cl, Br, I, -CN, methyl, ethyl, n-propyl, isopropyl, n-butyl, 2-butyl, isobutyl, tert.-butyl, -OH, oxo, thioxo, -O-CH₃, -O-C₂H₅, -O-C₃H₇, -O-phenyl, -O-CH₂-phenyl, -NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂H₅, -CH₂-naphthyl, benzyl and phenyl and/or may in each case be attached via an unsubstituted C₁₋₃ alkylene, C₂₋₃ alkenylene or C₂₋₃ alkynylene group and/or may in each case comprise 1 or 2 heteroatom(s) mutually independently selected from the group consisting of oxygen, sulfur and nitrogen (NH) as ring member(s); or
a residue selected from the group consisting of phenyl, naphthyl, indolizinyl, benzimidazolyl, tetrazolyl, triazinyl, isoxazolyl, phthalazinyl, carbazolyl, carbolinyl, diazanaphthyl, thienyl, furyl, pyrrolyl, pyrazolyl, pyrazinyl, pyranyl, triazolyl, pyridinyl, imidazolyl, indolyl, isoindolyl, benzo[b]furanyl, benzo[b]thiophenyl, benzo[d]thiazolyl, benzodiazolyl, benzotriazolyl, benzoxazolyl, benzisoxazolyl, thiazolyl, thiadiazolyl, oxazolyl, oxadiazolyl, isoxazolyl, pyridazinyl, indazolyl, quinoxalinyl, quinazolinyl, quinolinyl, naphthridinyl, thieno[2,3-d]pyrimidinyl and isoquinolinyl, which may in each case be attached via a C₁₋₃ alkylene, C₂₋₃ alkenylene or C₂₋₃ alkynylene group and/or may in each case be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, allyl, ethynyl, propynyl, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -C(=O)-O-CH(CH₃)₂, -C(=O)-O-(CH₂)₃-CH₃, -C(=O)-NH-C₂H₅, -C(=O)-NH-C(CH₃)₃, -C(=O)-N(C₂H₅)₂, cyclopropyl, cyclobutyl, cyclopentyl, cyclohexyl, phenyl, furyl (furanyl) and benzyl, wherein the cyclic substituents or the cyclic residues of these substituents themselves may in each case be substituted with 1, 2, 3, 4, or 5 substituents mutually independently selected from the group consisting of F, Cl, Br, I, -CN, -NO₂, -OH, -SH, -NH₂, methyl, ethyl, n-propyl, isopropyl, n-butyl, isobutyl, 2-butyl, tert.-butyl, n-pentyl, neopentyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ and -S-CH₂F.
R⁷, R⁸ and R⁹, mutually independently, in each case denote
C₁₋₆ alkyl, C₂₋₆ alkenyl or C₂₋₆ alkynyl, which may in each case be unsubstituted or substituted with 1, 2, 3, 4 or 5 substituents mutually independently selected from the group consisting of F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O-C₂H₅ and -C(=O)-O-C(CH₃)₃;
a residue selected from the group consisting of indolinyl, indanyl, (1,2,3,4)-tetrahydronaphthyl, (2,3)-dihydrobenzo[1.4]dioxinyl and benzo[1.3]dioxolyl, which may in each case be unsubstituted or substituted with 1, 2, 3, 4 or 5 substituents mutually independently selected from the group consisting of F, Cl, Br, I, -CN, methyl, ethyl, n-propyl, isopropyl, n-butyl, 2-butyl, isobutyl, tert.-butyl, -OH, oxo, thioxo, -O-CH₃, -O-C₂H₅, -O-C₃H₇, -O-phenyl, -O-CH₂-phenyl, -NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂H₅, -CH₂-naphthyl, benzyl and phenyl; or
a residue selected from the group consisting of phenyl, naphthyl, indolizinyl, benzimidazolyl, tetrazolyl, triazinyl, isoxazolyl, phthalazinyl, carbazolyl, carbolinyl, diazanaphthyl, thienyl, furyl, pyrrolyl, pyrazolyl, pyrazinyl, pyranyl, triazolyl, pyridinyl, imidazolyl, indolyl, isoindolyl, thiazolyl, thiadiazolyl, oxazolyl, oxadiazolyl, isoxazolyl and pyridazinyl, which may in each case be attached via a C₁₋₃ alkylene, C₂₋₃ alkenylene or C₂₋₃ alkynylene group and/or may in each case be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, allyl, ethynyl, propynyl, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -C(=O)-O-CH(CH₃)₂, -C(=O)-O-(CH₂)₃-CH₃, -C(=O)-NH-C₂H₅, -C(=O)-NH-C(CH₃)₃, -C(=O)-N(C₂H₅)₂, cyclopropyl, cyclobutyl, cyclopentyl, cyclohexyl, phenyl, furyl (furanyl) and benzyl,
in each case in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

2. Compounds according to claim 1, **characterised in that**
R¹ denotes C₁₋₆ alkyl, C₂₋₆ alkenyl or C₂₋₆ alkynyl, which may in each case be unsubstituted or substituted with 1, 2, 3, 4 or 5 substituents mutually independently selected from the group consisting of F, Cl, Br, 1, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O-C₂H₅, -C(=O)-O-C(CH₃)₃, -C(=O)-O-CH₂- phenyl, -C(=O)-NH-naphthyl, -N(CH₃)-phenyl, -N(C₂H₅)-phenyl, -N(C₂H₅)-(m-toluyl), -N(C₂H₅)-(p-toluyl), -N(CH₃)-(p-toluyl) and -NH-C(=O)-O-C(CH₃)₃;
2- to 6-membered heteroalkyl, which may in each case be unsubstituted or substituted with 1, 2, 3 4 or 5 substituents mutually independently selected from the group consisting of F, Cl, Br, I, -NO₂, -CN, -OH, -SH, and -NH₂ and may in each case comprise 1 or 2 heteroatom(s) mutually independently selected from the group consisting of oxygen, sulfur and nitrogen (NH) as a chain link(s);
C₃₋₇ cycloalkyl, C₅₋₆ cycloalkenyl, 5- to 7-membered heterocycloalkyl, 5- to 7-membered heterocycloalkenyl, (1,2,3,4)-tetrahydroquinolinyl, (1,2,3,4)-tetrahydroisoquinolinyl, (2,3)-dihydro-1H-isoindolyl, indolinyl, indanyl, (1,2,3,4)-tetrahydronaphthyl, (2,3)-dihydrobenzo[1.4]dioxinyl or benzo[1.3]dioxolyl, which may in each case be unsubstituted or substituted with 1, 2, 3, 4 or 5 substituents mutually independently selected from the group consisting of F, Cl, Br, I, -CN, methyl, ethyl, n-propyl, isopropyl, n-butyl, 2-butyl, isobutyl, tert.-butyl, -OH, oxo, thioxo, -O-CH₃, -O-C₂H₅, -O-C₃H₇, -O-phenyl, -O-CH₂-phenyl, -NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂H₅, -CH₂-naphthyl, benzyl and phenyl and/or may in each case be attached via an unsubstituted C₁₋₃ alkylene, C₂₋₃ alkenylene or C₂₋₃ alkynylene group and/or may in each case comprise 1 or 2 heteroatom(s) mutually independently selected from the group consisting of oxygen, sulfur and nitrogen (NH) as ring member(s);
phenyl, which may in each case be attached via a C₁₋₃-alkylene, C₂₋₃-alkenylene or C₂₋₃-alkynylene group, which may in each case be unsubstituted or substituted with 1 or 2 substituents selected from the group consisting of F, Cl, Br, -C(=O)-O-CH₃ and -C(=O)-O-C₂H₅, or via a -CH₂-CH₂-O, -CH₂-O- or -CH₂-CH₂-CH₂-O group and/or may in each case be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, allyl, ethynyl, propynyl, -C≡C-Si(CH₃)₃, -C≡C-Si(C₂H₅)₃, -CH₂-O-CH₃, -CH₂-O-C₂H₅, -NH₂, -S-CH₃, -S-C₂H₅, -S(=O)-CH₃, -S(=O)₂-CH₃, -S(=O)-C₂H₅, -S(=O)₂-C₂H₅, -O-CH₃**,** -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-phenyl, pyrazolyl, -N(CH₃)₂, -N(C₂H₅)₂, -NH-CH₃, -NH-C₂H₅, -CH₂-O-C(=O)-phenyl, -NH-S(=O)₂-CH₃, -C(=O)-H, -C(=O)-CH₃, -C(=O)-C₂H₅, -NH-C(=O)-CH₃, -NH-C(=O)-C₂H₅, -O-C(=O)-phenyl, -C(=O)-NH₂, -C(=O)-NH-CH₃, -C(=O)-NH-C₂H₅, -C(=O)-NH-C(CH₃)₃, -C(=O)-N(C₂H₅)₂, -S(=O)-NH₂, [1 ,2,3]-thiadiazolyl, cyclopropyl, cyclobutyl, cyclopentyl, cyclohexyl, piperazinyl, pyrrolidinyl, piperidinyl, phenyl, furyl (furanyl), thiadiazolyl, thiophenyl (thienyl) and benzyl, wherein the cyclic substituents or the cyclic residues of these substituents themselves may in each case be substituted with 1, 2, 3, 4, or 5 substituents mutually independently selected from the group consisting of F, Cl, Br, I, -CN, -NO₂, -OH, -SH, -NH₂, methyl, ethyl, n-propyl, isopropyl, n-butyl, isobutyl, 2-butyl, tert.-butyl, n-pentyl, neopentyl, -O-CH₃, -O-C₂H₅, -O-C₃H₇, -O-C(CH₃)₃, -CF₃, -CHF₂, -CH₂F, -O-CF₃, -O-CHF₂ and -O-CH₂F;
a residue selected from the group consisting of naphthyl, indolizinyl, benzimidazolyl, tetrazolyl, triazinyl, isoxazolyl, phthalazinyl, carbazolyl, carbolinyl, diazanaphthyl, thienyl, furyl, pyrrolyl, pyrazolyl, pyrazinyl, pyranyl, triazolyl, pyridinyl, imidazolyl, indolyl, isoindolyl, benzo[b]furanyl, benzo[b]thiophenyl, benzo[d]thiazolyl, benzodiazolyl, benzotriazolyl, benzoxazolyl, benzisoxazolyl, thiazolyl, thiadiazolyl, oxazolyl, oxadiazolyl, isoxazolyl, pyridazinyl, indazolyl, quinoxalinyl, quinazolinyl, quinolinyl, naphthridinyl and isoquinolinyl, which may in each case be attached via a C₁₋₃ alkylene, C₂₋₃ alkenylene or C₂₋₃ alkynylene group and/or may be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, allyl, ethynyl, propynyl, -C≡C-Si(CH₃)₃, -C≡C-Si(C₂H₅)₃, -CH₂-O-CH₃, -CH₂-O-C₂H₅, -NH₂, -C(=O)-OH, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-phenyl, pyrazolyl, -N(CH₃)₂, -N(C₂H₅)₂, -NH-CH₃, -NH-C₂H₅, -CH₂-O-C(=O)-phenyl, -NH-S(=O)₂-CH₃, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -O-C(=O)-phenyl, -C(=O)-NH₂, -C(=O)-NH-CH₃, -C(=O)-N(CH₃)₂, -C(=O)-O-CH(CH₃)₂, -C(=O)-O-(CH₂)₃-CH₃, -C(=O)-O-phenyl, -O-C(=O)-CH₃, -O-C(=O)-C₂H₅, -C(=O)-NH-C₂H₅, -C(=O)-NH-C(CH₃)₃, -C(=O)-N(C₂H₅)₂, -C(=O)-NH-phenyl, -C(=O)-N(CH₃)-phenyl, -C(=O)-N(C₂H₅)-phenyl, -S(=O)-NH₂, [1,2,3]-thiadiazolyl, cyclopropyl, cyclobutyl, cyclopentyl, cyclohexyl, piperazinyl, pyrrolidinyl, piperidinyl, phenyl, furyl (furanyl), thiadiazolyl, thiophenyl (thienyl) and benzyl, wherein the cyclic substituents or the cyclic residues of these substituents themselves may in each case be substituted with 1, 2, 3, 4, or 5 substituents mutually independently selected from the group consisting of F, Cl, Br, I, -CN, -NO₂, -OH, -SH, -NH₂, methyl, ethyl, n-propyl, isopropyl, n-butyl, isobutyl, 2-butyl, tert.-butyl, n-pentyl, neopentyl, ethenyl, allyl, ethynyl, propynyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ and -S-CH₂F;
or -NH-C(=O)-R⁵;
R² denotes H or C₁₋₆-alkyl, which may in each case be unsubstituted or substituted with 1, 2, 3, 4 or 5 substituents mutually independently selected from the group consisting of F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O-C₂H₅, -C(=O)-O-C(CH₃)₃, -C(=O)-O-CH₂-phenyl, -C(=O)-NH-naphthyl, -N(CH₃)-phenyl, -N(C₂H₅)-phenyl, -N(C₂H₅)-(m- toluyl), -N(C₂H₅)-(p-toluyl), -N(CH₃)-(p-toluyl) and -NH-C(=O)-O-C(CH₃)₃; or
a residue selected from the group consisting of phenyl and naphthyl, which may in each case be attached via a C₁₋₃ alkylene, C₂₋₃ alkenylene or C₂₋₃ alkynylene group and/or may in each case be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, allyl, ethynyl, propynyl, -C≡C-Si(CH₃)_{3,} -C≡C-Si(C₂H₅)₃, -CH₂-O-CH₃, -CH₂-O-C₂H₅, -NH₂, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃ and -NH-C₂H₅;
or R¹ and R², together with the nitrogen atom joining them together, form a residue selected from the group consisting of 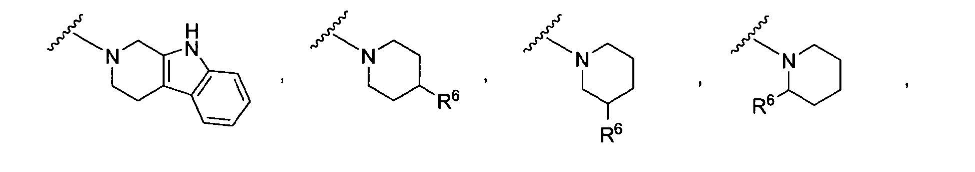 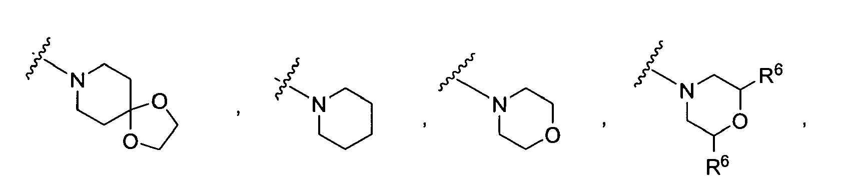 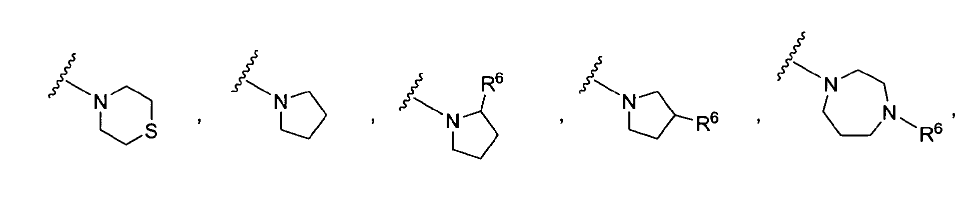 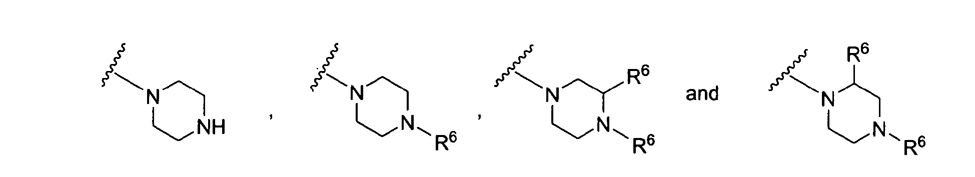
R³ denotes C₁₋₆ alkyl, which may in each case be unsubstituted or substituted with 1, 2, 3, 4 or 5 substituents mutually independently selected from the group consisting of F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O-C₂H₅ and -C(=O)-O-C(CH₃)₃; or
a residue selected from the group consisting of phenyl, isoxazolyl, thienyl, furyl, pyrrolyl, pyrazolyl, pyrazinyl, pyranyl, triazolyl, pyridinyl, imidazolyl, indolyl, isoindolyl, thiazolyl, thiadiazolyl, oxazolyl, oxadiazolyl, pyridazinyl and isoquinolinyl, which may in each case be attached via a C₁₋₃ alkylene, C₂₋₃ alkenylene or C₂₋₃ alkynylene group and/or may be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, allyl, ethynyl, propynyl, -CH₂-O-CH₃, -CH₂-O-C₂H₅, -NH₂, -C(=O)-OH, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-H, -C(=O)-CH₃, -C(=O)-C₂H₅, -C(=O)-NH₂, -C(=O)-NH-CH₃ and -C(=O)-N(CH₃)₂;
R⁴ denotes phenyl, which may in each case be unsubstituted or substituted with 1, 2, 3, 4 or 5 substituents mutually independently selected from the group consisting of F, Cl, Br, I, -CN, -CH₂-CN, -OH, -SH, -NH₂, -C(=O)-OH, methyl, ethyl, n-propyl, isopropyl, n-butyl, isobutyl, 2-butyl, tert.-butyl, n-pentyl, neopentyl, ethenyl, allyl, ethynyl, propynyl, -C≡C-Si(CH₃)₃, -C≡C-Si(C₂H₅)₃, -S-CH₃, -S-C₂H₅, -S-phenyl, -S-CH₂- phenyl, -O-CH₃, -O-C₂H₅, -O-C₃H₇, -O-C(CH₃)₃, -O-phenyl, -O-CH₂- phenyl, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-phenyl, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -S(=O)₂-NH₂, -S(=O)₂-NH-phenyl, [1,2,3]-thiadiazolyl, cyclopropyl, cyclobutyl, cyclopentyl, cyclohexyl, phenyl, benzyl and phenethyl, wherein the cyclic substituents or the cyclic residues of these substituents may themselves in each case be unsubstituted or substituted with 1, 2, 3, 4 or 5 substituents mutually independently selected from the group consisting of F, Cl, Br, I, -CN, -NO₂, -OH, -SH, -NH₂, -C(=O)-OH, methyl, ethyl, n-propyl, isopropyl, n-butyl, isobutyl, 2-butyl, tert.-butyl, n-pentyl, neopentyl, ethenyl, allyl, ethynyl, propynyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ and -S-CH₂F; or
a residue selected from the group consisting of thienyl, furyl, pyrrolyl, pyrazolyl, pyrazinyl, pyranyl, triazolyl, pyridinyl, imidazolyl, thiadiazolyl, oxadiazolyl and pyridazinyl, which may in each case be unsubstituted or substituted with 1, 2, 3, 4 or 5 substituents mutually independently selected from the group consisting of F, Cl, Br, I, -CN, -NO₂, -OH, -CH₂-CN, -SH, -NH₂, -C(=O)-OH, methyl, ethyl, n-propyl, isopropyl, n-butyl, isobutyl, 2-butyl, tert.-butyl, n-pentyl, neopentyl, ethenyl, allyl, ethynyl, propynyl, -S-CH₃, -S-C₂H₅, -S-phenyl, -S-CH₂-phenyl, -O-CH₃, -O-C₂H₅, -O-C₃H₇, -O-C(CH₃)₃, -O-phenyl, -O-CH₂-phenyl, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -S(=O)₂-NH₂, cyclopropyl, cyclobutyl, cyclopentyl, cyclohexyl, phenyl and benzyl, wherein the cyclic substituents or the cyclic residues of these substituents themselves may in each case be unsubstituted or substituted with 1, 2, 3, 4, or 5 substituents mutually independently selected from the group consisting of F, Cl, Br, I, -CN, -NO₂, -OH, -SH, -NH₂, -C(=O)-OH, methyl, ethyl, n-propyl, isopropyl, n-butyl, isobutyl, 2-butyl, tert.-butyl, n-pentyl, neopentyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ and -S-CH₂F;
R⁵ denotes a residue selected from the group consisting of phenyl, naphthyl, thienyl, furyl, pyrrolyl, pyrazolyl, pyrazinyl, pyranyl, triazolyl, pyridinyl, imidazolyl, indolyl, isoindolyl, thiazolyl, thiadiazolyl, oxazolyl, oxadiazolyl, isoxazolyl and pyridazinyl, which may in each case be attached via a C₁₋₃ alkylene, C₂₋₃ alkenylene or C₂₋₃ alkynylene group and/or may be unsubstituted or substituted with 1, 2, 3, 4 or 5 substituents mutually independently selected from the group consisting of F, Cl, Br, I, -CN, -CH₂-CN, -NO₂, -OH, -SH, methyl, ethyl, n-propyl, isopropyl, n-butyl, isobutyl, 2-butyl, tert.-butyl, n-pentyl, neopentyl, ethenyl, allyl, ethynyl, propynyl, -NH₂, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃ and -NH-C₂H₅;
R⁶ denotes -OH; F; Cl; Br; I; -SH; -NO₂; -NH₂; -NH-C(=O)-O-R⁷; -C(=O)-O-R⁸; -C(=O)-R⁹; C₁₋₆ alkyl, C₂₋₆ alkenyl or C₂₋₆ alkynyl, which may in each case be unsubstituted or substituted with 1, 2, 3, 4 or 5 substituents mutually independently selected from the group consisting of F, Cl, Br, I, -NO₂, -CN, -OH, -SH, -NH₂, -N(CH₃)₂, -C(=O)-pyrrolidinyl, -C(=O)-N(CH₃)- phenyl and -C(=O)-NH-CH(CH₃)₂, -N(C₂H₅)₂, -N(CH₃)(C₂H₅), -C(=O)-OH, -C(=O)-O-CH₃, -C(=O)-O-C₂H₅ and -C(=O)-O-C(CH₃)₃; C₃₋₇ cycloalkyl, C₅₋₆ cycloalkenyl, 5- to 7-membered heterocycloalkyl or 5- to 7-membered heterocycloalkenyl, which may in each case be unsubstituted or substituted with 1, 2, 3, 4 or 5 substituents mutually independently selected from the group consisting of F, Cl, Br, I, -CN, methyl, ethyl, n-propyl, isopropyl, n-butyl, 2-butyl, isobutyl, tert.-butyl, -OH, oxo, thioxo, -O-CH₃, -O-C₂H₅, -O-C₃H₇, -O-phenyl, -O-CH₂-phenyl, -NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂H₅, -CH₂-naphthyl, benzyl and phenyl and/or may in each case be attached via an unsubstituted C₁₋₃ alkylene, C₂₋₃ alkenylene or C₂₋₃ alkynylene group and/or may in each case comprise 1 or 2 heteroatom(s) mutually independently selected from the group consisting of oxygen, sulfur and nitrogen (NH) as ring member(s); or a residue selected from the group consisting of phenyl, naphthyl, indolizinyl, benzimidazolyl, tetrazolyl, triazinyl, isoxazolyl, phthalazinyl, carbazolyl, carbolinyl, diazanaphthyl, thienyl, furyl, pyrrolyl, pyrazolyl,
pyrazinyl, pyranyl, triazolyl, pyridinyl, imidazolyl, indolyl, isoindolyl, benzo[b]furanyl, benzo[b]thiophenyl, benzo[d]thiazolyl, benzodiazolyl, benzotriazolyl, benzoxazolyl, benzisoxazolyl, thiazolyl, thiadiazolyl, oxazolyl, oxadiazolyl, isoxazolyl, pyridazinyl, indazolyl, quinoxalinyl, quinazolinyl, quinolinyl, naphthridinyl, thieno[2,3-d]pyrimidinyl and isoquinolinyl, which may in each case be attached via a C₁₋₃ alkylene, C₂₋₃ alkenylene or C₂₋₃ alkynylene group and/or may be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, allyl, ethynyl, propynyl, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -C(=O)-O-CH(CH₃)₂, -C(=O)-O-(CH₂)₃-CH₃, -C(=O)-NH-C₂H₅, -C(=O)-NH-C(CH₃)₃, -C(=O)-N(C₂H₅)₂, cyclopropyl, cyclobutyl, cyclopentyl, cyclohexyl, phenyl, furyl (furanyl) and benzyl, wherein the cyclic substituents or the cyclic residues of these substituents themselves may in each case be substituted with 1, 2, 3, 4, or 5 substituents mutually independently selected from the group consisting of F, Cl, Br, I, -CN, -NO₂, -OH, -SH, -NH₂, methyl, ethyl, n-propyl, isopropyl, n-butyl, isobutyl, 2-butyl, tert.-butyl, n-pentyl, neopentyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ and -S-CH₂F
and R⁷, R⁸ and R⁹, mutually independently, in each case denote
C₁₋₆ alkyl, C₂₋₆ alkenyl or C₂₋₆ alkynyl, which may in each case be unsubstituted or substituted with 1, 2, 3, 4 or 5 substituents mutually independently selected from the group consisting of F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O-C₂H₅ and -C(=O)-O-C(CH₃)₃;
a residue selected from the group consisting of indolinyl, indanyl, (1,2,3,4)-tetrahydronaphthyl, (2,3)-dihydrobenzo[1.4]dioxinyl and benzo[1.3]dioxolyl, which may in each case be unsubstituted or substituted with 1, 2, 3, 4 or 5 substituents mutually independently selected from the group consisting of F, Cl, Br, I, -CN, methyl, ethyl, n-propyl, isopropyl, n-butyl, 2-butyl, isobutyl, tert.-butyl, -OH, oxo, thioxo, -O-CH₃, -O-C₂H₅, -O-C₃H₇, -O-phenyl, -O-CH₂-phenyl, -NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂H₅, -CH₂-naphthyl, benzyl and phenyl; or
a residue selected from the group consisting of phenyl, naphthyl, indolizinyl, benzimidazolyl, tetrazolyl, triazinyl, isoxazolyl, phthalazinyl, carbazolyl, carbolinyl, diazanaphthyl, thienyl, furyl, pyrrolyl, pyrazolyl, pyrazinyl, pyranyl, triazolyl, pyridinyl, imidazolyl, indolyl, isoindolyl, thiazolyl, thiadiazolyl, oxazolyl, oxadiazolyl, isoxazolyl and pyridazinyl, which may in each case be attached via a C₁₋₃ alkylene, C₂₋₃ alkenylene or C₂₋₃ alkynylene group and/or may be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, allyl, ethynyl, propynyl, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -C(=O)-O-CH(CH₃)₂, -C(=O)-O-(CH₂)₃-CH₃, -C(=O)-NH-C₂H₅, -C(=O)-NH-C(CH₃)₃, -C(=O)-N(C₂H₅)₂, cyclopropyl, cyclobutyl, cyclopentyl, cyclohexyl, phenyl, furyl (furanyl) and benzyl.

3. Compounds according to claim 1 or claim 2, **characterised in that**
R¹ denotes an alkyl residue selected from the group consisting of methyl, ethyl, n-propyl, isopropyl, n-butyl, 2-butyl, isobutyl, tert.-butyl, n-pentyl, sec.-pentyl, neopentyl, (3,3)-dimethylbutyl, 4-methyl-2-pentyl and n-hexyl, which may in each case be unsubstituted or substituted with 1, 2, 3, 4 or 5 substituents mutually independently selected from the group consisting of F, Cl, Br, I, -CN, -OH, -SH, -NH₂, -N(CH₃)₂, -N(C₂H₅)₂, -N(CH₃)(C₂H₅), -C(=O)-O-CH₃, -C(=O)-O-C₂H₅, -C(=O)-O-C(CH₃)₃, -C(=O)-O-CH₂-phenyl, -C(=O)-NH-naphthyl, -N(CH₃)-phenyl, -N(C₂H₅)- phenyl, -N(C₂H₅)-(m-toluyl), -N(C₂H₅)-(p-toluyl), -N(CH₃)-(p-toluyl) and -NH-C(=O)-O-C(CH₃)₃;
a heteroalkyl residue selected from the group consisting of -CH₂-O-CH₃, -CH₂-O-C₂H₅, -CH₂-CH₂-O-CH₃, -CH₂-CH₂-O-C₂H₅, -CH₂-CH₂-CH₂-O-CH₃ and -CH₂-CH₂-CH₂-O-C₂H₅, which may in each case be unsubstituted or substituted with 1, 2, 3, 4 or 5 substituents mutually independently selected from the group consisting of F, Cl and Br;
a residue selected from the group consisting of cyclopropyl, cyclobutyl, cyclopentyl, cyclohexyl, cycloheptyl, cyclohexenyl, cyclopentenyl, pyrrolidinyl, tetrahydrothiophenyl, tetrahydrofuranyl, piperidinyl, morpholinyl, piperazinyl, azepanyl, diazepanyl, (1,2,3,4)- tetrahydroquinolinyl, (1,2,3,4)-tetrahydroisoquinolinyl, (2,3)-dihydro- 1H-isoindolyl, indolinyl, indanyl, (1,2,3,4)-tetrahydronaphthyl, (2,3)-dihydrobenzo[1.4]dioxinyl and benzo[1.3]dioxolyl, which may in each case be unsubstituted or substituted with 1, 2, 3, 4 or 5 substituents mutually independently selected from the group consisting of F, Cl, Br, I, -CN, methyl, ethyl, n-propyl, isopropyl, n-butyl, 2-butyl, isobutyl, tert.-butyl, -OH, oxo, -O-CH₃, -O-C₂H₅, -O-C₃H₇, -O-phenyl, -O-CH₂-phenyl, -C(=O)-CH₃, -C(=O)-C₂H₅, -C(=O)-C(CH₃)₃, -C(=O)-NH-CH₃, -C(=O)-NH-C₂H₅, -CH₂-naphthyl, benzyl and phenyl and/or may in each case be attached via a -CH₂-, -CH(CH₃)-, -CH₂-CH₂-, -CH(CH₃)-CH₂-, -CH₂-CH(CH₃)- or -CH₂-CH₂-CH₂- group; phenyl, which may in each case be attached via a -CH₂-CH₂-O-, -CH₂-O-, -CH₂-CH₂-CH₂-O-, -CH[C(=O)-O-CH₃]-CH₂-, -CH[C(=O)-O-C₂H₅]-CH₂-, -CH₂-, -CH(CH₃)-, -CH₂-CH₂-, -CH(CH₃)-CH₂-, -CH₂-CH(CH₃)- or -CH₂-CH₂-CH₂- group and/or may in each case be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, allyl, ethynyl, propynyl, -NH₂, -S-CH₃, -S-C₂H₅ -O-CH₃, -O-C₂H₅, -O-C₃H₇, -O-C(CH₃)₃, -CF₃, -CHF₂, -CH₂F, -O-CF₃, -O-CHF₂, -O-CH₂F, -N(CH₃)₂, -N(C₂H₅)₂, -NH-CH₃, -NH-C₂H₅, -NH-S(=O)₂-CH₃, -C(=O)-H, -C(=O)-CH₃, -C(=O)-C₂H₅, -NH-C(=O)-CH₃, -NH-C(=O)-C₂H₅, -S(=O)-NH₂, [1,2,3]-thiadiazolyl, cyclopropyl, cyclobutyl, cyclopentyl, cyclohexyl, phenyl, furyl (furanyl), thiadiazolyl, thiophenyl (thienyl) and benzyl, wherein the cyclic substituents or the cyclic residues of these substituents themselves may in each case be substituted with 1, 2, 3, 4, or 5 substituents mutually independently selected from the group consisting of F, Cl, Br, I, -CN, -NO₂, methyl, ethyl, n-propyl, isopropyl, n-butyl, isobutyl, 2-butyl, tert.-butyl, -O-CH₃, -O-C₂H₅, -O-C₃H₇ and -O-C(CH₃)₃;
a residue selected from the group consisting of naphthyl, indolizinyl, benzimidazolyl, tetrazolyl, triazinyl, isoxazolyl, phthalazinyl, carbazolyl, carbolinyl, diazanaphthyl, thienyl, furyl, pyrrolyl, pyrazolyl, pyrazinyl, pyranyl, triazolyl, pyridinyl, imidazolyl, indolyl, isoindolyl, benzo[b]furanyl, benzo[b]thiophenyl, benzo[d]thiazolyl, benzodiazolyl, benzotriazolyl, benzoxazolyl, benzisoxazolyl, thiazolyl, thiadiazolyl, oxazolyl, oxadiazolyl, isoxazolyl, pyridazinyl, indazolyl, quinoxalinyl, quinazolinyl, quinolinyl, naphthridinyl and isoquinolinyl, which may in each case be attached via a -CH₂-, -CH(CH₃)-, -CH₂-CH₂-, -CH(CH₃)-CH₂-, -CH₂-CH(CH₃)- or -CH₂-CH₂-CH₂ group and/or may be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -C(=O)-NH₂, -C(=O)-NH-CH₃, -C(=O)-N(CH₃)₂, -C(=O)-O-CH(CH₃)₂ and -C(=O)-O-(CH₂)₃-CH₃,
or -NH-C(=O)-R⁵;
R² denotes H; an alkyl residue selected from the group consisting of methyl, ethyl, n-propyl, isopropyl, n-butyl, 2-butyl, isobutyl, tert.-butyl, n-pentyl, sec.-pentyl, neopentyl, (3,3)-dimethylbutyl, 4-methyl-2-pentyl and n-hexyl, which may in each case be unsubstituted; or
a residue selected from the group consisting of phenyl and naphthyl, which may in each case be attached via a -CH₂-, -CH(CH₃)-, -CH₂-CH₂-, -CH(CH₃)-CH₂-, -CH₂-CH(CH₃)- or -CH₂-CH₂-CH₂- group and/or may in each case be unsubstituted or substituted with 1, 2, 3, 4 or 5 substituents mutually independently selected from the group consisting of F, Cl, Br, I, -CN, methyl, ethyl, n-propyl, isopropyl, n-butyl, isobutyl, 2-butyl, tert.-butyl, n-pentyl, neopentyl, -O-CH₃, -O-C₂H₅, -O-C₃H₇, -O-C(CH₃)₃, -CF₃, -CHF₂ and -CH₂F; or R¹ and R², together with the nitrogen atom joining them together, form a residue selected from the group consisting of 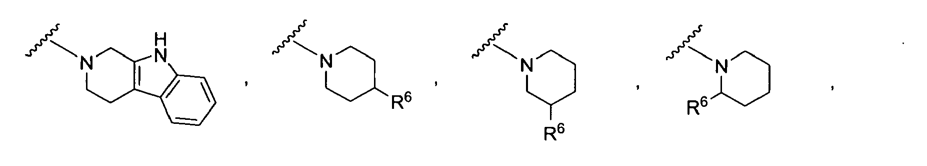 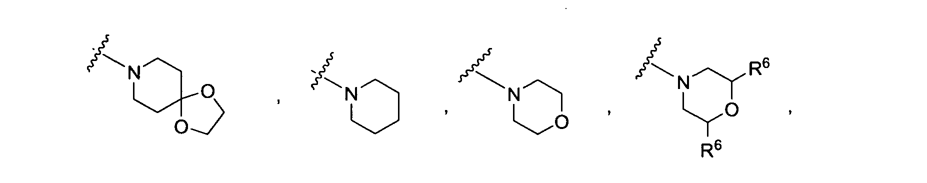 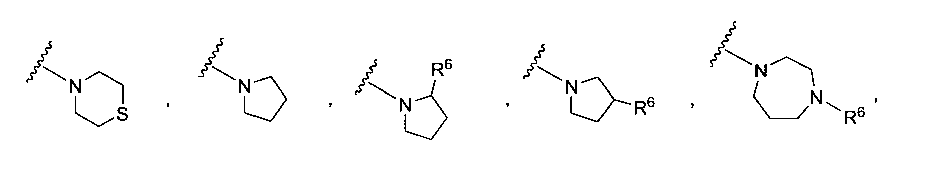 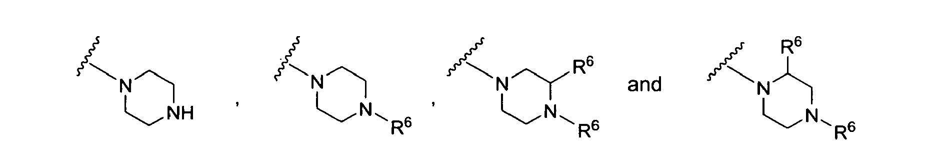
R³ denotes an alkyl residue selected from the group consisting of methyl, ethyl, n-propyl, isopropyl, n-butyl, 2-butyl, isobutyl, tert.-butyl, n-pentyl, sec.-pentyl, neopentyl, (3,3)-dimethylbutyl, 4-methyl-2-pentyl and n-hexyl, which may in each case be unsubstituted; or
a phenyl residue, which may in each case be attached via a -CH₂, -CH(CH₃)-, -CH₂-CH₂-, -CH(CH₃)-CH₂-, -CH₂-CH(CH₃)- or -CH₂-CH₂-CH₂ group and/or may in each case be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, allyl, ethynyl, propynyl, -CH₂-O-CH₃, -CH₂-O-C₂H₅, -NH₂, -C(=O)-OH, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂ and -O-CH₂F;
R⁴ denotes phenyl, which may in each case be unsubstituted or substituted with 1, 2, 3, 4 or 5 substituents mutually independently selected from the group consisting of F, Cl, Br, I, -CN, -CH₂-CN, -OH, -SH, -NH₂, -C(=O)-OH, methyl, ethyl, n-propyl, isopropyl, n-butyl, isobutyl, 2-butyl, tert.-butyl, n-pentyl, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂ and -NH-CH₃; or
a residue selected from the group consisting of thienyl, furyl, pyrrolyl, pyrazolyl, pyrazinyl, pyranyl, triazolyl, pyridinyl, imidazolyl, thiadiazolyl, oxadiazolyl and pyridazinyl, which may in each case be unsubstituted or substituted with 1, 2, 3, 4 or 5 substituents mutually independently selected from the group consisting of F, Cl, Br, I, -CN, -NO₂, -OH, -CH₂-CN, -SH, -NH₂, -C(=O)-OH, methyl, ethyl, n-propyl, isopropyl, n-butyl, isobutyl, 2-butyl, tert.-butyl, n-pentyl, neopentyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂ and -NH-CH₃;
R⁵ denotes a residue selected from the group consisting of phenyl and naphthyl, which may in each case be unsubstituted or substituted with 1, 2, 3, 4 or 5 substituents mutually independently selected from the group consisting of F, Cl, Br, I, methyl, ethyl, n-propyl, isopropyl, n-butyl, isobutyl, 2-butyl, tert.-butyl, n-pentyl, neopentyl, ethenyl, allyl, ethynyl, propynyl, -NH₂, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃ and -NH-C₂H₅;
R⁶ denotes -OH; F; Cl; Br; I; -SH; -NO₂; -NH₂; -NH-C(=O)-O-R⁷; -C(=O)-O-R⁸; -C(=O)-R⁹;
an alkyl residue selected from the group consisting of methyl, ethyl, n-propyl, isopropyl, n-butyl, 2-butyl, isobutyl, tert.-butyl, n-pentyl, sec.-pentyl, neopentyl, (3,3)-dimethylbutyl, 4-methyl-2-pentyl and n-hexyl, which may in each case be unsubstituted or substituted with 1, 2, 3, 4 or 5 substituents mutually independently selected from the group consisting of F, Cl, Br, I, -NO₂, -CN, -OH, -SH, -NH₂, -N(CH₃)₂, -N(C₂H₅)₂, -N(CH₃)(C₂H₅), -C(=O)-pyrrolidinyl, -C(=O)-N(CH₃)-phenyl and -C(=O)-NH-CH(CH₃)₂, -C(=O)-OH, -C(=O)-O-CH₃, -C(=O)-O-C₂H₅ and -C(=O)-O-C(CH₃)₃;
a residue selected from the group consisting of cyclopropyl, cyclobutyl, cyclopentyl, cyclohexyl, cycloheptyl, cyclohexenyl, cyclopentenyl, pyrrolidinyl, tetrahydrothiophenyl, tetrahydrofuranyl, piperidinyl, morpholinyl, piperazinyl, azepanyl and diazepanyl, which may in each case be unsubstituted or substituted with 1, 2, 3, 4 or 5 substituents mutually independently selected from the group consisting of methyl, ethyl, n-propyl, isopropyl, n-butyl, 2-butyl, isobutyl, tert.-butyl, -OH, -O-CH₃, -O-C₂H₅, -O-C₃H₇, -O-CF₃, -C(=O)-CH₃, -C(=O)-C₂H₅, -C(=O)-C(CH₃)₃, -C(=O)-NH-CH₃ and -C(=O)-NH-C₂H₅ and/or may in each case be attached via a -CH₂-, -CH(CH₃)-, -CH₂-CH₂-, -CH(CH₃)-CH₂-, -CH₂-CH(CH₃)- or -CH₂-CH₂-CH₂- group; or
a residue selected from the group consisting of phenyl, naphthyl, indolizinyl, benzimidazolyl, tetrazolyl, triazinyl, isoxazolyl, phthalazinyl, thienyl, furyl, pyrrolyl, pyrazolyl, pyrazinyl, pyranyl, triazolyl, pyridinyl, imidazolyl, indolyl, isoindolyl, thiazolyl, thiadiazolyl, oxazolyl, oxadiazolyl, isoxazolyl, pyridazinyl, thieno[2,3-d]pyrimidinyl and isoquinolinyl, which may in each case be attached via a CH₂-, -CH(CH₃)-, -CH₂-CH₂-, -CH(CH₃)-CH₂-, -CH₂-CH(CH₃)- or -CH₂-CH₂-CH₂ group and/or may be unsubstituted or substituted with 1, 2, 3, 4 or 5 substituents mutually independently selected from the group consisting of F, Cl, Br, I, -CN, -CH₂-CN, -NO₂, -OH, -SH, -O-phenyl, methyl, ethyl, n-propyl, isopropyl, n-butyl, isobutyl, 2-butyl, tert.-butyl, n-pentyl, neopentyl, ethenyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -C(=O)-H, -C(=O)-CH₃ and -C(=O)-C₂H₅;
and R⁷, R⁸ and R⁹, mutually independently, in each case denote
an alkyl residue selected from the group consisting of methyl, ethyl, n-propyl, isopropyl, n-butyl, 2-butyl, isobutyl, tert.-butyl, n-pentyl, sec.-pentyl, neopentyl, (3,3)-dimethylbutyl, 4-methyl-2-pentyl and n-hexyl, which may in each case be unsubstituted or substituted with 1, 2, 3, 4 or 5 substituents mutually independently selected from the group consisting of F, Cl, Br, I, -NO₂, -CN, -OH, -SH, -NH₂, -N(CH₃)₂, -N(C₂H₅)₂ and -N(CH₃)(C₂H₅);
a residue selected from the group consisting of indolinyl, indanyl, (1,2,3,4)-tetrahydronaphthyl, (2,3)-dihydrobenzo[1.4]dioxinyl and benzo[1.3]dioxolyl, which may in each case be unsubstituted;
or a residue selected from the group consisting of phenyl, naphthyl, isoxazolyl, thienyl, furyl, pyrrolyl, pyrazolyl, pyrazinyl, pyranyl, triazolyl, pyridinyl, imidazolyl, indolyl, isoindolyl, thiazolyl, thiadiazolyl, oxazolyl, oxadiazolyl, isoxazolyl and pyridazinyl, which may in each case be attached via a -CH₂-, -CH(CH₃)-, -CH₂-CH₂-, -CH(CH₃)-CH₂-, -CH₂-CH(CH₃)- or -CH₂-CH₂-CH₂- group and/or may in each case be unsubstituted or substituted with 1, 2, 3, 4 or 5 substituents mutually independently selected from the group consisting of F, Cl, Br, I, -CN, -NO₂, -O-phenyl, methyl, ethyl, n-propyl, isopropyl, n-butyl, isobutyl, 2-butyl, tert.-butyl, n-pentyl, neopentyl, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂ and -O-CH₂F;

4. Compounds according to one or more of claims 1 to 3, **characterised in that**
R¹ denotes an alkyl residue selected from the group consisting of methyl, ethyl, n-propyl, isopropyl, n-butyl, 2-butyl, isobutyl, tert.-butyl, n-pentyl, sec.-pentyl, neopentyl, (3,3)-dimethylbutyl, 4-methyl-2-pentyl and n-hexyl, which may in each case be unsubstituted or substituted with 1, 2 or 3 substituents mutually independently selected from the group consisting of -CN, -OH, -N(CH₃)₂, -N(C₂H₅)₂ -C(=O)-O-CH₃, -C(=O)-O-C₂H₅, -C(=O)-O-C(CH₃)₃, -C(=O)-O-CH₂-phenyl, -C(=O)-NH-naphthyl, -N(CH₃)-phenyl, -N(C₂H₅)-(m-toluyl) and -N(C₂H₅)-(p-toluyl);
a heteroalkyl residue selected from the group consisting of -CH₂-O-CH₃, -CH₂-O-C₂H₅, -CH₂-CH₂-O-CH₃, -CH₂-CH₂-O-C₂H₅, -CH₂-CH₂-CH₂-O-CH₃ and -CH₂-CH₂-CH₂-O-C₂H₅;
a residue selected from the group consisting of cyclopropyl, cyclobutyl, cyclopentyl, cyclohexyl, cycloheptyl, pyrrolidinyl, tetrahydrofuranyl, piperidinyl, morpholinyl, piperazinyl, azepanyl, dihydrofuran-2(3H)-only, indanyl and (1,2,3,4)-tetrahydronaphthyl, which may in each case be unsubstituted or substituted with 1, 2, 3, 4 or 5 substituents mutually independently selected from the group consisting of methyl, ethyl, n-propyl, isopropyl, n-butyl, 2-butyl, isobutyl, tert.-butyl, -CH₂-naphthyl, -O-phenyl, -O-CH₂-phenyl, benzyl and phenyl and/or may in each case be attached via a -CH₂, -CH(CH₃)-, -CH₂-CH₂-, -CH(CH₃)-CH₂-, -CH₂-CH(CH₃)- or -CH₂-CH₂-CH₂-group;
phenyl, which may in each case be attached via a -CH₂-CH₂-O-, -CH₂-O-, -CH₂-CH₂-CH₂-O-, -CH[C(=O)-O-CH₃]-CH₂-, -CH[C(=O)-O-C₂H₅]-CH₂-, -CH₂-, -CH(CH₃)-, -CH₂-CH₂-, -CH(CH₃)-CH₂-, -CH₂-CH(CH₃)- or -CH₂-CH₂-CH₂- group and/or may in each case be unsubstituted or substituted with 1, 2, 3, 4 or 5 substituents mutually independently selected from the group consisting of F, Cl, Br, I, -CH₂-CN, methyl, ethyl, n-propyl, isopropyl, n-butyl, isobutyl, 2-butyl, tert.-butyl, n-pentyl, neopentyl, -O-phenyl, -O-CH₃, -O-C₂H₅, -O-C₃H₇, -O-C(CH₃)₃, -CF₃, -CHF₂, -CH₂F, -O-CF₃, -O-CHF₂, -O-CH₂F, -N(CH₃)₂, -N(C₂H₅)₂, -S(=O)-NH₂ and [1,2,3]-thiadiazolyl;
a residue selected from the group consisting of thienyl, furyl, pyrrolyl, pyrazolyl, pyridinyl, imidazolyl, indolyl and isoindolyl, which may in each case be attached via a -CH₂, -CH(CH₃)-, -CH₂-CH₂-, -CH(CH₃)-CH₂-, -CH₂-CH(CH₃)- or -CH₂-CH₂-CH₂ group and/or may be unsubstituted or substituted with 1, 2 or 3 substituents mutually independently selected from the group consisting of -NO₂, -OH, methyl, ethyl, n-propyl, isopropyl, n-butyl, isobutyl, 2-butyl, tert.-butyl, n-pentyl, and neopentyl or
-NH-C(=O)-R⁵;
R² denotes H or an alkyl residue selected from the group consisting of methyl, ethyl, n-propyl, isopropyl, n-butyl, 2-butyl, isobutyl, tert.-butyl, n-pentyl, sec.-pentyl, neopentyl, (3,3)-dimethylbutyl, 4-methyl-2-pentyl and n-hexyl, which may in each case be unsubstituted; or
a residue selected from the group consisting of phenyl and naphthyl, which may in each case be attached via a -CH₂-, -CH(CH₃)-, -CH₂-CH₂-, -CH(CH₃)-CH₂-, -CH₂-CH(CH₃)- or -CH₂-CH₂-CH₂- group and/or may in each case be unsubstituted or substituted with 1, 2 or 3 substituents mutually independently selected from the group consisting of -O-CH₃, -O-C₂H₅, -O-C₃H₇ and -O-C(CH₃)₃; or
R¹ and R², together with the nitrogen atom joining them together, form a residue selected from the group consisting of 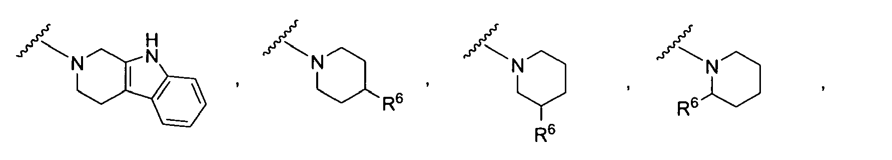 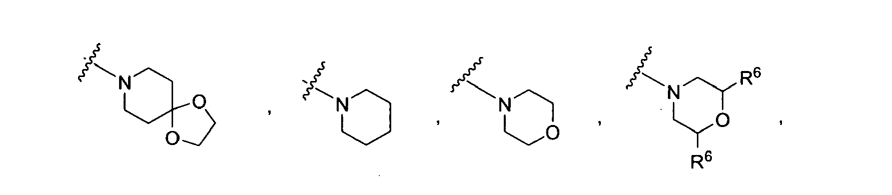 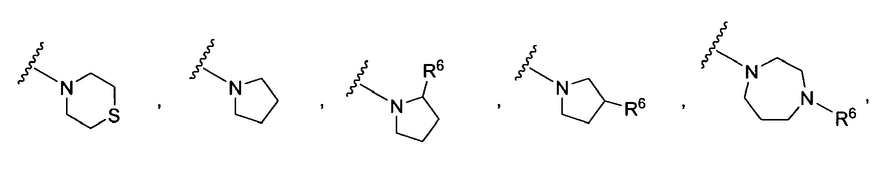 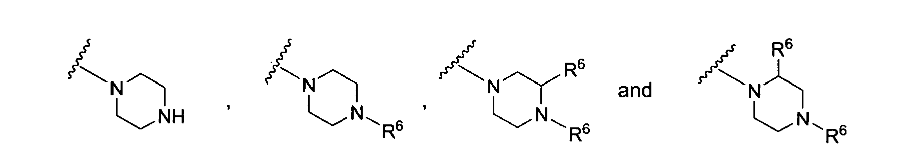
R³ denotes an alkyl residue selected from the group consisting of methyl, ethyl, n-propyl, isopropyl, n-butyl, 2-butyl, isobutyl, tert.-butyl, n-pentyl, sec.-pentyl, neopentyl, (3,3)-dimethylbutyl, 4-methyl-2-pentyl and n-hexyl, which may in each case be unsubstituted; or
a phenyl residue, which may in each case be attached via a -CH₂, -CH(CH₃)-, -CH₂-CH₂-, -CH(CH₃)-CH₂-, -CH₂-CH(CH₃)- or -CH₂-CH₂-CH₂ group and/or may in each case be unsubstituted or substituted with 1, 2, 3, 4 or 5 substituents mutually independently selected from the group consisting of F, Cl, Br, I, -CN, -CH₂-CN, -NO₂, methyl, ethyl, n-propyl, isopropyl, n-butyl, isobutyl, 2-butyl, tert.-butyl, n-pentyl, neopentyl, -O-CH₃, -O-C₂H₅, -O-C₃H₇, -O-C(CH₃)₃, -CF₃, -CHF₂, -CH₂F, -O-CF₃, -O-CHF₂ and -O-CH₂F;
R⁴ denotes phenyl, which may in each case be unsubstituted or substituted with 1, 2, 3, 4 or 5 substituents mutually independently selected from the group consisting of F, Cl, Br, I, methyl, ethyl, n-propyl, isopropyl, n-butyl, isobutyl, 2-butyl, tert.-butyl, n-pentyl, -O-CH₃, -O-C₂H₅, -O-C₃H₇, -O-C(CH₃)₃, -O-CF₃, -O-CHF₂ and -O-CH₂F; or
a residue selected from the group consisting of thienyl, furyl and pyrrolyl, which may in each case be unsubstituted or substituted with 1, 2, 3, 4 or 5 substituents mutually independently selected from the group consisting of F, Cl, Br, I, methyl, ethyl, n-propyl, isopropyl, n-butyl, isobutyl, 2-butyl, -O-CH₃, -O-C₂H₅, -O-C₃H₇ and -O-C(CH₃)₃;
R⁵ denotes a residue selected from the group consisting of phenyl and naphthyl, which may in each case be unsubstituted or substituted with 1, 2, 3, 4 or 5 substituents mutually independently selected from the group consisting of F, Cl, Br, I, methyl, ethyl, n-propyl, isopropyl, n-butyl, isobutyl, 2-butyl, tert.-butyl, -NH₂, -O-CH₃, -O-C₂H₅, -O-C₃H₇, -O-C(CH₃)₃, -N(CH₃)₂, -N(C₂H₅)₂, -NH-CH₃ and -NH-C₂H₅;
R⁶ denotes -OH; -NH-C(=O)-O-R⁷; -C(=O)-O-R⁸; -C(=O)-R⁹;
an alkyl residue selected from the group consisting of methyl, ethyl, n-propyl, isopropyl, n-butyl, 2-butyl, isobutyl, tert.-butyl, n-pentyl, sec.- pentyl, neopentyl, (3,3)-dimethylbutyl, 4-methyl-2-pentyl and n-hexyl, which may in each case be unsubstituted or substituted with 1, 2 or 3 substituents mutually independently selected from the group consisting of -OH, -N(CH₃)₂, -N(C₂H₅)₂, -C(=O)-pyrrolidinyl, -C(=O)-N(CH₃)-phenyl and -C(=O)-NH-CH(CH₃)₂;
a residue selected from the group consisting of cyclopropyl, cyclobutyl, cyclopentyl, cyclohexyl, cycloheptyl, pyrrolidinyl, piperidinyl and azepanyl, which may in each case be unsubstituted and/or may in each case be attached via a -CH₂-, -CH(CH₃)-, -CH₂-CH₂-, -CH(CH₃)-CH₂-, -CH₂-CH(CH₃)- or -CH₂-CH₂-CH₂- group; or
a residue selected from the group consisting of phenyl, naphthyl, thienyl, furyl, pyrazinyl, pyridinyl, pyridazinyl and thieno[2,3-d]pyrimidinyl, which may in each case be attached via a -CH₂, -CH(CH₃)-, -CH₂-CH₂-, -CH(CH₃)-CH₂-, -CH₂-CH(CH₃)- or -CH₂-CH₂-CH₂ group and/or may in each case be unsubstituted or substituted with 1, 2, 3, 4 or 5 substituents mutually independently selected from the group consisting of F, Cl, Br, I, -CN, methyl, tert.-butyl, -O-CH₃, -O-C₂H₅, -CF₃, -C(=O)-CH₃ and -C(=O)-C₂H₅;
and R⁷, R⁸ and R⁹, mutually independently, in each case denote
an alkyl residue selected from the group consisting of methyl, ethyl, n-propyl, isopropyl, n-butyl, 2-butyl, isobutyl, tert.-butyl, and n-pentyl, which may in each case be unsubstituted;
a residue selected from the group consisting of (2,3)-dihydrobenzo[1.4]dioxinyl and benzo[1.3]dioxolyl, which may in each case be unsubstituted; or
a residue selected from the group consisting of phenyl, thienyl, furyl and pyrazinyl, which may in each case be attached via a -CH₂, -CH(CH₃)-, -CH₂-CH₂-, -CH(CH₃)-CH₂-, -CH₂-CH(CH₃)- or -CH₂-CH₂-CH₂ group and/or may in each case be unsubstituted or substituted with 1, 2, 3, 4 or 5 substituents mutually independently selected from the group consisting of F, Cl, Br, I, methyl, ethyl, n-propyl, isopropyl, n-butyl, isobutyl, 2-butyl, tert.-butyl, n-pentyl, neopentyl, -O-CH₃, -O-C₂H₅, -O-C₃H₇ and -O-C(CH₃)₃.

5. Compounds according to one or more of claims 1 to 4, **characterised in that**
R¹ denotes a residue selected from the group consisting of -CH₂-CH₂-N(C₂H₅)-(m-toluyl), -CH₂-CH₂-N(C₂H₅)-(p-toluyl), -CH₂-CH₂-CH₂-N(CH₃)-phenyl, -CH₂-CH₂-CH₂-N(CH₃)₂, -CH₂-CH₂-CH₂-N(C₂H₅)₂, -CH(CH₃)-CH₂-CH₂-CH(CH₃)₂, -CH₂-CH₂-CN, -CH₂-CH₂-OH, -CH₂-CH₂-N(CH₃)₂, -CH₂-CH₂-N(C₂H₅)₂, -CH₂-CH₂-C(CH₃)₃, n-pentyl, n-butyl, methyl, ethyl, n-propyl, -CH(CH₃)-C(=O)-O-CH₂-phenyl, -CH[CH(CH₃)₂]-C(=O)-O-C(CH₃)₃, -CH₂-C(=O)-O-CH₂-phenyl, -CH₂-CH₂-C(=O)-O-C(CH₃)₃, -CH₂-CH₂-C(=O)-O-C₂H₅, -CH₂-C(=O)-NH-naphthyl and -CH₂-CH₂-CH₂-O-CH₃;
a residue selected from the group consisting of cyclopropyl, cyclobutyl, cyclopentyl, cyclohexyl, cycloheptyl, pyrrolidinyl, tetrahydrofuranyl, piperidinyl, morpholinyl, piperazinyl, azepanyl, dihydrofuran-2(3H)-only, indanyl and (1,2,3,4)-tetrahydronaphthyl, which may in each case be unsubstituted or substituted with 1, 2, 3, 4 or 5 substituents mutually independently selected from the group consisting of methyl, ethyl, -CH₂-naphthyl, -O-phenyl, -O-CH₂-phenyl and benzyl and/or may in each case be attached via a -CH₂, -CH₂-CH₂- or -CH₂-CH₂-CH₂ group;
phenyl, which may in each case be attached via a -CH₂-CH₂-O-, -CH[C(=O)-O-CH₃]-CH₂-, -CH[C(=O)-O-C₂H₅]-CH₂-, -CH₂-, -CH(CH₃)-, -CH₂-CH₂-, -CH(CH₃)-CH₂-, -CH₂-CH(CH₃)- or -CH₂-CH₂-CH₂- group and/or may in each case be unsubstituted or substituted with 1, 2, 3, 4 or 5 substituents mutually independently selected from the group consisting of F, Cl, Br, I, -CH₂-CN, methyl, ethyl, n-propyl, isopropyl, n-butyl, isobutyl, 2-butyl, tert.-butyl, -O-phenyl, -O-CH₃, -O-C₂H₅, -CF₃, -O-CF₃, -N(CH₃)₂, -N(C₂H₅)₂, -S(=O)-NH₂ and [1,2,3]-thiadiazolyl;
a residue selected from the group consisting of thienyl, furyl, pyridinyl, imidazolyl, indolyl and isoindolyl, which may in each case be attached via a -CH₂, -CH₂-CH₂- or -CH₂-CH₂-CH₂ group and/or may be unsubstituted or substituted with 1, 2 or 3 substituents mutually independently selected from the group consisting of -NO₂, -OH, methyl, ethyl and n-propyl
or -NH-C(=O)-R⁵;
R² denotes H or an alkyl residue selected from the group consisting of methyl, ethyl and n-propyl, which is in each case unsubstituted; or a residue selected from the group consisting of phenyl and naphthyl, which may in each case be attached via a -CH₂ group and/or may in each case be unsubstituted or substituted with 1, 2 or 3 substituents mutually independently selected from the group consisting of -O-CH₃, -O-C₂H₅, -O-C₃H₇ and -O-C(CH₃)₃; or
R¹ and R², together with the nitrogen atom joining them together, form a residue selected from the group consisting of 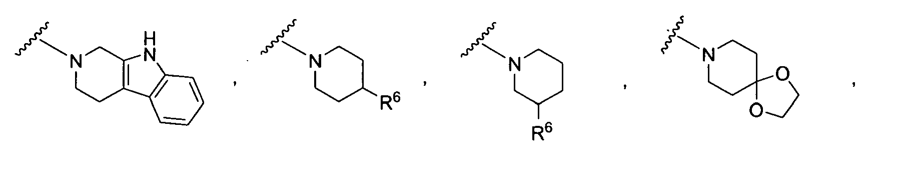 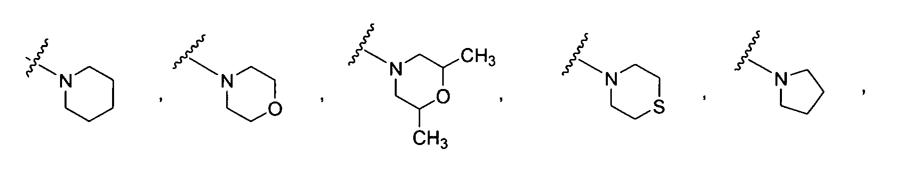 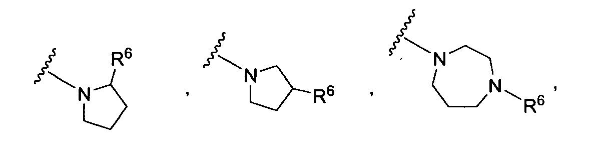 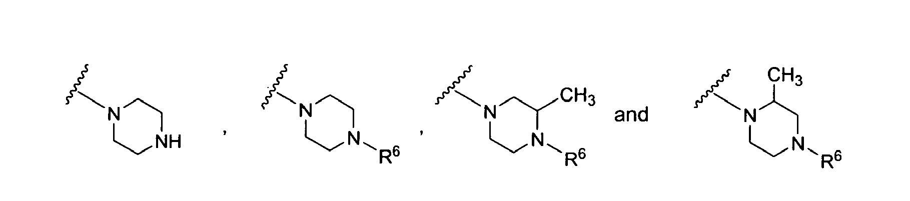
R³ denotes an alkyl residue selected from the group consisting of methyl, ethyl, n-propyl, isopropyl, n-butyl, 2-butyl, isobutyl and tert.-butyl, which may in each case be unsubstituted; or
a phenyl residue, which may in each case be attached via a -CH₂ group and/or may in each case be unsubstituted or substituted with 1, 2, 3, 4 or 5 substituents mutually independently selected from the group consisting of F, Cl, Br, methyl, ethyl, n-propyl, -O-CH₃, -O-C₂H₅, and -CF₃;
R⁴ denotes phenyl, which may in each case be unsubstituted or substituted with 1, 2, 3, 4 or 5 substituents mutually independently selected from the group consisting of F, Cl, Br, methyl, ethyl, n-propyl, -O-CH₃ and -O-C₂H₅;
or a residue selected from the group consisting of thienyl, furyl and pyrrolyl, which may in each case be unsubstituted;
R⁵ denotes a residue selected from the group consisting of phenyl and naphthyl, which may in each case be unsubstituted or substituted with 1, 2, 3, 4 or 5 substituents mutually independently selected from the group consisting of F, Cl, Br, methyl, ethyl, -O-CH₃, -O-C₂H₅, -N(CH₃)₂, -N(C₂H₅)₂, -NH-CH₃ and -NH-C₂H₅;
R⁶ denotes -OH; -NH-C(=O)-O-R⁷; -C(=O)-O-R⁸; -C(=O)-R⁹;
a residue selected from the group consisting of methyl, ethyl, n-propyl, isopropyl, n-butyl, -CH₃-CH(CH₃)₂, -CH₂-CH₂-OH, -CH₂-CH₂-N(CH₃)₂, -CH₂-CH₂-N(C₂H₅), -CH₂-C(=O)-pyrrolidinyl, -CH₂-C(=O)-NH-CH(CH₃)₂ and -CH₂-C(=O)-N(CH₃)-phenyl;
a residue selected from the group consisting of cyclopropyl, cyclobutyl, cyclopentyl, cyclohexyl, cycloheptyl, pyrrolidinyl, piperidinyl and azepanyl, which may in each case be unsubstituted and/or may in each case be attached via a -CH₂- group; or
a residue selected from the group consisting of phenyl, naphthyl, thienyl, pyrazinyl, pyridinyl and thieno[2,3-d] pyrimidinyl, which may in each case be attached via a -CH₂, -CH₂-CH₂- or -CH₂-CH₂-CH₂ group and/or may in each case be unsubstituted or substituted with 1, 2, 3, 4 or 5 substituents mutually independently selected from the group consisting of F, Cl, Br, -CN, methyl, tert.-butyl, -O-CH₃, -O-C₂H₅, -CF₃, -C(=O)-CH₃ and -C(=O)-C₂H₅;
R⁷ denotes an alkyl residue selected from the group consisting of methyl, ethyl, n-propyl, isopropyl, n-butyl, 2-butyl, isobutyl, tert.-butyl, and n-pentyl, which is in each case unsubstituted;
R⁸ denotes an alkyl residue selected from the group consisting of methyl, ethyl, n-propyl, isopropyl, n-butyl, 2-butyl, isobutyl, tert.-butyl, and n-pentyl, which is in each case unsubstituted; or
a phenyl or benzyl residue; and
R⁹ denotes a residue selected from the group consisting of (2,3)-dihydrobenzo[1.4]dioxinyl and benzo[1.3]dioxolyl, which is in each case unsubstituted;
or a residue selected from the group consisting of phenyl, thienyl, furyl and pyrazinyl, which may in each case be attached via a -CH₂, -CH₂-CH₂- or -CH₂-CH₂-CH₂ group and/or may in each case be unsubstituted or substituted with 1, 2, 3, 4 or 5 substituents mutually independently selected from the group consisting of F, Cl, Br, methyl, ethyl, -O-CH₃ and -O-C₂H₅.

6. Compounds according to one of claims 1 to 5 selected from the group consisting of
| | |
|---|---|
| [1] | 1-benzyl-4-methyl-3-p-tolyl-1H-pyrrole-2-carboxylic acid 4-[1,2,3]thiadiazol-4-ylbenzylamide, |
| [2] | 3-furan-2-yl-1,4-dimethyl-1H-pyrrole-2-carboxylic acid 4-sulfamoylbenzylamide, |
| [3] | 1-(4-fluorobenzyl)-3-furan-2-yl-4-methyl-1H-pyrrole-2-carboxylic acid 2,4-dimethoxybenzylamide, |
| [4] | 1-butyl-4-methyl-3-p-tolyl-1H-pyrrole-2-carboxylic acid (2-pyridin-2-ylethyl)-amide, |
| [5] | 1-(4-fluorobenzyl)-3-(4-methoxyphenyl)-4-methyl-1H-pyrrole-2-carboxylic acid [2-(5-hydroxy-1H-indol-3-yl)-ethyl]-amide, |
| [6] | (1,4-dimethyl-3-p-tolyl-1H-pyrrole-2-yl)-(4-pyrrolidin-1-ylpiperidin-1-yl)-methanone, |
| [7] | 1-benzyl-3-furan-2-yl-4-methyl-1H-pyrrole-2-carboxylic acid 4-bromo-2-fluorobenzylamide, |
| [8] | [1-benzyl-3-(4-chlorophenyl)-4-methyl-1H-pyrrol-2-yl]-[4-(2-chlorophenyl)-piperazin-1-yl]-methanone, |
| [9] | 1,4-dimethyl-3-phenyl-1H-pyrrole-2-carboxylic acid (3-dimethylamino-propyl)-amide, |
| [10] | 1,4-dimethyl-3-p-tolyl-1H-pyrrole-2-carboxylic acid (2-pyrrotidin-1-ylethyl)-amide, |
| [11] | 1-(4-fluorobenzyl)-3-furan-2-yl-4-methyl-1H-pyrrole-2-carboxylic acid 4-dimethylaminobenzylamide, |
| [12] | 1-[4-(1-benzyl-4-methyl-3-phenyl-1H-pyrrole-2-carbonyl)-piperazin-1-yl]-2-phenylethanone, |
| [13] | 1-benzyl-3-furan-2-yl-4-methyl-1H-pyrrole-2-carboxylic acid 2,5-difluorobenzylamide, |
| [14] | 1-(2,6-dichlorobenzyl)-4-methyl-3-p-tolyl-1H-pyrrole-2-carboxylic acid 3,5-difluorobenzylamide, |
| [15] | 1-(2-bromobenzyl)-3-(4-chlorophenyl)-4-methyl-1H-pyrrole-2-carboxylic acid (2-methylcyclohexyl)-amide, |
| [16] | 1,4-dimethyl-3-p-tolyl-1H-pyrrole-2-carboxylic acid (3-morpholin-4-ylpropyl)-amide, |
| [17] | 1-butyl-4-methyl-3-p-tolyl-1H-pyrrole-2-carboxylic acid (1,3-dimethylbutyl)-amide, |
| [18] | [4-(2,4-dimethylphenyl)-piperazin-1-yl]-[4-methyl-1-(4-methylbenzyl)-3-p-tolyl-1H-pyrrol-2-yl]-methanone, |
| [19] | 1-(2,6-dichlorobenzyl)-4-methyl-3-p-tolyl-1H-pyrrole-2-carboxylic acid methyl-(2-pyridin-2-ylethyl)-amide, |
| [20] | 1,4-dimethyl-3-phenyl-1H-pyrrole-2-carboxylic acid (2-cyano-ethyl)-methylamide, |
| [21] | 1-(2,6-dichlorobenzyl)-4-methyl-3-p-tolyl-1H-pyrrole-2-carboxylic acid (3-imidazol-1-ylpropyl)-amide, |
| [22] | 1-(4-fluorobenzyl)-3-(4-methoxyphenyl)-4-methyl-1H-pyrrole-2-carboxylic acid (1,3-dimethylbutyl)-amide, |
| [23] | 3-(4-methoxyphenyl)-1,4-dimethyl-1H-pyrrole-2-carboxylic acid 2-ethoxybenzylamide, |
| [24] | (4-cycloheptylpiperazin-1-yl)-(1,4-dimethyl-3-p-tolyl-1H-pyrrol-2-yl)-methanone, |
| [25] | 1-(4-fluorobenzyl)-3-(4-methoxyphenyl)-4-methyl-1H-pyrrole-2-carboxylic acid (2-dimethylaminoethyl)-amide, |
| [26] | 1-benzyl-4-methyl-3-phenyl-1H-pyrrole-2-carboxylic acid (pyridin-2-ylmethyl)-amide, |
| [27] | 3-(4-chlorophenyl)-1-ethyl-4-methyl-1H-pyrrole-2-carboxylic acid [2-(4-chlorophenyl)-propyl]-amide, |
| [28] | 3-(4-chlorophenyl)-1-(2-fluorobenzyl)-4-methyl-1H-pyrrole-2-carboxylic acid 3,5-difluorobenzylamide, |
| [29] | 2-{[1-(4-fluorobenzyl)-3-(4-methoxyphenyl)-4-methyl-1H-pyrrole-2-carbonyl]-amino}-propionic acid benzyl ester, |
| [30] | (1-benzyl-4-methyl-3-p-tolyl-1H-pyrrol-2-yl)-[4-(2-dimethylamino-ethyl)-piperazin-1-yl]-methanone, |
| [31] | 3-furan-2-yl-1-(4-methoxybenzyl)-4-methyl-1H-pyrrole-2-carboxylic acid (3,3-dimethylbutyl)-amide, |
| [32] | 3-(4-chlorophenyl)-1-isobutyl-4-methyl-1H-pyrrole-2-carboxylic acid 4-dimethylaminobenzylamide, |
| [33] | 3-(4-chlorophenyl)-1-ethyl-4-methyl-1H-pyrrole-2-carboxylic acid (2-pyridin-2-ylethyl)-amide, |
| [34] | 3-(4-chlorophenyl)-1-ethyl-4-methyl-1H-pyrrole-2-carboxylic acid 2,3-dimethoxybenzylamide, |
| [35] | 1-(4-fluorobenzyl)-3-(4-methoxyphenyl)-4-methyl-1H-pyrrole-2-carboxylic acid (2-thiophen-2-ylethyl)-amide, |
| [36] | 2-{[3-(4-chlorophenyl)-1-ethyl-4-methyl-1H-pyrrole-2-carbonyl]-amino}-3-methylbutyric acid tert.-butyl ester, |
| [37] | 3-furan-2-yl-1-(4-methoxybenzyl)-4-methyl-1H-pyrrole-2-carboxylic acid (1,2,3,4-tetrahydro-naphthalen-1-yl)-amide, |
| [38] | 3-furan-2-yl-4-methyl-1-(4-trifluoromethylbenzyl)-1H-pyrrole-2-carboxylic acid (2-pyrrolidin-1-ylethyl)-amide, |
| [39] | {[1-(4-methoxybenzyl)-4-methyl-3-p-tolyl-1H-pyrrole-2-carbonyl]-methylamino}-acetic acid benzyl ester, |
| [40] | 3-furan-2-yl-1-(4-methoxybenzyl)-4-methyl-1H-pyrrole-2-carboxylic acid [2-(1-methylpyrrolidin-2-yl)-ethyl]-amide, |
| [41] | [4-(5-bromo-2-ethoxybenzyl)-piperazin-1-yl]-(1,4-dimethyl-3-phenyl-1H-pyrrol-2-yl)-methanone, |
| [42] | (1-benzyl-3-furan-2-yl-4-methyl-1H-pyrrol-2-yl)-[4-(3-fluoro-4-methoxybenzyl)-piperazin-1-yl]-methanone, |
| [43] | 3-{[3-(4-chlorophenyl)-1-ethyl-4-methyl-1H-pyrrole-2-carbonyl]-amino}-propionic acid tert.-butyl ester, |
| [44] | 1-benzyl-4-methyl-3-phenyl-1H-pyrrole-2-carboxylic acid 3-methoxy-benzylamide, |
| [45] | (1-benzyl-3-furan-2-yl-4-methyl-1H-pyrrol-2-yl)-[4-(5-bromo-2-ethoxybenzyl)-piperazin-1-yl]-methanone, |
| [46] | [3-(4-chlorophenyl)-1,4-dimethyl-1H-pyrrol-2-yl]-(4-o-tolylpiperazin-1-yl)-methanone, |
| [47] | 1-(4-fluorobenzyl)-3-(4-methoxyphenyl)-4-methyl-1H-pyrrole-2-carboxylic acid [2-(4-ethoxyphenyl)-ethyl]-amide, |
| [48] | [1-(2,6-dichlorobenzyl)-4-methyl-3-p-tolyl-1H-pyrrol-2-yl]-(4-hydroxypiperidin-1-yl)-methanone, |
| [49] | (1,4-dimethyl-3-p-tolyl-1H-pyrrol-2-yl)-[4-(2,4,6-trimethoxybenzyl)-piperazin-1-yl]-methanone, |
| [50] | 1-(4-fluorobenzyl)-3-furan-2-yl-4-methyl-1H-pyrrole-2-carboxylic acid (2-morpholin-4-ylethyl)-amide, |
| [51] | 1-benzyl-3-(4-chlorophenyl)-4-methyl-1H-pyrrole-2-carboxylic acid [2-(4-fluorophenyl)-ethyl]-amide, |
| [52] | [4-(2,5-dimethylphenyl)-piperazin-1-yl]-[3-(4-methoxyphenyl)-1,4-dimethyl-1H-pyrrol-2-yl]-methanone, |
| [53] | 3-(4-methoxyphenyl)-1,4-dimethyl-1H-pyrrole-2-carboxylic acid (3-imidazol-1-ylpropyl)-amide, |
| [54] | 1-(4-fluorobenzyl)-3-(4-methoxyphenyl)-4-methyl-1H-pyrrole-2-carboxylic acid [2-(3-trifluoromethylphenyl)-ethyl]-amide, |
| [55] | [3-(4-chlorophenyl)-1-(2-fluorobenzyl)-4-methyl-1H-pyrrol-2-yl]-[4-(5-methylpyrazin-2-carbonyl)-piperazin-1-yl]-methanone, |
| [56] | 3-(4-chlorophenyl)-1-isobutyl-4-methyl-1H-pyrrole-2-carboxylic acid 3-fluoro-5-trifluoromethylbenzylamide, |
| [57] | [3-(4-chlorophenyl)-1,4-dimethyl-1H-pyrrol-2-yl]-(4-pyridin-4-ylpiperazin-1-yl)-methanone, |
| [58] | 3-(4-chlorophenyl)-1-(2-fluorobenzyl)-4-methyl-1H-pyrrole-2-carboxylic acid (3-imidazol-1-ylpropyl)-amide, |
| [59] | (1,4-dimethyl-3-phenyl-1H-pyrrol-2-yl)-[4-(3-fluoro-4-methoxybenzyl)-piperazin-1-yl]-methanone, |
| [60] | 1-benzyl-3-furan-2-yl-4-methyl-1H-pyrrole-2-carboxylic acid [2-(4-bromophenyl)-ethyl]-amide, |
| [61] | (4-cycloheptylpiperazin-1-yl)-(3-furan-2-yl-1,4-dimethyl-1H-pyrrol-2-yl)-methanone, |
| [62] | 1-benzyl-4-methyl-3-phenyl-1H-pyrrole-2-carboxylic acid [2-(4-methoxy-phenoxy)-ethyl]-amide, |
| [63] | 1-(4-fluorobenzyl)-3-furan-2-yl-4-methyl-1H-pyrrole-2-carboxylic acid (3-dimethylamino-propyl)-amide, |
| [64] | [1-(4-fluorobenzyl)-3-(4-methoxyphenyl)-4-methyl-1H-pyrrol-2-yl]-(2-pyrrolidin-1-ylmethylpyrrolidin-1-yl)-methanone, |
| [65] | 3-(4-chlorophenyl)-1-isobutyl-4-methyl-1H-pyrrole-2-carboxylic acid (3-diethylamino-propyl)-amide, |
| [66] | [3-(4-chlorophenyl)-1,4-dimethyl-1H-pyrrol-2-yl]-[4-(2-fluoro-5-methoxybenzyl)-piperazin-1-yl]-methanone, |
| [67] | 3-(4-methoxyphenyl)-1,4-dimethyl-1H-pyrrole-2-carboxylic acid pentylamide, |
| [68] | (1-butyl-4-methyl-3-p-tolyl-1H-pyrrol-2-yl)-[4-(2,5-dimethoxybenzyl)-piperazin-1-yl]-methanone, |
| [69] | [4-(2-diethylamino-ethyl)-piperazin-1-yl]-[4-methyl-1-(4-methylbenzyl)-3-p-tolyl-1H-pyrrol-2-yl]-methanone, |
| [70] | [4-(3-chlorophenyl)-piperazin-1-yl]-[3-(4-methoxyphenyl)-1,4-dimethyl-1H-pyrrol-2-yl]-methanone, |
| [71] | [1-(2-bromobenzyl)-3-(4-chlorophenyl)-4-methyl-1H-pyrrol-2-yl]-(4-isopropylpiperazin-1-yl)-methanone, |
| [72] | [4-(2-dimethylamino-ethyl)-piperazin-1-yl]-[1-(4-fluorobenzyl)-3-(4-methoxyphenyl)-4-methyl-1H-pyrrol-2-yl]-methanone, |
| [73] | 3-(4-chlorophenyl)-1-(2-fluorobenzyl)-4-methyl-1H-pyrrole-2-carboxylic acid 2,4-dimethoxybenzylamide, |
| [74] | 1-benzyl-4-methyl-3-p-tolyl-1H-pyrrole-2-carboxylic acid (2-pyrrolidin-1-ylethyl)-amide, |
| [75] | [1-(4-fluorobenzyl)-3-furan-2-yl-4-methyl-1H-pyrrol-2-yl]-[4-(3-fluoro-4-methoxybenzyl)-piperazin-1-yl]-methanone, |
| [76] | 3-(4-methoxyphenyl)-1,4-dimethyl-1H-pyrrole-2-carboxylic acid 2,4-dimethoxybenzylamide, |
| [77] | 1-benzyl-4-methyl-3-phenyl-1H-pyrrole-2-carboxylic acid (2-pyrrolidin-1-ylethyl)-amide, |
| [78] | 3-(4-chlorophenyl)-1,4-dimethyl-1H-pyrrole-2-carboxylic acid [3-(2-methylpiperidin-1-yl)-propyl]-amide, |
| [79] | 1-(4-fluorobenzyl)-3-furan-2-yl-4-methyl-1H-pyrrole-2-carboxylic acid cyclohexylamide, |
| [80] | 3-furan-2-yl-1,4-dimethyl-1H-pyrrole-2-carboxylic acid 4-fluoro-2-trifluoromethylbenzylamide, |
| [81] | 1-benzyl-3-furan-2-yl-4-methyl-1H-pyrrole-2-carboxylic acid (1-naphthalen-2-ylethyl)-amide, |
| [82] | 3-(4-chlorophenyl)-1-ethyl-4-methyl-1H-pyrrole-2-carboxylic acid (furan-2-ylmethyl)-amide, |
| [83] | 1-benzyl-4-methyl-3-phenyl-1H-pyrrole-2-carboxylic acid (3-dimethylamino-propyl)-methylamide, |
| [84] | [3-(4-methoxyphenyl)-1,4-dimethyl-1H-pyrrol-2-yl]-(4-p-tolylpiperazin-1-yl)-methanone, |
| [85] | 1-(4-methoxybenzyl)-4-methyl-3-p-tolyl-1H-pyrrole-2-carboxylic acid (thiophen-2-ylmethyl)-amide, |
| [86] | 4-methyl-1-(4-methylbenzyl)-3-p-tolyl-1H-pyrrole-2-carboxylic acid phenethylamide, |
| [87] | 1,4-dimethyl-3-p-tolyl-1H-pyrrole-2-carboxylic acid 3,5-difluorobenzyl-amide, |
| [88] | 3-(4-methoxyphenyl)-1,4-dimethyl-1H-pyrrole-2-carboxylic acid indan-1-ylamide, |
| [89] | 1-benzyl-4-methyl-3-phenyl-1H-pyrrole-2-carboxylic acid 4-dimethyl-aminobenzylamide, |
| [90] | 1-(4-fluorobenzyl)-3-furan-2-yl-4-methyl-1H-pyrrole-2-carboxylic acid (1-naphthalen-2-ylmethylpyrrolidin-3-yl)-amide, |
| [91] | [3-furan-2-yl-4-methyl-1-(4-trifluoromethylbenzyl)-1H-pyrrol-2-yl]-(4-methyl-[1,4]diazepan-1-yl)-methanone, |
| [92] | [1-(2-bromobenzyl)-3-(4-chlorophenyl)-4-methyl-1H-pyrrol-2-yl]-[4-(3-trifluoromethylphenyl)-piperazin-1-yl]-methanone, |
| [93] | [3-furan-2-yl-1-(4-methoxybenzyl)-4-methyl-1H-pyrrole-2-yl]-(4-thiophen-3-ylmethylpiperazin-1-yl)-methanone, |
| [94] | 1,4-dimethyl-3-p-tolyl-1H-pyrrole-2-carboxylic acid (2-benzyloxy-cyclohexyl)-amide, |
| [95] | 2-{4-[1-benzyl-3-(4-chlorophenyl)-4-methyl-1H-pyrrole-2-carbonyl]-piperazin-1-yl}-N-isopropylacetamide, |
| [96] | (2,6-dimethylmorpholin-4-yl)-[3-(4-methoxyphenyl)-1,4-dimethyl-1H-pyrrol-2-yl]-methanone, |
| [97] | 3-furan-2-yl-1-(4-methoxybenzyl)-4-methyl-1H-pyrrole-2-carboxylic acid (thiophen-2-ylmethyl)-amide, |
| [98] | 1,4-dimethyl-3-p-tolyl-1H-pyrrole-2-carboxylic acid (furan-2-ylmethyl)-amide, |
| [99] | 4-methyl-1-(4-methylbenzyl)-3-p-tolyl-1H-pyrrole-2-carboxylic acid (3-dimethylamino-propyl)-amide, |
| [100] | 1-(4-fluorobenzyl)-3-(4-methoxyphenyl)-4-methyl-1H-pyrrole-2-carboxylic acid (2-oxo-tetrahydro-furan-3-yl)-amide, |
| [101] | 3-(4-chlorophenyl)-1,4-dimethyl-1H-pyrrole-2-carboxylic acid (2-phenoxy-ethyl)-amide, |
| [102] | 3-(4-(1-benzyl-4-methyl-3-p-tolyl-1H-pyrrole-2-carbonyl)piperazin-1-yl)pyrazine-2-carbonitrile |
| [103] | 2-{[3-(4-methoxyphenyl)-1,4-dimethyl-1H-pyrrole-2-carbonyl]-amino}-3-phenylpropionic acid methyl ester, |
| [104] | 3-(4-chlorophenyl)-1,4-dimethyl-1H-pyrrole-2-carboxylic acid (2-morpholin-4-ylethyl)-amide, |
| [105] | 3-furan-2-yl-1,4-dimethyl-1H-pyrrole-2-carboxylic acid indan-2-ylamide, |
| [106] | (1-butyl-4-methyl-3-p-tolyl-1H-pyrrol-2-yl)-[4-(2-fluorophenyl)-piperazin-1-yl]-methanone, |
| [107] | [4-(3-chlorophenyl)-piperazin-1-yl]-(1,4-dimethyl-3-p-tolyl-1H-pyrrol-2-yl)-methanone, |
| [108] | [1-(4-fluorobenzyl)-3-furan-2-yl-4-methyl-1H-pyrrol-2-yl]-(4-naph-thalen-2-ylmethylpiperazin-1-yl)-methanone, |
| [109] | 3-(4-chlorophenyl)-1-isobutyl-4-methyl-1H-pyrrole-2-carboxylic acid p-tolylamide, |
| [110] | (1-benzyl-4-methyl-3-p-tolyl-1H-pyrrol-2-yl)-[4-(2-hydroxy-ethyl)-piperazin-1-yl]-methanone, |
| [111] | 1-butyl-4-methyl-3-p-tolyl-1H-pyrrole-2-carboxylic acid [2-(2,5-dimethoxyphenyl)-ethyl]-amide, |
| [112] | 1-butyl-4-methyl-3-p-tolyl-1H-pyrrole-2-carboxylic acid (3-imidazol-1-ylpropyl)-amide, |
| [113] | 1-benzyl-3-(4-chlorophenyl)-4-methyl-1H-pyrrole-2-carboxylic acid [2-(3,4-dimethoxyphenyl)-ethyl]-methylamide, |
| [114] | 4-methyl-1-(4-methylbenzyl)-3-p-tolyl-1H-pyrrole-2-carboxylic acid (3-morpholin-4-ylpropyl)-amide, |
| [115] | 4-methyl-1-(4-methylbenzyl)-3-p-tolyl-1H-pyrrole-2-carboxylic acid 4-fluoro-2-trifluoromethylbenzylamide, |
| [116] | 2-{[1-benzyl-3-(4-chlorophenyl)-4-methyl-1H-pyrrole-2-carbonyl]-amino}-3-(4-chlorophenyl)-propionic acid ethyl ester, |
| [117] | 1-benzyl-4-methyl-3-phenyl-1H-pyrrole-2-carboxylic acid 3-fluoro-4-trifluoromethylbenzylamide, |
| [118] | 1-(4-bromobenzyl)-4-methyl-3-p-tolyl-1H-pyrrole-2-carboxylic acid (2-pyridin-2-ylethyl)-amide, |
| [119] | 1-(4-fluorobenzyl)-3-furan-2-yl-4-methyl-1H-pyrrole-2-carboxylic acid benzyl-(2-hydroxy-ethyl)-amide, |
| [120] | 2-[4-(1-butyl-4-methyl-3-p-tolyl-1H-pyrrole-2-carbonyl)-piperazin-1-yl]-N-methyl-N-phenylacetamide, |
| [121] | [4-methyl-1-(4-methylbenzyl)-3-p-tolyl-1H-pyrrol-2-yl]-(4-phenylpiperazin-1-yl)-methanone, |
| [122] | 1-benzyl-4-methyl-3-p-tolyl-1H-pyrrole-2-carboxylic acid (1-benzyl-pyrrolidin-3-yl)-amide, |
| [123] | [1-(4-fluorobenzyl)-3-furan-2-yl-4-methyl-1H-pyrrol-2-yl]-[4-(3-trifluoro-methylphenyl)-piperazin-1-yl]-methanone, |
| [124] | 1-(4-methoxybenzyl)-4-methyl-3-p-tolyl-1H-pyrrole-2-carboxylic acid butylamide, |
| [125] | 1-benzyl-4-methyl-3-p-tolyl-1H-pyrrole-2-carboxylic acid [2-(2-fluoro-phenyl)-ethyl]-amide, |
| [126] | 4-methyl-1-(4-methylbenzyl)-3-p-tolyl-1H-pyrrole-2-carboxylic acid 3,4-dimethoxybenzylamide, |
| [127] | 3-(4-chlorophenyl)-1-isobutyl-4-methyl-1H-pyrrole-2-carboxylic acid (2-diethylamino-ethyl)-amide, |
| [128] | 1-{4-[3-(4-chlorophenyl)-1-ethyl-4-methyl-1H-pyrrole-2-carbonyl]-piperazin-1-yl}-2-phenylethanone, |
| [129] | 1-(4-fluorobenzyl)-4-methyl-3-p-tolyl-1H-pyrrole-2-carboxylic acid 3-trifluoromethoxybenzylamide, |
| [130] | [1-benzyl-3-(4-chlorophenyl)-4-methyl-1H-pyrrol-2-yl]-(3-methyl-piperidin-1-yl)-methanone, |
| [131] | 1-(4-fluorobenzyl)-4-methyl-3-p-tolyl-1H-pyrrole-2-carboxylic acid (2-p-tolylethyl)-amide, |
| [132] | 1-(4-methoxybenzyl)-4-methyl-3-p-tolyl-1H-pyrrole-2-carboxylic acid [2-(2-methyl-5-nitro-imidazol-1-yl)-ethyl]-amide, |
| [133] | 1,4-dimethyl-3-p-tolyl-1H-pyrrole-2-carboxylic acid 2,3-dimethoxybenzylamide, |
| [134] | 1-(2-bromobenzyl)-3-(4-chlorophenyl)-4-methyl-1H-pyrrole-2-carboxylic acid [2-(ethyl-m-tolylamino)-ethyl]-amide, |
| [135] | 1-(4-fluorobenzyl)-3-furan-2-yl-4-methyl-1H-pyrrole-2-carboxylic acid (4-isopropylphenyl)-amide, |
| [136] | 5-chloro-2-methoxybenzoic acid N'-[1-(4-fluorobenzyl)-3-(4-methoxy-phenyl)-4-methyl-1H-pyrrole-2-carbonyl]-hydrazide, |
| [137] | (1-benzyl-4-methyl-3-p-tolyl-1H-pyrrol-2-yl)-[1,4']bipiperidinyl-1'-ylmethanone, |
| [138] | 3-furan-2-yl-1,4-dimethyl-1H-pyrrole-2-carboxylic acid (4-butylphenyl)-amide, |
| [139] | 1-benzyl-4-methyl-3-phenyl-1H-pyrrole-2-carboxylic acid [2-(1H-indol-3-yl)-ethyl]-amide, |
| [140] | [4-(4-tert-butylbenzyl)-piperazin-1-yl]-[1-(4-fluorobenzyl)-4-methyl-3-p-tolyl-1H-pyrrol-2-yl]-methanone, |
| [141] | [1-(4-fluorobenzyl)-4-methyl-3-p-tolyl-1H-pyrrol-2-yl]-morpholin-4-ylmethanone, |
| [142] | 3-[(1-benzyl-3-furan-2-yl-4-methyl-1H-pyrrole-2-carbonyl)-amino]-propionic acid ethyl ester, |
| [143] | 1,4-dimethyl-3-phenyl-1H-pyrrole-2-carboxylic acid (naphthalen-2-ylcarbamoylmethyl)-amide, |
| [144] | [3-(4-chlorophenyl)-1,4-dimethyl-1H-pyrrol-2-yl]-[4-(2-ethoxyphenyl)-piperazin-1-yl]-methanone, |
| [145] | 1-(4-fluorobenzyl)-3-furan-2-yl-4-methyl-1H-pyrrole-2-carboxylic acid (4-cyanomethylphenyl)-amide, |
| [146] | 4-[1-benzyl-3-(4-chlorophenyl)-4-methyl-1H-pyrrole-2-carbonyl]-piperazine-1-carboxylic acid tert.-butyl ester, |
| [147] | (1,4-dioxa-8-aza-spiro[4.5]dec-8-yl)-[1-(4-fluorobenzyl)-3-furan-2-yl-4-methyl-1H-pyrrol-2-yl]-methanone, |
| [148] | 3-furan-2-yl-4-methyl-1-(4-trifluoromethylbenzyl)-1H-pyrrole-2-carboxylic acid pentylamide, |
| [149] | 3-furan-2-yl-1-(4-methoxybenzyl)-4-methyl-1H-pyrrole-2-carboxylic acid [3-(methylphenylamino)-propyl]-amide, |
| [150] | 1-(2,6-dichlorobenzyl)-4-methyl-3-p-tolyl-1H-pyrrole-2-carboxylic acid (2-azepan-1-ylethyl)-amide, |
| [151] | 1-(4-bromobenzyl)-4-methyl-3-p-tolyl-1H-pyrrole-2-carboxylic acid cyclopentylamide, |
| [152] | [1-(2,6-dichlorobenzyl)-4-methyl-3-p-tolyl-1H-pyrrol-2-yl]-[4-(2,4-dimethoxyphenyl)-piperazin-1-yl]-methanone, |
| [153] | 3-(4-chlorophenyl)-1-(2-fluorobenzyl)-4-methyl-1H-pyrrole-2-carboxylic acid [2-(2-chlorophenoxy)-ethyl]-amide, |
| [154] | 1-(2,6-dichlorobenzyl)-4-methyl-3-p-tolyl-1H-pyrrole-2-carboxylic acid (3,3-dimethylbutyl)-amide, |
| [155] | 1,4-dimethyl-3-p-tolyl-1H-pyrrole-2-carboxylic acid (2-benzyloxy-cyclohexyl)-amide, |
| [156] | [4-methyl-1-(4-methylbenzyl)-3-p-tolyl-1H-pyrrol-2-yl]-thiomorpholin-4-ylmethanone, |
| [157] | 3-(4-chlorophenyl)-1-isobutyl-4-methyl-1H-pyrrole-2-carboxylic acid (3-dimethylamino-propyl)-methylamide, |
| [158] | [4-(2,5-dimethylphenyl)-piperazin-1-yl]-[3-furan-2-yl-1-(4-methoxy-benzyl)-4-methyl-1H-pyrrol-2-yl]-methanone, |
| [159] | 5-chloro-2-methoxybenzoic acid N'-[3-(4-methoxyphenyl)-1,4-dimethyl-1H-pyrrole-2-carbonyl]-hydrazide, |
| [160] | 2-(3,4-difluorophenyl)-1-{4-[1-(4-fluorobenzyl)-3-furan-2-yl-4-methyl-1H-pyrrole-2-carbonyl]-piperazin-1-yl}-ethanone, |
| [161] | 2-{4-[1-(4-fluorobenzyl)-3-furan-2-yl-4-methyl-1H-pyrrole-2-carbonyl]-piperazin-1-yl}-N-isopropylacetamide, |
| [162] | 3-furan-2-yl-4-methyl-1-(4-trifluoromethylbenzyl)-1H-pyrrole-2-carboxylic acid [1-(3-methoxyphenyl)-ethyl]-amide, |
| [163] | 2-[(1-benzyl-3-furan-2-yl-4-methyl-1H-pyrrole-2-carbonyl)-amino]-3-(4-chlorophenyl)-propionic acid methyl ester, |
| [164] | [3-furan-2-yl-1-(4-methoxybenzyl)-4-methyl-1H-pyrrol-2-yl]-(4-methyl-piperazin-1-yl)-methanone, |
| [165] | 1,4-dimethyl-3-phenyl-1H-pyrrole-2-carboxylic acid (1-benzyl-pyrrolidin-3-yl)-amide, |
| [166] | (1,4-dimethyl-3-p-tolyl-1H-pyrrol-2-yl)-(4-thieno[2,3-d]pyrimidin-4-ylpiperazin-1-yl)-methanone, |
| [167] | 4-methyl-1-(4-methylbenzyl)-3-p-tolyl-1H-pyrrole-2-carboxylic acid (3-methoxypropyl)-amide, |
| [168] | [3-(4-methoxyphenyl)-1,4-dimethyl-1H-pyrrol-2-yl]-[4-(5-trifluoromethyl-pyridin-2-yl)-piperazin-1-yl]-methanone, |
| [169] | 1-(4-bromobenzyl)-4-methyl-3-p-tolyl-1H-pyrrole-2-carboxylic acid (3-morpholin-4-ylpropyl)-amide, |
| [170] | 1,4-dimethyl-3-p-tolyl-1H-pyrrole-2-carboxylic acid [2-(2,3-dimethoxy-phenyl)-ethyl]-amide, |
| [171] | [1-(4-methoxybenzyl)-4-methyl-3-p-tolyl-1H-pyrrol-2-yl]-(3-methyl-4-p-tolylpiperazin-1-yl)-methanone, |
| [172] | [3-(4-chlorophenyl)-1-ethyl-4-methyl-1H-pyrrol-2-yl]-[4-(2,4-dimethoxy-phenyl)-piperazin-1-yl]-methanone, |
| [173] | 2-{4-[1-(2-bromobenzyl)-3-(4-chlorophenyl)-4-methyl-1H-pyrrole-2-carbonyl]-piperazin-1-yl}-benzonitrile, |
| [174] | 1-(4-bromobenzyl)-4-methyl-3-p-tolyl-1H-pyrrole-2-carboxylic acid (1-ethylpyrrolidin-2-ylmethyl)-amide, |
| [175] | 3-(4-chlorophenyl)-1-isobutyl-4-methyl-1H-pyrrole-2-carboxylic acid [2-(2-chlorophenoxy)-ethyl]-amide, |
| [176] | 3-(4-chlorophenyl)-1,4-dimethyl-1H-pyrrole-2-carboxylic acid [2-(1H-indol-3-yl)-ethyl]-amide, |
| [177] | 1-(4-fluorobenzyl)-4-methyl-3-p-tolyl-1H-pyrrole-2-carboxylic acid (naphthalen-2-ylcarbamoylmethyl)-amide, |
| [178] | 4-(1-benzyl-3-furan-2-yl-4-methyl-1H-pyrrole-2-carbonyl)-piperazin-1-carboxylic acid benzyl ester, |
| [179] | 1,4-dimethyl-3-phenyl-1H-pyrrole-2-carboxylic acid (1-benzyl-pyrrolidin-3-yl)-amide, |
| [180] | [3-(4-chlorophenyl)-1-(2-fluorobenzyl)-4-methyl-1H-pyrrol-2-yl]-[4-(4-trifluoromethylphenyl)-piperazin-1-yl]-methanone, |
| [181] | [3-(4-chlorophenyl)-1-isobutyl-4-methyl-1H-pyrrol-2-yl]-(4-phenyl-piperazin-1-yl)-methanone, |
| [182] | 2-{4-[1-butyl-3-(4-chlorophenyl)-4-methyl-1H-pyrrole-2-carbonyl]-piperazin-1-yl}-1-pyrrolidin-1-ylethanone, |
| [183] | [1-benzyl-3-(4-chlorophenyl)-4-methyl-1H-pyrrol-2-yl]-[4-(2-chloro-4-fluorobenzoyl)-piperazin-1-yl]-methanone, |
| [184] | (4-benzoylpiperidin-1-yl)-[1-(4-methoxybenzyl)-4-methyl-3-p-tolyl-1H-pyrrol-2-yl]-methanone, |
| [185] | 3-furan-2-yl-1,4-dimethyl-1H-pyrrole-2-carboxylic acid ethylpyridin-4-ylmethylamide, |
| [186] | 1-(4-bromobenzyl)-4-methyl-3-p-tolyl-1H-pyrrole-2-carboxylic acid (2-pyrrolidin-1-ylethyl)-amide, |
| [187] | [3-(4-chlorophenyl)-1-isobutyl-4-methyl-1H-pyrrol-2-yl]-[4-(4-methyl-benzoyl)-piperazin-1-yl]-methanone, |
| [188] | [1-benzyl-3-(4-chlorophenyl)-4-methyl-1H-pyrrol-2-yl]-(4-thieno-[2,3-d]pyrimidin-4-ylpiperazin-1-yl)-methanone, |
| [189] | 1-(1,4-dimethyl-3-phenyl-1H-pyrrole-2-carbonyl)-hydroxypiperidine-3-carboxylic acid ethyl ester, |
| [190] | 1,4-dimethyl-3-phenyl-1H-pyrrole-2-carboxylic acid (1-phenylethyl)-amide, |
| [191] | [3-furan-2-yl-1-(4-methoxybenzyl)-4-methyl-1H-pyrrol-2-yl]-(1,3,4,9-tetrahydro-b-carbolin-2-yl)-methanone, |
| [192] | 1-{4-[1-benzyl-3-(4-chlorophenyl)-4-methyl-1H-pyrrole-2-carbonyl]-piperazin-1-yl}-2-(3,4-difluorophenyl)-ethanone, |
| [193] | [1-(4-bromobenzyl)-4-methyl-3-p-tolyl-1H-pyrrol-2-yl]-[4-(3-phenyl-propyl)-piperazin-1-yl]-methanone, |
| [194] | 1-(2-bromobenzyl)-3-(4-chlorophenyl)-4-methyl-1H-pyrrole-2-carboxylic acid [2-(1H-indol-3-yl)-ethyl]-methylamide, |
| [195] | 1-(4-{4-[1-(4-bromobenzyl)-4-methyl-3-p-tolyl-1H-pyrrole-2-carbonyl]-piperazin-1-yl}-phenyl)-ethanone, |
| [196] | 1,4-dimethyl-3-phenyl-1H-pyrrole-2-carboxylic acid 4-fluoro-2-trifluoromethylbenzylamide, |
| [197] | 4-methyl-1-(4-methylbenzyl)-3-p-tolyl-1H-pyrrole-2-carboxylic acid (4-phenoxyphenyl)-amide, |
| [198] | (4-hydroxy-piperidin-1-yl)-[1-(4-methoxybenzyl)-4-methyl-3-p-tolyl-1H-pyrrol-2-yl]-methanone, |
| [199] | 4-diethylaminobenzoic acid N'-(1-benzyl-4-methyl-3-p-tolyl-1H-pyrrole-2-carbonyl)-hydrazide, |
| [200] | [3-(4-chlorophenyl)-1-isobutyl-4-methyl-1H-pyrrol-2-yl]-[4-(3-chloro-phenyl)-piperazin-1-yl]-methanone, |
| [201] | 1-(4-bromobenzyl)-4-methyl-3-p-tolyl-1H-pyrrole-2-carboxylic acid [2-(3,4-dichlorophenyl)-ethyl]-amide, |
| [202] | 3-(4-chlorophenyl)-1-ethyl-4-methyl-1H-pyrrole-2-carboxylic acid [2-(1H-indol-3-yl)-ethyl]-amide, |
| [203] | [1-(4-fluorobenzyl)-4-methyl-3-p-tolyl-1H-pyrrol-2-yl]-[4-(5-trifluoro-methylpyridin-2-yl)-piperazin-1-yl]-methanone, |
| [204] | 1,4-dimethyl-3-phenyl-1H-pyrrole-2-carboxylic acid 3-trifluoromethoxy-benzylamide, |
| [205] | [1-(4-fluorobenzyl)-4-methyl-3-p-tolyl-1H-pyrrol-2-yl]-[4-(4-methyl-benzoyl)-piperazin-1-yl]-methanone, |
| [206] | [4-(3,4-dichlorophenyl)-piperazin-1-yl]-[1-(4-fluorobenzyl)-4-methyl-3-p-tolyl-1H-pyrrol-2-yl]-methanone, |
| [207] | 3-(4-chlorophenyl)-1-ethyl-4-methyl-1H-pyrrole-2-carboxylic acid 2,6-dimethoxybenzylamide, |
| [208] | 4-methyl-1-(4-methylbenzyl)-3-p-tolyl-1H-pyrrole-2-carboxylic acid [2-(3,4-dimethoxyphenyl)-ethyl]-amide, |
| [209] | 1-{4-[3-(4-chlorophenyl)-1-isobutyl-4-methyl-1H-pyrrole-2-carbonyl]-piperazin-1-yl}-2-(3,4-difluorophenyl)-ethanone, |
| [210] | [1-(4-fluorobenzyl)-4-methyl-3-p-tolyl-1H-pyrrol-2-yl]-[4-(2-methoxy-phenyl)-piperidin-1-yl]-methanone, |
| [211] | [3-furan-2-yl-4-methyl-1-(4-trifluoromethylbenzyl)-1H-pyrrol-2-yl]-thiomorpholin-4-ylmethanone, |
| [212] | 1,4-dimethyl-3-phenyl-1H-pyrrole-2-carboxylic acid (2-thiophen-2-ylethyl)-amide, |
| [213] | 4-diethylaminobenzoic acid N'-(1-butyl-4-methyl-3-p-tolyl-1H-pyrrole-2-carbonyl)-hydrazide, |
| [214] | {1-[1-(4-fluorobenzyl)-3-(4-methoxyphenyl)-4-methyl-1H-pyrrole-2-carbonyl]-pyrrolidin-3-yl}-carbamic acid tert.-butyl ester, |
| [215] | 2-{4-[3-(4-chlorophenyl)-1-isobutyl-4-methyl-1H-pyrrole-2-carbonyl]-piperazin-1-yl}-1-pyrrolidin-1-ylethanone, |
| [216] | [4-(2,3-dihydrobenzo[1,4]dioxine-2-carbonyl)-piperazin-1-yl]-(1,4-dimethyl-3-phenyl-1H-pyrrol-2-yl)-methanone, |
| [217] | 3-furan-2-yl-1,4-dimethyl-1H-pyrrole-2-carboxylic acid 3,4-dimethoxybenzylamide, |
| [218] | [3-furan-2-yl-4-methyl-1-(4-trifluoromethylbenzyl)-1H-pyrrol-2-yl]-(4-pyridin-2-ylpiperazin-1-yl)-methanone, |
| [219] | [4-(furan-2-carbonyl)-piperazin-1-yl]-[3-furan-2-yl-4-methyl-1-(4-trifluoro-methylbenzyl)-1H-pyrrol-2-yl]-methanone, |
| [220] | 3-furan-2-yl-4-methyl-1-(4-trifluoromethylbenzyl)-1H-pyrrole-2-carboxylic acid (4-tert.-butylphenyl)-amide, |
| [221] | 2-{4-[3-(4-chlorophenyl)-1-isobutyl-4-methyl-1H-pyrrole-2-carbonyl]-piperazin-1-yl}-benzonitrile, |
| [222] | (3-furan-2-yl-1,4-dimethyl-1H-pyrrol-2-yl)-[4-(4-trifluoromethylpyridin-2-yl)-piperazin-1-yl]-methanone, |
| [223] | 1,4-dimethyl-3-phenyl-1H-pyrrole-2-carboxylic acid 3,5-difluorobenzyl-amide, |
| [224] | 1,4-dimethyl-3-phenyl-1H-pyrrole-2-carboxylic acid (pyridin-4-ylmethyl)-amide, |
| [225] | 2-[4-(3-furan-2-yl-1,4-dimethyl-1H-pyrrole-2-carbonyl)-piperazin-1-yl]-benzonitrile, |
| [226] | 3-furan-2-yl-1,4-dimethyl-1H-pyrrole-2-carboxylic acid methyl-(2-pyridin-2-ylethyl)-amide, |
| [227] | 3-furan-2-yl-1,4-dimethyl-1H-pyrrole-2-carboxylic acid (3-methoxy-benzyl)-(tetrahydro-furan-2-ylmethyl)-amide, |
| [228] | 3-furan-2-yl-1,4-dimethyl-1H-pyrrole-2-carboxylic acid (4-phenoxyphenyl)-amide, |
| [229] | 3-furan-2-yl-1,4-dimethyl-1H-pyrrole-2-carboxylic acid pentylamide, |
| [230] | 2-(3,4-difluorophenyl)-1-[4-(3-furan-2-yl-1,4-dimethyl-1H-pyrrole-2-carbonyl)-piperazin-1-yl]-ethanone, |
| [231] | 3-furan-2-yl-1,4-dimethyl-1H-pyrrole-2-carboxylic acid phenylamide, |
| [232] | [4-(2,4-dimethoxyphenyl)-piperazin-1-yl]-(3-furan-2-yl-1,4-dimethyl-1H-pyrrol-2-yl)-methanone, |
| [233] | 3-furan-2-yl-1,4-dimethyl-1H-pyrrole-2-carboxylic acid [2-(3,4-dichloro-phenyl)-ethyl]-amide, |
| [234] | 3-furan-2-yl-1,4-dimethyl-1H-pyrrole-2-carboxylic acid [2-(2-chloro-phenoxy)-ethyl]-amide, |
| [235] | 3-furan-2-yl-1,4-dimethyl-1H-pyrrole-2-carboxylic acid 3-methoxy-benzylamide, |
| [236] | 3-furan-2-yl-4-methyl-1-(4-trifluoromethylbenzyl)-1H-pyrrole-2-carboxylic acid phenethylamide, |
| [237] | 3-furan-2-yl-1,4-dimethyl-1H-pyrrole-2-carboxylic acid cyclopentylamide, |
| [238] | 3-furan-2-yl-1,4-dimethyl-1H-pyrrole-2-carboxylic acid (pyridin-2-ylmethyl)-amide, |
| [239] | 3-furan-2-yl-4-methyl-1-(4-trifluoromethylbenzyl)-1H-pyrrole-2-carboxylic acid [2-(3,4-dimethoxyphenyl)-ethyl]-amide, |
| [240] | [1-(4-bromobenzyl)-4-methyl-3-p-tolyl-1H-pyrrol-2-yl]-(2,6-dimethylmorpholin-4-yl)-methanone, |
| [241] | 1,4-dimethyl-3-phenyl-1H-pyrrole-2-carboxylic acid (2-p-tolylethyl)-amide, |
| [242] | (1,4-dimethy)-3-phenyl-1H-pyrrol-2-yl)-[4-(4-fluorophenyl)-piperazin-1-yl]-methanone and |
| [243] | 4-methyl-1-(4-methylbenzyl)-3-p-tolyl-1H-pyrrole-2-carboxylic acid [2-(1H-indol-3-yl)-ethyl]-methylamide, |
in each case in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

7. A method for the manufacture of 1,3-disubstituted 4-methyl-1H-pyrrole-2-carboxamides of the general formula I according to one or more of claims 1 to 6, **characterised in that** at least one compound of the general formula II, 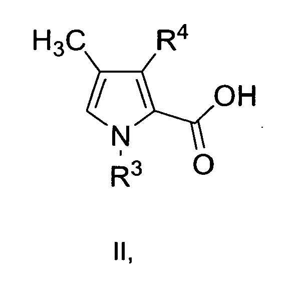 in which R³ and R⁴ have the meaning according to one or more of claims 1 to 6, is converted, in at least one reaction medium, in the presence of at least one suitable coupling agent, in the presence of at least one base, preferably at a temperature of -70°C to 100°C, by reaction with at least one compound of the general formula HNR¹R², in which R¹ and R² have the meaning according 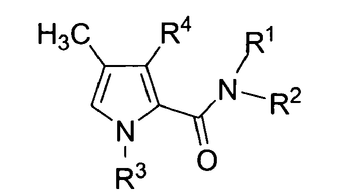 to one or more of claims 1 to 6, into a corresponding compound of the general formula I, optionally in the form of a corresponding salt,
I,
in which R¹, R², R³ and R⁴ have the above-stated meaning, and this is purified and/or isolated.

8. A medicament containing at least one compound according to one or more of claims 1 to 6 and one or more pharmaceutical auxiliary substances.

9. A medicament according to claim 8 for the prevention and/or treatment of pain, preferably of pain selected from the group consisting of acute pain, chronic pain and neuropathic pain.

10. A medicament according to claim 8 or 9 for the prevention and/or treatment of migraine; depression; urinary incontinence; coughing; neurodegenerative diseases, preferably selected from the group consisting of Parkinson's disease, Huntington's chorea, Alzheimer's disease and multiple sclerosis; disorders of food intake, preferably selected from the group consisting of bulimia, anorexia, obesity and cachexia; cognitive dysfunction, preferably memory disorders; epilepsy; diarrhoea; pruritus; abuse of alcohol and/or drugs and/or medicines; alcohol and/or drug dependency and/or dependency on medicines, preferably for the prevention and/or reduction of withdrawal symptoms associated with alcohol and/or drug dependency and/or dependency on medicines; for the prevention and/or reduction of the development of tolerance to medicines, in particular medicines based on opioids; for regulating food intake; for modulating locomotor activity; for regulating the cardiovascular system; for local anaesthesia; for anxiolysis; for increasing vigilance; for increasing libido; for diuresis, and/or for antinatriuresis.

11. Use of at least one compound according to one of claims 1 to 6 for the manufacture of a medicament for the prevention and/or treatment of pain, preferably of pain selected from the group consisting of acute pain, chronic pain and neuropathic pain.

12. Use of at least one compound according to one or more of claims 1 to 6 for the manufacture of a medicament for the prevention and/or treatment of migraine; depression; urinary incontinence; coughing; neurodegenerative diseases, preferably selected from the group consisting of Parkinson's disease, Huntington's chorea, Alzheimer's disease and multiple sclerosis; disorders of food intake, preferably selected from the group consisting of bulimia, anorexia, obesity and cachexia; cognitive dysfunction, preferably memory disorders; epilepsy; diarrhoea; pruritus; abuse of alcohol and/or drugs and/or medicines; alcohol and/or drug dependency and/or dependency on medicines, preferably for the prevention and/or reduction of withdrawal symptoms associated with alcohol and/or drug dependency and/or dependency on medicines; for the prevention and/or reduction of the development of tolerance to medicines, in particular medicines based on opioids; for regulating food intake; for modulating locomotor activity; for regulating the cardiovascular system; for local anaesthesia; for anxiolysis; for increasing vigilance; for increasing libido; for diuresis, and/or for antinatriuresis.

## Revendications

1. Amides de l'acide 4-méthyl-1H-pyrrole-2-carboxylique disubstitué en positions 1,3 de formule générale I, 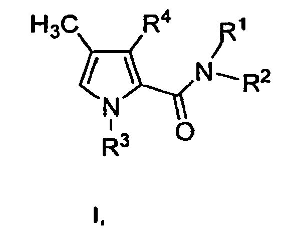 où
R¹ représente C₁₋₆-alkyle, C₂₋₆-alcényle ou C₂₋₆- alcynyle, qui peut à chaque fois être non substitué ou substitué par 1, 2, 3, 4 ou 5 substituants choisis indépendamment l'un de l'autre dans le groupe constitué par F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O- C₂H₅, -C(=O)-O-C(CH₃)₃, -C(=O)-O-CH₂-phényle, -C(=O)-NH-naphtyle, -N(CH₃)-phényle, -N(C₂H₅)- phényle, -N(C₂H₅)-(m-toluyle), -N(C₂H₅)-(p-toluyle), -N(CH₃)-(p-toluyle) et -NH-C(=O)-O-C(CH₃)₃ ; représente hétéroalkyle, hétéroalcényle ou hétéroalcynyle de 2 à 6 chaînons, qui peut à chaque fois être non substitué ou substitué par 1, 2, 3, 4 ou 5 substituants choisis indépendamment l'un de l'autre dans le groupe constitué par F, Cl, Br, I, -NO₂, -CN, -OH, -SH et -NH₂ et qui peut présenter à chaque fois 1 ou 2 hétéroatome(s) choisi(s) indépendamment l'un de l'autre dans le groupe constitué par oxygène, soufre et azote (NH) comme élément(s) de chaîne ;
représente C₃₋₇-cycloalkyle, C₅₋₆-cycloalcényle, hétérocycloalkyle de 5 à 7 chaînons, hétérocycloalcényle de 5 à 7 chaînons, (1,2,3,4)-tétrahydroquinoléinyle, (1,2,3,4)-tétrahydro-isoquinoléinyle, (2,3)-dihydro-1H-iso-indolyle, indolinyle, indanyle, (1,2,3,4)-tétrahydronaphtyle, (2,3)-dihydrobenzo[1,4]dioxinyle ou benzo[1,3]dioxolyle, qui peut à chaque fois être non substitué ou substitué par 1, 2, 3, 4 ou 5 substituants choisis indépendamment l'un de l'autre dans le groupe constitué par F, Cl, Br, I, -CN, méthyle, éthyle, n-propyle, isopropyle, n-butyle, 2-butyle, isobutyle, tert-butyle, -OH, oxo, thioxo, -O-CH₃, -O-C₂H₅, -O-C₃H₇, -O-phényle, -O-CH₂-phényle, -NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂Hₛ, -CH₂-naphtyle, benzyle et phényle et/ou qui peut être lié à chaque fois via un groupe C₁₋₃-alkylène, C₂₋₃-alcénylène ou C₂₋₃-alcynylène non substitué et/ou qui peut présenter à chaque fois 1 ou 2 hétéroatome(s) choisi(s) indépendamment l'un de l'autre dans le groupe constitué par l'oxygène, le soufre et l'azote (NH) comme élément(s) de cycle ;
représente phényle, qui peut être lié à chaque fois via un groupe C₁₋₃-alkylène, C₂₋₃-alcénylène ou C₂₋₃-alcynylène, qui peut à chaque fois être non substitué ou substitué par 1 ou 2 substituants choisis dans le groupe constitué par F, C, Br, -C(=O)-O-CH₃ et -C(=O)-O-C₂H₅ ou lié via un groupe -CH₂-CH₂-O-, -CH₂-O- ou -CH₂-CH₂-CH₂-O- et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O-phényle, méthyle, éthyle, n-propyle, isopropyle, n-butyle, isobutyle, 2-butyle, tert-butyle, n-pentyle, néo-pentyle, éthényle, allyle, éthynyle, propynyle, -C≡C-Si(CH₃)₃, -C≡C-Si(C₂H₅)₃, -CH₂-O-CH₃, -CH₂-O-C₂H₅, -NH₂, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-phényle, pyrazolyle, -N(CH₃)₂, -N(C₂H₅)₂, -NH-CH₃, -NH-C₂H₅, -CH₂-O-C(=O)-phényle, -NH-S(=O)₂-CH₃, -C(=O)-H, -C(=O)-CH₃, -C(=O)-C₂H₅, -NH-C(=O)-CH₃, -NH-C(=O)-C₂H₅, -OC(=O)-phényle, -C(=O)-NH₂, -C(=O)-NH-CH₃, -C(=O)-NH-C₂H₅, -C(=O)-NH-C(CH₃)₃, -C(=O)-N(C₂H₅)₂, -S(=O)-NH₂, [1,2,3]-thiadiazolyle, cyclopropyle, cyclobutyle, cyclopentyle, cyclohexyle, pipérazinyle, pyrrolidinyle, pipéridinyle, phényle, furyle (furannyle), thiadiazolyle, thiophényle (thiényle) et benzyle, les substituants cycliques ou les radicaux cycliques de ces substituants pouvant eux-mêmes à chaque fois être substitués par 1, 2, 3, 4, ou 5 substituants choisis indépendamment l'un de l'autre dans le groupe constitué par F, Cl, Br, I, -CN, -NO₂, -OH, -SH, -NH₂, méthyle, éthyle, n-propyle, isopropyle, n-butyle, isobutyle, 2-butyle, tert-butyle, n-pentyle, néo-pentyle, -O-CH₃, -O-C₂H₅, -O-C₃H₇, -O-C(CH₃)₃, -CF₃, -CHF₂, -CH₂F, -O-CF₃, -O-CHF₂ et -O-CH₂F ;
représente un radical choisi dans le groupe constitué par naphtyle, indolizinyle, benzimidazolyle, tétrazolyle, triazinyle, isoxazolyle, phtalazinyle, carbazolyle, carbolinyle, diaza-naphtyle, thiényle, furyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, benzo[d]thiazolyle, benzodiazolyle, benzotriazolyle, benzoxazolyle, benzo-isoxazolyle, thiazolyle, thiadiazolyle, oxazolyle, oxadiazolyle, isoxazolyle, pyridazinyle, indazolyle, quinoxalinyle, quinazolinyle, quinoléinyle, naphtridinyle et isoquinoléinyle, qui peut à chaque fois être lié via un groupe C₁₋₃-alkylène, C₂₋₃-alcénylène ou C₂₋₃-alcynylène et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O-phényle, méthyle, éthyle, n-propyle, isopropyle, n-butyle, isobutyle, 2-butyle, tert-butyle, n-pentyle, néo-pentyle, éthényle, allyle, éthynyle, propynyle, -C≡C-Si(CH₃)₃, -C≡C-Si(C₂H₅)₃, -CH₂-O-CH₃, -CH₂-OC₂H₅, -NH₂, -C(=O)-OH, -S-CH₃, -S-C₂H₅, -S(=O)-CH₃, -S(=O)₂-CH₃, -S(=O)-C₂H₅, -S(=O)₂-C₂H₅, -O-CH₃, -OC₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-phényle, pyrazolyle, -N(CH₃)₂, -N(C₂H₅)₂, -NH-CH₃, -NH-C₂H₅, -CH₂-O-C(=O) -phényle, -NH-S(=O)₂-CH₃, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -O-C(=O)-phényle, -C(=O)-NH₂, -C(=O)-NH-CH₃, -C(=O)-N(CH₃)₂, -C(=O)-O-CH(CH₃)₂, -C(=O)-O-(CH₂)₃-CH₃, -C(=O)-O-phényle, -OC(=O)-CH₃, -O-C(=O)-C₂H₅, -C(=O)-NH-C₂H₅, -C(=O)-NH-C(CH₃)₃, -C(=O)-N(C₂H₅)₂, -C(=O)-NH-phényle, -C(=O)-N(CH₃)-phényle, -C(=O)-N(C₂H₅)-phényle, -S(=O)-NH₂, [1,2,3]-thiadiazolyle, cyclopropyle, cyclobutyle, cyclopentyle, cyclohexyle, pipérazinyle, pyrrolidinyle, pipéridinyle, phényle, furyle (furannyle), thiadiazolyle, thiophényle (thiényle) et benzyle, les substituants cycliques ou les radicaux cycliques de ces substituants pouvant eux-mêmes à chaque fois être substitués par 1, 2, 3, 4, ou 5 substituants choisis indépendamment l'un de l'autre dans le groupe constitué par F, Cl, Br, I, -CN, -NO₂, -OH, -SH, -NH₂, méthyle, éthyle, n-propyle, isopropyle, n-butyle, isobutyle, 2-butyle, tert-butyle, n-pentyle, néo- pentyle, éthényle, allyle, éthynyle, propynyle, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ et -S-CH₂F ; ou représente -NH-C(=O)-R⁵,
R² représente H ou C₁₋₆-alkyle, C₂₋₆-alcényle ou C₂₋₆- alcynyle, qui peut à chaque fois être non substitué ou substitué par 1, 2, 3, 4 ou 5 substituants choisis indépendamment l'un de l'autre dans le groupe constitué par F, Cl, Br, I, -NO₂, -CN, -OH, -SH, -NH₂, -N(CH₃)₂, -N (C₂H₅)₂, -N(CH₃)(C₂H₅), -C(=O)-OH, -C(=O)-O-CH₃, -C(=O)-O- C₂H₅, -C(=O)-O-C(CH₃)₃, -C(=O)-O-CH₂-phényle, -C(=O)-NH-naphtyle, -N(CH₃)-phényle, -N(C₂H₅)- phényle, -N(C₂H₅)-(m-toluyle), -N(C₂H₅)-(p-toluyle), -N(CH₃)-(p-toluyle) et -NH-C(=O)-O-C(CH₃)₃ ; représente un radical choisi dans le groupe constitué par phényle et naphtyle, qui peut être lié à chaque fois via un groupe C₁₋₃-alkylène, C₂₋₃- alcénylène ou C₂₋₃-alcynylène et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O- phényle, méthyle, éthyle, n-propyle, isopropyle, n-butyle, isobutyle, 2-butyle, tert-butyle, n- pentyle, néo-pentyle, éthényle, allyle, éthynyle, propynyle, -C≡C-Si(CH₃)₃, -C≡C-Si(C₂H₅)₃, -CH₂-O-CH₃, -CH₂-O-C₂H₅, -NH₂, -S-CH₃, -S-C₂H₅, -S(=O)-CH₃, -S(=O)₂-CH₃, -S(=O)-C₂H₅, -S(=O)₂-C₂H₅, -O-CH₃, -O- C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S- CH₂F, -S(=O)₂-phényle, pyrazolyle, -N(CH₃)₂, -N(C₂H₅)₂, -NH-CH₃, -NH-C₂H₅, -CH₂-O-C(=O)-phényle, -NH-S(=O)₂-CH₃, -C(=O)-H, -C(=O)-CH₃, -C(=O)-C₂H₅, -NH-C(=O)-CH₃, -NH-C(=O)-C₂H₅, -O-C(=O)-phényle, -C(=O)-NH₂, -C(=O)-NH-CH₃, -C(=O)-NH-C₂H₅, -C(=O)- NH-C(CH₃)₃, -C(=O)-N(C₂H₅)₂, -S(=O)-NH₂, [1,2,3]- thiadiazolyle, cyclopropyle, cyclobutyle, cyclopentyle, cyclohexyle, pipérazinyle, pyrrolidinyle, pipéridinyle, phényle, furyle (furannyle), thiadiazolyle, thiophényle (thiényle) et benzyle, les substituants cycliques ou les radicaux cycliques de ces substituants pouvant eux-mêmes à chaque fois être substitués par 1, 2, 3, 4, ou 5 substituants choisis indépendamment l'un de l'autre dans le groupe constitué par F, Cl, Br, I, -CN, -NO₂, -OH, -SH, -NH₂, méthyle, éthyle, n-propyle, isopropyle, n-butyle, isobutyle, 2-butyle, tert-butyle, n-pentyle, néo- pentyle, -O-CH₃, -O-C₂H₅, -O-C₃H₇, -O-C(CH₃)₃, -CF₃, -CHF₂, -CH₂F, -O-CF₃, -O-CHF₂ et -O-CH₂F, ou
R¹ et R² forment ensemble avec l'atome d'azote qui les relie un radical choisi dans le groupe constitué par imidazolidinyle, [1,3,4,9]-tétrahydro-b- carbolinyle, pipéridinyle, morpholinyle, thiomorpholinyle, pyrrolidinyle, pipérazinyle, azépanyle, diazépanyle et (1,4)-dioxo-8- azaspiro[4,5]décyle, qui peut à chaque fois être non substitué ou substitué par 1 ou 2 radicaux R⁶.
R³ représente C₁₋₆-alkyle, C₂₋₆-alcényle ou C₂₋₆- alcynyle, qui peut à chaque fois être non substitué ou substitué par 1, 2, 3, 4 ou 5 substituants choisis indépendamment l'un de l'autre dans le groupe constitué par F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O- C₂H₅, -C(=O)-O-C(CH₃)₃, -C(=O)-O-CH₂-phényle, -C(=O)-NH-naphtyle, -N(CH₃)-phényle, -N(C₂H₅)- phényle, -N(C₂H₅)-(m-toluyle), -N(C₂H₅)-(p-toluyle), -N(CH₃)-(p-toluyle) et -NH-C(=O)-O-C(CH₃)₃ représente un radical choisi dans le groupe constitué par phényle, naphtyle, indolizinyle, benzimidazolyle, tétrazolyle, triazinyle, isoxazolyle, phtalazinyle, carbazolyle, carbolinyle, diazanaphtyle, thiényle-, furyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, benzo[d]thiazolyle, benzodiazolyle, benzotriazolyle, benzoxazolyle, benzisoxazolyle, thiazolyle, thiadiazolyle, oxazolyle, oxadiazolyle, isoxazolyle, pyridazinyle, indazolyle, quinoxalinyle, quinazolinyle, quinoléinyle, naphtridinyle et isoquinoléinyle, qui peut à chaque fois être lié via un groupe C₁₋₃-alkylène, C₂₋₃-alcénylène ou C₂₋₃- alcynylène et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O-phényle, méthyle, éthyle, n-propyle, isopropyle, n-butyle, isobutyle, 2-butyle, tert-butyle, n-pentyle, néo- pentyle, éthényle, allyle, éthynyle, propynyle, -CH₂-O-CH₃, -CH₂-O-C₂H₅, -NH₂, -C(=O)-OH, -S-CH₃, -S- C₂H₅, -S(=O)-CH₃, -S(=O)₂-CH₃, -S(=O)-C₂H₅, -S(=O)₂- C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N (C₂H₅)₂, -NH- CH₃, -NH-C₂H₅, -C(=O)-H, -C(=O)-CH₃, -C(=O)-C₂H₅, -C(=O)-NH₂, -C(=O)-NH-CH₃ et -C(=O)-N(CH₃)₂,
R⁴ représente phényle, qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -OH, -SH, -NH₂, -C(=O)-OH, méthyle, éthyle, n-propyle, isopropyle, n-butyle, isobutyle, 2-butyle, tert-butyle, n-pentyle, néo- pentyle, éthényle, allyle, éthynyle, propynyle, -C≡C-Si(CH₃)₃, -C≡C-Si(C₂H₅)₃, -S-CH₃, -S-C₂H₅, -S- phényle, -S-CH₂-phényle, -O-CH₃, -O-C₂H₅, -O-C₃H₇, -O-C(CH₃)₃, -O-phényle, -O-CH₂-phényle, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-phényle, -N(CH₃)₂, -N(C₂H₅)₂, -NH-CH₃, -NH-C₂H₅, -C(=O)-O-CH₃, -C(=O)-OC₂H₅, -C(=O) -O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -S(=O)₂-NH₂, -S(=O)₂-NH-phényle, [1,2,3]-thiadiazolyle, cyclopropyle, cyclobutyle, cyclopentyle, cyclohexyle, phényle, benzyle et phénéthyle, les substituants cycliques ou les radicaux cycliques de ces substituants pouvant eux-mêmes à chaque fois être non substitués ou substitués par 1, 2, 3, 4 ou 5 substituants choisis indépendamment l'un de l'autre dans le groupe constitué par F, Cl, Br, I, -CN, -NO₂, -OH, -SH, -NH₂, -C(=O)-OH, méthyle, éthyle, n-propyle, isopropyle, n-butyle, isobutyle, 2-butyle, tert-butyle, n-pentyle, néo-pentyle, éthényle, allyle, éthynyle, propynyle, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ et -S-CH₂F ; ou
représente un radical choisi dans le groupe constitué par thiényle, furyle, pyrrolyle, pyrazolyle, pyrazinyle, pyrannyle, triazolyle, pyridinyle, imidazolyle, thiadiazolyle, oxadiazolyle et pyridazinyle, qui peut à chaque fois être non substitué ou substitué par 1, 2, 3, 4 ou 5 substituants choisis indépendamment l'un de l'autre dans le groupe constitué par F, Cl, Br, I, -CN, -NO₂, -OH, -CH₂-CN, -SH, -NH₂, -C(=O)-OH, méthyle, éthyle, n-propyle, isopropyle, n-butyle, isobutyle, 2-butyle, tert-butyle, n-pentyle, néo-pentyle, éthényle, allyle, éthynyle, propynyle, -S-CH₃, -S-C₂H₅, -S-phényle, -S-CH₂-phényle, -O-CH₃, -O-C₂H₅, -O-C₃H₇, -O-C(CH₃)₃, -O-phényle, -O-CH₂-phényle, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-O-CH₃, -C(=O)-OC₂H₅, -C(=O)-O-C(CH₃)₃, -C(=O)-H-C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -S(=O)₂-NH₂, cyclopropyle, cyclobutyle, cyclopentyle, cyclohexyle, phényle et benzyle, les substituants cycliques ou les radicaux cycliques de ces substituants pouvant eux-mêmes à chaque fois être non substitués ou substitués par 1, 2, 3, 4 ou 5 substituants choisis indépendamment l'un de l'autre dans le groupe constitué par F, Cl, Br, I, -CN, -NO₂, -OH, -SH, -NH₂, -C(=O)-OH, méthyle, éthyle, n-propyle, isopropyle, n-butyle, isobutyle, 2-butyle, tert-butyle, n-pentyle, néo- pentyle, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ et -S-CH₂F ;
R⁵ représente un radical choisi dans le groupe constitué par phényle, naphtyle, thiényle, furyle, pyrrolyle, pyrazolyle, pyrazinyle, pyrannyle, triazolyle, pyridinyle, imidazolyle, indolyle, iso-indolyle, thiazolyle, thiadiazolyle, oxazolyle, oxadiazolyle, isoxazolyle, pyridazinyle, indazolyle, quinoxalinyle, quinazolinyle, quinoléinyle, naphtridinyle et isoquinoléinyle, qui peut à chaque fois être lié via un groupe C₁₋₃-alkylène, C₂₋₃-alcénylène ou C₂₋₃- alcynylène et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O-phényle, méthyle, éthyle, n-propyle, isopropyle, n-butyle, isobutyle, 2-butyle, tert-butyle, n-pentyle, néo- pentyle, éthényle, allyle, éthynyle, propynyle, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O- CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-H, -C(=O)-CH₃, -C(=O)-C₂H₅, -C(=O)-NH₂, -C(=O)-NH-CH₃ et -C(=O)-N(CH₃)₂ ;
R⁶ représente -OH ; F ; Cl ; Br ; I ; -SH ; -NO₂ ; -NH₂ ; -NH-C(=O)-O-R⁷ ; -C(=O)-O-R⁸ ; -C(=O)-R⁹ ; représente C₁₋₆-alkyle, C₂₋₆-alcényle ou C₂₋₆- alcynyle, qui peut à chaque fois être non substitué ou substitué par 1, 2, 3, 4 ou 5 substituants choisis indépendamment l'un de l'autre dans le groupe constitué par F, Cl, Br, I, -NO₂, -CN, -OH, -SH, -NH₂, -N(CH₃)₂, -N(C₂H₅)₂, -C(=O)-pyrrolidinyle, -C(=O)-N(CH₃)-phényle et -C(=O)-NH-CH(CH₃)₂, -N(CH₃)(C₂H₅), -C(=O)-OH, -C(=O)-O-CH₃, -C(=O)-O-C₂H₅ et -C(=O)-O-C(CH₃)₃ ; représente C₃₋₇-cycloalkyle, C₅₋₆-cycloalcényle, hétérocycloalkyle de 5 à 7 chaînons ou hétérocycloalcényle de 5 à 7 chaînons, qui peut à chaque fois être non substitué ou substitué par 1, 2, 3, 4 ou 5 substituants choisis indépendamment l'un de l'autre dans le groupe constitué par F, Cl, Br, I, -CN, méthyle, éthyle, n-propyle, isopropyle, n-butyle, 2-butyle, isobutyle, tert- butyle, -OH, oxo, thioxo, -O-CH₃, -O-C₂H₅, -O-C₃H₇, -O-phényle, -O-CH₂-phényle, -NH₂, -N(CH₃)₂, -N(C₂H₅)₂, -NH-CH₃, -NH-C₂H₅, -NO₂, -CF₃, -O-CF₃, -S- CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂H₅, -CH₂- naphtyle, benzyle et phényle et/ou qui peut à chaque fois être lié via un groupe C₁₋₃-alkylène, C₂₋₃-alcénylène ou C₂₋₃-alcynylène non substitué et/ou qui peut présenter à chaque fois 1 ou 2 hétéroatome(s) choisi(s) indépendamment l'un de l'autre dans le groupe constitué par oxygène, soufre et azote (NH) comme élément(s) de cycle ;
ou
représente un radical choisi dans le groupe constitué par phényle, naphtyle, indolizinyle, benzimidazolyle, tétrazolyle, triazinyle, isoxazolyle, phtalazinyle, carbazolyle, carbolinyle, diazanaphtyle, thiényle, furyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, benzo[d]thiazolyle, benzodiazolyle, benzotriazolyle, benzoxazolyle, benzisoxazolyle, thiazolyle, thiadiazolyle, oxazolyle, oxadiazolyle, isoxazolyle, pyridazinyle, indazolyle, quinoxalinyle, quinazolinyle, quinoléinyle, naphtridinyle, thiéno[2,3-d]pyrimidinyle et isoquinoléinyle, qui peut à chaque fois être lié via un groupe C₁₋₃-alkylène, C₂₋₃-alcénylène ou C₂₋₃-alcynylène et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O-phényle, méthyle, éthyle, n-propyle, isopropyle, n-butyle, isobutyle, 2-butyle, tert-butyle, n-pentyle, néo-pentyle, éthényle, allyle, éthynyle, propynyle, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -C(=O)-O-CH(CH₃)₂, -C(=O)-O-(CH₂)₃-CH₃, -C(=O)-NH-C₂H₅, -C(=O) -NH-C(CH₃)₃, -C(=O)-N(C₂H₅)₂, cyclopropyle, cyclobutyle, cyclopentyle, cyclohexyle, phényle, furyle (furannyle) et benzyle, les substituants cycliques ou les radicaux cycliques de ces substituants pouvant eux-mêmes à chaque fois être substitués par 1, 2, 3, 4, ou 5 substituants choisis indépendamment l'un de l'autre dans le groupe constitué par F, Cl, Br, I, -CN, -NO₂, -OH, -SH, -NH₂, méthyle, éthyle, n-propyle, isopropyle, n-butyle, isobutyle, 2-butyle, tert-butyle, n-pentyle, néo-pentyle, -O-CH₃, -O-C₂H₅, -O-C₃H₇, -OC(CH₃)₃, -CF₃, -CHF₂, -CH₂F, -O-CF₃, -O-CHF₂, -O-CH₂F, -C(=O)-CF₃, -S-CF₃, -S-CHF₂ et -S-CH₂F ; et
R⁷, R⁸ et R⁹ représentent, indépendamment l'un de l'autre, à chaque fois
C₁₋₆-alkyle, C₂₋₆-alcényle ou C₂₋₆-alcynyle, qui peut à chaque fois être non substitué ou substitué par 1, 2, 3, 4 ou 5 substituants choisis indépendamment l'un de l'autre dans le groupe constitué par F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O-C₂H₅ et -C(=O)-O-C(CH₃)₃ ;
un radical choisi dans le groupe constitué par indolinyle, indanyle, (1,2,3,4)-tétrahydronaphtyle, (2,3)-dihydrobenzo[1,4]dioxinyle et benzo[1,3]dioxolyle, qui peut à chaque fois être non substitué ou substitué par 1, 2, 3, 4 ou 5 substituants choisis indépendamment l'un de l'autre dans le groupe constitué par F, Cl, Br, I, -CN, méthyle, éthyle, n-propyle, isopropyle, n-butyle, 2-butyle, isobutyle, tert-butyle, -OH, oxo, thioxo, -O-CH₃, -O-C₂H₅, -O-C₃H₇, -O-phényle, -O-CH₂-phényle, -NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -C(=P)-CH₃, -C(=O)-C₂H₅, -C(=O)-C(CH₃)₃, -C(=O)-NH-CH₃, -C(=O)-NH-C₂H₅, -CH₂-naphtyle, benzyle et phényle ; ou
un radical choisi dans le groupe constitué par phényle, naphtyle, indolizinyle, benzimidazolyle, tétrazolyle, triazinyle, isoxazolyle, phtalazinyle, carbazolyle, carbolinyle, diazanaphtyle, thiényle, furyle, pyrrolyle, pyrazolyle, pyrazinyle, pyrannyle, triazolyle, pyridinyle, imidazolyle, indolyle, iso-indolyle, thiazolyle, thiadiazolyle, oxazolyle, oxadiazolyle, isoxazolyle et pyridazinyle, qui peut à chaque fois être lié via un groupe C₁₋₃-alkylène, C₂₋₃-alcénylène ou C₂₋₃-alcynylène et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O-phényle, méthyle, éthyle, n-propyle, isopropyle, n-butyle, isobutyle, 2-butyle, tert-butyle, n-pentyle, néo-pentyle, éthényle, allyle, éthynyle, propynyle, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -C(=O)-O-CH(CH₃)₂, -C(=O)-O-(CH₂)₃-CH₃, -C(=O)-NH-C₂H₅, -C(=O)-NH-C(CH₃)₃, -C(=O)-N(C₂H₅)₂, cyclopropyle, cyclobutyle, cyclopentyle, cyclohexyle, phényle, furyle (furannyle) et benzyle,
à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

2. Composés selon la revendication 1, **caractérisés en ce que**
R¹ représente C₁₋₆-alkyle, C₂₋₆-alcényle ou C₂₋₆- alcynyle, qui peut à chaque fois être non substitué ou substitué par 1, 2, 3, 4 ou 5 substituants choisis indépendamment l'un de l'autre dans le groupe constitué par F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O- C₂H₅, -C(=O)-O-C(CH₃)₃, -C(=O)-O-CH₂-phényle, -C(=O)-NH-naphtyle, -N(CH₃)-phényle, -N(C₂H₅)- phényle, -N(C₂H₅)-(m-toluyle), -N(C₂H₅)-(p-toluyle), -N(CH₃)-(p-toluyle) et -NH-C(=O)-O-C(CH₃)₃ ;
représente hétéroalkyle de 2 à 6 chaînons, qui peut à chaque fois être non substitué ou substitué par 1, 2, 3, 4 ou 5 substituants choisis indépendamment l'un de l'autre dans le groupe constitué par F, Cl, Br, I, -NO₂, -CN, -OH, -SH et -NH₂ et qui peut présenter à chaque fois 1 ou 2 hétéroatome(s) choisi(s) indépendamment l'un de l'autre dans le groupe constitué par oxygène, soufre et azote (NH) comme élément(s) de chaîne ;
représente C₃₋₇-cycloalkyle, C₅₋₆-cycloalcényle, hétérocycloalkyle de 5 à 7 chaînons, hétérocycloalcényle de 5 à 7 chaînons, (1,2,3,4)-tétrahydroquinoléinyle, (1,2,3,4)-tétrahydro-isoquinoléinyle, (2,3)-dihydro-1H-iso-indolyle, indolinyle, indanyle, (1,2,3,4)-tétrahydronaphtyle, (2,3)-dihydrobenzo[1,4]dioxinyle ou benzo[1,3]dioxolyle, qui peut à chaque fois être non substitué ou substitué par 1, 2, 3, 4 ou 5 substituants choisis indépendamment l'un de l'autre dans le groupe constitué par F, Cl, Br, I, -CN, méthyle, éthyle, n-propyle, isopropyle, n-butyle, 2-butyle, isobutyle, tert-butyle, -OH, oxo, thioxo, -O-CH₃, -O-C₂H₅, -O-C₃H₇, -O-phényle, -O-CH₂-phényle, -NH₂, -N(CH3₎2, -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂H₅, -CH₂-naphtyle, benzyle et phényle et/ou qui peut être lié à chaque fois via un groupe C₁₋₃-alkylène, C₂₋₃-alcénylène ou C₂₋₃-alcynylène non substitué et/ou qui peut présenter à chaque fois 1 ou 2 hétéroatome(s) choisi(s) indépendamment l'un de l'autre dans le groupe constitué par l'oxygène, le soufre et l'azote (NH) comme élément(s) de cycle ;
représente phényle, qui peut être lié à chaque fois via un groupe C₁₋₃-alkylène, C₂₋₃-alcénylène ou C₂₋₃-alcynylène, qui peut à chaque fois être non substitué ou substitué par 1 ou 2 substituants choisis dans le groupe constitué par F, Cl, Br, -C(=O)-O-CH₃ et -C(=O)-O-C₂H₅ ou lié via un groupe -CH₂-CH₂-O-, -CH₂-O- ou -CH₂-CH₂-CH₂-O- et/ou qui peut être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O-phényle, méthyle, éthyle, n-propyle, isopropyle, n-butyle, isobutyle, 2-butyle, tert-butyle, n-pentyle, néo-pentyle, éthényle, allyle, éthynyle, propynyle, -C≡C-Si(CH₃)₃, -C≡C-Si(C₂H₅)₃, -CH₂-O-CH₃, -CH₂-OC₂H₅, -NH₂, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-phényle, pyrazolyle, -N(CH₃)₂, -N(C₂H₅)₂, -NH-CH₃, -NH-C₂H₅, -CH₂-O-C(=O)-phényle, -NH-S(=O)₂-CH₃, -C(=O)-H, -C(=O)-CH₃, -C(=O)-C₂H₅, -NH-C(=O)-CH₃, -NH-C(=O)-C₂H₅, -O-C(=O)-phényle, -C(=O)-NH₂, -C(=O)-NH-CH₃, -C(=O)-NH-C₂H₅, -C(=O)-NH-C(CH₃)₃, -C(=O)-N(C₂H₅)₂, -S(=O)-NH₂, [1,2,3]-thiadiazolyle, cyclopropyle, cyclobutyle, cyclopentyle, cyclohexyle, pipérazinyle, pyrrolidinyle, pipéridinyle, phényle, furyle (furannyle), thiadiazolyle, thiophényle (thiényle) et benzyle, les substituants cycliques ou les radicaux cycliques de ces substituants pouvant eux-mêmes à chaque fois être substitués par 1, 2, 3, 4, ou 5 substituants choisis indépendamment l'un de l'autre dans le groupe constitué par F, Cl, Br, I, -CN, -NO₂, -OH, -SH, -NH₂, méthyle, éthyle, n-propyle, isopropyle, n-butyle, isobutyle, 2-butyle, tert-butyle, n-pentyle, néo-pentyle, -O-CH₃, -O-C₂H₅, -O-C₃H₇, -O-C(CH₃)₃, -CF₃, -CHF₂, -CH₂F, -O-CF₃, -O-CHF₂ et -O-CH₂F ;
représente un radical choisi dans le groupe constitué par naphtyle, indolizinyle, benzimidazolyle, tétrazolyle, triazinyle, isoxazolyle, phtalazinyle, carbazolyle, carbolinyle, diazanaphtyle, thiényle, furyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, benzo[d]thiazolyle, benzodiazolyle, benzotriazolyle, benzoxazolyle, benzisoxazolyle, thiazolyle, thiadiazolyle, oxazolyle, oxadiazolyle, isoxazolyle, pyridazinyle, indazolyle, quinoxalinyle, quinazolinyle, quinoléinyle, naphtridinyle et isoquinoléinyle, qui peut à chaque fois être lié via un groupe C₁₋₃-alkylène, C₂₋₃-alcénylène ou C₂₋₃-alcynylène et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O-phényle, méthyle, éthyle, n-propyle, isopropyle, n-butyle, isobutyle, 2-butyle, tert-butyle, n-pentyle, néo-pentyle, éthényle, allyle, éthynyle, propynyle, -C≡C-Si(CH₃)₃, -C≡C-Si(C₂H₅)₃, -CH₂-O-CH₃, -CH₂-OC₂H₅, -NH₂, -C(=O)-OH, -S-CH₃, -S-C₂H₅, -S(=O)-CH₃, -S(=O)₂-CH₃, -S(=O)-C₂H₅, -S(=O)₂-C₂H₅, -O-CH₃, -OC₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-phényle, pyrazolyle, -N(CH₃)_{2,} -N(C₂H₅)₂, -NH-CH₃, -NH-C₂H₅, -CH₂-O-C(=O)-phényle, -NH-S(=O)₂-CH₃, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -O-C(=O)-phényle, -C(=O)-NH₂, -C(=O)-NH-CH₃, -C(=O)-N(CH₃)₂, -C(=O)-O-CH(CH₃)₂, -C(=O)-O-(CH₂)₃-CH₃, -C(=O)-O-phényle, -OC(=O)-CH₃, -O-C(=O)-C₂H₅, -C(=O)-NH-C₂H₅, -C(=O)-NH-C(CH₃)₃, -C(=O)-N(C₂H₅)₂, -C(=O)-NH-phényle, -C(=O)-N(CH₃)-phényle, -C(=O)-N(C₂H₅) -phényle, -S(=O)-NH₂, [1,2,3]-thiadiazolyle, cyclopropyle, cyclobutyle, cyclopentyle, cyclohexyle, pipérazinyle, pyrrolidinyle, pipéridinyle, phényle, furyle (furannyle), thiadiazolyle, thiophényle (thiényle) et benzyle, les substituants cycliques ou les radicaux cycliques de ces substituants pouvant eux-mêmes à chaque fois être substitués par 1, 2, 3, 4, ou 5 substituants choisis indépendamment l'un de l'autre dans le groupe constitué par F, Cl, Br, I, -CN, -NO₂, -OH, -SH, -NH₂, méthyle, éthyle, n-propyle, isopropyle, n-butyle, isobutyle, 2-butyle, tert-butyle, n-pentyle, néo- pentyle, éthényle, allyle, éthynyle, propynyle, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ et -S-CH₂F ; ou représente -NH-C(=O)-R⁵ ;
R² représente H ou C₁₋₆-alkyle, qui peut à chaque fois être non substitué ou substitué par 1, 2, 3, 4 ou 5 substituants choisis indépendamment l'un de l'autre dans le groupe constitué par F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O- C₂H₅, -C(=O)-O-C(CH₃)₃, -C(=O)-O-CH₂-phényle, -C(=O)-NH-naphtyle, -N(CH₃)-phényle, -N(C₂H₅)- phényle, -N(C₂H₅)-(m-toluyle), -N(C₂H₅)-(p-toluyle), -N(CH₃)-(p-toluyle) et -NH-C(=O)-O-C(CH₃)₃ ; ou représente un radical choisi dans le groupe constitué par phényle et naphtyle, qui peut à chaque fois être lié via un groupe C₁₋₃-alkylène, C₂₋₃-alcénylène ou C₂₋₃-alcynylène et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O- phényle, méthyle, éthyle, n-propyle, isopropyle, n-butyle, isobutyle, 2-butyle, tert-butyle, n- pentyle, néo-pentyle, éthényle, allyle, éthynyle, propynyle, -C≡C-Si(CH₃)₃, -C≡C-Si(C₂H₅)₃, -CH₂-O-CH₃, -CH₂-O-C₂H₅, -NH₂, -S-CH₃, -S-C₂H₅, -S(=O)-CH₃, -S(=O)₂-CH₃, -S(=O)-C₂H₅, -S(=O)₂-C₂H₅, -O-CH₃, -O- C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S- CH₂F, -N(CH₃)₂, -N(C₂H₅)₂, -NH-CH₃ et -NH-C₂H₅ ; ou R¹ et R² forment ensemble avec l'atome d'azote qui les relie un radical choisi dans le groupe constitué par 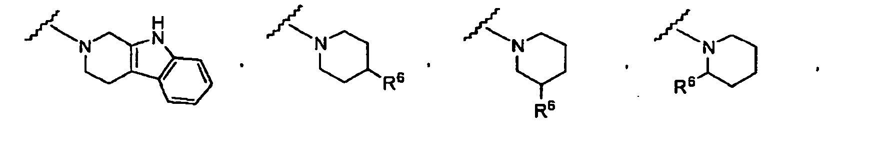 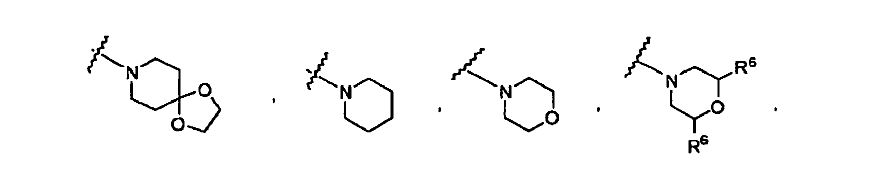 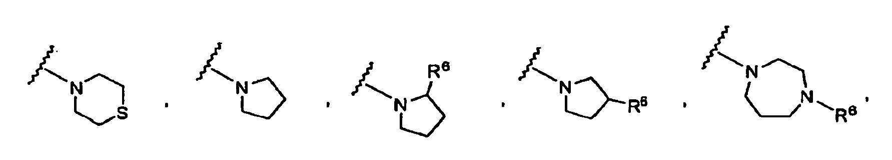 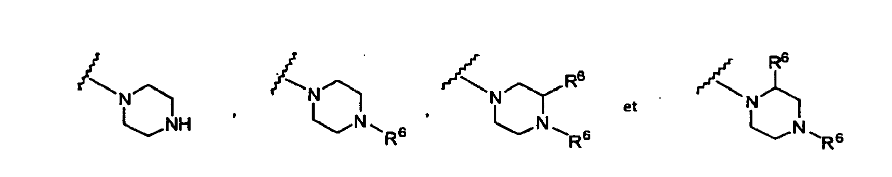
R³ représente C₁₋₆-alkyle, qui peut à chaque fois être non substitué ou substitué par 1, 2, 3, 4 ou 5 substituants choisis indépendamment l'un de l'autre dans le groupe constitué par F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O- C₂H₅ et -C(=O)-O-C(CH₃)₃ ; ou
représente un radical choisi dans le groupe constitué par phényle, isoxazolyle, thiényle, furyle, pyrrolyle, pyrazolyle, pyrazinyle, pyrannyle, triazolyle, pyridinyle, imidazolyle, indolyle, iso-indolyle, thiazolyle, thiadiazolyle, oxazolyle, oxadiazolyle, pyridazinyle et isoquinoléinyle, qui peut à chaque fois être lié via un groupe C₁₋₃-alkylène, C₂₋₃-alcénylène ou C₂₋₃- alcynylène et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O-phényle, méthyle, éthyle, n-propyle, isopropyle, n-butyle, isobutyle, 2-butyle, tert-butyle, n-pentyle, néo- pentyle, éthényle, allyle, éthynyle, propynyle, -CH₂-O-CH₃, -CH₂-O-C₂H₅, -NH₂, -C(=O)-OH, -S-CH₃, -S- C₂H₅, -S(=O)-CH₃, -S(=O)₂-CH₃, -S(=O)-C₂H₅, -S(=O)₂- C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH- CH₃, -NH-C₂H₅, -C(=O)-H, -C(=O)-CH₃, -C(=O)-C₂H₅, -C(=O)-NH₂, -C(=O)-NH-CH₃ et -C(=O)-N(CH₃)₂ ;
R⁴ représente phényle, qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -OH, -SH, -NH₂, -C(=O)-OH, méthyle, éthyle, n-propyle, isopropyle, n-butyle, isobutyle, 2-butyle, tert-butyle, n-pentyle, néo- pentyle, éthényle, allyle, éthynyle, propynyle, -C≡C-Si(CH₃)₃, -C≡C-Si(C₂H₅)₃, -S-CH₃, -S-C₂H₅, -S- phényle, -S-CH₂-phényle, -O-CH₃, -O-C₂H₅, -O-C₃H₇, -O-C(CH₃)₃, -O-phényle, -O-CH₂-phényle, -CF₃, -CHF₂, -CH₂F, -O-CF₃, -O-CHF₂, -O-CH₂F, -C(=O)-CF₃, -S-CF₃, -S-CHF₂, -S-CH₂F, -S(=O)₂-phényle, -N(CH₃)₂, -N(C₂H₅)₂, -NH-CH₃, -NH-C₂H₅, -C(=O)-O-CH₃, -C(=O)-O- C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)- NH₂, -C(=O)-NH-CH₃, -C(=O)-N(CH₃)₂, -S(=O)₂-NH₂, -S(=O)₂-NH-phényle, [1,2,3]-thiadiazolyle, cyclopropyle, cyclobutyle, cyclopentyle, cyclohexyle, phényle, benzyle et phénéthyle, les substituants cycliques ou les radicaux cycliques de ces substituants pouvant eux-mêmes à chaque fois être non substitués ou substitués par 1, 2, 3, 4 ou 5 substituants choisis indépendamment l'un de l'autre dans le groupe constitué par F, Cl, Br, I, -CN, -NO₂, -OH, -SH, -NH₂, -C(=0)-OH, méthyle, éthyle, n-propyle, isopropyle, n-butyle, isobutyle, 2-butyle, tert-butyle, n-pentyle, néo-pentyle, éthényle, allyle, éthynyle, propynyle, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ et -S-CH₂F ; ou
représente un radical choisi dans le groupe constitué par thiényle, furyle, pyrrolyle, pyrazolyle, pyrazinyle, pyrannyle, triazolyle, pyridinyle, imidazolyle, thiadiazolyle, oxadiazolyle et pyridazinyle, qui peut à chaque fois être non substitué ou substitué par 1, 2, 3, 4 ou 5 substituants choisis indépendamment l'un de l'autre dans le groupe constitué par F, Cl, Br, I, -CN, -NO₂, -OH, -CH₂-CN, -SH, -NH₂, -C(=O)-OH, méthyle, éthyle, n-propyle, isopropyle, n-butyle, isobutyle, 2-butyle, tert-butyle, n-pentyle, néo-pentyle, éthényle, allyle, éthynyle, propynyle, -S-CH₃, -S-C₂H₅, -S-phényle, -S-CH₂-phényle, -O-CH₃, -O-C₂H₅, -O-C₃H₇, -O-C(CH₃)₃, -O-phényle, -O-CH₂-phényle, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-O-CH₃, -C(=O)-OC₂H₅, -C(=O)-O-C(CH₃)₃, -C(=O)-H -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -S(=O)₂-NH₂, cyclopropyle, cyclobutyle, cyclopentyle, cyclohexyle, phényle et benzyle, les substituants cycliques ou les radicaux cycliques de ces substituants pouvant eux-mêmes à chaque fois être non substitués ou substitués par 1, 2, 3, 4 ou 5 substituants choisis indépendamment l'un de l'autre dans le groupe constitué par F, Cl, Br, I, -CN, -NO₂, -OH, -SH, -NH₂, -C(=O)-OH, méthyle, éthyle, n-propyle, isopropyle, n-butyle, isobutyle, 2-butyle, tert-butyle, n-pentyle, néo-pentyle, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂ et -S-CH₂F ;
R⁵ représente un radical choisi dans le groupe constitué par phényle, naphtyle, thiényle, furyle, pyrrolyle, pyrazolyle, pyrazinyle, pyrannyle, triazolyle, pyridinyle, imidazolyle, indolyle, iso-indolyle, thiazolyle, thiadiazolyle, oxazolyle, oxadiazolyle, isoxazolyle et pyridazinyle, qui peut à chaque fois être lié via un groupe C₁₋₃-alkylène, C₂₋₃-alcénylène ou C₂₋₃- alcynylène et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, méthyle, éthyle, n- propyle, isopropyle, n-butyle, isobutyle, 2- butyle, tert-butyle, n-pentyle, néo-pentyle, éthényle, allyle, éthynyle, propynyle, -NH₂, -S- CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃ et -NH-C₂H₅ ;
R⁶ représente -OH; F; Cl; Br; I; -SH; -NO₂; -NH₂; -NH-C(=O)-O-R⁷; -C(=O)-O-R⁸; -C(=O)-R⁹; représente C₁₋₆-alkyle, C₂₋₆-alcényle ou C₂₋₆- alcynyle, qui peut à chaque fois être non substitué ou substitué par 1, 2, 3, 4 ou 5 substituants choisis indépendamment l'un de l'autre dans le groupe constitué par F, Cl, Br, I, -NO₂, -CN, -OH, -SH, -NH₂, -N(CH₃)₂, -C(=O)- pyrrolidinyle, -C(=O)-N(CH₃)-phényle et -C(=O)-NH- CH(CH₃)₂, -N(C₂H₅)₂, -N(CH₃)(C₂H₅), -C(=O)-OH, -C(=O)-O-CH₃, -C(=O)-O-C₂H₅ et -C(=O)-O-C(CH₃)₃ ; représente C₃₋₇-cycloalkyle, C₅₋₆-cycloalcényle, hétérocycloalkyle de 5 à 7 chaînons ou hétérocycloalcényle de 5 à 7 chaînons, qui peut à chaque fois être non substitué ou substitué par 1, 2, 3, 4 ou 5 substituants choisis indépendamment l'un de l'autre dans le groupe constitué par F, Cl, Br, I, -CN, méthyle, éthyle, n-propyle, isopropyle, n-butyle, 2-butyle, isobutyle, tert- butyle, -OH, oxo, thioxo, -O-CH₃, -O-C₂H₅, -O-C₃H₇, -O-phényle, -O-CH₂-phényle, -NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H_{5,} -C(=O)-CH₃, -C(=O)-C₂H₅, -C(=O)-C(CH₃)₃, -C(=O)-NH-CH₃, -C(=O)-NH-C₂H₅, -CH₂-naphtyle, benzyle et phényle et/ou qui peut à chaque fois être lié via un groupe C₁₋₃-alkylène, C₂₋₃-alcénylène ou C₂₋₃-alcynylène non substitué et/ou qui peut présenter à chaque fois 1 ou 2 hétéroatome(s) choisi(s) indépendamment l'un de l'autre dans le groupe constitué par oxygène, soufre et azote (NH) comme élément(s) de cycle ;
ou
représente un radical choisi dans le groupe constitué par phényle, naphtyle, indolizinyle, benzimidazolyle, tétrazolyle, triazinyle, isoxazolyle, phtalazinyle, carbazolyle, carbolinyle, diazanaphtyle, thiényle, furyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, benzo[d]thiazolyle, benzodiazolyle, benzotriazolyle, benzoxazolyle, benzisoxazolyle, thiazolyle, thiadiazolyle, oxazolyle, oxadiazolyle, isoxazolyle, pyridazinyle, indazolyle, quinoxalinyle, quinazolinyle, quinoléinyle, naphtridinyle, thiéno[2,3-d]pyrimidinyle et isoquinoléinyle, qui peut à chaque fois être lié via un groupe C₁₋₃-alkylène, C₂₋₃-alcénylène ou C₂₋₃-alcynylène et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O-phényle, méthyle, éthyle, n-propyle, isopropyle, n-butyle, isobutyle, 2-butyle, tert-butyle, n-pentyle, néo-pentyle, éthényle, allyle, éthynyle, propynyle, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -C(=O)-O-CH(CH₃)₂, -C(=O)-O-(CH₂)₃-CH₃, -C(=O)-NH-C₂H₅, -C(=O)-NH-C(CH₃)₃, -C(=O)-N(C₂H₅)₂, cyclopropyle, cyclobutyle, cyclopentyle, cyclohexyle, phényle, furyle (furannyle) et benzyle, les substituants cycliques ou les radicaux cycliques de ces substituants pouvant eux-mêmes à chaque fois être substitués par 1, 2, 3, 4, ou 5 substituants choisis indépendamment l'un de l'autre dans le groupe constitué par F, Cl, Br, I, -CN, -NO₂, -OH, -SH, -NH₂, méthyle, éthyle, n-propyle, isopropyle, n-butyle, isobutyle, 2-butyle, tert-butyle, n-pentyle, néo-pentyle, -O-CH₃, -O-C₂H₅, -O-C₃H₇, -OC(CH₃)₃, -CF₃, -CHF₂, -CH₂F, -O-CF₃, -O-CHF₂, -O-CH₂F, -C(=O)-CF₃, -S-CF₃, -S-CHF₂ et -S-CH₂F ; et
et R⁷, R⁸ et R⁹, représentent, indépendamment l'un de l'autre, à chaque fois
C₁₋₆-alkyle, C₂₋₆-alcényle ou C₂₋₆-alcynyle, qui peut à chaque fois être non substitué ou substitué par 1, 2, 3, 4 ou 5 substituants choisis indépendamment l'un de l'autre dans le groupe constitué par F, Cl, Br, I, -NO₂, -CN, -OH, -SH, -NH₂, -N(CH₃)₂, -N(C₂H₅)₂, -N(CH₃)(C₂H₅), -C(=O)-OH, -C(=O)-O-CH₃, -C(=O)-O-C₂H₅ et -C(=O)-O-C(CH₃)₃ ;
un radical choisi dans le groupe constitué par indolinyle, indanyle, (1,2,3,4)-tétrahydronaphtyle, (2,3)-dihydrobenzo[1,4]dioxinyle et benzo[1,3]dioxolyle, qui peut à chaque fois être non substitué ou substitué par 1, 2, 3, 4 ou 5 substituants choisis indépendamment l'un de l'autre dans le groupe constitué par F, Cl, Br, I, -CN, méthyle, éthyle, n-propyle, isopropyle, n-butyle, 2-butyle, isobutyle, tert-butyle, -OH, oxo, thioxo, -O-CH₃, -O-C₂H₅, -O-C₃H₇, -O-phényle, -O-CH₂-phényle, -NH₂, -N(CH₃)₂, -N(C₂H₅)₂, -NH-CH₃, -NH-C₂H₅, -NO₂, -CF₃, -O-CF₃, -S-CF₃, -SH, -S-CH₃, -S-C₂H₅, -C(=O)-CH₃, -C(=O)-C₂H₅, -C(=O)-C(CH₃)₃, -C(=O)-NH-CH₃, -C(=O)-NH-C₂H₅, -CH₂-naphtyle, benzyle et phényle ; ou
un radical choisi dans le groupe constitué par phényle, naphtyle, indolizinyle, benzimidazolyle, tétrazolyle, triazinyle, isoxazolyle, phtalazinyle, carbazolyle, carbolinyle, diazanaphtyle, thiényle, furyle, pyrrolyle, pyrazolyle, pyrazinyle, pyrannyle, triazolyle, pyridinyle, imidazolyle, indolyle, iso-indolyle, thiazolyle, thiadiazolyle, oxazolyle, oxadiazolyle, isoxazolyle et pyridazinyle, qui peut à chaque fois être lié via un groupe C₁₋₃-alkylène, C₂₋₃-alcénylène ou C₂₋₃-alcynylène et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O-phényle, méthyle, éthyle, n-propyle, isopropyle, n-butyle, isobutyle, 2-butyle, tert-butyle, n-pentyle, néo-pentyle, éthényle, allyle, éthynyle, propynyle, -NH₂, -C(=O)-OH, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂, -N(C₂H₅)₂, -NH-CH₃, -NH-C₂H₅, -C(=O)-O-CH₃, -C(=O)-O-C₂H₅, -C(=O)-O-C(CH₃)₃, -C(=O)-H, -C(=O)-CH₃, -C(=O)-C₂H₅, -NH-C(=O)-CH₃, -NH-C(=O)-C₂H₅, -C(=O)-NH₂, -C(=O)-NH-CH₃, -C(=O)-N(CH₃)₂, -C(=O)-O-CH(CH₃)₂, -C(=O)-O-(CH₂)₃-CH₃, -C(=O)-NH-C₂H₅, -C(=O)-NH-C(CH₃)₃, -C(=O)-N(C₂H₅)₂, cyclopropyle, cyclobutyle, cyclopentyle, cyclohexyle, phényle, furyle (furannyle) et benzyle.

3. Composés selon la revendication 1 ou 2, **caractérisés**
**en ce que**
R¹ représente un radical alkyle choisi dans le groupe constitué par méthyle, éthyle, n-propyle, isopropyle, n-butyle, 2-butyle, isobutyle, tert- butyle, n-pentyle, sec-pentyle, néo-pentyle, (3,3)-diméthylbutyle, 4-méthyl-2-pentyle et n- hexyle, qui peut à chaque fois être non substitué ou substitué par 1, 2, 3, 4 ou 5 substituants choisis indépendamment l'un de l'autre dans le groupe constitué par F, Cl, Br, I, -CN, -OH, -SH, -NH₂, -N(CH₃)₂, -N(C₂H₅)₂, -N(CH₃)(C₂H₅), -C(=O)-O- CH₃, -C(=O)-O-C₂H₅, -C(=O)-O-C(CH₃)₃, -C(=O)-O-CH₂- phényle, -C(=O)-NH-naphtyle, -N(CH₃)-phényle, -N(C₂H₅)-phényle, -N(C₂H₅)-(m-toluyle), -N(C₂H₅)-(p- toluyle), -N(CH₃)-(p-toluyle) et -NH-C(=O)-O- C(CH₃)₃ ;
représente un radical hétéroalkyle, choisi dans le groupe constitué par -CH₂-O-CH₃, -CH₂-O-C₂H₅, -CHF- CH₂-O-CH₃, -CH₂-CH₂-O-C₂H₅, -CH₂-CH₂-CH₂-O-CH₃ et -CH₂-CH₂-CH₂-O-C₂H₁₅, qui peut à chaque fois être non substitué ou substitué par 1, 2, 3, 4 ou 5 substituants choisis indépendamment l'un de l'autre dans le groupe constitué par F, Cl et Br ; représente un radical choisi dans le groupe constitué par cyclopropyle, cyclobutyle, cyclopentyle, cyclohexyle, cycloheptyle, cyclohexényle, cyclopentényle, pyrrolidinyle, tétrahydrothiophényle, tétrahydrofurannyle, pipéridinyle, morpholinyle, pipérazinyle, azépanyle, diazépanyle, (1,2,3,4)- tétrahydroquinoléinyle, (1,2,3,4)-tétrahydro- isoquinoléinyle, (2,3)-dihydro-1H-iso-indolyle, indolinyle, indanyle, (1,2,3,4)- tétrahydronaphtyle, (2,3)- dihydrobenzo[1,4]dioxinyle et benzo[1,3]dioxolyle, qui peut à chaque fois être non substitué ou substitué par 1, 2, 3, 4 ou 5 substituants choisis indépendamment l'un de l'autre dans le groupe constitué par F, Cl, Br, I, -CN, méthyle, éthyle, n-propyle, isopropyle, n-butyle, 2-butyle, isobutyle, tert-butyle, -OH, oxo, -O-CH₃, -O-C₂H₅, -O-C₃H₇, -O-phényle, -O-CH₂-phényle, -C(=O)-CH₃, -C(=O)-C₂H₅, -C(=O)-C(CH₃)₃, -C(=O)-NH-CH₃, -C(=O)-NH-C₂H₅, -CH₂-naphtyle, benzyle et phényle et/ou qui peut à chaque fois être lié via un groupe -CH₂-, -CH(CH₃)-, -CH₂-CH₂-, -CH(CH₃)-CH₂-, -CH₂-CH(CH₃)- ou -CH₂-CH₂-CH₂- ;
représente phényle, qui peut à chaque fois être lié via un groupe -CH₂-CH₂-O-, -CH₂-O-, -CH₂-CH₂-CH₂-O-, -CH[C(=O)-O-CH₃]-CH₂-, -CH[C(=O)-O-C₂H₅]-CH₂-, -CH₂-, -CH(CH₃)-, -CH₂-CH₂-, -CH(CH₃)-CH₂-, -CH₂-CH(CH₃)- ou -CH₂-CH₂-CH₂- et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O-phényle, méthyle, éthyle, n-propyle, isopropyle, n-butyle, isobutyle, 2-butyle, tert-butyle, n-pentyle, néo-pentyle, éthényle, allyle, éthynyle, propynyle, -NH₂, -S-CH₃, -S-C₂H₅ -O-CH₃, -O-C₂H₅, -OC₃H₇, -O-C(CH₃)₃, -CF₃, -CHF₂, -CH₂F, -O-CF₃, -O-CHF₂, -O-CH₂F, -N(CH₃)₂, -N(C₂H₅)₂, -NH-CH₃, -NH-C₂H₅, -NH-S(=O)₂-CH₃, -C(=O)-H, -C(=O)-CH₃, -C(=O)-C₂H₅, -NH-C(=O)-CH₃, -NH-C(=O)-C₂H₅, -S(=O)-NH₂, [1,2,3]-thiadiazolyle, cyclopropyle, cyclobutyle, cyclopentyle, cyclohexyle, phényle, furyle (furannyle), thiadiazolyle, thiophényle (thiényle) et benzyle, où les substituants cycliques ou les radicaux cycliques de ces substituants peuvent eux-mêmes à chaque fois être substitués par 1, 2, 3, 4, ou 5 substituants choisis indépendamment l'un de l'autre dans le groupe constitué par F, Cl, Br, I, -CN, -NO₂, méthyle, éthyle, n-propyle, isopropyle, n-butyle, isobutyle, 2-butyle, tert-butyle, -O-CH₃, -O-C₂H₅, -O-C₃H₇ et -O-C(CH₃)₃ ;
représente un radical choisi dans le groupe constitué par naphtyle, indolizinyle, benzimidazolyle, tétrazolyle, triazinyle, isoxazolyle, phtalazinyle, carbazolyle, carbolinyle, diazanaphtyle, thiényle, furyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, benzo[d]thiazolyle, benzodiazolyle, benzotriazolyle, benzoxazolyle, benzisoxazolyle, thiazolyle, thiadiazolyle, oxazolyle, oxadiazolyle, isoxazolyle, pyridazinyle, indazolyle, quinoxalinyle, quinazolinyle, quinoléinyle, naphtridinyle et isoquinoléinyle, qui peut à chaque fois être lié via un groupe -CH₂-, -CH(CH₃)-, -CH₂-CH₂-, -CH(CH₃)- CH₂-, -CH₂-CH(CH₃)- ou -CH₂-CH₂-CH₂- et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O-phényle, méthyle, éthyle, n-propyle, isopropyle, n-butyle, isobutyle, 2-butyle, tert- butyle, n-pentyle, néo-pentyle, -O-CH₃, -O-C₂H₅, -O-C₃H₇, -O-C(CH₃)₃, -CF₃, -CHF₂, -CH₂F, -O-CF₃, -O- CHF₂, -O-CH₂F, -C(=O)-CF₃, -S-CF₃, -S-CHF₂, -S-CH₂F, -C(=O)-NH₂, -C(=O)-NH-CH₃, -C(=O)-N(CH₃)₂, -C(=O)-O- CH(CH₃)₂ et -C(=O)-O-(CH₂)₃-CH₃, ou représente -NH-C(=O)-R⁵ ;
R² représente H ou un radical alkyle, choisi dans le groupe constitué par méthyle, éthyle, n-propyle, isopropyle, n-butyle, 2-butyle, isobutyle, tert- butyle, n-pentyle, sec-pentyle, néo-pentyle, (3,3)-diméthylbutyle, 4-méthyl-2-pentyle et n- hexyle, qui peut à chaque fois être non substitué ; ou
représente un radical choisi dans le groupe constitué par phényle et naphtyle, qui peut être lié à chaque fois via un groupe -CH₂-, -CH(CH₃)-, -CH₂-CH₂-, -CH(CH₃)-CH₂-_{,} -CH₂-CH(CH₃)- ou -CH₂-CH₂- CH₂- et/ou qui peut être chaque fois non substitué ou substitué par 1, 2, 3, 4 ou 5 substituants choisis indépendamment l'un de l'autre dans le groupe constitué par F, Cl, Br, I, -CN, méthyle, éthyle, n-propyle, isopropyle, n-butyle, isobutyle, 2-butyle, tert-butyle, n-pentyle, néo-pentyle, -O-CH₃, -O-C₂H₅, -O-C₃H₇, -O-C(CH₃)₃, -CF₃, -CHF₂ et -CH₂F ;
ou R¹ et R² forment ensemble avec l'atome d'azote qui les relie un radical choisi dans le groupe constitué par 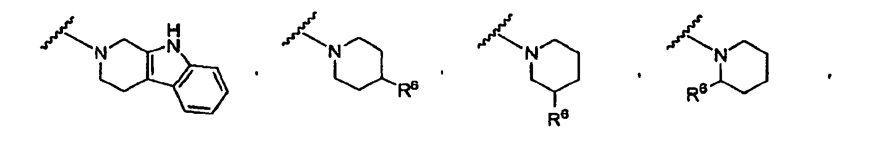 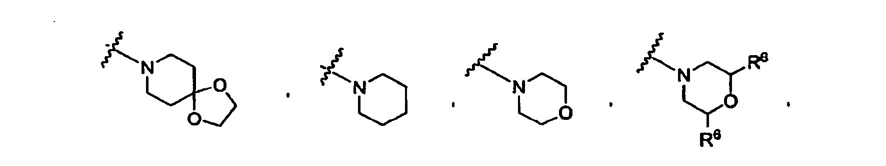 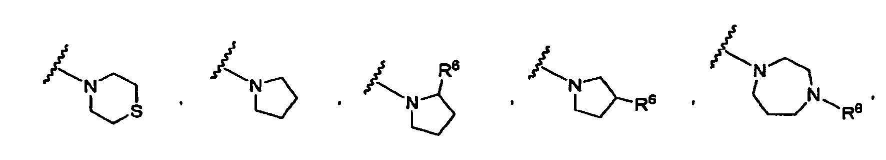 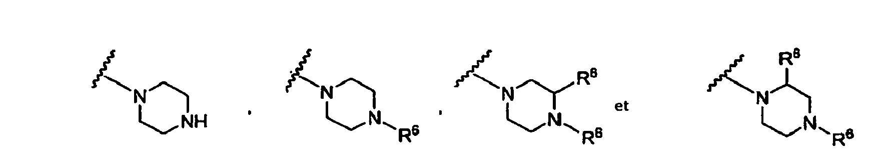
R³ représente un radical alkyle, choisi dans le groupe constitué par méthyle, éthyle, n-propyle, isopropyle, n-butyle, 2-butyle, isobutyle, tert- butyle, n-pentyle, sec-pentyle, néo-pentyle, (3,3)-diméthylbutyle, 4-méthyl-2-pentyle et n- hexyle, qui est à chaque fois non substitué ; ou
représente un radical phényle, qui est à chaque fois lié via un groupe -CH₂-, -CH(CH₃)-, -CH₂-CH₂-, -CH(CH₃)-CH₂-, -CH₂-CH(CH₃)- ou -CH₂-CH₂-CH₂- et /ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O-phényle, méthyle, éthyle, n-propyle, isopropyle, n-butyle, isobutyle, 2-butyle, tert- butyle, n-pentyle, néo-pentyle, éthényle, allyle, éthynyle, propynyle, -CH₂-O-CH₃, -CH₂-O-C₂H₅, -NH₂, -C(=O)-OH, -S-CH₃, -S-C₂H₅, -S(=O)-CH₃, -S(=O)₂-CH₃, -S(=O)-C₂H₅, -S(=O)₂-C₂H₅, -O-CH₃, -O-C₂H₅, -O-C₃H₇, -O-C(CH₃)₃, -CF₃, -CHF₂, -CH₂F, -O-CF₃, -O-CHF₂ et -O-CH₂F ;
R⁴ représente phényle, qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -OH, -SH, -NH₂, -C(=O)-OH, méthyle, éthyle, n-propyle, isopropyle, n-butyle, isobutyle, 2-butyle, tert-butyle, n-pentyle, -S- CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂ et -NH-CH₃ ; ou
représente un radical choisi dans le groupe constitué par thiényle, furyle, pyrrolyle, pyrazolyle, pyrazinyle, pyrannyle, triazolyle, pyridinyle, imidazolyle, thiadiazolyle, oxadiazolyle et pyridazinyle, qui peut à chaque fois être non substitué ou substitué par 1, 2, 3, 4 ou 5 substituants choisis indépendamment l'un de l'autre dans le groupe constitué par F, Cl, Br, I, -CN, -NO₂, -OH, -CH₂-CN, -SH, -NH₂, -C(=O)-OH, méthyle, éthyle, n-propyle, isopropyle, n-butyle, isobutyle, 2-butyle, tert-butyle, n-pentyle, néo- pentyle, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -S-CF₃, -S-CHF₂, -S-CH₂F, -N(CH₃)₂ et -NH-CH₃ ;
R⁵ représente un radical choisi dans le groupe constitué par phényle et naphtyle, qui peut à chaque fois être non substitué ou substitué par 1, 2, 3, 4 ou 5 substituants choisis indépendamment l'un de l'autre dans le groupe constitué par F, Cl, Br, I, méthyle, éthyle, n-propyle, isopropyle, n-butyle, isobutyle, 2-butyle, tert-butyle, n- pentyle, néo-pentyle, éthényle, allyle, éthynyle, propynyle, -NH₂, -O-CH₃, -O-C₂H₅, -O-C₃H₇, -O- C(CH₃)₃, -CF₃, -CHF₂, -CH₂F, -O-CF₃, -O-CHF₂, -O- CH₂F, -C(=O)-CF₃, -S-CF₃, -S-CHF₂, -S-CH₂F, -N(CH₃)₂, -N(C₂H₅)₂, -NH-CH₃ et -NH-C₂H₅ ;
R⁶ représente -OH ; F ; Cl ; Br ; I ; -SH ; -NO₂ ; -NH₂ ; -NH-C(=O)-O-R⁷ ; -C(=O)-O-R⁸ ; -C(=O)-R⁹ ; représente un radical alkyle choisi dans le groupe constitué par méthyle, éthyle, n-propyle, isopropyle, n-butyle, 2-butyle, isobutyle, tert- butyle, n-pentyle, sec-pentyle, néo-pentyle, (3,3)-diméthylbutyle, 4-méthyl-2-pentyle et n- hexyle, qui peut à chaque fois être non substitué ou substitué par 1, 2, 3, 4 ou 5 substituants choisis indépendamment l'un de l'autre dans le groupe constitué par F, Cl, Br, I, -NO₂, -CN, -OH, -SH, -NH₂, -N(CH₃)₂, -N(C₂H₅)₂, -N(CH₃)(C₂H₅), -C(=O)-pyrrolidinyle, -C(=O)-N(CH₃)-phényle et -C(=O)-NH-CH(CH₃)₂, -C(=O)-OH, -C(=O)-O-CH₃, -C(=O)-O-C₂H₅ et -C(=O)-O-C(CH₃)₃ ;
représente un radical choisi dans le groupe constitué par cyclopropyle, cyclobutyle, cyclopentyle, cyclohexyle, cycloheptyle, cyclohexényle, cyclopentényle, pyrrolidinyle, tétrahydrothiophényle, tétrahydrofurannyle, pipéridinyle, morpholinyle, pipérazinyle, azépanyle et diazépanyle, qui peut à chaque fois être non substitué ou substitué par 1, 2, 3, 4 ou 5 substituants choisis indépendamment l'un de l'autre dans le groupe constitué par méthyle, éthyle, n-propyle, isopropyle, n-butyle, 2-butyle, isobutyle, tert-butyle, -OH, -O-CH₃, -O-C₂H₅, -O- C₃H₇, -O-CF₃, -C(=O)-CH₃, -C(=O)-C₂H₅, -C(=O)- C(CH₃)₃, -C(=O)-NH-CH₃ et -C(=O)-NH-C₂H₅ et/ou qui peut à chaque fois être lié via un groupe -CH₂-, -CH(CH₃)-, -CH₂-CH₂-, -CH(CH₃)-CH₂-, -CH₂-CH(CH₃)- ou -CH₂-CH₂-CH₂- ; ou
représente un radical choisi dans le groupe constitué par phényle, naphtyle, indolizinyle, benzimidazolyle, tétrazolyle, triazinyle, isoxazolyle, phtalazinyle, thiényle, furyle, pyrrolyle, pyrazolyle, pyrazinyle, pyrannyle, triazolyle, pyridinyle, imidazolyle, indolyle, iso-indolyle, thiazolyle, thiadiazolyle, oxazolyle, oxadiazolyle, isoxazolyle, pyridazinyle, thiéno[2,3-d]pyrimidinyle et isoquinoléinyle, qui peut à chaque fois être lié via un groupe -CH₂-, -CH(CH₃)-, -CH₂-CH₂-, -CH(CH₃)-CH₂-, -CH₂-CH(CH₃)- ou -CH₂-CH₂-CH₂- et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, -OH, -SH, -O-phényle, méthyle, éthyle, n-propyle, isopropyle, n-butyle, isobutyle, 2-butyle, tert-butyle, n-pentyle, néo-pentyle, éthényle, -O-CH₃, -O-C₂H₅, -O-C₃H₇, -O-C(CH₃)₃, -CF₃, -CHF₂, -CH₂F, -O-CF₃, -O-CHF₂, -O-CH₂F, -C(=O)-CF₃, -C(=O)-H, -C(=O)-CH₃ et -C(=O)-C₂H₅ ;
et R⁷, R⁸ et R⁹, représentent, indépendamment l'un de l'autre, à chaque fois
un radical alkyle choisi dans le groupe constitué par méthyle, éthyle, n-propyle, isopropyle, n-butyle, 2-butyle, isobutyle, tert-butyle, n-pentyle, sec-pentyle, néo-pentyle, (3,3)-diméthylbutyle, 4-méthyl-2-pentyle et n-hexyle, qui peut à chaque fois être non substitué ou substitué par 1, 2, 3, 4 ou 5 substituants choisis indépendamment l'un de l'autre dans le groupe constitué par F, Cl, Br, I, -NO₂, -CN, -OH, -SH, -NH₂, -N(CH₃)₂, -N(C₂H₅)₂ et -N(CH₃)(C₂H₅) ;
un radical choisi dans le groupe constitué par indolinyle, indanyle, (1,2,3,4)-tétrahydronaphtyle, (2,3)-dihydrobenzo[1,4]dioxinyle et benzo[1,3]dioxolyle, qui est à chaque fois non substitué ; ou
un radical choisi dans le groupe constitué par phényle, naphtyle, isoxazolyle, thiényle, furyle, pyrrolyle, pyrazolyle, pyrazinyle, pyrannyle, triazolyle, pyridinyle, imidazolyle, indolyle, iso-indolyle, thiazolyle, thiadiazolyle, oxazolyle, oxadiazolyle, isoxazolyle et pyridazinyle, qui peut à chaque fois être lié via un groupe -CH₂-, -CH(CH₃)-, -CH₂-CH₂-, -CH(CH₃)-CH₂, -CH₂-CH(CH₃)- ou -CH₂-CH₂-CH₂- et/ou qui peut à chaque fois être non substitué ou substitué par 1, 2, 3, 4 ou 5 substituants choisis indépendamment l'un de l'autre dans le groupe constitué par F, Cl, Br, I, -CN, -NO₂, -O-phényle, méthyle, éthyle, n-propyle, isopropyle, n-butyle, isobutyle, 2-butyle, tert-butyle, n-pentyle, néo-pentyle, -S-CH₃, -S-C₂H₅, -O-CH₃, -O-C₂H₅, -O-C₃H₇, -O-C(CH₃)₃, -CF₃, -CHF₂, -CH₂F, -O-CF₃, -O-CHF₂ et -O-CH₂F.

4. Composés selon l'une ou plusieurs des revendications 1 à 3, **caractérisés**
**en ce que**
R¹ représente un radical alkyle choisi dans le groupe constitué par méthyle, éthyle, n-propyle, isopropyle, n-butyle, 2-butyle, isobutyle, tert- butyle, n-pentyle, sec-pentyle, néo-pentyle, (3,3)-diméthylbutyle, 4-méthyl-2-pentyle et n- hexyle, qui peut à chaque fois être non substitué ou substitué par 1, 2 ou 3 substituants choisis indépendamment l'un de l'autre dans le groupe constitué par -CN, -OH, -N(CH₃)₂, -N(C₂H₅)₂ -C(=O)- O-CH₃, -C(=O)-O-C₂H₅, -C(=O)-O-C(CH₃)₃, -C(=O)-O- CH₂-phényle, -C(=O)-NH-naphtyle, -N(CH₃)-phényle, -N(C₂H₅)-(m-toluyle) et -N(C₂H₅)-(p-toluyle) ;
représente un radical hétéroalkyle choisi dans le groupe constitué par -CH₂-O-CH₃, -CH₂-O-C₂H₅, -CH₂- CH₂-O-CH₃, -CH₂-CH₂-O-C₂H₅, -CH₂-CH₂-CH₂-O-CH₃ et -CH₂-CH₂-CH₂-O-C₂H₅ ;
représente un radical choisi dans le groupe constitué par cyclopropyle, cyclobutyle, cyclopentyle, cyclohexyle, cycloheptyle, pyrrolidinyle, tétrahydrofurannyle, pipéridinyle, morpholinyle, pipérazinyle, azépanyle, dihydrofurann-2(3H)-onyle, indanyle et (1,2,3,4)-tétrahydronaphtyle, qui peut à chaque fois être non substitué ou substitué par 1, 2, 3, 4 ou 5 substituants choisis indépendamment l'un de l'autre dans le groupe constitué par méthyle, éthyle, n-propyle, isopropyle, n-butyle, 2-butyle, isobutyle, tert-butyle, -CH₂-naphtyle, -O-phényle, -O-CH₂-phényle, benzyle et phényle et/ou qui peut à chaque fois être lié via un groupe -CH₂-, -CH(CH₃), -CH₂-CH₂-, -CH(CH₃)-CH₂-, -CH₂-CH(CH₃)- ou -CH₂-CH₂-CH₂- ;
représente phényle, qui peut à chaque fois être lié via un groupe -CH₂-CH₂-O-, -CH₂-O-, -CH₂-CH₂-CH₂-O-, -CH[C(=O)-O-CH₃]-CH₂-, -CH[C(=O)-O-C₂H₅]-CH₂-, -CH₂-, -CH(CH₃)-, -CH₂-CH₂-, -CH(CH₃)-CH₂-, -CH₂-CH(CH₃)- ou -CH₂-CH₂-CH₂- et/ou qui peut à chaque fois être non substitué ou substitué par 1, 2, 3, 4 ou 5 substituants choisis indépendamment l'un de l'autre dans le groupe constitué par F, Cl, Br, I, -CH₂-CN, méthyle, éthyle, n-propyle, isopropyle, n-butyle, isobutyle, 2-butyle, tert-butyle, n-pentyle, néo-pentyle, -O-phényle, -O-CH₃, -O-C₂H₅, -O-C₃H₇, -O-C(CH₃)₃, -CF₃, -CHF₂, -CH₂F, -O-CF₃, -O-CHF₂, -O-CH₂F, -N(CH₃)₂, -N(C₂H₅)₂, -S(=O)-NH₂ et [1,2,3]-thiadiazolyle ;
représente un radical choisi dans le groupe constitué par thiényle, furyle, pyrrolyle, pyrazolyle, pyridinyle, imidazolyle, indolyle et iso-indolyle, qui peut à chaque fois être lié via un groupe -CH₂-, -CH(CH₃)-, -CH₂-CH₂-, -CH(CH₃)-CH₂, -CH₂-CH(CH₃)- ou -CH₂-CH₂-CH₂- et/ou qui peut à chaque fois être non substitué ou substitué par 1, 2 ou 3 substituants choisis indépendamment l'un de l'autre dans le groupe constitué par -NO₂, -OH, méthyle, éthyle, n-propyle, isopropyle, n-butyle, isobutyle, 2-butyle, tert-butyle, n-pentyle et néo-pentyle ou
représente -NH-C(=O)-R⁵ ;
R² représente H ou un radical alkyle, choisi dans le groupe constitué par méthyle, éthyle, n-propyle, isopropyle, n-butyle, 2-butyle, isobutyle, tert- butyle, n-pentyle, sec-pentyle, néo-pentyle, (3,3)-diméthylbutyle, 4-méthyl-2-pentyle et n- hexyle, qui peut à chaque fois être non substitué ; ou
représente un radical choisi dans le groupe constitué par phényle et naphtyle, qui peut à chaque fois être lié via un groupe -CH₂-, -CH(CH₃)-, -CH₂-CH₂-, -CH(CH₃)-CH₂-, -CH₂-CH(CH₃)- ou -CH₂-CH₂-CH₂- et/ou qui peut être chaque fois non substitué ou substitué par 1, 2 ou 3 substituants choisis indépendamment l'un de l'autre dans le groupe constitué par -O-CH₃, -O-C₂H₅, -O-C₃H₇ et -O- C(CH₃)₃ ; ou
R¹ et R² forment ensemble avec l'atome d'azote qui les relie un radical choisi dans le groupe constitué par 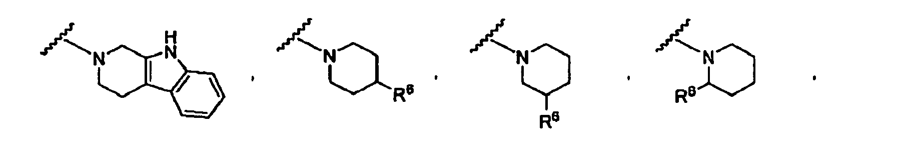 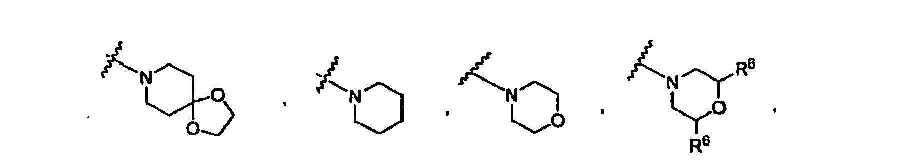 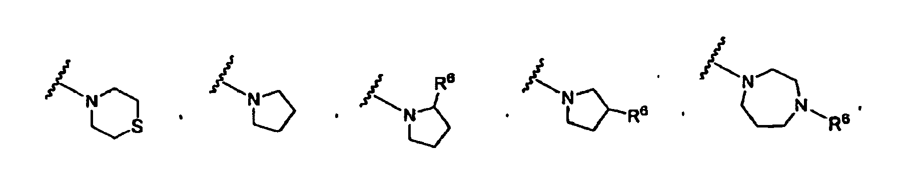 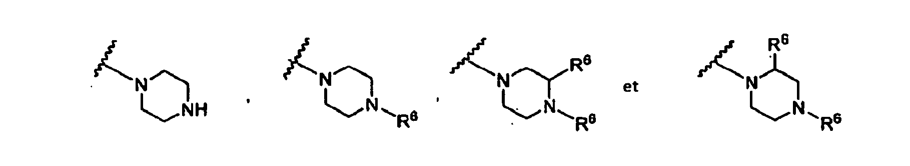
R³ représente un radical alkyle, choisi dans le groupe constitué par méthyle, éthyle, n-propyle, isopropyle, n-butyle, 2-butyle, isobutyle, tert- butyle, n-pentyle, sec-pentyle, néo-pentyle, (3,3)-diméthylbutyle, 4-méthyl-2-pentyle et n- hexyle, qui peut à chaque fois être non substitué ; ou
représente un radical phényle, qui est à chaque fois lié via un groupe -CH₂-, -CH(CH₃)-, -CH₂-CH₂-, -CH(CH₃)-CH₂-, -CH₂-CH(CH₃)- ou -CH₂-CH₂-CH₂- et/ou qui peut à chaque fois être non substitué ou substitué par 1, 2, 3, 4 ou 5 substituants choisis indépendamment l'un de l'autre dans le groupe constitué par F, Cl, Br, I, -CN, -CH₂-CN, -NO₂, méthyle, éthyle, n-propyle, isopropyle, n-butyle, isobutyle, 2-butyle, tert-butyle, n-pentyle, néo- pentyle, -O-CH₃, -O-C₂H₅, -O-C₃H₇, -O-C(CH₃)₃, -CF₃, -CHF, -CH₂F, -O-CF₃, -O-CHF₂ et -O-CH₂F ;
R⁴ représente phényle, qui peut à chaque fois être non substitué ou substitué par 1, 2, 3, 4 ou 5 substituants choisis indépendamment l'un de l'autre dans le groupe constitué par F, Cl, Br, I, méthyle, éthyle, n-propyle, isopropyle, n-butyle, isobutyle, 2-butyle, tert-butyle, n-pentyle, -O- CH₃, -O-C₂H₅, -O-C₃H₇, -O-C(CH₃)₃, -O-CF₃, -O-CHF₂ et -O-CH₂F ; ou
représente un radical choisi dans le groupe constitué par thiényle, furyle et pyrrolyle, qui peut à chaque fois être non substitué ou substitué par 1, 2, 3, 4 ou 5 substituants choisis indépendamment l'un de l'autre dans le groupe constitué par F, Cl, Br, I, méthyle, éthyle, n- propyle, isopropyle, n-butyle, isobutyle, 2- butyle, -O-CH₃, -O-C₂H₅, -O-C₃H₇ et -O-C(CH₃)₃ ;
R⁵ représente un radical choisi dans le groupe constitué par phényle et naphtyle, qui peut à chaque fois être non substitué ou substitué par 1, 2, 3, 4 ou 5 substituants choisis indépendamment l'un de l'autre dans le groupe constitué par F, Cl, Br, I, méthyle, éthyle, n-propyle, isopropyle, n-butyle, isobutyle, 2-butyle, tert-butyle, -NH₂, -O-CH₃, -O-C₂H₅, -O-C₃H₇, -O-C(CH₃)₃, -N(CH₃)₂, -N(C₂H₅)2, -NH-CH₃ et -NH-C₂H₅ ;
R⁶ représente -OH ; -NH-C(=O)-O-R₇ ; -C(=O)-O-R⁸ ; -C(=O)-R⁹,
représente un radical alkyle choisi dans le groupe constitué par méthyle, éthyle, n-propyle, isopropyle, n-butyle, 2-butyle, isobutyle, tert- butyle, n-pentyle, sec-pentyle, néo-pentyle, (3,3)-diméthylbutyle, 4-méthyl-2-pentyle et n- hexyle, qui peut à chaque fois être non substitué ou substitué par 1, 2 ou 3 substituants choisis indépendamment l'un de l'autre dans le groupe constitué par -OH, -N(CH₃)₂, -N(C₂H₅)₂, -C(=O)- pyrrolidinyle, -C(=O)-N(CH3)-phényle et -C(=O)-NH- CH(CH₃)₂ ;
représente un radical choisi dans le groupe constitué par cyclopropyle, cyclobutyle, cyclopentyle, cyclohexyle, cycloheptyle, pyrrolidinyle, pipéridinyle et azépanyle, qui est à chaque fois non substitué et/ou qui peut à chaque fois être lié via un groupe -CH₂-, -CH(CH₃)-, -CH₂-CH₂-, -CH(CH₃)-CH₂-, -CH₂-CH(CH₃)- ou -CH₂-CH₂-CH₂- ; ou
représente un radical choisi dans le groupe constitué par phényle, naphtyle, thiényle, furyle, pyrazinyle, pyridinyle, pyridazinyle et thiéno[2,3-d]pyrimidinyle, qui peut à chaque fois être lié via un groupe -CH₂-, -CH(CH₃)-, -CH₂-CH₂-, -CH(CH₃)-CH₂-, -CH₂-CH(CH₃)- ou -CH₂-CH₂-CH₂- et/ou qui peut à chaque fois être non substitué ou substitué par 1, 2, 3, 4 ou 5 substituants choisis indépendamment l'un de l'autre dans le groupe constitué par F, Cl, Br, I, -CN, méthyle, tert- butyle, -O-CH₃, -O-C₂H₅, -CF₃, -C(=O)-CH₃ et -C(=O)- C₂H₅ ;
et R⁷, R⁸ et R⁹ représentent, indépendamment l'un de l'autre, à chaque fois
un radical alkyle choisi dans le groupe constitué par méthyle, éthyle, n-propyle, isopropyle, n-butyle, 2-butyle, isobutyle, tert-butyle et n-pentyle, qui peut à chaque fois être non substitué ;
un radical choisi dans le groupe constitué par (2,3)-dihydrobenzo[1,4]dioxinyle et benzo[1,3]dioxolyle, qui peut à chaque fois être non substitué ; ou
un radical choisi dans le groupe constitué par phényle, thiényle, furyle et pyrazinyle, qui peut être lié à chaque fois via un groupe -CH₂-, -CH(CH₃)-, -CH₂-CH₂-, -CH(CH₃)-CH₂-, -CH₂-CH(CH₃)- ou -CH₂-CH₂-CH₂- et/ou qui peut à chaque fois être non substitué ou substitué par 1, 2, 3, 4 ou 5 substituants choisis indépendamment l'un de l'autre dans le groupe constitué par F, Cl, Br, I, méthyle, éthyle, n-propyle, isopropyle, n-butyle, isobutyle, 2-butyle, tert-butyle, n-pentyle, néo-pentyle, -O-CH₃, -O-C₂H₅, -O-C₃H₇ et -O-C(CH₃)₃.

5. Composés selon l'une ou plusieurs des revendications 1 à 4, **caractérisés**
**en ce que**
R¹ représente un radical choisi dans le groupe constitué par -CH₂-CH₂-N(C₂H₅)-(m-toluyle), -CH₂- CH₂-N(C₂H₅)-(p-toluyle), -CH₂-CH₂-CH₂-N (CH₃) -phényle, -CH₂-CH₂-CH₂-N(CH₃)₂, -CH₂-CH₂-CH₂-N(C₂H₅)₂, -CH(CH₃)- CH₂-CH₂-CH(CH₃)₂, -CH₂-CH₂-CN, -CH₂-CH₂-OH, -CH₂-CH₂- N(CH₃)₂, -CH₂-CH₂-N(C₂H₅)₂, -CH₂-CH₂-C(CH₃)₃, n- pentyle, n-butyle, méthyle, éthyle, n-propyle, -CH(CH₃)-C(=O)-O-CH₂-phényle, -CH[CH(CH₃)₂]-C(=O)-O- C(CH₃)₃, -CH₂-C(=O)-O-CH₂-phényle, -CH₂-CH₂-C(=O)-O- C(CH₃)₃, -CH₂-CH₂-C(=O)-O-C₂H₅, -CH₂-C(=O)-NH- naphtyle et -CH₂-CH₂-CH₂-O-CH₃ ;
représente un radical choisi dans le groupe constitué par cyclopropyle, cyclobutyle, cyclopentyle, cyclohexyle, cycloheptyle, pyrrolidinyle, tétrahydrofurannyle, pipéridinyle, morpholinyle, pipérazinyle, azépanyle, dihydrofurann-2(3H)-onyle, indanyle et (1,2,3,4)- tétrahydronaphtyle, qui peut à chaque fois être non substitué ou substitué par 1, 2, 3, 4 ou 5 substituants choisis indépendamment l'un de l'autre dans le groupe constitué par méthyle, éthyle, -CH₂-naphtyle, -O-phényle, -O-CH₂-phényle et benzyle et/ou qui peut à chaque fois être lié via un groupe -CH₂-, -CH₂-CH₂- ou -CH₂-CH₂-CH₂- ;
représente phényle, qui peut à chaque fois être lié via un groupe -CH₂-CH₂-O-, -CH[C(=O)-O-CH₃]- CH₂-, -CH[C(=O)-O-C₂H₅]-CH₂-, -CH₂-, -CH(CH₃)-, -CH₂- CH₂-, -CH(CH₃)-CH₂-, -CH₂-CH(CH₃)- ou -CH₂-CH₂-CH₂- et/ou qui peut à chaque fois être non substitué ou substitué par 1, 2, 3, 4 ou 5 substituants choisis indépendamment l'un de l'autre dans le groupe constitué par F, Cl, Br, I, -CH₂-CN, méthyle, éthyle, n-propyle, isopropyle, n-butyle, isobutyle, 2-butyle, tert-butyle, -O-phényle, -O- CH₃, -O-C₂H₅, -CF₃, -O-CF₃, -N(CH₃)₂, -N(C₂H₅)₂, -S(=O)-NH₂ et [1,2,3]-thiadiazolyle ;
représente un radical choisi dans le groupe constitué par thiényle, furyle, pyridinyle, imidazolyle, indolyle et iso-indolyle, qui peut à chaque fois être lié via un groupe -CH₂-, -CH₂-CH₂- ou -CH₂-CH₂-CH₂- et/ou qui peut à chaque fois être non substitué ou substitué par 1, 2 ou 3 substituants choisis indépendamment l'un de l'autre dans le groupe constitué par -NO₂, -OH, méthyle, éthyle et n-propyle
ou représente -NH-C(=O)-R⁵ ;
R² représente H ou un radical alkyle choisi dans le groupe constitué par méthyle, éthyle et n-propyle, qui est à chaque fois non substitué ; ou représente un radical choisi dans le groupe constitué par phényle et naphtyle, qui peut être lié à chaque fois via un groupe -CH₂-, et/ou qui peut être chaque fois non substitué ou substitué par 1, 2 ou 3 substituants choisis indépendamment l'un de l'autre dans le groupe constitué par -O-CH₃, -O-C₂H₅, -O-C₃H₇ et -O-C(CH₃)₃ ; ou
R¹ et R² forment ensemble avec l'atome d'azote qui les relie un radical choisi dans le groupe constitué par 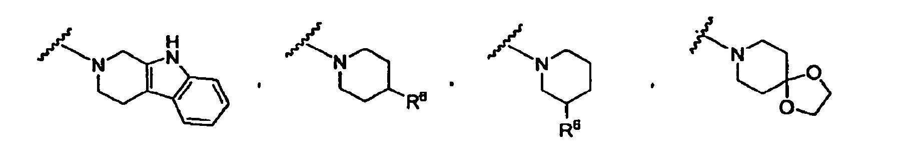 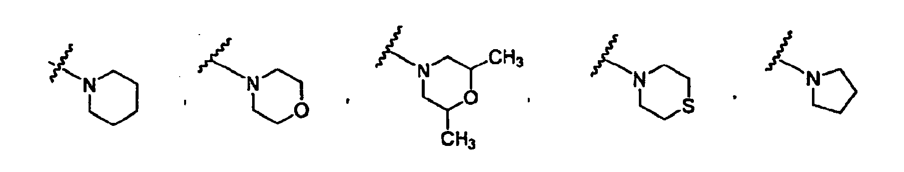 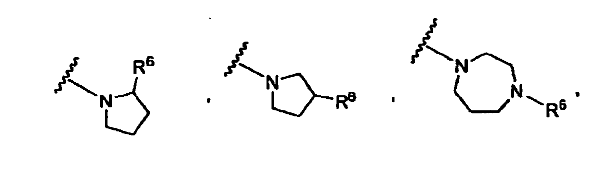 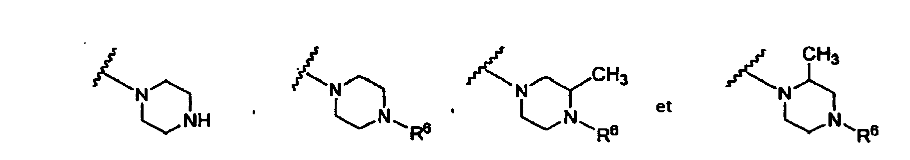
R³ représente un radical alkyle choisi dans le groupe constitué par méthyle, éthyle, n-propyle, isopropyle, n-butyle, 2-butyle, isobutyle et tert- butyle, qui peut à chaque fois être non substitué ; ou
représente un radical phényle, qui est à chaque fois lié via un groupe -CH₂- et/ou qui peut à chaque fois être non substitué ou substitué par 1, 2, 3, 4 ou 5 substituants choisis indépendamment l'un de l'autre dans le groupe constitué par F, Cl, Br, méthyle, éthyle, n-propyle, -O-CH₃, -O- C₂H₅, et -CF₃ ;
R⁴ représente phényle, qui peut à chaque fois être non substitué ou substitué par 1, 2, 3, 4 ou 5 substituants choisis indépendamment l'un de l'autre dans le groupe constitué par F, Cl, Br, méthyle, éthyle, n-propyle, -O-CH₃ et -O-C₂H₅ ; ou représente un radical choisi dans le groupe constitué par thiényle, furyle et pyrrolyle, qui peut à chaque fois être non substitué ;
R⁵ représente un radical choisi dans le groupe constitué par phényle et naphtyle, qui peut à chaque fois être non substitué ou substitué par 1, 2, 3, 4 ou 5 substituants choisis indépendamment l'un de l'autre dans le groupe constitué par F, Cl, Br, méthyle, éthyle, -O-CH₃, -O-C₂H₅, -N(CH₃)₂, -N(C₂H₅)₂, -NH-CH₃ et -NH-C₂H₅ ;
R⁶ représente -OH ; -NH-C(=O)-O-R⁷ ; -C(=O)-O-R⁸ ; -C(=O)-R⁹,
représente un radical choisi dans le groupe constitué par méthyle, éthyle, n-propyle, isopropyle, n-butyle, -CH₃-CH(CH₃)₂, -CH₂-CH₂-OH, -CH₂-CH₂-N(CH₃)₂, -CH₂-CH₂-N(C₂H₅), -CH₂-C(=O)- pyrrolidinyle, -CH₂-C(=O)-NH-CH(CH₃)₂ et -CH₂-C(=O)- N(CH₃)-phényle ;
représente un radical choisi dans le groupe constitué par cyclopropyle, cyclobutyle, cyclopentyle, cyclohexyle, cycloheptyle, pyrrolidinyle, pipéridinyle et azépanyle, qui est à chaque fois non substitué et/ou qui peut à chaque fois être lié via un groupe -CH₂- ; ou
représente un radical choisi dans le groupe constitué par phényle, naphtyle, thiényle, pyrazinyle, pyridinyle et thiéno[2,3- d]pyrimidinyle, qui peut à chaque fois être lié via un groupe -CH₂-, -CH₂-CH₂- ou -CH₂-CH₂-CH₂- et/ou qui peut à chaque fois être non substitué ou substitué par 1, 2, 3, 4 ou 5 substituants choisis indépendamment l'un de l'autre dans le groupe constitué par F, Cl, Br, -CN, méthyle, tert- butyle, -O-CH₃, -O-C₂H₅, -CF₃, -C(=O)-CH₃ et -C(=O)- C₂H₅ ;
R⁷ représente un radical alkyle choisi dans le groupe constitué par méthyle, éthyle, n-propyle, isopropyle, n-butyle, 2-butyle, isobutyle, tert- butyle et n-pentyle, qui est à chaque fois non substitué ;
R⁸ représente un radical alkyle choisi dans le groupe constitué par méthyle, éthyle, n-propyle, isopropyle, n-butyle, 2-butyle, isobutyle, tert- butyle et n-pentyle, qui est à chaque fois non substitué ; ou
représente un radical phényle ou benzyle ; et
R⁹ représente un radical choisi dans le groupe constitué par (2,3)-dihydrobenzo[1,4]dioxinyle et benzo[1,3]dioxolyle, qui est à chaque fois non substitué ; ou
représente un radical choisi dans le groupe constitué par phényle, thiényle, furyle et pyrazinyle, qui peut à chaque fois être lié via un groupe -CH₂-, -CH₂-CH₂- ou -CH₂-CH₂-CH₂- et/ou qui peut à chaque fois être non substitué ou substitué par 1, 2, 3, 4 ou 5 substituants choisis indépendamment l'un de l'autre dans le groupe constitué par F, Cl, Br, méthyle, éthyle -O-CH₃ et -O-C₂H₅5.

6. Composés selon l'une quelconque des revendications 1 à 5 choisis dans le groupe constitué par
| | |
|---|---|
| 1. | 4-[1,2,3]thiadiazol-4-ylbenzylamide de l'acide 1-benzyl-4-méthyl-3-p-toluyl-1H-pyrrole-2-carboxylique, |
| 2. | 4-sulfamoylbenzylamide de l'acide 3-furann-2-yl-1,4-diméthyl-1H-pyrrole-2-carboxylique, |
| 3. | 2,4-diméthoxybenzylamide de l'acide 1-(4-fluorobenzyl)-3-furann-2-yl-4-méthyl-1H-pyrrole-2-carboxylique, |
| 4. | (2-pyridin-2-yléthyl)-amide de l'acide 1-butyl-4-méthyl-3-p-toluyl-1H-pyrrole-2-carboxylique, |
| 5. | [2-(5-hydroxy-1H-indol-3-yl)-éthyl]-amide de l'acide 1-(4-fluorobenzyl)-3-(4-méthoxyphényl)-4-méthyl-1H-pyrrole-2-carboxylique, |
| 6. | (1,4-diméthyl-3-p-toluyl-1H-pyrrol-2-yl)-(4-pyrrolidin-1-ylpipéridin-1-yl)-méthanone, |
| 7. | 4-bromo-2-fluorobenzylamide de l'acide 1-benzyl-3-furann-2-yl-4-méthyl-1H-pyrrole-2-carboxylique, |
| 8. | [1-benzyl-3-(4-chlorophényl)-4-méthyl-1H-pyrrol-2-yl]-[4-(2-chlorophényl)-pipérazin-1-yl]-méthanone, |
| 9. | (3-diméthylaminopropyl)-amide de l'acide 1,4-diméthyl-3-phényl-1H-pyrrole-2-carboxylique, |
| 10. | (2-pyrrolidin-1-yl-éthyl)-amide de l'acide 1,4-diméthyl-3-p-toluyl-1H-pyrrole-2-carboxylique, |
| 11. | 4-diméthylaminobenzylamide de l'acide 1-(4-fluorobenzyl)-3-furann-2-yl-4-méthyl-1H-pyrrole-2-carboxylique, |
| 12. | 1-[4-(1-benzyl-4-méthyl-3-phényl-1H-pyrrole-2-carbonyl)-pipérazin-1-yl]-2-phényléthanone, |
| 13. | 2,5-difluorobenzylamide de l'acide 1-benzyl-3-furann-2-yl-4-méthyl-1H-pyrrole-2-carboxylique, |
| 14. | 3,5-difluorobenzylamide de l'acide 1-(2,6-dichlorobenzyl)-4-méthyl-3-p-toluyl-1H-pyrrole-2-carboxylique, |
| 15. | (2-méthylcyclohexyl)-amide de l'acide 1-(2-bromobenzyl)-3-(4-chlorophényl)-4-méthyl-1H-pyrrole-2-carboxylique, |
| 16. | (3-morpholin-4-ylpropyl)-amide de l'acide 1,4-diméthyl-3-p-toluyl-1H-pyrrole-2-carboxylique, |
| 17. | (1,3-diméthylbutyl)-amide de l'acide 1-butyl-4-méthyl-3-p-toluyl-1H-pyrrole-2-carboxylique, |
| 18. | [4-(2,4-diméthylphényl)-pipérazin-1-yl]-[4-méthyl-1-(4-méthylbenzyl)-3-p-toluyl-1H-pyrrol-2-yl]-méthanone, |
| 19. | méthyl-(2-pyridin-2-yléthyl)-amide de l'acide 1-(2,6-dichlorobenzyl)-4-méthyl-3-p-toluyl-1H-pyrrole-2-carboxylique, |
| 20. | (2-cyanoéthyl)-méthylamide de l'acide 1,4-diméthyl-3-phényl-1H-pyrrole-2-carboxylique, |
| 21. | (3-imidazol-1-ylpropyl)-amide de l'acide 1-(2,6-dichlorobenzyl)-4-méthyl-3-p-toluyl-1H-pyrrole-2-carboxylique, |
| 22. | (1,3-diméthylbutyl)-amide de l'acide 1-(4-fluorobenzyl)-3-(4-méthoxyphényl)-4-méthyl-1H-pyrrole-2-carboxylique, |
| 23. | 2-éthoxybenzylamide de l'acide 3-(4-méthoxyphényl)-1,4-diméthyl-1H-pyrrole-2-carboxylique, |
| 24. | (4-cycloheptylpipérazin-1-yl)-(1,4-diméthyl-3-p-toluyl-1H-pyrrol-2-yl)-méthanone, |
| 25. | (2-diméthylaminoéthyl)-amide de l'acide 1-(4-fluorobenzyl)-3-(4-méthoxyphényl)-4-méthyl-1H-pyrrole-2-carboxylique, |
| 26. | (pyridin-2-ylméthyl)-amide de l'acide 1-benzyl-4-méthyl-3-phényl-1H-pyrrole-2-carboxylique, |
| 27. | [2-(4-chlorophényl)-propyl]-amide de l'acide 3-(4-chlorophényl)-1-éthyl-4-méthyl-1H-pyrrole-2-carboxylique, |
| 28. | 3,5-difluorobenzylamide de l'acide 3-(4-chlorophényl)-1-(2-fluorobenzyl)-4-méthyl-1H-pyrrole-2-carboxylique, |
| 29. | ester benzylique de l'acide 2-{[1-(4-fluorobenzyl)-3-(4-méthoxyphényl)-4-méthyl-1H-pyrrole-2-carbonyl]-amino}-propionique, |
| 30. | (1-benzyl-4-méthyl-3-p-toluyl-1H-pyrrol-2-yl)-[4-(2-diméthylaminoéthyl)-pipérazin-1-yl]-méthanone, |
| 31. | (3,3-diméthylbutyl)-amide de l'acide 3-furann-2-yl-1-(4-méthoxybenzyl)-4-méthyl-1H-pyrrole-2-carboxylique, |
| 32. | 4-diméthylaminobenzylamide de l'acide 3-(4-chlorophényl)-1-isobutyl-4-méthyl-1H-pyrrole-2-carboxylique, |
| 33. | (2-pyridin-2-yléthyl)-amide de l'acide 3-(4-chlorophényl)-1-éthyl-4-méthyl-1H-pyrrole-2-carboxylique, |
| 34. | 2,3-diméthoxybenzylamide de l'acide 3-(4-chlorophényl)-1-éthyl-4-méthyl-1H-pyrrole-2-carboxylique, |
| 35. | (2-thiophén-2-yléthyl)-amide de l'acide 1-(4-fluorobenzyl)-3-(4-méthoxyphényl)-4-méthyl-1H-pyrrole-2-carboxylique, |
| 36. | ester tert-butylique de l'acide 2-{[3-(4-chlorophényl)-1-éthyl-4-méthyl-1H-pyrrole-2-carbonyl]-amino}-3-méthylbutyrique, |
| 37. | (1,2,3,4-tétrahydronaphtalén-1-yl)-amide de l'acide 3-furann-2-yl-1-(4-méthoxybenzyl)-4-méthyl-1H-pyrrole-2-carboxylique, |
| 38. | (2-pyrrolidin-1-yléthyl)-amide de l'acide 3-furann-2-yl-4-méthyl-1-(4-trifluorométhylbenzyl)-1H-pyrrole-2-carboxylique, |
| 39. | ester benzylique de l'acide {[1-(4-méthoxybenzyl)-4-méthyl-3-p-toluyl-1H-pyrrole-2-carbonyl]-méthylamino}-acétique, |
| 40. | [2-(1-méthylpyrrolidin-2-yl)-éthyl]-amide de l'acide 3-furann-2-yl-1-(4-méthoxybenzyl)-4-méthyl-1H-pyrrole-2-carboxylique, |
| 41. | [4-(5-bromo-2-éthoxybenzyl)-pipérazin-1-yl]-(1,4-diméthyl-3-phényl-1H-pyrrol-2-yl)-méthanone, |
| 42. | (1-benzyl-3-furann-2-yl-4-méthyl-1H-pyrrol-2-yl)-[4-(3-fluoro-4-méthoxybenzyl)-pipérazin-1-yl]-méthanone, |
| 43. | ester tert-butylique de l'acide 3-{[3-(4-chlorophényl)-1-éthyl-4-méthyl-1H-pyrrole-2-carbonyl]-amino}-propionique, |
| 44. | 3-méthoxybenzylamide de l'acide 1-benzyl-4-méthyl-3-phényl-1H-pyrrole-2-carboxylique, |
| 45. | (1-benzyl-3-furann-2-yl-4-méthyl-1H-pyrrol-2-yl)-[4-(5-bromo-2-éthoxybenzyl)-pipérazin-1-yl]-méthanone, |
| 46. | [3-(4-chlorophényl)-1,4-diméthyl-1H-pyrrol-2-yl]-(4-o-toluylpipérazin-1-yl)-méthanone, |
| 47. | [2-(4-éthoxyphényl)-éthyl]-amide de l'acide 1-(4-fluorobenzyl)-3-(4-méthoxyphényl)-4-méthyl-1H-pyrrole-2-carboxylique, |
| 48. | [1-(2,6-dichlorobenzyl)-4-méthyl-3-p-toluyl-1H-pyrrol-2-yl]-(4-hydroxypipéridin-1-yl)-méthanone, |
| 49. | (1,4-diméthyl-3-p-toluyl-1H-pyrrol-2-yl)-[4-(2,4,6-triméthoxybenzyl)-pipérazin-1-yl]-méthanone, |
| 50. | (2-morpholin-4-yléthyl)-amide de l'acide 1-(4-fluorobenzyl)-3-furann-2-yl-4-méthyl-1H-pyrrole-2-carboxylique, |
| 51. | [2-(4-fluorophényl)-éthyl]-amide de l'acide 1-benzyl-3-(4-chlorophényl)-4-méthyl-1H-pyrrole-2-carboxylique, |
| 52. | [4-(2,5-diméthylphényl)-pipérazin-1-yl]-[3-(4-méthoxyphényl)-1,4-diméthyl-1H-pyrrol-2-yl]-méthanone, |
| 53. | (3-imidazol-1-ylpropyl)-amide de l'acide 3-(4-méthoxyphényl)-1,4-diméthyl-1H-pyrrole-2-carboxylique, |
| 54. | [2-(3-trifluorométhylphényl)-éthyl]-amide de l'acide 1-(4-fluorobenzyl)-3-(4-méthoxyphényl)-4-méthyl-1H-pyrrole-2-carboxylique, |
| 55. | [3-(4-chlorophényl)-1-(2-fluorobenzyl)-4-méthyl-1H-pyrrol-2-yl]-[4-(5-méthylpyrazine-2-carbonyl)-pipérazin-1-yl]-méthanone, |
| 56. | 3-fluoro-5-trifluorométhylbenzylamide de l'acide 3-(4-chlorophényl)-1-isobutyl-4-méthyl-1H-pyrrole-2-carboxylique, |
| 57. | [3-(4-chlorophényl)-1,4-diméthyl-1H-pyrrol-2-yl]-(4-pyridin-4-ylpipérazin-1-yl)-méthanone, |
| 58. | (3-imidazol-1-ylpropyl)-amide de l'acide 3-(4-chlorophényl)-1-(2-fluorobenzyl)-4-méthyl-1H-pyrrole-2-carboxylique, |
| 59. | (1,4-diméthyl-3-phényl-1H-pyrrol-2-yl)-[4-(3-fluoro-4-méthoxybenzyl)-pipérazin-1-yl]-méthanone, |
| 60. | [2-(4-bromophényl)-éthyl]-amide de l'acide 1-benzyl-3-furann-2-yl-4-méthyl-1H-pyrrole-2-carboxylique, |
| 61. | (4-cycloheptylpipérazin-1-yl)-(3-furann-2-yl-1,4-diméthyl-1H-pyrrol-2-yl)-méthanone, |
| 62. | [2-(4-méthoxyphénoxy)-éthyl]-amide de l'acide 1-benzyl-4-méthyl-3-phényl-1H-pyrrole-2-carboxylique, |
| 63. | (3-diméthylaminopropyl)-amide de l'acide 1-(4-fluorobenzyl)-3-furann-2-yl-4-méthyl-1H-pyrrole-2-carboxylique, |
| 64. | [1-(4-fluorobenzyl)-3-(4-méthoxyphényl)-4-méthyl-1H-pyrrol-2-yl]-(2-pyrrolidin-1-ylméthylpyrrolidin-1-yl)-méthanone, |
| 65. | (3-diéthylaminopropyl)-amide de l'acide 3-(4-chlorophényl)-1-isobutyl-4-méthyl-1H-pyrrole-2-carboxylique, |
| 66. | [3-(4-chlorophényl)-1,4-diméthyl-1H-pyrrol-2-yl]-[4-(2-fluoro-5-méthoxybenzyl)-pipérazin-1-yl]-méthanone, |
| 67. | pentylamide de l'acide 3-(4-méthoxyphényl)-1,4-diméthyl-1H-pyrrole-2-carboxylique, |
| 68. | (1-butyl-4-méthyl-3-p-toluyl-1H-pyrrol-2-yl)-[4-(2,5-diméthoxybenzyl)-pipérazin-1-yl]-méthanone, |
| 69. | [4-(2-diéthylaminoéthyl)-pipérazin-1-yl]-[4-méthyl-1-(4-méthylbenzyl)-3-p-toluyl-1H-pyrrol-2-yl]-méthanone, |
| 70. | [4-(3-chlorophényl)-pipérazin-1-yl]-[3-(4-méthoxyphényl)-1,4-diméthyl-1H-pyrrol-2-yl]-méthanone, |
| 71. | [1-(2-bromobenzyl)-3-(4-chlorophényl)-4-méthyl-1H-pyrrol-2-yl]-(4-isopropylpipérazin-1-yl)-méthanone, |
| 72. | [4-(2-diméthylaminoéthyl)-pipérazin-1-yl]-[1-(4-fluorobenzyl)-3-(4-méthoxyphényl)-4-méthyl-1H-pyrrol-2-yl]-méthanone, |
| 73. | 2,4-diméthoxybenzylamide de l'acide 3-(4-chlorophényl)-1-(2-fluorobenzyl)-4-méthyl-1H-pyrrole-2-carboxylique, |
| 74. | (2-pyrrolidin-1-yléthyl)-amide de l'acide 1-benzyl-4-méthyl-3-p-toluyl-1H-pyrrole-2-carboxylique, |
| 75. | [1-(4-fluorobenzyl)-3-furann-2-yl-4-méthyl-1H-pyrrol-2-yl]-[4-(3-fluoro-4-méthoxybenzyl)-pipérazin-1-yl]-méthanone, |
| 76. | 2,4-diméthoxybenzylamide de l'acide 3-(4-méthoxyphényl)-1,4-diméthyl-1H-pyrrole-2-carboxylique, |
| 77. | (2-pyrrolidin-1-yléthyl)-amide de l'acide 1-benzyl-4-méthyl-3-phényl-1H-pyrrole-2-carboxylique, |
| 78. | [3-(2-méthylpipéridin-1-yl)-propyl]-amide de l'acide 3-(4-chlorophényl)-1,4-diméthyl-1H-pyrrole-2-carboxylique, |
| 79. | cyclohexylamide de l'acide 1-(4-fluorobenzyl)-3-furann-2-yl-4-méthyl-1H-pyrrole-2-carboxylique, |
| 80. | 4-fluoro-2-trifluorométhylbenzylamide de l'acide 3-furann-2-yl-1,4-diméthyl-1H-pyrrole-2-carboxylique, |
| 81. | (1-naphtalén-2-yl-éthyl)-amide de l'acide 1-benzyl-3-furann-2-yl-4-méthyl-1H-pyrrole-2-carboxylique, |
| 82. | (furann-2-ylméthyl)-amide de l'acide 3-(4-chlorophényl)-1-éthyl-4-méthyl-1H-pyrrole-2-carboxylique, |
| 83. | (3-diméthylaminopropyl)-méthylamide de l'acide 1-benzyl-4-méthyl-3-phényl-1H-pyrrole-2-carboxylique, |
| 84. | [3-(4-méthoxyphényl)-1,4-diméthyl-1H-pyrrol-2-yl]-(4-p-toluylpipérazin-1-yl)-méthanone, |
| 85. | (thiophén-2-ylméthyl)-amide de l'acide 1-(4-méthoxybenzyl)-4-méthyl-3-p-toluyl-1H-pyrrole-2-carboxylique, |
| 86. | phénéthylamide de l'acide 4-méthyl-1-(4-méthylbenzyl)-3-p-toluyl-1H-pyrrole-2-carboxylique, |
| 87. | 3,5-difluorobenzylamide de l'acide 1,4-diméthyl-3-p-toluyl-1H-pyrrole-2-carboxylique, |
| 88. | indan-1-ylamide de l'acide 3-(4-méthoxyphényl)-1,4-diméthyl-1H-pyrrole-2-carboxylique, |
| 89. | 4-diméthylaminobenzylamide de l'acide 1-benzyl-4-méthyl-3-phényl-1H-pyrrole-2-carboxylique, |
| 90. | (1-naphtalén-2-ylméthylpyrrolidin-3-yl)-amide de l'acide 1-(4-fluorobenzyl)-3-furann-2-yl-4-méthyl-1H-pyrrole-2-carboxylique, |
| 91. | [3-furann-2-yl-4-méthyl-1-(4-trifluorométhylbenzyl)-1H-pyrrol-2-yl]-(4-méthyl-[1,4]diazépan-1-yl)-méthanone, |
| 92. | [1-(2-bromobenzyl)-3-(4-chlorophényl)-4-méthyl-1H-pyrrol-2-yl]-[4-(3-trifluorométhylphényl)-pipérazin-1-yl]-méthanone, |
| 93. | [3-furann-2-yl-1-(4-méthoxybenzyl)-4-méthyl-1H-pyrrol-2-yl]-(4-thiophén-3-ylméthylpipérazin-1-yl)-méthanone, |
| 94. | (2-benzyloxycyclohexyl)-amide de l'acide 1,4-diméthyl-3-p-toluyl-1H-pyrrole-2-carboxylique, |
| 95. | 2-{4-[1-benzyl-3-(4-chlorophényl)-4-méthyl-1H-pyrrole-2-carbonyl]-pipérazin-1-yl}-N-isopropylacétamide, |
| 96. | (2,6-diméthylmorpholin-4-yl)-[3-(4-méthoxyphényl)-1,4-diméthyl-1H-pyrrol-2-yl]-méthanone, |
| 97. | (thiophén-2-ylméthyl)-amide de l'acide 3-furann-2-yl-1-(4-méthoxybenzyl)-4-méthyl-1H-pyrrole-2-carboxylique, |
| 98. | (furann-2-ylméthyl)-amide de l'acide 1,4-diméthyl-3-p-toluyl-1H-pyrrole-2-carboxylique, |
| 99. | (3-diméthylaminopropyl)-amide de l'acide 4-méthyl-1-(4-méthylbenzyl)-3-p-toluyl-1H-pyrrole-2-carboxylique, |
| 100. | (2-oxotétrahydrofurann-3-yl)-amide de l'acide 1-(4-fluorobenzyl)-3-(4-méthoxyphényl)-4-méthyl-1H-pyrrole-2-carboxylique, |
| 101. | (2-phénoxyéthyl)-amide de l'acide 3-(4-chlorophényl)-1,4-diméthyl-1H-pyrrole-2-carboxylique, |
| 102. | 3-(4-(1-benzyl-4-méthyl-3-p-toluyl-1H-pyrrole-2-carbonyl)pipérazin-1-yl)pyrazine-2-carbonitrile |
| 103. | ester méthylique de l'acide 2-{[3-(4-méthoxyphényl)-1,4-diméthyl-1H-pyrrole-2-carbonyl]-amino}-3-phénylpropionique, |
| 104. | (2-morpholin-4-yléthyl)-amide de l'acide 3-(4-chlorophényl)-1,4-diméthyl-1H-pyrrole-2-carboxylique, |
| 105. | indan-2-ylamide de l'acide 3-furann-2-yl-1,4-diméthyl-1H-pyrrole-2-carboxylique, |
| 106. | (1-butyl-4-méthyl-3-p-toluyl-1H-pyrrol-2-yl)-[4-(2-fluorophényl)-pipérazin-1-yl]-méthanone, |
| 107. | [4-(3-chlorophényl)-pipérazin-1-yl]-(1,4-diméthyl-3-p-toluyl-1H-pyrrol-2-yl)-méthanone, |
| 108. | [1-(4-fluorobenzyl)-3-furann-2-yl-4-méthyl-1H-pyrrol-2-yl]-(4-naphtalén-2-ylméthylpipérazin-1-yl)-méthanone, |
| 109. | p-toluylamide de l'acide 3-(4-chlorophényl)-1-isobutyl-4-méthyl-1H-pyrrole-2-carboxylique, |
| 110. | (1-benzyl-4-méthyl-3-p-toluyl-1H-pyrrol-2-yl)-[4-(2-hydroxyéthyl)-pipérazin-1-yl]-méthanone, |
| 111. | [2-(2,5-diméthoxyphényl)-éthyl]-amide de l'acide 1-butyl-4-méthyl-3-p-toluyl-1H-pyrrole-2-carboxylique, |
| 112. | (3-imidazol-1-ylpropyl)-amide de l'acide 1-butyl-4-méthyl-3-p-toluyl-1H-pyrrole-2-carboxylique, |
| 113. | [2-(3,4-diméthoxyphényl)-éthyl]-méthylamide de l'acide 1-benzyl-3-(4-chlorophényl)-4-méthyl-1H-pyrrole-2-carboxylique, |
| 114. | (3-morpholin-4-ylpropyl)-amide de l'acide 4-méthyl-1-(4-méthylbenzyl)-3-p-toluyl-1H-pyrrole-2-carboxylique, |
| 115. | 4-fluoro-2-trifluorométhylbenzylamide de l'acide 4-méthyl-1-(4-méthylbenzyl)-3-p-toluyl-1H-pyrrole-2-carboxylique, |
| 116. | ester éthylique de l'acide 2-{[1-benzyl-3-(4-chlorophényl)-4-méthyl-1H-pyrrole-2-carbonyl]-amino}-3-(4-chlorophényl)-propionique, |
| 117. | 3-fluoro-4-trifluorométhylbenzylamide de l'acide 1-benzyl-4-méthyl-3-phényl-1H-pyrrole-2-carboxylique, |
| 118. | (2-pyridin-2-yléthyl)-amide de l'acide 1-(4-bromobenzyl)-4-méthyl-3-p-toluyl-1H-pyrrole-2-carboxylique, |
| 119. | benzyl-(2-hydroxyéthyl)-amide de l'acide 1-(4-fluorobenzyl)-3-furann-2-yl-4-méthyl-1H-pyrrole-2-carboxylique, |
| 120. | 2-[4-(1-butyl-4-méthyl-3-p-toluyl-1H-pyrrole-2-carbonyl)-pipérazin-1-yl]-N-méthyl-N-phénylacétamide, |
| 121. | [4-méthyl-1-(4-méthylbenzyl)-3-p-toluyl-1H-pyrrol-2-yl]-(4-phénylpipérazin-1-yl)-méthanone, |
| 122. | (1-benzylpyrrolidin-3-yl)-amide de l'acide 1-benzyl-4-méthyl-3-p-toluyl-1H-pyrrole-2-carboxylique, |
| 123. | [1-(4-fluorobenzyl)-3-furann-2-yl-4-méthyl-1H-pyrrol-2-yl]-[4-(3-trifluorométhylphényl)-pipérazin-1-yl]-méthanone, |
| 124. | butylamide de l'acide 1-(4-méthoxybenzyl)-4-méthyl-3-p-toluyl-1H-pyrrole-2-carboxylique, |
| 125. | [2-(2-fluorophényl)-éthyl]-amide de l'acide 1-benzyl-4-méthyl-3-p-toluyl-1H-pyrrole-2-carboxylique, |
| 126. | 3,4-diméthoxybenzylamide de l'acide 4-méthyl-1-(4-méthylbenzyl)-3-p-toluyl-1H-pyrrole-2-carboxylique, |
| 127. | (2-diéthylaminoéthyl)-amide de l'acide 3-(4-chlorophényl)-1-isobutyl-4-méthyl-1H-pyrrole-2-carboxylique, |
| 128. | 1-{4-[3-(4-chlorophényl)-1-éthyl-4-méthyl-1H-pyrrole-2-carbonyl]-pipérazin-1-yl}-2-phényléthanone, |
| 129. | 3-trifluorométhoxybenzylamide de l'acide 1-(4-fluorobenzyl)-4-méthyl-3-p-toluyl-1H-pyrrole-2-carboxylique, |
| 130. | [1-benzyl-3-(4-chlorophényl)-4-méthyl-1H-pyrrol-2-yl]-(3-méthylpipéridin-1-yl)-méthanone, |
| 131. | (2-p-toluyléthyl)-amide de l'acide 1-(4-fluorobenzyl)-4-méthyl-3-p-toluyl-1H-pyrrole-2-carboxylique, |
| 132. | [2-(2-méthyl-5-nitro-imidazol-1-yl)-éthyl]-amide de l'acide 1-(4-méthoxybenzyl)-4-méthyl-3-p-toluyl-1H-pyrrole-2-carboxylique, |
| 133. | 2,3-diméthoxybenzylamide de l'acide 1,4-diméthyl-3-p-toluyl-1H-pyrrole-2-carboxylique, |
| 134. | [2-(éthyl-m-toluylamino)-éthyl]-amide de l'acide 1-(2-bromobenzyl)-3-(4-chlorophényl)-4-méthyl-1H-pyrrole-2-carboxylique, |
| 135. | (4-isopropylphényl)-amide de l'acide 1-(4-fluorobenzyl)-3-furann-2-yl-4-méthyl-1H-pyrrole-2-carboxylique, |
| 136. | N'-[1-(4-fluorobenzyl)-3-(4-méthoxyphényl)-4-méthyl-1H-pyrrole-2-carbonyl]-hydrazide de l'acide 5-chloro-2-méthoxybenzoïque, |
| 137. | (1-benzyl-4-méthyl-3-p-toluyl-1H-pyrrol-2-yl)-[1,4']bipipéridinyl-1'-ylméthanone, |
| 138. | (4-butylphényl)-amide de l'acide 3-furann-2-yl-1,4-diméthyl-1H-pyrrole-2-carboxylique, |
| 139. | [2-(1H-indol-3-yl)-éthyl]-amide de l'acide 1-benzyl-4-méthyl-3-phényl-1H-pyrrole-2-carboxylique, |
| 140. | [4-(4-tert-butylbenzyl)-pipérazin-1-yl]-[1-(4-fluorobenzyl)-4-méthyl-3-p-toluyl-1H-pyrrol-2-yl]-méthanone, |
| 141. | [1-(4-fluorobenzyl)-4-méthyl-3-p-toluyl-1H-pyrrol-2-yl]-morpholin-4-ylméthanone, |
| 142. | ester éthylique de l'acide 3-[(1-benzyl-3-furann-2-yl-4-méthyl-1H-pyrrole-2-carbonyl)-amino]-propionique, |
| 143. | (naphtalén-2-ylcarbamoylméthyl)-amide de l'acide 1,4-diméthyl-3-phényl-1H-pyrrole-2-carboxylique, |
| 144. | [3-(4-chlorophényl)-1,4-diméthyl-1H-pyrrol-2-yl]-[4-(2-éthoxyphényl)-pipérazin-1-yl]-méthanone, |
| 145. | (4-cyanométhylphényl)-amide de l'acide 1-(4-fluorobenzyl)-3-furann-2-yl-4-méthyl-1H-pyrrole-2-carboxylique, |
| 146. | ester tert-butylique de l'acide 4-[1-benzyl-3-(4-chlorophényl)-4-méthyl-1H-pyrrole-2-carbonyl]-pipérazine-1-carboxylique, |
| 147. | (1,4-dioxa-8-azaspiro[4,5]déc-8-yl)-[1-(4-fluorobenzyl)-3-furann-2-yl-4-méthyl-1H-pyrrol-2-yl]-méthanone, |
| 148. | pentylamide de l'acide 3-furann-2-yl-4-méthyl-1-(4-trifluorométhylbenzyl)-1H-pyrrole-2-carboxylique, |
| 149. | [3-(méthylphénylamino)-propyl]-amide de l'acide 3-furann-2-yl-1-(4-méthoxybenzyl)-4-méthyl-1H-pyrrole-2-carboxylique, |
| 150. | (2-azépan-1-yléthyl)-amide de l'acide 1-(2,6-dichlorobenzyl)-4-méthyl-3-p-toluyl-1H-pyrrole-2-carboxylique, |
| 151. | cyclopentylamide de l'acide 1-(4-bromobenzyl)-4-méthyl-3-p-toluyl-1H-pyrrole-2-carboxylique, |
| 152. | [1-(2,6-dichlorobenzyl)-4-méthyl-3-p-toluyl-1H-pyrrol-2-yl]-[4-(2,4-diméthoxyphényl)-pipérazin-1-yl]-méthanone, |
| 153. | [2-(2-chlorophénoxy)-éthyl]-amide de l'acide 3-(4-chlorophényl)-1-(2-fluorobenzyl)-4-méthyl-1H-pyrrole-2-carboxylique, |
| 154. | (3,3-diméthylbutyl)-amide de l'acide 1-(2,6-dichlorobenzyl)-4-méthyl-3-p-toluyl-1H-pyrrole-2-carboxylique, |
| 155. | (2-benzyloxycyclohexyl)-amide de l'acide 1,4-diméthyl-3-p-toluyl-1H-pyrrole-2-carboxylique, |
| 156. | [4-méthyl-1-(4-méthylbenzyl)-3-p-toluyl-1H-pyrrol-2-yl]-thiomorpholin-4-ylméthanone, |
| 157. | (3-diméthylaminopropyl)-méthylamide de l'acide 3-(4-chlorophényl)-1-isobutyl-4-méthyl-1H-pyrrole-2-carboxylique, |
| 158. | [4-(2,5-diméthylphényl)-pipérazin-1-yl]-[3-furann-2-yl-1-(4-méthoxybenzyl)-4-méthyl-1H-pyrrol-2-yl]-méthanone, |
| 159. | N'-[3-(4-méthoxyphényl)-1,4-diméthyl-1H-pyrrole-2-carbonyl]-hydrazide de l'acide 5-chloro-2-méthoxybenzoïque, |
| 160. | 2-(3,4-difluorophényl)-1-{4-[1-(4-fluorobenzyl)-3-furann-2-yl-4-méthyl-1H-pyrrole-2-carbonyl]-pipérazin-1-yl}-éthanone, |
| 161. | 2-{4-[1-(4-fluorobenzyl)-3-furann-2-yl-4-méthyl-1H-pyrrole-2-carbonyl]-pipérazin-1-yl}-N-isopropylacétamide, |
| 162. | [1-(3-méthoxyphényl)-éthyl]-amide de l'acide 3-furann-2-yl-4-méthyl-1-(4-trifluorométhylbenzyl)-1H-pyrrole-2-carboxylique, |
| 163. | ester méthylique de l'acide 2-[(1-benzyl-3-furann-2-yl-4-méthyl-1H-pyrrole-2-carbonyl)-amino]-3-(4-chlorophényl)-propionique, |
| 164. | [3-furann-2-yl-1-(4-méthoxybenzyl)-4-méthyl-1H-pyrrol-2-yl]-(4-méthylpipérazin-1-yl)-méthanone, |
| 165. | (1-benzylpyrrolidin-3-yl)-amide de l'acide 1,4-diméthyl-3-phényl-1H-pyrrole-2-carboxylique, |
| 166. | (1,4-diméthyl-3-p-toluyl-1H-pyrrol-2-yl)-(4-thiéno[2,3-d]pyrimidin-4-ylpipérazin-1-yl)-méthanone, |
| 167. | (3-méthoxypropyl)-amide de l'acide 4-méthyl-1-(4-méthylbenzyl)-3-p-toluyl-1H-pyrrole-2-carboxylique, |
| 168. | [3-(4-méthoxyphényl)-1,4-diméthyl-1H-pyrrol-2-yl]-[4-(5-trifluorométhylpyridin-2-yl)-pipérazin-1-yl]-méthanone, |
| 169. | (3-morpholin-4-yl-propyl)-amide de l'acide 1-(4-bromobenzyl)-4-méthyl-3-p-toluyl-1H-pyrrole-2-carboxylique, |
| 170. | [2-(2,3-diméthoxyphényl)-éthyl]-amide de l'acide 1,4-diméthyl-3-p-toluyl-1H-pyrrole-2-carboxylique, |
| 171. | [1-(4-méthoxybenzyl)-4-méthyl-3-p-toluyl-1H-pyrrol-2-yl]-(3-méthyl-4-p-toluylpipérazin-1-yl)-méthanone, |
| 172. | [3-(4-chlorophényl)-1-éthyl-4-méthyl-1H-pyrrol-2-yl]-[4-(2,4-diméthoxyphényl)-pipérazin-1-yl]-méthanone, |
| 173. | 2-{4-[1-(2-bromobenzyl)-3-(4-chlorophényl)-4-méthyl-1H-pyrrole-2-carbonyl]-pipérazin-1-yl}-benzonitrile, |
| 174. | (1-éthylpyrrolidin-2-ylméthyl)-amide de l'acide 1-(4-bromobenzyl)-4-méthyl-3-p-toluyl-1H-pyrrole-2-carboxylique, |
| 175. | [2-(2-chlorophénoxy)-éthyl]-amide de l'acide 3-(4-chlorophényl)-1-isobutyl-4-méthyl-1H-pyrrole-2-carboxylique, |
| 176. | [2-(1H-indol-3-yl)-éthyl]-amide de l'acide 3-(4-chlorophényl)-1,4-diméthyl-1H-pyrrole-2-carboxylique, |
| 177. | (naphtalén-2-ylcarbamoylméthyl)-amide de l'acide 1-(4-fluorobenzyl)-4-méthyl-3-p-toluyl-1H-pyrrole-2-carboxylique, |
| 178. | ester benzylique de l'acide 4-(1-benzyl-3-furann-2-yl-4-méthyl-1H-pyrrole-2-carbonyl)-pipérazine-1-carboxylique, |
| 179. | (1-benzylpyrrolidin-3-yl)-amide de l'acide 1,4-diméthyl-3-phényl-1H-pyrrole-2-carboxylique, |
| 180. | [3-(4-chlorophényl)-1-(2-fluorobenzyl)-4-méthyl-1H-pyrrol-2-yl]-[4-(4-trifluorométhylphényl)-pipérazin-1-yl]-méthanone, |
| 181. | [3-(4-chlorophényl)-1-isobutyl-4-méthyl-1H-pyrrol-2-yl]-(4-phénylpipérazin-1-yl)-méthanone, |
| 182. | 2-{4-[1-butyl-3-(4-chlorophényl)-4-méthyl-1H-pyrrole-2-carbonyl]-pipérazin-1-yl}-1-pyrrolidin-1-yléthanone, |
| 183. | [1-benzyl-3-(4-chlorophényl)-4-méthyl-1H-pyrrol-2-yl]-[4-(2-chloro-4-fluorobenzoyl)-pipérazin-1-yl]-méthanone, |
| 184. | (4-benzoylpipéridin-1-yl)-[1-(4-méthoxybenzyl)-4-méthyl-3-p-toluyl-1H-pyrrol-2-yl]-méthanone, |
| 185. | éthylpyridin-4-ylméthylamide de l'acide 3-furann-2-yl-1,4-diméthyl-1H-pyrrole-2-carboxylique, |
| 186. | (2-pyrrolidin-1-yléthyl)-amide de l'acide 1-(4-bromobenzyl)-4-méthyl-3-p-toluyl-1H-pyrrole-2-carboxylique, |
| 187. | [3-(4-chlorophényl)-1-isobutyl-4-méthyl-1H-pyrrol-2-yl]-[4-(4-méthylbenzoyl)-pipérazin-1-yl]-méthanone, |
| 188. | [1-benzyl-3-(4-chlorophényl)-4-méthyl-1H-pyrrol-2-yl]-(4-thiéno[2,3-d]pyrimidin-4-yl-pipérazin-1-yl)-méthanone, |
| 189. | ester éthylique de l'acide 1-(1,4-diméthyl-3-phényl-1H-pyrrole-2-carbonyl)-pipéridine-3-carboxylique, |
| 190. | (1-phényléthyl)-amide de l'acide 1,4-diméthyl-3-phényl-1H-pyrrole-2-carboxylique, |
| 191. | [3-furann-2-yl-1-(4-méthoxybenzyl)-4-méthyl-1H-pyrrol-2-yl]-(1,3,4,9-tétrahydro-b-carbolin-2-yl)-méthanone, |
| 192. | 1-{4-[1-benzyl-3-(4-chlorophényl)-4-méthyl-1H-pyrrole-2-carbonyl]-pipérazin-1-yl}-2-(3,4-difluorophényl)-éthanone, |
| 193. | [1-(4-bromobenzyl)-4-méthyl-3-p-toluyl-1H-pyrrol-2-yl]-[4-(3-phénylpropyl)-pipérazin-1-yl]-méthanone, |
| 194. | [2-(1H-indol-3-yl)-éthyl]-méthylamide de l'acide 1-(2-bromobenzyl)-3-(4-chlorophényl)-4-méthyl-1H-pyrrole-2-carboxylique, |
| 195. | 1-(4-{4-[1-(4-bromobenzyl)-4-méthyl-3-p-toluyl-1H-pyrrole-2-carbonyl]-pipérazin-1-yl)-phényl)-éthanone, |
| 196. | 4-fluoro-2-trifluorométhylbenzylamide de l'acide 1,4-diméthyl-3-phényl-1H-pyrrole-2-carboxylique, |
| 197. | (4-phénoxyphényl)-amide de l'acide 4-méthyl-1-(4-méthylbenzyl)-3-p-toluyl-1H-pyrrole-2-carboxylique, |
| 198. | (4-hydroxypipéridin-1-yl)-[1-(4-méthoxybenzyl)-4-méthyl-3-p-toluyl-1H-pyrrol-2-yl]-méthanone, |
| 199. | N'-(1-benzyl-4-méthyl-3-p-toluyl-1H-pyrrole-2-carbonyl)-hydrazide de l'acide 4-diéthylaminobenzoïque, |
| 200. | [3-(4-chlorophényl)-1-isobutyl-4-méthyl-1H-pyrrole-2-yl]-[4-(3-chlorophényl)-pipérazin-1-yl]-méthanone, |
| 201. | [2-(3,4-dichlorophényl)-éthyl]-amide de l'acide 1-(4-bromobenzyl)-4-méthyl-3-p-toluyl-1H-pyrrole-2-carboxylique, |
| 202. | [2-(1H-indol-3-yl)-éthyl]-amide de l'acide 3-(4-chlorophényl)-1-éthyl-4-méthyl-1H-pyrrole-2-carboxylique, |
| 203. | [1-(4-fluorobenzyl)-4-méthyl-3-p-toluyl-1H-pyrrol-2-yl]-[4-(5-trifluorométhylpyridin-2-yl)-pipérazin-1-yl]-méthanone, |
| 204. | 3-trifluorométhoxybenzylamide de l'acide 1,4-diméthyl-3-phényl-1H-pyrrole-2-carboxylique, |
| 205. | [1-(4-fluorobenzyl)-4-méthyl-3-p-toluyl-1H-pyrrol-2-yl]-[4-(4-méthylbenzoyl)-pipérazin-1-yl]-méthanone, |
| 206. | [4-(3,4-dichlorophényl)-pipérazin-1-yl]-[1-(4-fluorobenzyl)-4-méthyl-3-p-toluyl-1H-pyrrol-2-yl]-méthanone, |
| 207. | 2,6-diméthoxybenzylamide de l'acide 3-(4-chlorophényl)-1-éthyl-4-méthyl-1H-pyrrole-2-carboxylique, |
| 208. | [2-(3,4-diméthoxyphényl)-éthyl]-amide de l'acide 4-méthyl-1-(4-méthylbenzyl)-3-p-toluyl-1H-pyrrole-2-carboxylique, |
| 209. | 1-{4-[3-(4-chlorophényl)-1-isobutyl-4-méthyl-1H-pyrrole-2-carbonyl]-pipérazin-1-yl)-2-(3,4-difluorophényl)-éthanone, |
| 210. | [1-(4-fluorobenzyl)-4-méthyl-3-p-toluyl-1H-pyrrol-2-yl]-[4-(2-méthoxyphényl)-pipéridin-1-yl]-méthanone, |
| 211. | [3-furann-2-yl-4-méthyl-1-(4-trifluorométhylbenzyl)-1H-pyrrol-2-yl]-thiomorpholin-4-ylméthanone, |
| 212. | (2-thiophén-2-yléthyl)-amide de l'acide 1,4-diméthyl-3-phényl-1H-pyrrole-2-carboxylique, |
| 213. | N'-(1-butyl-4-méthyl-3-p-toluyl-1H-pyrrole-2-carbonyl)-hydrazide de l'acide 4-diéthylaminobenzoïque, |
| 214. | ester tert-butylique de l'acide {1-[1-(4-fluorobenzyl)-3-(4-méthoxyphényl)-4-méthyl-1H-pyrrole-2-carbonyl]-pyrrolidin-3-yl}-carbamique, |
| 215. | 2-{4-[3-(4-chlorophényl)-1-isobutyl-4-méthyl-1H-pyrrole-2-carbonyl]-pipérazin-1-yl}-1-pyrrolidin-1-yléthanone, |
| 216. | [4-(2,3-dihydrobenzo[1,4]dioxine-2-carbonyl)-pipérazin-1-yl]-(1,4-diméthyl-3-phényl-1H-pyrrol-2-yl)-méthanone, |
| 217. | 3,4-diméthoxybenzylamide de l'acide 3-furann-2-yl-1,4-diméthyl-1H-pyrrole-2-carboxylique, |
| 218. | [3-furann-2-yl-4-méthyl-1-(4-trifluorométhylbenzyl)-1H-pyrrol-2-yl]-(4-pyridin-2-ylpipérazin-1-yl)-méthanone, |
| 219. | [4-(furann-2-carbonyl)-pipérazin-1-yl]-[3-furann-2-yl-4-méthyl-1-(4-trifluorométhylbenzyl)-1H-pyrrol-2-yl]-méthanone, |
| 220. | (4-tertbutylphényl)-amide de l'acide 3-furann-2-yl-4-méthyl-1-(4-trifluorométhylbenzyl)-1H-pyrrole-2-carboxylique, |
| 221. | 2-{4-[3-(4-chlorophényl)-1-isobutyl-4-méthyl-1H-pyrrole-2-carbonyl]-pipérazin-1-yl]-benzonitrile, |
| 222. | (3-furann-2-yl-1,4-diméthyl-1H-pyrrol-2-yl)-[4-(4-trifluorométhylpyridin-2-yl)-pipérazin-1-yl]-méthanone, |
| 223. | 3,5-difluorobenzylamide de l'acide 1,4-diméthyl-3-phényl-1H-pyrrole-2-carboxylique, |
| 224. | (pyridin-4-ylméthyl)-amide de l'acide 1,4-diméthyl-3-phényl-1H-pyrrole-2-carboxylique, |
| 225. | 2-[4-(3-furann-2-yl-1,4-diméthyl-1H-pyrrole-2-carbonyl)-pipérazin-1-yl]-benzonitrile, |
| 226. | méthyl-(2-pyridin-2-yléthyl)-amide de l'acide 3-furann-2-yl-1,4-diméthyl-1H-pyrrole-2-carboxylique, |
| 227. | (3-méthoxybenzyl)-(tétrahydrofurann-2-ylméthyl)-amide de l'acide 3-furann-2-yl-1,4-diméthyl-1H-pyrrole-2-carboxylique, |
| 228. | (4-phénoxyphényl)-amide de l'acide 3-furann-2-yl-1,4-diméthyl-1H-pyrrole-2-carboxylique, |
| 229. | pentylamide de l'acide 3-furann-2-yl-1,4-diméthyl-1H-pyrrole-2-carboxylique, |
| 230. | 2-(3,4-difluorophényl)-1-[4-(3-furann-2-yl-1,4-diméthyl-1H-pyrrole-2-carbonyl)-pipérazin-1-yl]-éthanone, |
| 231. | phénylamide de l'acide 3-furann-2-yl-1,4-diméthyl-1H-pyrrole-2-carboxylique, |
| 232. | [4-(2,4-diméthoxyphényl)-pipérazin-1-yl]-(3-furann-2-yl-1,4-diméthyl-1H-pyrrol-2-yl)-méthanone, |
| 233. | [2-(3,4-dichlorophényl)-éthyl]-amide de l'acide 3-furann-2-yl-1,4-diméthyl-1H-pyrrole-2-carboxylique, |
| 234. | [2-(2-chlorophénoxy)-éthyl]-amide de l'acide 3-furann-2-yl-1,4-diméthyl-1H-pyrrole-2-carboxylique, |
| 235. | 3-méthoxybenzylamide de l'acide 3-furann-2-yl-1,4-diméthyl-1H-pyrrole-2-carboxylique, |
| 236. | phénéthylamide de l'acide 3-furann-2-yl-4-méthyl-1-(4-trifluorométhylbenzyl)-1H-pyrrole-2-carboxylique, |
| 237. | cyclopentylamide de l'acide 3-furann-2-yl-1,4-diméthyl-1H-pyrrole-2-carboxylique, |
| 238. | (pyridin-2-ylméthyl)-amide de l'acide 3-furann-2-yl-1,4-diméthyl-1H-pyrrole-2-carboxylique, |
| 239. | [2-(3,4-diméthoxyphényl)-éthyl]-amide de l'acide 3-furann-2-yl-4-méthyl-1-(4-trifluorométhylbenzyl)-1H-pyrrole-2-carboxylique, |
| 240. | [1-(4-bromobenzyl)-4-méthyl-3-p-toluyl-1H-pyrrol-2-yl]-(2,6-diméthylmorpholin-4-yl)-méthanone, |
| 241. | (2-p-toluyléthyl)-amide de l'acide 1,4-diméthyl-3-phényl-1H-pyrrole-2-carboxylique, |
| 242. | (1,4-diméthyl-3-phényl-1H-pyrrol-2-yl)-[4-(4-fluorophényl)-pipérazin-1-yl]-méthanone et |
| 243. | [2-(1H-indol-3-yl)-éthyl]-méthylamide de l'acide 4-méthyl-1-(4-méthylbenzyl)-3-p-toluyl-1H-pyrrole-2-carboxylique, |
à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

7. Procédé pour la préparation d'amides de l'acide 4-méthyl-1H-pyrrole-2-carboxylique disubstitué en positions 1,3 de formule générale I selon l'une quelconque des revendications 1 à 6, **caractérisé en ce qu'**on transforme au moins un composé de formule générale II, 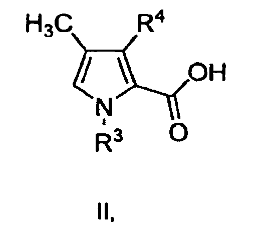 où R³ et R⁴ ont la signification selon les revendications 1 à 6, dans au moins un mélange réactionnel, en présence d'au moins un agent de couplage approprié, en présence d'au moins une base, de préférence à une température de -70°C à 100°C, par transformation avec au moins un composé de formule générale HNR¹R², où R¹ et R² ont la signification selon l'une quelconque des revendications 1 à 6, en un composé correspondant de formule générale I, sous forme d'un sel correspondant, 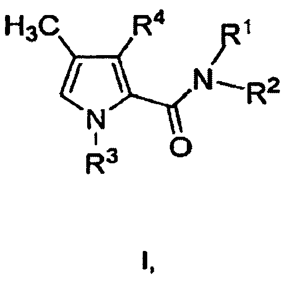 où R¹, R², R³ et R⁴ ont la signification susmentionnée et ce composé est le cas échéant purifié et/ou isolé.

8. Médicament contenant au moins un composé selon l'une quelconque des revendications 1 à 6 et un ou plusieurs adjuvants pharmaceutiques.

9. Médicament selon la revendication 8 pour la prophylaxie et/ou le traitement de la douleur, de préférence de la douleur choisie dans le groupe constitué par la douleur aiguë, la douleur chronique et la douleur neuropathique.

10. Médicament selon la revendication 8 ou 9 pour la prophylaxie et/ou le traitement de migraines ; de dépressions ; d'incontinence urinaire ; de la toux ; de maladies de neurodégénérescence, de préférence choisies dans le groupe constitué par la maladie de Parkinson, de Huntington, d'Alzheimer et la sclérose en plaques ; de troubles de l'absorption alimentaire, de préférence choisis dans le groupe constitué par la boulimie, l'anorexie, l'obésité et la cachexie ; de dysfonctionnements cognitifs, de préférence les troubles de la mémoire ; de l'épilepsie ; de la diarrhée ; du prurit ; de l'abus d'alcool et/ou de drogues et/ou de médicaments ; de la dépendance de l'alcool et/ou de drogues et/ou de médicaments, de préférence pour la prophylaxie et/ou la diminution de symptômes de sevrage lors d'une dépendance de l'alcool et/ou de drogues et/ou des médicaments ; pour la prophylaxie et/ou la diminution d'un développement de tolérances à des médicaments, en particulier de médicaments à base d'opioïdes ; pour la régulation de l'absorption d'aliments ; pour la modulation de l'activité mobile ; pour la régulation du système cardiovasculaire ; pour l'anesthésie locale ; pour l'anxiolyse ; pour l'augmentation de la vigilance ; pour l'augmentation de la libido ; pour la diurèse et/ou pour l'antinatriurèse.

11. Utilisation d'au moins un composé selon l'une quelconque des revendications 1 à 6 pour la préparation d'un médicament destiné à la prophylaxie et/ou au traitement de la douleur, de préférence de la douleur choisie dans le groupe constitué par la douleur aiguë, la douleur chronique et la douleur neuropathique.

12. Utilisation d'au moins un composé selon l'une quelconque des revendications 1 à 6 pour la préparation d'un médicament pour la prophylaxie et/ou le traitement de migraines ; de dépressions ; d'incontinence urinaire ; de la toux ; de maladies de neurodégénérescence, de préférence choisies dans le groupe constitué par la maladie de Parkinson, de Huntington, d'Alzheimer et la sclérose en plaques ; de troubles de l'absorption alimentaire, de préférence choisis dans le groupe constitué par la boulimie, l'anorexie, l'obésité et la cachexie ; de dysfonctionnements cognitifs, de préférence les troubles de la mémoire ; de l'épilepsie ; de la diarrhée ; du prurit ; de l'abus d'alcool et/ou de drogues et/ou de médicaments ; de la dépendance de l'alcool et/ou de drogues et/ou de médicaments, de préférence pour la prophylaxie et/ou la diminution de symptômes de sevrage lors d'une dépendance de l'alcool et/ou de drogues et/ou de médicaments ; pour la prophylaxie et/ou la diminution d'un développement de tolérances à des médicaments, en particulier des médicaments à base d'opioïdes ; pour la régulation de l'absorption d'aliments ; pour la modulation de l'activité mobile ; pour la régulation du système cardiovasculaire ; pour l'anesthésie locale ; pour l'anxiolyse ; pour l'augmentation de la vigilance ; pour l'augmentation de la libido ; pour la diurèse et/ou pour l'antinatriurèse.
